(19) **Europäisches Patentamt**

**European Patent Office**

**Office européen des brevets**

(11) **EP 1 250 351 B1**

(12) **EUROPEAN PATENT SPECIFICATION**

(45) Date of publication and mention
of the grant of the patent:
**30.11.2005 Bulletin 2005/48**

(51) Int Cl.[7]: **C07K 7/06**, C07K 14/16,
G01N 33/569, G01N 33/68

(21) Application number: **01946867.7**

(22) Date of filing: **29.01.2001**

(86) International application number:
**PCT/DK2001/000059**

(87) International publication number:
**WO 2001/055177 (02.08.2001 Gazette 2001/31)**

(54) **METHODS TO IDENTIFY CTL EPITOPES OF HIV**

VERFAHREN ZU IDENTIFIZIERUNG VON HIV-EPITOPEN, DIE DURCH ZYTOTOXISCHE
T-ZELLEN ERKANNT WERDEN KÖNNEN

METHODES D'IDENTIFICATION DE CTL EPITOPES DU VIH

(84) Designated Contracting States:
**AT BE CH CY DE DK ES FI FR GB GR IE IT LI LU
MC NL PT SE TR**

(30) Priority: **28.01.2000 EP 00610017
31.01.2000 US 179333 P**

(43) Date of publication of application:
**23.10.2002 Bulletin 2002/43**

(73) Proprietor: **Statens Serum Institut
2300 Copenhagen S (DK)**

(72) Inventors:
  • **FOMSGAARD, Anders
    DK-1954 Frederiksberg C (DK)**
  • **BRUNAK, Soren
    DK-2900 Hellerup (DK)**
  • **BUUS, Soren
    DK-2700 Bronshoj (DK)**
  • **CORBET, Sylvie
    DK-1954 Frederiksberg C (DK)**
  • **LAUEMOLLER, Sanne, Lise
    DK-3000 Helsingor (DK)**
  • **HANSEN, Jan
    DK-2830 Virum (DK)**

(74) Representative: **Plougmann & Vingtoft A/S
Sundkrogsgade 9
P.O. Box 831
2100 Copenhagen OE (DK)**

(56) References cited:
  • **MEISTER, G.E. ET AL.: "Two novel T cell epitope
    prediction algorithms based on MHC-binding
    motifs; comparison of predicted and published
    epitopes from Mycobacterium tuberculosis and
    HIV protein sequences" VACCINE, vol. 13, no. 6,
    1995, pages 581-591, XP004057605 cited in the
    application**
  • **FERRER, M. ET AL.: "Peptide Ligands to Human
    Immunodeficiency Virus Type I gp120 Identified
    from Phage Display Libraries" JOURNAL OF
    VIROLOGY, vol. 73, no. 7, July 1999 (1999-07),
    pages 5795-5802, XP000925407**
  • **VASMATZIS, G. ET AL.: "Computational
    Determination of Side Chain Specificity for
    Pockets in Class I MHC Molecules"
    MOLECULAR IMMUNOLOGY, vol. 33, no. 16,
    1996, pages 1231-1239, XP000925328 cited in the
    application**
  • **MILIK, M. ET AL.: "Application of an artificial
    neural network to predict specific class I MHC
    binding peptide sequences" NATURE
    BIOTECHNOLOGY, vol. 16, August 1998
    (1998-08), pages 753-756, XP000929462**
  • **STRYHN, A. ET AL.: "Peptide binding specificity
    of major histocompatibility complex class I
    resolved into an array of apparently independent
    subspecificities: quantitation by peptide
    libraries and improved prediction of binding"
    EUROPEAN JOURNAL OF IMMUNOLOGY, vol.
    26, 1996, pages 1911-1918, XP000925378**

- ALTUVIA, Y. ET AL.: "Ranking Potential Binding Peptides to MHC Molecules by a Computational Threading Approach" JOURNAL OF MOLECULAR BIOLOGY, vol. 249, no. 1, 26 May 1995 (1995-05-26), pages 244-250, XP000925520 cited in the application

- ALTUVIA, Y. ET AL.: "A Structure-Based Algorithm to Predict Potential Binding Peptides to MHC Molecules with Hydrophobic Binding Pockets" HUMAN IMMUNOLOGY, vol. 58, no. 1, 1997, pages 1-11, XP000925379 cited in the application

**Description**

**FIELD OF INVENTION**

The present invention relates to the identification of CTL epitopes by the combination of biochemical assays, statistical matrix calculations, and artificial neural networks. One aspect of the invention relates to the use of the identified and tested CTL epitopes for the preparation of diagnostic, prophylactic and therapeutic agents.

**BACKGROUND OF THE INVENTION**

A virus is an intracellular organism. Therefore, cytotoxic T-cell lymphocytes (CTL) are a major protective mechanism against viral diseases. Antibodies may neutralise *extracellular* Human Immune deficiency Virus (HIV) and thus limit or prevent *infection* of cells in the host (Shibata et al., 1999), whereas CTL will limit the viral production by killing the cell, inducing apoptosis, inducing antiviral substances, and/or inducing increased intracellular lysis in already infected cells and thus cure or prevent the *disease.*

A considerable amount of evidence suggests that CD8[+] CTL plays a major role both in preventing and limiting the *initial* HIV infection (Koup et al., 1994) as well as being active *throughout* the HIV infection (Harrer et al., 1994). Correlations between CTL activity and protection have been observed in monkeys (Schmitz et al., 1999) and in challenge experiments (Gallimore et al., 1995). Thus, in order to prevent and/or treat HIV infections by a vaccination activating CTL, the identification of new CTL epitopes is of great importance.

Progress made over the last two decades has conclusively demonstrated that one of the most important immune recognitions - that of T-cells - uses peptides as one part of a complicated target structure. T-cells are specific for peptides presented in the context of Major Histocompatibility Complex (MHC) molecules; a phenomenon which is known as MHC restriction. During ontogeny, the MHC molecules available to the host are involved in shaping the T-cell repertoire through selection processes, which results in T-cell recognition being restricted by the host MHC molecules. Later in life, only pathogen-derived peptides presented in the context of one of the host MHC molecules can be recognised by the host T-cell immune system. Thus, the particular MHC molecules available to the host have a very direct impact on the specificity of the T-cell immune system. The specificity of the MHC molecules is therefore of considerable scientific and practical interest.

A good correlation between the MHC restriction and the ability of the MHC molecule to bind e.g. a putative CTL epitope in a biochemical assay has been established. The major advantage of the biochemical approach is that binding can be accurately quantified and the MHC specificity can be addressed in isolation, very pointedly and under highly controlled conditions. The present belief is that the MHC specificities, such as the primary anchors, secondary anchors, and disfavoured amino acids, can be investigated through the interaction between purified MHC and the peptide.

The biochemical specificity of some MHC molecules have now been described in great detail (Falk et al., 1991, Ruppert et al., 1993) and explained structurally (Madden et al., 1995). As expected, the peptide binding specificity is quite broad. This broad specificity is obtained through the recognition of peptide motifs; a recognition mode which requires the presence of important structural requirements such as the presence and proper spacing of particular amino acids in certain anchor positions (Falk et al., 1991, Sette et al., 1987, Rammensee et al., 1995). Thus, peptides binding to the same MHC allotype exhibit some degree of similarity, but there is no requirement for identity. X-ray crystallography has identified a unique peptide binding site at the outer polymorphic domains of the MHC (Fremont et al., 1992). These outer domains form a groove which can be subdivided into various pockets, A through F (Garrett et al., 1989). The majority of the peptide-MHC bonds involve peptide main chain (or backbone) atoms including the termini for MHC class I (Fremont et al., 1992). This explains how one MHC haplotype can perform high affinity ($K_D = 10^{-8} - 10^{-9}$M) binding of a large and diverse repertoire of peptides, as backbone atoms are common to all peptides. Only the minority of the binding energy involves peptide side chain atoms. These interactions, however, are believed to explain the specificity of the MHC (Matsumura et al., 1992).

The MHC specificities have been represented in various ways, the most elaborate of these being complete statistical matrices representing an indication of the frequency of a particular amino add in a given position of the epitope. Such matrix can be based on the analysis of large series of analogues (a biased matrix), or on the analysis of peptide libraries (an unbiased matrix) - the latter yielding significantly better predictions. All matrix-driven predictions, however, make the assumption that the specificity of each sub-site is independent of the residues present in the neighbouring sub-sites. The reasonable success of the matrix-driven predictions would support this as a general assumption. However, crystal structures have clearly identified examples that violate this assumption. Further improvement in binding predictions must therefore take the entire sequence into account including more or less long ranging correlated effects. It is difficult to envision any simple set of rules that would include these non-linear effects.

It was recently demonstrated that a completely random 8-mer peptide library contains sufficient peptide binders to be detected in an MHC binding assay. These findings suggest a strategy which requires that all possible peptides of a given size and composition under consideration are represented by a systematic set of sub-libraries. In each sub-library, one amino acid in one position is kept constant whereas the remaining positions contain mixtures of amino acids. A similar design was recently reported by Houghten and co-workers who termed it "positional scanning combinatorial peptide library" (PSCPL).

To avoid labour intensive classical experimental epitope mapping, the identification of CTL epitopes in computerised predictions from linear protein sequences may be used to limit the amount of putative epitopes to be tested. For this purpose, more or less accurate prediction programs have been developed (Schafer et al., 1998) and some of these are also available on the internet. Artificial Neural Networks are particularly well suited to perform complex pattern recognition and have already shown some promise in predicting MHC binding.

An illustration of the potential of using trained neural networks in the identification of class I binding peptides is provided by Milik et al. studying peptide binding to mouse MHC class I molecule H2-k$^b$. The authors describe the training of an artificial neural network on binding and non-binding phage followed by a blind test of the network on chicken ovalbumin containing a well characterised H2-k$^b$ epitope.

**Detailed disclosure of the invention**

The broadest aspect of the invention relates to a method to identify a CTL epitope comprising the steps of:

(a) generating primary position specific prediction means from experimental MHC class I structural or peptide binding data;

(b) identifying potentially high affinity binding epitopes by scanning a set of protein sequences and calculating the binding affinity according to the primary prediction means obtained in step (a);

(c) optionally reducing the high number of peptides identified in step (b) by exclusion means based on sequence similarity;

(d) generating experimental binding data for the peptides identified in step (c) or (b);

(e) training one or more artificial neural networks to predict binding affinities to MHC class I, using the experimental binding data from step (d) such that the individual peptide binding data examples, weighted according to their frequency in sub-intervals in the binding affinity range of 1nM to 50,000 nM, are equally presented; and

(f) estimating the binding affinity of a query peptide by testing said query peptide in each of the artificial neural networks trained in step (e) obtaining an approximate binding affinity of the query peptide from each of the artificial neural networks, and calculating the weighted average of the approximate bindings thereby obtaining the estimated binding affinity of the query peptide;

the CTL epitope having a weighted average of the MHC class I binding affinity of less than 500 nM.

Primary position specific prediction means can be generated in many ways. In one embodiment of the invention, primary position specific prediction means are generated by Pool sequencing as described by Falk et al., 1991. This procedure leads to the identification of a simple natural motif and can be represented as rough classifications of more or less preferred amino acids in various positions. In another embodiment, primary position specific prediction means are generated by extended motifs as described by Ruppert et al., 1993. This procedure containing biochemical experiments can be reported as a complete statistical matrix stating the frequency of all amino acids in each position. Such experiments are based on experiments using panels of more or less randomly selected peptides (e.g. as described by Ruppert et al., 1993, Parker et al., 1992) or on peptide library approaches (e.g. as described by Stryhn et al., 1996). In yet another embodiment, primary position specific prediction means are generated by structural information.

It is preferred that the simple motifs and the statistical binding matrices are used to perform a crude search for MHC binding peptides (e.g. as described by Sette et al., 1989, Meister et al., 1995). Predictions are improved considerably when the extended motifs rather than the simple motifs are used. Statistical matrices, such as those generated by the PSCPL approach, are used in a straightforward fashion to calculate the predicted binding. Assuming that each amino acid in each position contributes with a certain binding energy independent of the neighbouring residues ('independent binding of side-chains') and that the binding of a given peptide is the result of combining the contributions from the different residues (Parker et al., 1992), multiplying the relevant matrix values gives an indication of the binding affinity of the corresponding peptide. Yet other embodiments of the invention relates to knowledge-based predictions using approaches such as molecular dynamics (e.g. as described by Rognan et al., 1994, Mata et al., 1998), computational threading (e.g. as described by Altuvia et al., 1995, Altuvia et al., 1997), and computational approaches to address the specificity of MHC pockets (e.g. as described by Vasmatzis et al., 1996, Zhang et al., 1998).

The PSCPL approach has several important advances compared to previous approaches used to determine MHC class I specificities. In particular, it is universal and unbiased. The PSCPL approach identifies all functionally important components of MHC class I binding. The PSCPL approach appears to be more sensitive to secondary anchors and disfavored amino acids than an analogue approach. In fact, it allows a detailed quantitative description of the combinatorial specificity of MHC class I specificities and generates a complete binding matrix specific for the MHC class I in question. These matrices can be used to improve predictions of binding (Stryhn et al., 1996). The PSCPL approach is universal since many different MHC molecules can be addressed with the very same set of PSCPL sub-libraries. This will reduce the costs, experiments and data handling that are otherwise associated with past technologies significantly. A detailed mapping of many MHC specificities can therefore easily be envisioned.

A very robust, low-tech, yet accurate and high-throughput technology for analysing peptide-MHC binding has been developed. A modified spun column gel filtration assay was optimized for efficient separation of free peptides and MHC-bound peptides through a novel principle, which has been termed "gradient centrifugation" (Buus et al., 1995). It has several advantages compared to the classical gel filtration assay: The sensitivity and the throughput is much better, it demands fewer resources both in terms of unique reagents and labour, and it generates less hazardous waste. Once a binding assay is set up, it is easy to determine the binding capacity of any other preparation by using this unknown preparation as inhibitor of the known interaction. The less inhibitor needed to obtain 50% inhibition (the $IC_{50}$). the better the binding. The uninhibited maximum binding must be less than 30% so that the measured $IC_{50}$ will be an approximation of the $K_D$ (otherwise ligand depletion is experienced and the $IC_{50}$ values cannot be trusted).

Natural HLA molecules are commonly obtained from EBV transformed B cell lines (e.g. from homozygous cell lines from the 12.th IHWC). They are usually grown in large scale *in vitro* culture, extracted in detergent, purified by affinity chromatography and concentrated.

In the present working examples, complete 8-mer, 9-mer and 10-mer sets of PSCPL were synthesised as described in table 1, table 6 and in example 1.

Table 1:

| PSCPL design and nomenclature | | | | |
|---|---|---|---|---|
| Position 1 | Position 2 | Position 3 | | Position 8 |
| AXXXXXXX (AX7) | XAXXXXXX(XAX6) | XXAXXXXX (X2AX5) | etc | XXXXXXXA (X7A) |
| CXXXXXXX(CXV) | XCXXXXXX(XCX6) | XXCXXXXX (X2CX5) | etc | XXXXXXXC (X7C) |
| DXXXXXXX (DX7) | XDXXXXXX (XDX6) | XXDXXXXX(X2DX5) | etc | XXXXXXXD (X7D) |
| etc | etc | etc | etc | etc |
| YXXXXXXX(YX7) | XYXXXXXX (XYX6) | XXYXXXXX (X2YX5) | etc | XXXXXXXY (X7Y) |

For each position, 20 sub-libraries were synthesized, each representing one of the 20 naturally occurring L-amino acids at that position. All other positions contained mixtures of amino acids. This lead to the synthesis of a total of 8 x 20 = 160 sub-libraries representing all 25.6 x $10^9$ possible 8-mer peptides. Each PSCPL sub-library was tested for its ability to bind to three different class I molecules in a biochemical binding assay. Strikingly, strong MHC dependent signals were detected (anchor residues represented up to 90% of the amino acids found at the corresponding anchor positions, whereas less than 1% disfavored amino acids were found in some positions) showing that the MHC indeed did select peptides from these unbiased peptide libraries. The PSCPL approach clearly identified the primary anchor residues, and it also identified secondary anchor residues and disfavoured residues. Corresponding to every position throughout the 8-mer motifs of the three MHC class I molecules tested, the PSCPL identified amino adds which could increase or decrease binding, ie. every position can contribute to the overall binding specificity. Reassuringly, the known specificities were confirmed for three out of three MHC class I molecules examined (Stryhn et al., 1996). Furthermore, several minor, but significant, positive contributions and so far unknown numbers of disfavored amino acids were identified. Particularly noteworthy, 12-14 of the 20 amino acids were unacceptable in the known anchor positions, and disfavoured amino acids were also frequently found scattered throughout the remaining positions.

The data can be reported as statistical matrices consisting of relative binding factors. These are calculated for each amino acid at each position as $IC_{50}$ (X8) divided with $IC_{50}$ (PSCPL sub-library), as whether a given amino acid in a particular position increases (RB> 1) or decreases (RB< 1) binding compared to an "average amino acid" in that position. The data can be tabulated and used in a convenient way to illustrate the features of the binding specificity in quantitative terms (see table 6 for an example).

Improved predictions can be performed using matrix values generated by the PSCPL. Assuming that each amino acid in each position contributes with a certain binding energy independent of the neighbouring residues and that the binding of a given peptide is the result of combining the contributions from the different residues (Parker et al., 1992), multiplying the relative binding factors of the different residues of a given peptide indicates to what extent the peptide binds better or worse than a complete random library. The relative success of the PSCPL derived matrix-driven prediction would suggest that the binding of an unknown peptide to a given MHC molecule is to some extent the result of a combinatorial specificity - confirming the assumption.

Thus, in a preferred method according to the invention a PSCPL is used to generate primary position specific prediction means in step (a).

The Major Histocompatibility Complex (MHC) is essential for vertebrate immunology especially for the T-cell mediated immune response. In humans, the term HLA complex is used, whereas the term H2 complex is used in mice. The structural characteristics of the HLA and H2 complexes are fairly similar. Thus, in a preferred embodiment, the MHC class I structural or peptide binding affinity data in step (a) are HLA structural or peptide binding affinity data. By using HLA structural or peptide binding affinity data, the CTL epitopes identified by the method according to the invention will be CTL epitopes binding to the HLA complex, and thus especially preferred in the treatment, prophylaxis and/or diagnosis of humans. In a preferred embodiment the HLA subtypes A1, A2, AB, A11 and/or A24 are selected. Throughout the present application, the term "MHC class I" is used. However, as it should be obvious to the person skilled in the art, if CTL epitopes are to be used in human medicine they should bind to HLA.

One aspect of the present invention relates to the use of primary position specific prediction means, such as unbiased matrix predictions, to scan the non-redundant part of a protein database, such as the TrEMBL database or the SWISS-PROT databases, for potentially high affinity binding epitopes. In this context, high affinity binding epitopes are defined as epitopes with a predicted binding affinity in the primary position specific prediction matrix of $IC_{50}$ less than 100 nM, that is an approximation of $K_D \leq 100$ nM. It is contemplated that this scanning will result in a large selection of potentially high affinity binding epitopes.

Therefore, the high number of peptides are optionally reduced by exclusion means based on sequence similarity. In a preferred embodiment of the invention, the sequence similarity reduction is performed ad modum Hobohm.

The next step in the method of the invention implies that the resulting representative sample of potentially high affinity binders is synthesised and tested in the biochemical binding assay. An illustrative, but not limiting, example of synthesising and testing epitopes is given in example 1.

Although the sequence independent combinatorial specificity may be correct as an average consideration, it is certainly known to be wrong for individual peptides. Based on crystal structures of the MHC molecules, it is unlikely that independent combinatorial specificity is correct. In contrast, it has been demonstrated that artificial neural networks are particularly well suited to handle and recognise any non-linear sequence dependent information or interaction. Such relations can be trained into neural network algorithms comprising an input layer, one or more hidden layers, and an output layer, where the units in one layer are connected by weights to the units in the subsequent layer. Such artificial neural networks can be trained to recognise inputs (i.e. peptides) associated with a given output (i.e. MHC binding affinity values). Once trained, the network will recognise the complicated peptide patterns compatible with binding. In the artificial neural network approach, the size and quality of the training set become of major importance. This is particularly true for the HLA, since only about 0,1-1% of a random set of peptides will bind to any given HLA. Thus, to generate as little as e.g. 100 examples of peptide binders will, if random peptides are screened, require the synthesis and testing of some 100 000 peptides. This will be a very resource and labor intensive proposition even at this modest number of binders in the training set. However, by initial PSCPL selection of peptides, the number of peptides needed to be synthesised to get a reasonable amount of true HLA binders is dramatically reduced.

These data are subsequently used to train the artificial neural networks. The network is trained to predict the actual real-valued strength of the MHC binding, and not, as in previous approaches, artificially selected categories of binders and non-binders. Using a balanced training scheme, where the original data, having a skew distribution over the binding affinity interval ranging from 1 nM to 50 000 nM, is selected, such that examples from sub-intervals are presented equally often, results in a network that is able to predict almost equally well over the entire range. Thereby, over-prediction, or under-prediction is avoided that would bias the network such that it mostly would identify either binders or non-binders.

The next step of the method according to the invention comprises estimating the binding affinity of a query peptide. The query peptide can be derived from any source of peptide libraries such as random peptide combinations or commercial, private, or public databases of protein sequences. In a preferred aspect of the invention, the protein database is a HIV-1 or HIV-2 protein sequence database, or a part thereof. A preferred database comprises full-length sequenced HIV-1 genes, such as The Los Alamos 1998/1999 HIV sequences database (http://hiv-web.lanl.gov/). These proteins are the products of translations of all available full-length sequenced HIV-1 genes and reflect the principal genetic diversity of HIV-1.

In one embodiment of the invention, the artificial neural network comprises three layers of neurons. In another embodiment, the query peptide is presented to the artificial neural network as a binary number of 20 digits. As a result of this embodiment, the input layer will consist of 20 times the number of amino acids in the query peptide.

As described above, it can be of significant value to have the binding affinity estimated by several artificial neural networks. Thus, in one embodiment of the invention, one or more artificial neural networks are trained. The training is preferably performed on different parts of the training data to incorporate as many features as possible to the network. In one aspect of the invention, only one artificial neural network is trained. In another embodiment 2, 3, 4, 5, 6, 7, 8, 9, 10, 11, 12, 13, 14, 15, 20, 25 or 30 artificial neural networks are trained. In a most preferred embodiment, 7 artificial neural networks are trained.

In a preferred embodiment, the training algorithm is a gradient descent type, where the adjustable network weights iteratively are modified in order to make the network produce the correct output upon presentations of the sequence fragments. In an even more preferred embodiment, the networks are trained using the error function suggested by McClelland.

In one embodiment of the invention, each artificial neural network is tested. The testing is preferably performed by cross validation. An illustrative example of cross validation testing is given in example 2.

The essential step in the use of the artificial neural networks trained relates to estimating the binding of a query peptide. This is carried out by testing said query peptide in each of the artificial neural networks trained obtaining an approximate binding affinity of the query peptide from each of the artificial neural networks, and calculating the weighted average of the approximate bindings, thereby obtaining the estimated binding affinity of the query peptide.

Based on phylogenetic studies, HIV-1 viruses are classified into 3 genetic groups; group M for Major, group O for Outer and group N for New. While HIV-1 group M viruses are responsible for the majority of HIV-infections worldwide, HIV-1 group O and N are mainly found in restricted areas in Central Africa (mostly Cameroon). HIV-1group M is currently subdivided into 8 reference genetic subtypes: A, B, C, D, F, G, H, J (available at http://hiv-web.lanl.gov/). However, recombination events between distinct subtypes have been demonstrated in individuals who are co-infected with more than one genetic subtype. Subtypes AB, AC, AD, ADI, AE, AG, AGI, AGJ, BF, CD are intersubtype-recombinant HIV-1 sequences. Albeit, viruses belonging to group M are responsible for most of the HIV-1 infection cases reported by WHO. The world-wide spreading potential of HIV-1group O and N viruses is not known and should not be underestimated. Moreover, the recent characterisation of HIV-1M/O recombinant viruses raises new speculations on the pandemic spreading of such viruses.

After the identification of a CTL epitope, based on the artificial neural network estimation of the binding affinity of a query peptide, the CTL epitope must, in most embodiments of the invention, have further properties to be well suited in medical use such as incorporation into a vaccine or a diagnostic agent. One such property is a certain conservation of the CTL epitope among HIV groups and subtypes. Thus, in one embodiment of the present invention, the method to identify a CTL epitope further comprises the step of:

(k) determining the global conservation of the query peptide across a set of HIV protein sequences;

the CTL epitope of step (k) having an MHC class I binding affinity of less than 500 nM.

In this context, the term "global conservation" should be understood as a number representative for, among the 9 HIV-1 proteins Gag, Pol, Env, Vif, Vpr, Vpu, Tat, Rev and Nef, how often the CTL epitope is found in all subtypes and groups of HIV-1. The percent global conservation is calculated as the number of protein sequences harbouring the query sequence divided by the total number of protein sequences times 100. The term "intra-subtype conservation" should be understood as a number representative for, among the 9 HIV proteins Gag, Pol, Env, Vif, Vpr, Vpu, Tat, Rev and Nef, how often the CTL epitope is found within one subtype of HIV. For example, if one epitope is found in 6 out of 8 HIV-1 subtype A Gag proteins, but also in 10 out of 32 HIV-1 subtype B Gag proteins and in 4 out of 8 HIV-1 subtype C Gag proteins, its intra-subtype A, B and C conservation will be 75%, 31,25% and 50% respectively. In one embodiment of the invention, the global conservation is determined by comparing across all subtypes of HIV protein sequences in the Los Alamos HIV-1 protein database.

In a preferred embodiment of the invention, the global conservation is more than 1%. In a related aspect, it is preferred that the method according to the invention is carried out with a global conservation of more than 1% and a cut off value for the weighted average of the MHC class I binding affinity for the query peptide of less than 100 nM. Hereby, a CTL epitope with high affinity binding and an intermediate global conservation is obtained. This especially preferred embodiment is further described in example 4. An example of specific CTL epitopes identified by the method according to the invention, satisfying these criteria are given in table 5A.

In a preferred embodiment, the global conservation is more than 8% across HIV protein sequences. In a related aspect, it is preferred that the method according to the invention is carried out with a global conservation of more than 8% and a cut off value for the weighted average of the MHC class I binding affinity for the query peptide of less than 500 nM. Hereby, a CTL epitope with an intermediate affinity binding and a high global conservation is obtained. In a especially related embodiment, only a CLT epitope with intermediate binding is identified by the further criteria that the weighted average of the MHC class I binding affinity is more than 50 nM. This especially preferred embodiment is further described in example 4. An example of specific CTL epitopes identified by the method according to the invention, satisfying these criteria are given in tables 5B and 5C.

In a general aspect of the invention, the weighted average MHC class I binding affinity is less than 100 nM.

As will be recognised by the person skilled in the art, peptides naturally presented by MHC class I molecules are 8 to 10 amino-acid long and contain specific anchor residues that share properties like hydrophobicity or charge. The anchor residue side chains are deeply embedded within the antigen-presenting groove of the MHC molecule, suggesting that anchor residues are primarily involved in MHC class I binding. Because anchor residue side chains are buried in the peptide binding groove, it is believed that anchor residues are not directly involved in the T-cell response. This suggest that anchor residues can be replaced without affecting T-cell recognition.

In the case of the HLA complex, it is generally accepted that the binding affinity of an HLA-A2 peptide epitope can be improved by replacing non-optimal amino acids in primary anchor positions with more optimal amino acids in said primary anchor positions. It is contemplated that this is the case for all CTL epitopes according to the invention. Thus, using HLA-A2 as an example, the primary anchor positions are defined as the second amino acid in the epitope (2) and the terminal amino acid in the epitope ($\Omega$). For example, the primary anchor positions of a 9 amino acid long HLA-A2 binding motif are located at position 2 and position 9. In a preferred embodiment, the amino acids in anchor position 2 are replaced with Leucine, Methionine, Glutamine, or Isoleucine. The most preferred optimal primary anchor amino acids in this context is Leucine at position 2. In another embodiment of the invention, the amino acids replaced in anchor position $\Omega$ are Valine, Isoleucine, Leucine, or Alanine. The most preferred optimal primary anchor amino acids in this context is a Valine at position 9.

It is presently contemplated that immunisation with a modified CTL epitope with improved binding affinity will induce a higher CTL response than that induced with the natural CTL epitope. Therefore, the identification of intermediate CTL epitope binders in the present invention is very important Such intermediate binders are potential new targets against HIV infected cells. During the infection, these intermediate binding epitopes bind to MHC-I with an affinity that is too low to induce sufficient immunity but high enough to provide an excellent target if such sufficient immunity can be induced. In one preferred embodiment of the invention, the non-immunogenic intermediate binders, identified by the method described in the present invention, can be turned into immunogenic by exchange of amino adds in anchor positions. The possibilities to exchange anchor positioned amino acids can further be evaluated for effects on the binding affinity of the epitope with the artificial neural network. Thus, the combination of identifying intermediate binders which are antigenic but not sufficient immunogenic, taken together with the exchange of amino acids in anchor positions for improving MHC-I binding affinity is new and provides many new possibilities for a successful HIV vaccine. The CTL effectors raised against the improved epitope will be able to recognise and subsequently lyse target cells presenting the natural epitope at their surface.

Table 3

| | Anchor pos.2 | | | | | | | Anchor pos.9 |
|---|---|---|---|---|---|---|---|---|
| X | LEU MET GLN ILE | X | X | X | X | X | X | VAL ILE LEU ALA |

All combinations of amino acid modification in table 3 at positions 2 and/or $\Omega$ are possible, however, they will not yield the same binding affinity for MHC class I. The binding affinity is also influenced by secondary anchor positions.

Thus, in one embodiment of the invention, the method of identifying a CTL epitope further comprises the steps of:

(l) modifying the CTL epitope by computationally replacing amino acids in the anchor positions; and
(m) estimating the binding affinity of the modified CTL epitope of step (l) by testing said modified CTL epitope in each of the artificial neural networks trained in steps (e) or (i) obtaining an approximate binding affinity of the CTL epitope from each of the artificial neural networks, and calculating the weighted average of the approximate bindings thereby obtaining the estimated binding affinity of the CTL epitope;

the modified CTL epitope having an improved predicted MHC class I binding affinity compared to the natural CTL epitope. By improved binding affinity is obviously understood a lower $IC_{50}$ value.

An illustrative example of the improvement of the binding affinity by substitution of amino acids at the anchor positions is given in example 4. An example of specific CTL epitopes identified by the method according to the invention, satisfying these criteria are given in table 5D. Thus, in an especially preferred embodiment of the present invention, a modified CTL epitope will be able to raise an immune response against a plethora of natural CTL epitopes sharing immune-dominant parts of the epitope, that is epitopes that only differ in the amino acids in the anchor positions. A method to validate if improved CTL epitope is able to raise a CTL immune response that cross-reacts with the natural epitope is described in Example 7.

When the term "improved binding" or the like is used herein it is reflected as a decreased binding affinity (or in short binding). Thus, a CTL epitope binding with 50nM has a binding of less than 500nM, and the CTI epitope binding with 50nM has an improved binding compared to the CTL eptipe with a binding for 400 nM.

The CTL epitope identified by the present invention must satisfy the criteria which the cytotoxic T-cells respond to. Presently, data suggest that T-cells will respond to CTL epitopes consisting of 7, 8, 9, 10 or 11 amino acids.

As should be evident from the method according to the invention, one purpose is the identification of a CTL epitope. Thus, one aspect of the invention relates to a CTL epitope having the sequence of SEQ ID NO:1.

In a preferred embodiment, the invention relates to a CTL epitope with a global conservation of more than 1% and a weighted average of the MHC class I binding affinity for the query peptide of less than 100 nM.

The preparation of vaccines which contain peptide sequences, e.g. CTL epitopes, as active ingredients is generally well understood in the art, as exemplified by U.S. Patents 4,608,251; 4,601,903; 4,599,231 and 4,599,230. Typically, such vaccines are prepared as injectables either as liquid solutions or suspensions; solid forms suitable for solution in liquid or suspension in liquid prior to injection may also be prepared. The preparation may also be emulsified. The active immunogenic ingredient is often mixed with excipients which are pharmaceutically acceptable and compatible with the active ingredient. Suitable excipients are, for example, water, saline, dextrose, glycose, glycerol, ethanol, or the like, and combinations thereof. In addition, if desired, the vaccine may contain minor amounts of auxiliary substances such as wetting or emulsifying agents, pH buffering agents, bupivacain, or adjuvants which enhance the effectiveness of the vaccines.

The vaccines are conventionally administered parenterally, by injection, for example either subcutaneously or intramuscularly. Additional formulations which are suitable for other modes of administration include suppositories and, in some cases, oral formulations. For suppositories, traditional binders and carriers may include, for example, polyalkalene glycols or triglycerides; such suppositories may be formed from mixtures containing the active ingredient in the range of 0.5% to 10%, preferably 1-2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10-95% of active ingredient, preferably 25-70%.

The CTL epitope or epitopes may be formulated into the vaccine as neutral or salt forms. Pharmaceutically acceptable salts include acid addition salts (formed with the free amino groups of the peptide) and acid addition salts that are formed with inorganic acids such as, for example, hydrochloric or phosphoric acids, or such organic acids as acetic, oxalic, tartaric, mandelic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The vaccines are administered in a manner compatible with the dosage formulation, and in such an amount as will be therapeutically effective and immunogenic. The quantity to be administered depends on the subject to be treated, including, e.g., the capacity of the individual's immune system to mount an immune response, and the degree of protection desired. Suitable dosage ranges are of the order of several hundred micrograms of active ingredient per vaccination with a preferred range from about 0.1 µg to 1000 µg, such as in the range from about 1 µg to 300 µg, and especially in the range from about 10 µg to 50 µg. Suitable regimes for initial administration and booster shots are also variable but are typified by an initial administration followed by subsequent inoculations or other administrations.

The manner of application may be varied widely. Any of the conventional methods for administration of a vaccine are applicable. Preferred routes of administration are the parenteral route such as the intravenous, intraperitoneal, intramuscular, subcutaneous, intracutaneous or intradermal routes; the oral (on a solid physiologically acceptable base or in a physiologically acceptable dispersion), buccal, sublingual, nasal, rectal or transdermal routes. The dosage of the vaccine will depend on the route of administration and will vary according to the age of the person to be vaccinated and, to a lesser degree, the weight of the person to be vaccinated.

Some of the CTL epitopes are sufficiently immunogenic in a vaccine, but for some of the others, the immune response will be enhanced if the vaccine further comprises an adjuvant substance. Various methods of achieving adjuvant effect for the vaccine include use of agents such as aluminum hydroxide or phosphate (alum), commonly used as a 0.05 to 0.1 percent solution in phosphate buffered saline, admixture with synthetic polymers of sugars (Carbopol) used as a 0.25 percent solution, aggregation of the CTL epitope in the vaccine by heat treatment with temperatures ranging between 70° to 101°C for 30 second to 2 minute periods respectively. Aggregation by reactivating with pepsin treated (Fab) antibodies to albumin, mixture with bacterial cells such as *C. parvum* or endotoxins or lipopolysaccharide or lipid A components of gram-negative bacteria, emulsion in physiologically acceptable oil vehicles such as mannide mono-oleate (Aracel A) or emulsion with 20 percent solution of a perfluorocarbon (Fluosol-DA) used as a block substitute may also be employed. According to the invention, DDA (dimethyldioctadecylammonium bromide) is an interesting candidate for an adjuvant, but also Freund's complete and incomplete adjuvants as well as QuilA, Quil A derivates and RIBI adjuvants are interesting possibilities.

Other possibilities to enhance the immunogenic effect involve the use of immune modulating substances such as lymphokines (e.g. IFN-γ, IL-2 and IL-12) or synthetic IFN-γ inducers such as poly I:C in combination with the abovementioned adjuvants.

Thus, the invention also relates to the use of a CTL epitope having the sequence of SEQ ID NO:1 for the manufacture of a vaccine.

Finally, one aspect of the invention pertains to the use of a CTL epitope having the sequence of SEQ ID NO: 1 for the manufacture of a diagnostic agent.

## Examples

### *Example 1: Peptide binding*

Cells: An EBV transformed cell line, RML, were used from the HLA-A*0204 production.

MHC purification: Cells were lysed with detergent and MHC class I molecules were affinity purified as previously described (Buus et al., 1995). The monoclonal antibody used for affinity purifications was BB7.2. Human β2-microglobulin was obtained from the urine of uraemic patients and purified to homogeneity by gelfiltration and chromatofoccusing, or purchased from Sigma.

Peptide synthesis: Peptides were synthesised on a parallel multiple column peptide synthesiser (Holm et al., 89) on a PepSyn KA resin with the first amino acid attached and using the FMOC-protection strategy. After completion of all coupling according to the sequence, the protection groups were removed, and the peptides cleaved off the resin with 95% aqueous trifluoro acetic acid for 2 hours. The peptides were precipitated with ether and ether/ethylacetate, and lyophilised. The peptides were analysed by reversed-phase high performance liquid chromatography (HPLC), and by mass spectroscopy (MS).

Peptide radiolabelling: 2-10μg peptide was labelled with 1mCi $^{125}$Iodine (Amersham, Sweden) using chloramine T as previously described (Buus et al., 1986). The specific activity was measured to 1-5 x $10^8$cpm/mg peptide.

Peptide-MHC class I binding assay: In general, affinity purified MHC class I molecules at 2-10 μM were incubated at 18°C for 48 h with approximately 10-100 nM radiolabelled peptide in a reaction mixture containing a protease inhibitor cocktail as previously described (Buus et al., 1986). The final detergent concentration in the reaction mixture was 0.05% NP-40 (Sigma). Peptide-MHC class complexes were separated (in duplicate) from free peptide by gel filtration using G25 spun columns (Buus et al., 1995).The radioactivity of the excluded "void" volume and of the included volume were measured by gamma spectrometry (Packard). The fraction of peptide bound to MHC class I relative to the total amount of offered peptide was calculated and corrected by subtracting the fraction of peptide (usually less than 1%) which appeared in the void volume in the absence of MHC. The spun columns and the previously reported Sephadex G50 column chromatography assay gave similar results (Buus et al., 1995). The recovery of complexes and the exclusion of ligand were 95-100%. Binding data are illustrated in table 11.

### *Example 2: Neural Networks*

A data driven neural network algorithm was used, with network architectures comprising an input layer encoding the sequence information, one layer of hidden units, and a single output unit representing the floating point value for the binding affinity (normalized to the interval between zero and one). During training on the data consisting of pairs of sequence fragments and the associated binding affinity, the weights were adjusted essentially according to the method described by Rumelhart et al. (1986). Hence, each neuron (unit) besides those in the input layer, calculates a weighted sum of its inputs and passes this sum through a sigmoidal function to produce the output

$$O = \sigma(\sum_{n=1}^{N} w_n I_n - t)$$

where N is the number of neurons in a layer, $I_n$ is the nth input to the neuron and $W_n$ is the weight of this input. σ is the sigmoidal function

$$\sigma(x) = \frac{1}{1 + \exp(-x)}$$

and t is its threshold.

The training algorithm was of the gradient descent type, where the adjustable network weights iteratively are modified in order to make the network produce the correct output upon presentations of the sequence fragments. When training the networks we used a slightly more powerful error function suggested by McClelland

$$E = -\sum_{\alpha,i} \log(1 - (O_i^\alpha - T_i^\alpha)^2)$$

instead of the conventional error function

$$E = \sum_{\alpha,i} (O_i^\alpha - T_i^\alpha)^2$$

This logaritmic error function reduced the convergence time considerably, and has also the property of making a given network learn more complex tasks (compared to the standard error measure) without increasing the network size. The network was trained in experimentally determined relations between amino acid sequence and the experimentally determined binding affinity. The purpose of the training is to adjust the weights and thresholds in the network to quantitatively minimize the error between the prediction and the experimentally determined binding affinity.

As the experimental data typically is not evenly distributed over a given range of binding affinities, a balancing scheme was designed with the purpose of avoiding that the network only would obtain sufficient prediction accuracy in the most populated part of the range.

Typically, the most populated part of the range is "non-binders" which can be defined quantitatively using a specific threshold. The output interval (zero to one) was divided into a number of bins, and for each bin, the number of examples found was calculated. In the balancing training scheme, the examples were selected for presentation to the neural network such that all intervals were represented equally often. Specifically, the examples from the least populated bin were always selected in a training epoch, while randomly selected examples from the other more populated bins were skipped, such that, on average, all bins were represented equally often.

Each amino acid in the sequence fragments was represented as a binary string of 20 bits thus using 20 input neurons for each residue. Alanine and cysteine were, for example, represented as the strings 10000000000000000000 and 01000000000000000000, respectively. The output was a single real value representing the affinity renormalized using various forms of logarithmic scaling.

To find the best network, the performance of different network architectures (different numbers of units in the input (8, 9, or 10-mers) and in the hidden layer) were tested by training using part of the data as described above and testing the performance of the network on the remaining sequence fragments, using the classification correlation coefficient C (Mathews 1975, Brunak 1991) as quality measure as well as the standard Pearson correlation measure. For example, the classification coefficient is defined as follows (when a specific threshold separating binders and non-binders has been defined),

$$C = \frac{P_x N_x - N_{fx} P_{fx}}{\sqrt{(N_x + N_{fx})(N_x + P_{fx})(P_x + N_{fx})(P_x + P_{fx})}}$$

In this equation $P_x$ is the number of true positives (experimentally determined binders, predicted binders), $N_x$ the number of true negatives (experimentally non-binders, predicted non-binders), $P_{fx}$ the number of false positives (experimentally non-binders, predicted binders), and $N_{fx}$ the number of false negatives (experimentally binders, predicted non-binders). It is common practice to obtain the final prediction for a query fragment by averaging over the prediction from networks of different architectures, where the networks possibly have been trained on different parts of the data available. Here, the test performances have been calculated by cross-validation: The data set was divided into seven approximately equal-sized parts, and then every network run was carried out with one part as test data and the other six parts as training data. The performance measures were then calculated as an average over the seven different data set divisions (the output from some of the networks may be renormalized before averaging). The number of cross-validation networks is typically set by the amount of data available (if the data is sparse, it makes little sense to train hundreds of networks), and the amount of computer time one wants to spend. In this case, seven networks were chosen because it gave a resonable size of the training and test sets.

For four out of four peptide-MHC class I combinations examined, the artificial neural networks performed better than the matrix-driven prediction. The predictions had been generated in a fashion predicting the actual binding $IC_{50}$ value rather than an arbitrary classification into binders vs. non-binders. Indeed, it had been possible to predict binders over a large range leading to the identification of high affinity binders as well as binders of lower affinity and non-binders. For the purpose of the invention, the fidelity of the HLA-A*0204 prediction is most important. 316 8-mer and 398 9-mer peptides were synthesised and tested for binding. Each of the 7 artificial neural networks were trained on 6/7 of the peptides leaving the remaining 1/7 for test. Each of the 7 artificial neural networks used a different 1/7 of the peptides in a cross-validation scheme. Compiling the average predicted vs. the observed values (after a logarithmic transformation) allowed a linear regression analysis which (without correction) yielded a line close to the expected y = x (for the 9 mers the line was log (obs) = log (pred) x 0.9734 + 0.1807 with a Pearson coefficient of 0.87). Analysing the same data set with alternative predictions gave the following Pearson coefficients: 0,78 (Rammensee http://134.2.96.221/scripts/hlaserver.dll/home.htm), 0,83 (Parker, hftp://bimas.dcrt.nih.gov/molbio/hla_bind/) and 0,85 (Stryhn et al., 1996). Even a first generation artificial neural network is better than any of the alternative prediction methods.

The final network ensemble thus consists of 7 artificial neural networks, where the prediction on query peptides results from an average over the 7 output values.

### Example 3: Selection Criteria

The HIV-1 protein sequences for the query epitope were obtained from The Los Alamos 1998/1999 HIV sequences database (http://hiv-web.lanl.gov/). These proteins are the products of translation of all available full-length sequenced HIV-1 proteins and reflect the principal genetic diversity of HIV-1 (Reference Sequences Representing the Principal Genetic Diversity of HIV-1 in the Pandemic available at http://hiv-web.lanl.gov/ ). All available Gag (96 seq.), Pol (86 seq.), Vif (265 seq.), Vpr (173 seq.), Vpu (156 seq.), Tat (101 seq.), Rev (105 seq.), Env (213 seq.) and Nef (251 seq.) protein sequences were extracted from the HIV-1/SIVcpz protein alignments. All residual gaps that were inserted in the sequences for alignment purposes were removed. Amino acid sequences from Los Alamos HIV database (containing also all gene bank HIV sequences) were used and only *complete* sequences of each protein were selected.

Table 7 summarises the number of HIV-1 protein sequences from which HLA-A2 epitopes were predicted and their distribution within the genetic subtypes composing group M (subtypes A, AB, AC, AD, ADI, AE, AG, AGI, AGJ, B, BF, C, CD, D, F, G, H, J) or within the groups N or group O. HIV-1-related sequences such as SIVcpz were included since SIVcpz viruses share a high genetic homology with HIV-1 group N in Env.

All proteins were individually scanned for peptides of 9 or 8 residues length that displayed HLA-A2 binding motifs using the trained artificial neural network (as described above). The cut-off for the maximum value of the predicted peptide-HLA-A2 binding affinity was set at 500nM.

A total of 4215 HLA-A2 binding motifs with $IC_{50} < 500nM$ and with a global HIV-I conservation >0% were found after the scanning of all HIV proteins (see table 2).

### Example 4: Characterisation of the predicted epitopes

Clinically relevant HLA-A2 epitopes to be included in a vaccine should be able to potentially elicit an immune response against either most of the viruses of a same HIV-1 subtype or against viruses belonging to more than one HIV-1 genetic subtype or against viruses belonging to different HIV-1 genetic groups.

Table 8 shows that the HIV-1 subtype B sequences often dominated in the database that was used for predicting HLA-A2 epitopes. For example, 66,5% of the Nef proteins that were used for predicting Net-specific HLA-A2 epitopes belonged to the HIV-1 subtype B. This bias renders difficulty for the evaluation of whether or not a single epitope will cover different HIV-1 subtypes or will represent only HIV-1 subtype B. During a first attempt to select the most clinically relevant HLA-A2 epitopes, the global conservation cut-off was placed at 50%. Later it was observed, that the epitopes were counterselected that were found in HIV-1 subtypes other than subtype B (but not in subtype B). These subtypes were less represented in the database. For example, an epitope predicted in Nef with a global conservation of 10,35% could in fact be present in all proteins within subtype C (7,97%), G (1,19%) and H (1,19%). In order to take this bias of the database into account in the selection criteria, all epitopes with a global conservation of more than 8% were analysed. This cut-off allowed for picking up epitopes that are conserved among minor subtypes.

It was identified which HIV-1 subtypes were related to each epitope that harboured at least a global conservation of 8%. According to the database nomenclature, all HIV-1 sequences are named according to the genetic subtypes they belong. Thus, the first letter appearing in their names corresponds to the subtype. The names of the protein sequences that harbour 100% amino acid sequence homology with each epitope were sorted and it was counted how many times any of the letters A, AB, AC, AD, ADI, AE, AG, AGI, AGJ, B, BF, C, CD, D, F, G, H, J or O or N appeared in the names.

**Positioning of each epitope within the reference HIV-1 strain HXB2.**

In order to better evaluate whether some epitopes could be variants of each other's, the epitopes within the 9 proteins of the HXB2 reference strain were mapped. This positioning was performed by locating the region of HXB2 that harboured the best homology with each epitope analysed. Each epitope was aligned with the corresponding HXB2 protein using classical pairwise homology search.

**Ranking the predicted epitopes by their binding affinity for the molecule HLA-A2**

Peptide epitopes with a binding affinity of $IC_{50} \leq 50nM$ are generally recognised as good binders towards HLA-A2, whereas peptide epitopes with a $IC_{50}$ binding affinity between 50nM and 500nM are considered intermediate binders. Good binders will harbour a good capacity in eliciting a CTL response whereas intermediate binders will not elicit such a good CTL response. On the other hand, they provide good targets, since infected cells in the host can present these CTL epitopes that can function as a target without necessarily being able to induce CTL immunity. However, the neural network could mispredict the binding affinity by a factor 2. Thus, in order to be inclusive, the $IC_{50}$ affinity threshold that defined HLA-A2 good binders was raised to 100nM and such HLA-A2 epitopes were considered as natural immunogenic. All HLA-A2 epitopes with a binding affinity $IC_{50} \leq 100nM$ and with more than 1% of global conservation were sorted separately. They were considered natural immunogenic HIV-1 HLA-A2 epitopes. These peptide epitopes are listed in table 5A. Table 5A contains 213 HLA-A2 epitopes. They are grouped by protein family and ranked according to their length (9 or 8 amino acid residues) and to their global conservation. All predicted HLA-A2 epitopes with a binding affinity $IC_{50}$ between 50nM and 500nM and with a global conservation above 8% were sorted and considered natural HLA-A2 intermediate binders with potentially low capacity to elicit a good CTL response. Those intermediate binders were regarded as low immunogenic but antigenic. All predicted HLA-A2 epitopes with a binding affinity encomprised between 50nM and 500nM and with a global conservation above or equal to 8% were sorted separately in tables 5B and 5C. 158 (110 9-mers (table 5B) and 48 8-mers (table 5C)) HLA-A2 natural epitopes were found. These 158 HLA-A2 epitopes were regarded as natural intermediate binders whose binding affinity could be increased to obtain an improved binding affinity of $IC_{50}$ below 100nM by modifying one or two of the primary anchor positions. The improved epitopes, and their predicted binding, is listed in tables 5B and 5C. In a few cases an improved epitope will cover more than one natural epitope. Thus table 5B lists 100 improved epitopes, and table 5C lists 46 improved epitopes. The selection criteria for "good and intermediate HLA-A2 binders" are listed in table 2.

Table 2.

| Criteria for selection of predicted 8+9 mer HIV HLA-A2 CTL epitopes for vaccines | | | | |
|---|---|---|---|---|
| Table | $IC_{50}$ binding affinity | global HIV conservation | # epitopes | immunogenic characterisation |
| | < 500 nM | > 0% | 4215 | all possible, natural |
| 5A | ≤ 100 nM | >1% | 213 | all immunogenic, natural |
| 5B, 5C | 50-500 nM | > 8% | 158 | low immunogenic but antigenic with possibility of optimising immunogenicity by designing best anchor residues |
| 5D | 50-500 nM | > 8% | 812 | immunogenic relevant, new designed |
| 5E | 50-500 nM | >8% | 32 | low immunogenic that cannot be improved by anchor exchange |

**Improvement prediction from natural intermediate binders**

it was evaluated, whether or not the binding affinity of each of the best 146 intermediate binders (table 5B) could be improved by exchanging one or two of the primary anchor residues. For that purpose, each peptide sequence was designed by permutating the primary anchor residues with each of the amino acids described above. All combinations were analysed. For a natural intermediate binder whose sequence is XLXXXXXXA and binding affinity $IC_{50}$>100nM, 15 epitopes were artificially designed, as seen in table 4:

Table 4

| | | | |
|---|---|---|---|
| XLXXXXXXV | XMXXXXXXV | XQXXXXXXV | XIXXXXXXV |
| XLXXXXXXI | XMXXXXXXI | XQXXXXXXI | XIXXXXXXI |
| XLXXXXXXL | XMXXXXXXL | XQXXXXXXL | XIXXXXXXL |

Table 4   (continued)

| XLXXXXXXV | XMXXXXXXV | XQXXXXXXV | XIXXXXXXV |
|---|---|---|---|
| | XMXXXXXXA | XQXXXXXXA | XIXXXXXXA |

The HLA-A2 binding affinity of these 15 designed HLA-A2 epitopes were then predicted using the artificial neural networks described in the present invention. The affinity threshold of the predicted binding was set at 100nM in order to obtain immunogenicity. All designed epitopes with a predicted binding affinity $IC_{50}$ below 100nM and with an $IC_{50}$ value lower than the $IC_{50}$ value of the natural epitope were regarded as improved HLA-A2 epitopes. The total amount of 958 improved epitopes is listed in tables 5B, 5C and 5D.

From these, the best new improved binder for each natural epitope was selected (see tables 5B and 5C).

All HLA-A2 predicted epitopes from table 5B that showed an affinity between 50nM and 100nM and with a global conservation above 8% were assayed for affinity improvement. 20 peptide epitopes were sorted from table 5B in which the affinity could theoretically be improved and were compared to the binding affinity of the respective natural epitope.

***Example 5: Selection of clinically relevant new HLA-A2 epitopes for building HIV-1 polytope vaccines.***

An ideal "cover-all" candidate vaccine will be composed of 9 epitope-sets. Each set will be specific to one of the 9 HIV-1 proteins and will be composed of 1 to 10 epitopes fulfilling the above-described criteria.

| Set 1 | 1 to 10 epitopes being specific for | Vpu |
|---|---|---|
| Set 2 | " " | Vpr |
| Set 3 | " " | Vif |
| Set 4 | " " | Rev |
| Set 5 | " " | Tat |
| Set 6 | " " | Nef |
| Set 7 | " " | Gag |
| Set 8 | " " | Pol |
| Set 9 | " " | Env |

A minimum of one epitope per set could be envisaged, however, several reasons advice against a "minimal selective approach":

-1) very few single epitopes can fulfil all selection criteria and in particular the "cover-all" conservation criteria. This is especially true for the A2-epitopes predicted in regulatory or accessory viral proteins such as Rev, Tat, Vpu, Vpr, Vif and Nef. The combination of several epitopes is necessary in order to cover as much as possible the diversity of HIV-1 strains.

-2) since the virus is able to escape the host immune response by changing its protein sequence, choosing only one single epitope per viral protein may enhance the chance for the virus to escape the CTL response elicited by the vaccine. In order to fight the virus, as many epitopes as possible should be included in a set. Two strategies can be chosen in order to overcome the viral escape. One strategy is to select all A2-epitopes that form an "epitopic" family and thus are epitope variants of each other. By immunising with such a family of variants one expects to induce an immune response directed against as many escape variants as present in the database. However, such epitope variant families are located in some region of the viral protein that can undergo high mutation rates without being too damaging for the virus. The virus has then more chance to introduce new escape mutations in this region under the immune selective pressure due to the vaccine.

Another and complementary approach is to select highly conserved single epitopes located within highly conserved region of the viral proteins. Such a high conservation might reflect functional domains of the protein that cannot undergo escape mutations without being deleterious for the virus.

-3) Incomplete data are nowadays available on the selective mechanisms that rule the antigen-processing of a protein into peptides (see www.cbs.dtu.dk/services). It is believed that the excision of a particular peptide from a whole protein depends on the recognition by proteolytic enzymes of particular residues that flank that peptide. Since these flanking residues are not well known, it is uncertain if each selected epitope will be properly processed from the native viral protein in the form of a peptide (at the surface of the virus infected cells) and then be antigenically available. Albeit the selected epitopes are able to raise a good immune response through vaccination, such immune response can only be efficient if these epitopes are presented *in vivo* as peptide-HLA-A2 complexes at the surface of infected cells. In order to overcome this uncertainty, several epitopes located in different regions (thus theoretically in distinct flanking environments) of the viral protein should be selected.

Based on the criteria described above, 53 out of 354 epitopes were selected to be incorporated in a synthetic polytope vaccine. These 53 epitopes are shown in tables 9 and 10.They are divided in 8 sets:

Set 1: 4 epitopes located in Vpu
Set 2: 4 epitopes located in Vpr
Set 3: 6 epitopes located in Vif
Set 4: 4 epitopes located in Rev
Set 5: 5 epitopes located in Nef
Set 6: 10 epitopes located in Pol from which 4 can be located in the Reverse Transcriptase polypeptide (p51) and one epitope in the integrase polypeptide (p31).
Set 7: 10 epitopes located in Gag
Set 8: 10 epitopes located in Env

Few epitopes can be selected for regulatory and auxiliary proteins such as Vpu, Vpr, Vif, Rev and Nef, compared to structural proteins such as Pol, Gag and Env. This is explained by the smaller size and the higher variability of some of these regulatory and auxiliary proteins that renders it difficult for all selective criteria to be fulfilled by the epitopes found.
For example, no epitope could be selected for Tat, since the few epitopes predicted by the artificial neural networks did not full fill the conservation criteria.
The few epitopes selected for Vpu, Vpr, Vif, Rev were not highly conserved in all HIV-1 subtypes, however they presented a certain interest because they were conserved among more than 20 % of the strains within at least one HIV-1 subtype. For example, in set 1, the natural epitope IVGLIVAL was conserved in 66,6% of HIV-1 AE recombinant strains despite its global conservation of 3,2% among all HIV-1 strains. This epitope was regarded as a good candidate immunogen for raising a CT1 response against HIV-1 AE recombinant strains.
Most of the selected epitopes were improved for their binding to HLA-A2. The use of improved epitopes was dictated by the observation that most of the best-conserved natural epitopes were intermediate or low A2-binders, also, these improved epitopes could be expected to raise a CTL response against the natural epitopes from which they were derived. In some cases, a particular improved epitope was an improved version of several natural epitopes, thus the immune response raised against such improved epitope was able to be directed against all HIV-1 strains expressing the different natural epitopes.
For example the improved epitope ILAIVVVWTV was related to two natural epitopes: IIAIVVWTI (Kd=373,1nM) and ILAIVVWTI (Kd=39,9nM) that were found respectively, in 32,1% and 9,6% of all HIV-1 strains tested. Thus an immunisation performed with this improved epitope was expected to raise an immune response directed against 41,7% (32,1%+9,6%) of HIV-1 strains (% of global coverage). The natural epitope IIAIVVWTI was conserved in 33,3% of HIV-1 subtype A strains, 32% of HIV-1 subtype B strains, 54% of HIV-1 subtype C strains, 56,2% of HIV-1 subtype D strains, 33,3% of recombinant AE strains, 66,65% of subtype F and 66,65 of subtype H. The natural epitope ILAIVVWTI was found in 41,6% of HIV-1 subtype A strains, 33,3% of AC recombinant strains, 3,1% of HIV-1 subtype B strains, 31,2% of HIV-1 subtype D strains. The improved epitope ILAIVVVWTV was therefore expected to be able to induce a CTL immune response directed against 74,9% (33,3% + 41,6%) of HIV-1 subtype A strains, 35% (32%+3%) of subtype B strains, 54% of subtype B strains, 87,4% (56,2%+31,2%) of subtype D strains.

### *Example 6: Designing and building of a "cover-all" polytope vaccine*

A "cover-all" polytope vaccine will be composed of a string of pearls, each "pearl" being one peptide-epitope. In a first step, a synthetic protein sequence will be computer-designed. All peptide-epitope sequences will be placed one after each other without incorporating any amino-acid linkers in between each peptide-epitope. The peptide-epitope order within this synthetic protein will be defined randomly or by the use of a computer programme allowing the evaluation of different proteolysis-specific signalisation sites that are created by a combinatorial positioning of all peptide-epitopes.
In a second step, the designed polyepitopic protein sequence will be back-translated into a nucleotide sequence using software such as DNAstar (Lasergene) or any other commonly available programme. For each amino-acid residue, a highly expressed mammalian codon will be chosen in order to enhance the transcription and translation levels of the engineered synthetic gene.
In a last step, the designed nucleotide sequence will be modified in order to introduce silent mutations that generate unique restriction enzyme sites. Such unique restriction enzyme sites will allow the building of this synthetic gene as a system of several cassettes. Each cassette will contain several peptides encoding nucleic sequences (so called minigenes). Finally, specific restriction sites will be added to the 5'- and 3'-ends of the gene in order to clone the full-length synthesised gene of the "cover-all" polytope into a suitable mammalian expression vector and/or a viral vector.

**Building the synthetic polytope gene**

The synthetic "cover-all" polytope gene will be constructed using a long oligonucleotide complementary annealing technique (Fomsgaard et al, 1999). The nucleotide sequence of the gene will be subdivided in fragments of approximately 100 to 120 base pairs. For example, a synthetic gene with a length of 1417 base pairs (39 9mer-peptides and 14 8mer-peptides) could be divided into 12 to 14 fragments. Each of these double stranded DNA fragments will be synthesised as a pair of complementary oligonucleotides (sense and anti-sense strands). Each extremity of each of these paired oligonucleotides will be designed in such a manner that after their annealing, the double stranded DNA fragment will harbour complementary overhanging extremities with the 5'and 3' neighbouring DNA fragments. Each pair of complementary sense and anti-sense oligonucleotides will be annealed individually. A ligation procedure will be performed by using 3 to 4 distinctly re-annealed double stranded DNA fragments in order to allow the assembly of a 300 to 480bp gene cassette and its insertion into a suitable vector such as pMOSBLUE or pBlueScript. XL1-Blue bacteria or any suitable E.Coli strain will be transformed with the ligation product. Positive recombinant clones will be amplified. The recombinant plasmid DNAs will be purified and their sequence will be checked by sequencing. This procedure will be performed until all designed cassettes (300-480 bp fragments) are synthesised and cloned. All cassettes (between 5 or 3 cassettes containing between 11 to 18 peptide-epitopes) will then be ligated to each other in order to reconstitute the full-length gene and be cloned into a suitable mammalian expression vector and/or a viral vector.

***Example 7: Mouse experiments***

To analyse immunogenicity of a predicted epitope, HLA-A2 transgenic mice were immunised with the peptide representing the epitope. Immunisation and CTL methods for this have been described in the literature as seen in (Sette et al., 1994,) and the experiments were performed largely in accordance to this paper. Briefly, stock solutions were made of the query peptide (4 mg/ml in water) and a T-helper epitope peptide (amino acid sequence: TPPAYRPPNAPIL) (Milich et al., 1988) at 4.8 mg/ml in water and incomplete Freund Adjuvant (from Statens Serum Institut, DK). A mixture of IFA (0.2 ml) plus Th-peptide (0.2 ml) plus the test epitope peptide (0.2 ml) was made on ice. 100 μl of the IFA/peptide mixture were injected intracutaneously (i.c.) at the root of the tail of HLA-A2 transgenic mice (a gift from Dr Nicholas Holmes, Camebridge, UK). Usually 3 mice were injected per query peptide. CTL was measured at day 10.

**Cytotoxic T Lymphocyte (CTL) assay**

CTL assay methods have been described in the literature (Sette et al., 1994), and the experiments were performed largely in accordance to that paper. Briefly, the spleen of peptide immunised HLA-A2 transgenic mice were collected aseptically 10 days after immunisation and placed in 5 ml cell medium (RPMI 1640, peniccilin+steptomycin (P+S), 2% Hepes buffer, 10% Fetal calf serum) on ice. The splenocytes were cultured for 6 days in the presence of LPS blasts coated with 100 μg/ml of the peptide (stimulator cells) and then assayed for peptide-specific A*0201/Kb-restricted CTL activity by using EL4-A2 and EL4 cell lines in the presence or absence of the query peptide as the target cells. The splenocytes from immunised mice were cultured for 6 days mixed with stimulator cells at a responder/stimulator cell ratio of 2.5/1 are called "effector cells".

LPS blasts were prepared from 1 normal spleen (non-immunised HLA-A2 mouse). 3-5 x $10^6$ splenocytes per ml of LPS medium in a final volume of 40 ml. LPS medium was RPMI 1640, 1 % penicillin-streptomycin, 2% Hepes, 10% FCS, 7 μg/100 ml medium of dextrane sulphate (Pharmacia #17-0340-01) and 25 ug/100 ml medium of LPS (Sigma #L-2387). After 3 days of culturing the splenocytes in LPS medium, the LPS blasts were treated with mitomycin to inhibit cell division. LPS blasts were treated with mitomycin at the same time as they were loaded with the target peptide. 2 ml of blasts were mixed with 20 μg of peptide and 100 μg of mitomycin in PBS. Incubate 1 hour at 37°C in 5% $CO_2$ in an incubator. LPS blasts were then washed in media and adjusted to 2 x $10^7$ cells/ml of medium (RPMI 1640, P+S, Hepes, 50 μM mercaptoethanol).

In the present invention EL-4-A2 or EL4 cells or EL4-A2kb Cell line are interchangeable and described as follows: The plasmid pA2kb is a kind gift from Nikolas Holmes. $10^7$ EL4 cells (ATCC TIB-39) are electroporated with 20μg of pA2kb using the Gene Pulser system (BIO-RAD)(Potter et al., 1993). Electroporation was performed according to the manufactor's recommendations. Selection is undertaken after 48hr with 600μg/ml geneticin (G418 Gibco BRL). Cell clones are screened with the HLA-A2 specific monoclonal antibody (BB7.2 ATCC-HB82) by FACS analysis.

A standard $^{51}$Cr release CTL assay was done according to (Marker et al., 1973). Briefly, target cells were EL-4-A2 or EL4 cells that had been incubated for 1 hour at 37 °C in the presence of 200 µl of sodium $^{51}$Cr chromate, washed 3 times, and resuspended in RPMI 1640, 10% FCS at a concentration of $10^5$ cells/ml in the absence of presence of 10 µg/ml of the appropriate peptide. For the assay, 100 µl of target cells were incubated with 100µl of different concentrations of effector cells in U-bottom 96-well plates. Supernatants (100 µl) were removed after 6 hours at 37 °C and the percent lysis determined by the formula:

$$\text{% release} = 100 \times (\text{experimental release - spontaneous release}) / (\text{maximum release - spontaneous release})$$

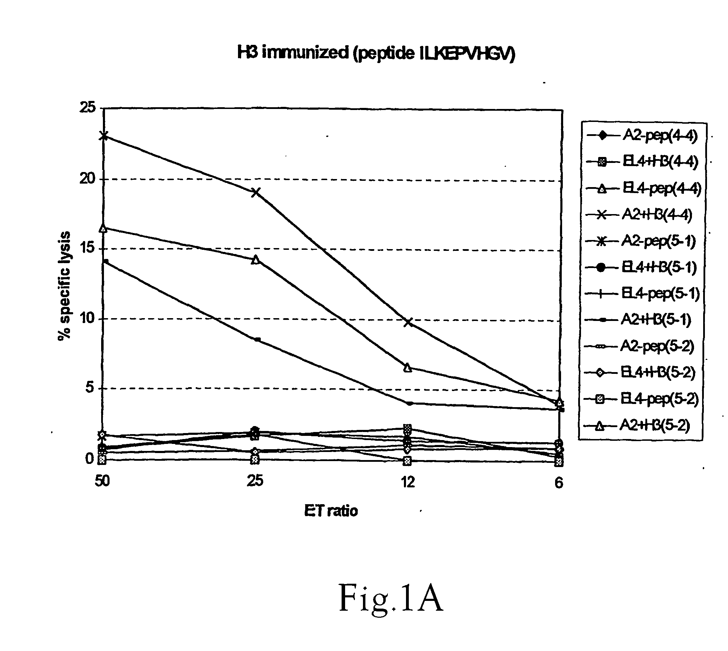

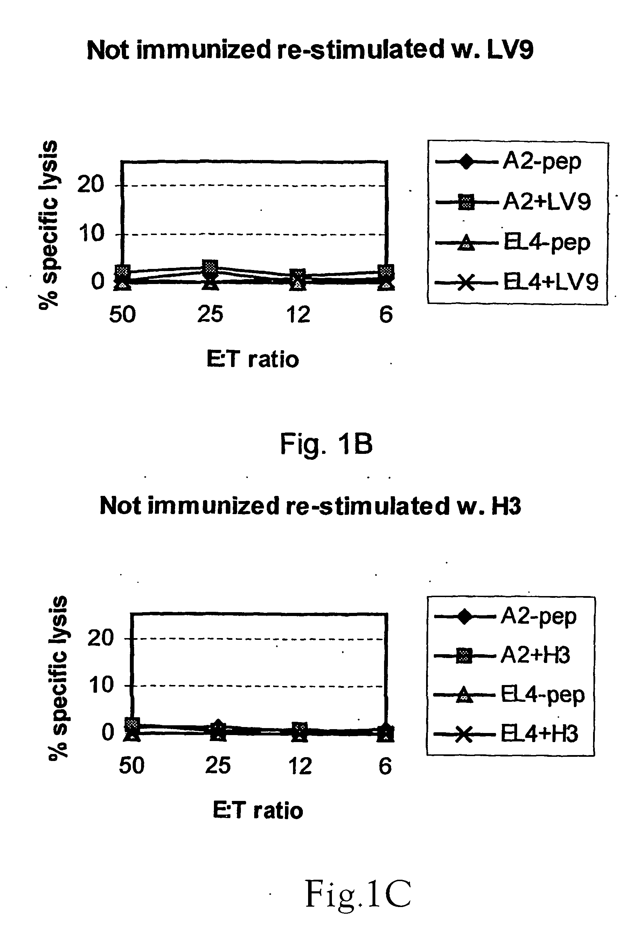

An example of the CTL assay data is shown in figure 1.

Of 3 HLA-A2 transgenic C57BU6 mice (4-4, 5-1, 5-2) all (A2+H3 (4-4), A2+H3 (5-2), A2+H3 (5-1)) responded (>10% specific lysis at E: T=50) to vaccination with a peptide (H3) representing a predicted CTL epitope (amino acid sequence ILKEPVHGV) that is already described in the literature as an HLA-A2 CTL epitope. The higher the effector cell to EL4-A2 Target cell ratio (E: T ratio) the higher the specific % lysis. No unspecific lysis of non-peptide loaded EL4-A2 target cells were seen (A2 -pep (4-4), A2 -pep (5-1), A2- pep (5-2)). No unspecific lysis of HLA-A2 negative EL4 target cells were seen whether H3 peptide loaded (EL4+H3 (4-4), EL4+H3 (5-1), EL4+H3 (5-2)) or not (EL4 -pep (4-4), EL4 - pep (5-1), EL4 -pep (5-2)).

Two control experiments were performed to secure that the process of *in vitro* stimulation of effector cells for 5 days could not in it self generate effector cells that would be able to lyse target cells.

"Not immunised re-stimulated w. H3": HLA-A2 transgenic mice which were not immunised with the H3 peptide, but whose effector splenocytes had been stimulated in vitro with H3 loaded stimulator blasts did not react against EL4-A2 target cells loaded with H3 peptide (A2+H3) or not (A2 -pep). This demonstrated that the process of in vitro stimulation for 5 days in itself could not result in effector cells reacting towards specific peptide loaded EL4-A2 target cells. This confirmed the specific induction of CTL by the peptide immunisation. The *in vitro* stimulated effector cells from these non-immunised HLA-A2 mice did not react against HLA-A2 negative EL4 target cells whether H3 loaded (EL4+H3) or not (EL4 -pep).

The control experiment "Not immunised re-stimulated w. LV9" showed that splenocytes from non immunised HLA-A2 transgenic mice could not be made reactive to another known HLA-A2 restricted epitope peptide (LV9, sequence LLGRNSFEV) from the tumour antigen p53 by 5 days of *in vitro* stimulation with LV9 peptide loaded blasts cells. Thus, the figure showed no reaction to EL4 target cells (Kd positive, HLA-A2 negative) or EL4-A2 target cells loaded with LV9 (EL4+LV9 and A2+LV9, respectively) or not peptide loaded (EL4 -pep and A2 -pep, respectively) (the results are shown in figure 1).

### *Example 8: Mouse experiment to validate cross-reaction of the improved epitope and the natural epitope*

The method to validate an improved epitope for its ability to raise a CTL immune response that cross-reacts with the natural epitope is similar to that described in example 7. A2 transgenic immunisation: Mice were immunised with the selected improved epitope using 100µl out of 600µ of a mixture containing IFA (30%), HBV T-helper epitope (960µg) and test epitope (800µg). CTL was measured at day 10 post-immunisation.

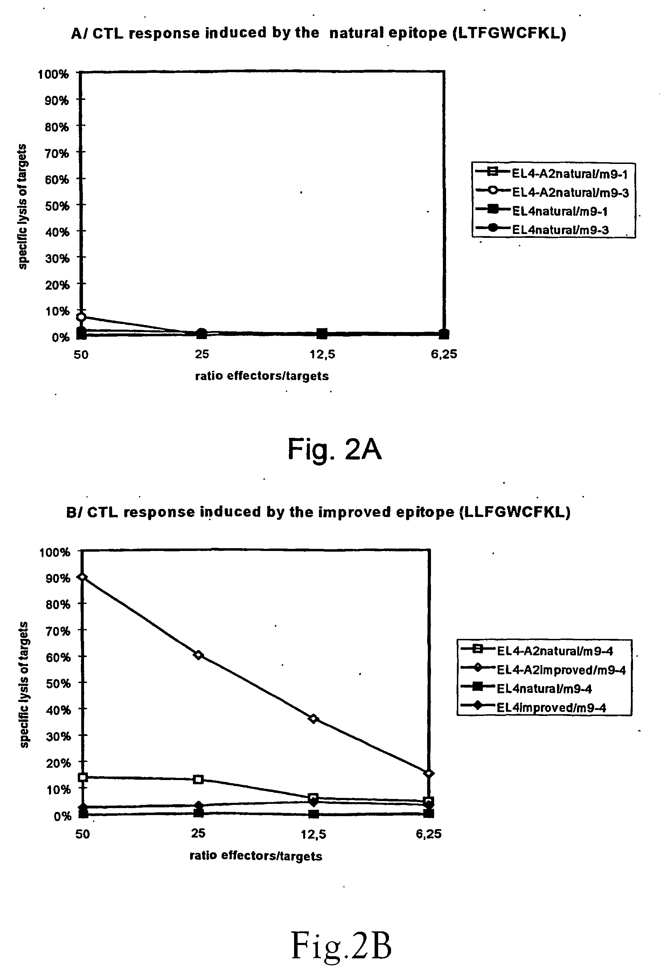

*In vitro* stimulation of CTL effectors: Splenocytes obtained from the immunised mice were divided in two parts. The first half of the splenocytes were stimulated in the presence of LPS blasts loaded with the related natural epitope peptide. Stimulation was allowed 5 to 6 days (the results are shown in figure 2).

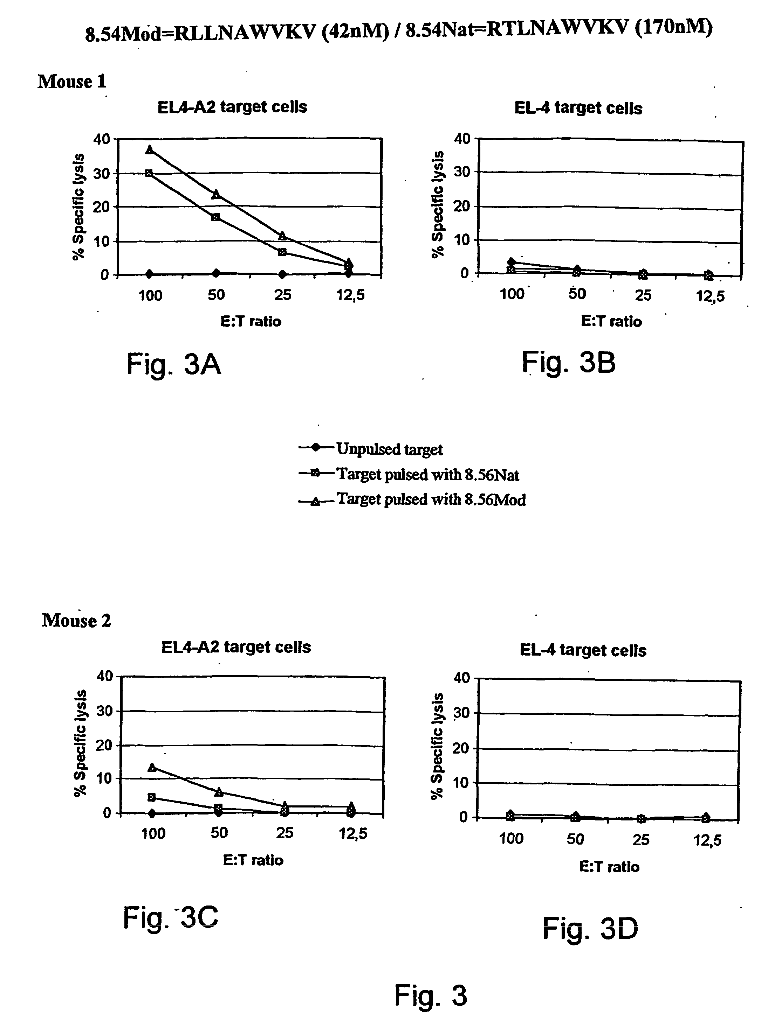

*Cytotoxic T-lymphocyte assays:* The stimulated CTL effectors were assayed for their ability to lyse peptide-loaded or unloaded EL4-A2$^+$ and EL4 (A2$^-$) cell lines. CTL effectors that were *in vitro* stimulated with improved epitope were tested for a specific lysis of target cells loaded with improved peptide. Reciprocally, CTL effectors *in vitro* stimulated with the natural epitope were tested for a specific lysis of natural peptide loaded target cells. The percent of specific lysis obtained with improved peptide loaded target cells was then compared with that obtained with natural peptide loaded target cells. A A2-restricted specific lysis of more than 10% of target cells loaded with the improved peptide was indicative of this improved peptide being immunogenic. A A2-restricted specific lysis of more than 10% of target cells loaded with the natural peptide was indicating that CTL effectors raised *in vivo* against the improved peptide were able to recognise the natural peptide. In a parallel experiment, the natural epitope-peptide was tested for its ability to induce a CTL response in A2 transgenic mice. Immunogenicity of the natural and improved peptides was then compared. An enhanced immunogenicity of the improved peptide towards the natural peptide was concluded if the specific lysis directed against the natural peptide was significantly higher when CTL effectors originating from mice immunised with improved peptide were used. See figure 3 and the legend thereto for specific results.

**Tables**

*Table 5A:*

CTL epitopes with high affinity binding and an intermediate global conservation, meaning with a global conservation of more than 1% and a cut off value for the weighted average of the MHC class I binding for the query peptide of less than 100 nM. This especially preferred embodiment is further described in example 4. Table 5A contains 213 new HLA-A2 epitopes. They are grouped by protein family and ranked according to their length (9 or 8 amino acid residues) and to their global conservation.

## Table 5A

| NAME | LENGTH | PEPTIDE | Kd nM | HXB2 MAPPING | GLOBAL CONSERVATION | % OF INTRA-SUBTYPES CONSERVATION | | | | | | | | | | |
|------|--------|---------|-------|--------------|---------------------|---|---|---|---|----|---|---|---|---|---|---|
| | | | | | | A | B | C | D | AE | F | G | H | J | N | O |
| 4.1 | 9 | LLAGVDYRI | 83,9 | Vpu(4-7) | 1,3% | | 15,3 | | | | | | | | | |
| 4.2 | 9 | LLAKVDYRL | 44,6 | Vpu(4-7) | 1,3% | | 15,3 | | | | | | | | | |
| 4.3 | 9 | ILAIVVWTI | 39,9 | Vpu(17-25) | 9,6% | 41,6 | 3,1 | | 31,2 | 33,3 | | | | | | |
| 4.4 | 9 | KLVEMGHHA | 71,5 | Vpu(66-74) | 2,6% | | 1,6 | | 18,7 | | | | | | | |
| 4.5 | 9 | ALMEMGHHA | 47,9 | Vpu(66-74) | 1,9% | | 4,7 | | | | | | | | | |
| 4.6 | 9 | ALVEMGHLA | 76,4 | Vpu(66-74) | 1,3% | | 3,1 | | | | | | | | | |
| 4.7 | 9 | ALGEMGPFI | 73,8 | Vpu(66-74) | 1,3% | | | | | | | 25 | | | | |
| 4.8 | 8 | IVGLIVAL | 67,8 | Vpu(9-16) | 3,2% | 16,6 | | | | | | | 66,6 | | | |
| 4.9 | 8 | IVGLVAV | 50,2 | Vpr(9-16) | 1,3% | 25agi | | | | | | | | | | |
| 4.10 | 9 | ALIRTLQQL | 38,6 | Vpr(59-67) | 3,5% | | 2,9 | 12,5 | | | | | | 2 | | |
| 4.11 | 9 | ALIRILQQL | 52,8 | Vpr(59-67) | 3,5% | | 4,8 | | 20 | | | | | | | |
| 4.12 | 9 | ALIRMLQQL | 26,9 | Vpr(59-67) | 2,3% | | 3,8 | | | | | | | | | |
| 4.13 | 8 | LFIHFRIGC | 96,4 | Vpr(59-67) | 4,0% | | | 62,5 | | | | | | | 50 | |
| 4.14 | 9 | SLVKHHMYV | 26,6 | Vif(23-31) | 26,0% | 60 | 16 | 75 | 60,8 | | | | 33,3 | 33,3 | 100 | |
| 4.15 | 9 | SLVKHHMHV | 51,2 | Vif(23-31) | 1,5% | 5 | 1,8 | | | | | | | | | |
| 4.16 | 9 | SLVKHHIYV | 67,7 | Vif(23-31) | 1,5% | | 2,4 | | | | | | | | | |
| 4.17 | 9 | LVIRTYWGL | 88 | Vif(64-72) | 1,5% | | 0,6 | | 66,6 | | | | | | | |
| 4.18 | 9 | RLRRYSTQV | 76,7 | Vif(90-98) | 1,5% | | 12,5 | | | | | | | | | |
| 4.19 | 9 | RLKRYSTQV | 60,1 | Vif(90-98) | 1,1% | 10 | | | | | | | 33,3 | | | |
| 4.20 | 9 | GLADQLIHL | 90,6 | Vif(101-109) | 14,3% | | 23 | | 4,3 | | | | | | | |
| 4.21 | 9 | NLADQLIHL | 78,3 | Vif(101-109) | 10,2% | | 16 | | | | | | | | | |
| 4.22 | 8 | FLGQYIHV | 45,4 | Vif(55-65) | 26,0% | 50 | 32,7 | | 13 | | | | 33,3 | | | |

EP 1 250 351 B1

| NAME | LENGTH | PEPTIDE | Kd nM | HXB2 MAPPING | GLOBAL CONSERVATION | % OF INTRA-SUBTYPES CONSERVATION | | | | | | | | | | |
|---|---|---|---|---|---|---|---|---|---|---|---|---|---|---|---|---|
| | | | | | | A | B | C | D | AE | F | G | H | J | N | O |
| 4.23 | 8 | RLGDAKLV | 63,9 | Vif(58-65) | 19,2% | 5 | 28,4 | | | | | 66,6 | | | | |
| 4.24 | 8 | RLGEARLV | 92,6 | Vif(58-65) | 17,0% | 15 | 6 | 62,5 | 78,2 | 66,6 | | | 33,3 | 50 | | |
| 4.25 | 8 | RLGDATLV | 35,9 | Vif(58-65) | 4,9% | 5 | 1,8 | | | | | 33,3 | | | | |
| 4.26 | 8 | KIGSLQYL | 56,2 | Vif(141-148) | 1,6% | | 1,8 | 12,5 | | | | | | | | |
| 4.27 | 8 | TLRKGRL | 42,2 | Tat(68-74) | 20% | 10 | | | | | | | 33,3 | | | |
| 4.28 | 9 | KLLYQSNPL | 46,9 | Rev(20-28) | 3,8% | | 11,7 | | | | | | | | | |
| 4.29 | 9 | CLGRPAEPV | 35,1 | Rev(63-71) | 15,2% | 36,3 | | 41,6 | | | 25 | | 100 | | | |
| 4.30 | 9 | YLGRPAEPV | 22,4 | Rev(63-71) | 10,5% | | 20,6 | | 12,5 | | | 33,3 | | | | |
| 4.31 | 9 | FLGRPAEPV | 12,7 | Rev(63-71) | 3,8% | | 5,8 | 16,7 | | | | | | | | |
| 4.32 | 9 | CLGRPTEPV | 37,7 | Rev(63-71) | 3,8% | 9 | 8,3 | | | | | | | | | |
| 4.33 | 9 | CLGRPEEPV | 59,8 | Rev(63-71) | 3,8% | | | | | | 50 | 33,3 | | | | |
| 4.34 | 9 | FLGRPEEPV | 18,7 | Rev(63-71) | 2,9% | | 5,8 | | 12,5 | | | | | | | |
| 4.35 | 9 | YLGRPEEPV | 36 | Rev(63-71) | 2,9% | | | | 12,5 | | | | | | | |
| 4.36 | 9 | CLGRPPEPV | 18,3 | Rev(63-71) | 1,9% | | | | | | | | 66,6 | | | |
| 4.37 | 9 | YLGRPTEPV | 23,8 | Rev(63-71) | 1,9% | | 5,8 | | | | | | | | | |
| 4.38 | 9 | FLGRSAEPV | 46,2 | Rev(63-71) | 1,9% | | 2,9 | | | | | | | | | |
| 4.39 | 9 | HLGRPAEPV | 96,6 | Rev(63-71) | 1,9% | | 5,8 | | | | | | | | | |
| 4.40 | 9 | GMGSPQILV | 55,9 | Rev(96-104) | 2,9% | | 8,8 | | | | | | | | | |
| 4.41 | 9 | ILVESPTVL | 74,4 | Rev(102-110) | 9,5% | | 26,5 | | | | | | | | | |
| 4.42 | 9 | VLVEPPVVL | 47,5 | Rev(102-110) | 1,9% | | | | | | | 33,3 | | | | |
| 4.43 | 9 | ILVESPTIL | 84,7 | Rev(102-110) | 1,9% | | 5,8 | | | | | | | | | |
| 4.44 | 8 | GMGSPQIL | 68,3 | Rev(96-103) | 2,9% | | 8,8 | | | | | | | | | |
| 4.45 | 8 | ISCERCMV | 86,8 | Rev(102-109) | 1,9% | | | | | | | | | 100 | | |
| 4.46 | 8 | ISCKECAV | 95,1 | Rev(102-109) | 1,9% | 18,2 | | | | | | | | | | |
| 4.47 | 9 | LLGRWKPKM | 65,1 | p15(38-46) | 1,2% | | | | | | | | 33,3 | | | |
| 4.48 | 9 | YMEAEVIPA | 98 | p31(87-95) | 4,7% | | 37,5 | | | | | | | | | |
| 4.49 | 9 | YLEAEVIPA | 63,4 | p31(87-95) | 3,5% | | | | | | | 50,0 | | | | 100 |

EP 1 250 351 B1

| NAME | LENGTH | PEPTIDE | Kd nM | HXB2 MAPPING | GLOBAL CONSERVATION | % OF INTRA-SUBTYPES CONSERVATION | | | | | | | | | | |
|---|---|---|---|---|---|---|---|---|---|---|---|---|---|---|---|---|
| | | | | | | A | B | C | D | AE | F | G | H | J | N | O |
| 4.50 | 9 | LAGRWPVKV | 91,9 | p31(104-112) | 40,7% | 75 | 3,5 | 75 | 80 | 66,6 | | 33,3 | | 50 | 100 | |
| 4.51 | 9 | LAARWPVKV | 60,9 | p31(104-112) | 2,3% | | | | | | | | | | | 100 |
| 4.52 | 9 | LTLAGRWPV | 70,2 | p31(106-114) | 1,2% | | 3,5 | | | | | | | | | |
| 4.53 | 9 | AMKAACWWA | 77,7 | p31(125-133) | 1,2% | 20ag | | | | | | | | | | |
| 4.54 | 9 | ALQKQITKI | 55,2 | p31(216-224) | 1,2% | | | | | 33,3 | | | | | | |
| 4.55 | 9 | ELQKQITKV | 60 | p31(216-224) | 1,2% | | | 12,5 | | | | | | | | |
| 4.56 | 9 | NLQTQILKV | 85,7 | p31(216-224) | 1,2% | | | | | | | | | | 100 | |
| 4.57 | 9 | ILKIQNFRV | 74,6 | p31(217-225) | 1,2% | | | 12,5 | | | | | | | | |
| 4.58 | 9 | DLGDAYFSV | 83,4 | p51(110-118) | 1,2% | 12,5 | | | | | | | | | | |
| 4.59 | 9 | KLHPEQARA | 62,8 | p51(233-240) | 1,2% | | | | | | | 50 | | | | |
| 4.60 | 9 | ILASQIQTT | 71,4 | p51(265-273) | 2,3% | | | | | | | | | | | 100 |
| 4.61 | 9 | LTAEAEMEL | 71,4 | p51(295-303) | 2,3% | | | | | | | | 33,3 | | | |
| 4.62 | 9 | MTAEAEMEL | 84,8 | p51(295-303) | 1,2% | | | | | | | | 33,3 | | | |
| 4.63 | 9 | ILKEPVHGA | 40,9 | p51(309-317) | 7,0% | | | | 20 | | 50 | | 33,3 | | | |
| 4.64 | 9 | ILKDPVHGV | 15 | p51(309-317) | 5,8% | 50 | | | | | | | | | | |
| 4.65 | 9 | ILREPVHGV | 17,7 | p51(309-317) | 3,5% | | 3,5 | | | | | | 33,3 | 33,3 | | |
| 4.66 | 9 | ILKTPVHGV | 35,1 | p51(309-317) | 2,3% | | | | | 66,6 | | | | | | |
| 4.67 | 9 | ILKDPVHGA | 40,2 | p51(309-317) | 2,3% | 25 | | | | | | | | | | |
| 4.68 | 9 | KLKEPVHGV | 11,1 | p51(309-317) | 1,2% | | | | | | | | | | | 50 |
| 4.69 | 9 | ILKAPVHGV | 12,3 | p51(309-317) | 1,2% | 12,5 | | | | | | | | | | |
| 4.70 | 9 | ILKEPIHGV | 16,6 | p51(309-317) | 1,2% | | | 12,5 | | | | | | | | |
| 4.71 | 9 | ILRDPVHGV | 17,4 | p51(309-317) | 1,2% | 20ac | | | | | | | | | | |
| 4.72 | 9 | ILKDPVHWV | 18,8 | p51(309-317) | 1,2% | 20ac | | | | | | | | | | |
| 4.73 | 9 | ILREPIHGV | 19,6 | p51(309-317) | 1,2% | | | | | | | | 33,3 | | | |
| 4.74 | 9 | ILKEPVHEV | 20,2 | p51(309-317) | 1,2% | | 3,5 | | | | | | | | | |
| 4.75 | 9 | ILKEPLHGV | 20,6 | p51(309-317) | 1,2% | | | | | | | | | | 100 | |
| 4.76 | 9 | ILKEPMHGV | 27,6 | p51(309-317) | 1,2% | | | | 20 | | | | | | | |
| 4.77 | 9 | RLKQPVHGV | 44,8 | p51(309-317) | 1,2% | | | | | | | | | | | 50 |

| NAME | LENGTH | PEPTIDE | Kd nM | HXB2 MAPPING | GLOBAL CONSERVATION | % OF INTRA-SUBTYPES CONSERVATION | | | | | | | | | | |
|---|---|---|---|---|---|---|---|---|---|---|---|---|---|---|---|---|
| | | | | | | A | B | C | D | AE | F | G | H | J | N | O |
| 4.78 | 9 | ILRIPVHGV | 49,4 | p51(309-317) | 1,2% | | | | | 33,3 | | | | | | |
| 4.79 | 9 | ILKESVHGV | 58,4 | p51(309-317) | 1,2% | | 3,5 | | | | | | | | | |
| 4.80 | 9 | ILRKPVHEV | 67,8 | p51(309-317) | 1,2% | | 3,5 | | | | | | | | | |
| 4.81 | 9 | ILKVPVHGV | 68,1 | p51(309-317) | 1,2% | | 3,5 | | | | | | | | | |
| 4.82 | 9 | QLAEVVQKV | 20,6 | p51(367-375) | 10,5% | 62,5 | | | | 33,3 | | | | | | 50 |
| 4.83 | 9 | QLTEVVQKV | 55,5 | p51(367-375) | 5,8% | 12,5 | 3,5 | | | 33,3 | | | | | | |
| 4.84 | 9 | QLTEAVQKV | 46,5 | p51(367-375) | 3,5% | | 10,7 | | | | | | | | | |
| 4.85 | 9 | QLAEVVQKI | 84,4 | p51(367-375) | 3,5% | | | | 20 | | | | | 50 | | |
| 4.86 | 9 | QLAEAVQKI | 70,5 | p51(367-375) | 2,3% | | | 12,5 | | | | | | | | |
| 4.87 | 9 | QLAEMVQKV | 14,9 | p51(367-375) | 1,2% | 12,5 | | | | | | | | | | |
| 4.88 | 9 | QLAEVIQKV | 22,7 | p51(367-375) | 1,2% | | | | | | | | | | | 50 |
| 4.89 | 9 | QLTAVVQKV | 40 | p51(367-375) | 1,2% | 20ac | | | | | | | | | | |
| 4.90 | 9 | QLVEVVQKV | 52,3 | p51(367-375) | 1,2% | 12,5 | | | | | | | | | | |
| 4.91 | 9 | YLLEEDPIV | 45,1 | p51(427-435) | 1,2% | 12,5 | | | | | | | | | | |
| 4.92 | 9 | NLAFPQWKA | 31,7 | Pol(5-13) | 1,2% | | | | 20 | | | | | | | |
| 4.93 | 9 | KLSSEQTRA | 66,6 | pol(15-23) | 2,3% | | 3,5 | | | | | | | | | |
| 4.94 | 9 | SLSFPQITL | 99,5 | Pol(53-61) | 9,3% | | 7,1 | | 20 | | 100 | 33,3 | 33,3 | | | |
| 4.95 | 9 | SLSLPQITL | 78,9 | Pol(53-61) | 4,7% | | 10,7 | | | | | | | | | |
| 4.96 | 9 | SLNFPQITL | 81 | Pol(53-61) | 3,5% | 20ag | 3,5 | 12,5 | | | | | | | | |
| 4.97 | 9 | ALNFPQITL | 96 | Pol(53-61) | 2,3% | 20ac | | 12,5 | | | | | | | | |
| 4.98 | 9 | SLSFPQTTL | 48 | Pol(53-61) | 1,2% | | 3,5 | | | | | | | | | |
| 4.99 | 9 | SLCFPQITL | 63,6 | Pol(53-61) | 1,2% | | | | | | | | 33,3 | | | |
| 4.100 | 9 | TLNCPQITL | 98,2 | pol(53-61) | 1,2% | | | 12,5 | | | | | | | | |
| 4.101 | 9 | IIGAETFYV | 51,8 | Pol(589-597) | 15,1% | | 25 | | 80 | | | | | | | |
| 4.102 | 9 | IMGAETFYV | 12,9 | Pol(589-597) | 3,5% | | | | | 33,3 | | | | 100 | | |
| 4.103 | 9 | ILGAETFYV | 9,9 | Pol(589-597) | 1,2% | 20ac | | | | | | | | | | |
| 4.104 | 9 | IMGAETYYV | 20,7 | Pol(589-597) | 1,2% | | | | | | | | | | | 50 |
| 4.105 | 9 | ITGAETFYV | 29,5 | Pol(589-597) | 1,2% | | 3,5 | | | | | | | | | |

|  |  |  |  | GLOBAL | % OF INTRA-SUBTYPES CONSERVATION | | | | | | | | | | |
| NAME | LENGTH | PEPTIDE | Kd nM | HXB2 MAPPING | CONSERVATION | A | B | C | D | AE | F | G | H | J | N | O |
|---|---|---|---|---|---|---|---|---|---|---|---|---|---|---|---|---|
| 4.106 | 9 | IVGADSFFV | 92,8 | Pol(589-597) | 1,2% | 12,5 | | | | | | | | | | |
| 4.107 | 9 | ITLWQPPLV | 71,3 | Pol(59-67) | 1,2% | 25agi | | | | | | | | | | |
| 4.108 | 9 | ELQAILMAL | 80,8 | Pol(633-641) | 2,3% | | | | | | | | | | 100 | |
| 4.109 | 9 | YLALQDSGV | 44,9 | Pol(638-646) | 1,2% | | 3,5 | | | | | | | | | |
| 4.110 | 9 | ALQDSGPEV | 86,5 | pol(640-648) | 3,5% | | | | | 33,3 | | | | | | |
| 4.111 | 9 | ALQDSQSEV | 64,8 | pol(640-648) | 1,2% | | | | | | | 33,3 | | | | |
| 4.112 | 9 | ALQESGPEV | 92,4 | pol(640-648) | 1,2% | | | | | | | | 33,3 | | | |
| 4.113 | 8 | KIGGQLKV | 95,4 | p15(64-71) | 1,2% | | 3,5 | | | | | | | | | |
| 4.114 | 8 | VLIGPTPV | 218 | p15(75-82) | 2,3% | | | | | | | | | 100 | | |
| 4.115 | 8 | ILVGPTPV | 64,5 | p15(75-82) | 1,2% | 100adi | | | | | | | | | | |
| 4.116 | 8 | ALTDIVPL | 78,9 | p51(288-295) | 26,7% | rec | | 50 | | 66,6 | 50 | 33,3 | | 100 | | |
| 4.117 | 8 | VLTDIVPL | 74,5 | p51(288-295) | 1,2% | | 12,5 | | | | | | | | | |
| 4.118 | 8 | TLTDIVPL | 93,1 | p51(288-295) | 1,2% | | 12,5 | | | | | | | | | |
| 4.119 | 8 | FVNTPPLV | 97,6 | p51(416-423) | 86,0% | 100 | 89,3 | 100 | 80 | 100 | 100 | 100 | | 100 | 100 | |
| 4.120 | 8 | FVNTPHLV | 68,8 | p51(416-423) | 7,5% | | | | | | | | 100 | | | |
| 4.121 | 8 | FVNTPLV | 68,6 | p51(416-423) | 1,2% | 25agi | | | | | | | | | | |
| 4.122 | 8 | FGGKARL | 69,4 | pol(9-16) | 1,2% | | 3,5 | | | | | | | | | |
| 4.123 | 8 | KLVGKAGYV | 91,7 | pol(606-613) | 57,7% | 87,5 | 78,5 | 12,5 | 80 | 66,6 | | 100 | | 33,3 | | 100 |
| 4.124 | 9 | LTFGWCFKL | 29,6 | Nef(137-145) | 65,7% | 87,5 | 73 | 100 | | 50 | | 100 | 100 | 50 | | |
| 4.125 | 9 | LTLGWCFKL | 50,5 | Nef(137-145) | 4,8% | | 6,6 | | | | | | | | | |
| 4.126 | 9 | LTPGWCFKL | 94,9 | Nef(137-145) | 1,2% | | 1,8 | | | | | | | | | |
| 4.127 | 9 | RLAYHHMAR | 74,8 | Nef(188-196) | 1,2% | | 1,8 | | | | | | | | | |
| 4.128 | 9 | LTLGWCFKL | 35,6 | Nef(137-145) | 4,8% | | 6,6 | | | | | | | | | |
| 4.129 | 9 | YMMKHLVWA | 80,7 | Pr55(29-37) | 4,2% | 16ac | | 12,5 | | | | | | | | |
| 4.130 | 9 | SLYNTVAVL | 71,3 | Pr55(77-85) | 7,3% | 12,5 | 9,4 | | | | | 33,3 | | | | |
| 4.131 | 9 | SLYNTIATL | 71,8 | Pr55(77-85) | 4,2% | 40ag | 3,1 | | | 33,3 | | | | | | |
| 4.132 | 9 | SLFNTVAVL | 51,1 | Pr55(77-85) | 3,1% | 12,5 | 6,2 | | | | | | | | | |
| 4.133 | 9 | SLFNTIATL | 52,4 | Pr55(77-85) | 2,1% | 20ag | | | | 33,3 | | | | | | |

EP 1 250 351 B1

GLOBAL ... % OF INTRA-SUBTYPES CONSERVATION

| NAME | LENGTH | PEPTIDE | Kd nM | HXB2 MAPPING | GLOBAL CONSERVATION | A | B | C | D | AE | F | G | H | J | N | O |
|---|---|---|---|---|---|---|---|---|---|---|---|---|---|---|---|---|
| 4.134 | 9 | SLYNAVATL | 56,6 | Pr55(77-85) | 2,1% | | 3,2 | | | | | 33,3 | | | | |
| 4.135 | 9 | NTIATLWCV | 65,8 | Pr55(80-88) | 5,2% | 60ag | | | | 66,6 | | | | | | |
| 4.136 | 9 | DLNAMLNTV | 91,1 | Pr55(183-191) | 3,1% | | | | | | | | 100 | | | |
| 4.137 | 9 | TLQEQITWM | 84,3 | Pr55(242-250) | 3,1% | 20ag | | 12,5 | | | 16,6 | | | | | |
| 4.138 | 9 | SLQEQIAWM | 72,5 | Pr55(242-250) | 2,1% | | | 25 | | | | | | | | |
| 4.139 | 9 | MTNNPPIPV | 71,3 | Pr55(250-258) | 29,2% | 16,6ac | 78,1 | | | 66,6 | | | | | | |
| 4.140 | 9 | MTSNPPIPV | 87,8 | Pr55(250-258) | 29,2% | 37,5 | 9,4 | 100 | 33,3 | 16,6 | 33,3 | | | | | |
| 4.141 | 9 | MTGNPPIPV | 42,8 | Pr55(250-258) | 10,4% | 62,5 | | | | | | 33,3 | 33,3 | 100 | | |
| 4.142 | 9 | MTSNPPVPV | 78,1 | Pr55(250-258) | 8,3% | 25agi | | 25 | | | 50 | 33,3 | | | | |
| 4.143 | 9 | MTGNPPVPV | 38,4 | Pr55(250-258) | 6,2% | | | 50 | | | 16,6 | | | | | |
| 4.144 | 9 | MTNNPPVPV | 64,2 | Pr55(250-258) | 3,1% | | | 25 | | | 16,6 | | | | | |
| 4.145 | 9 | MTHNPPIPV | 95,4 | Pr55(250-258) | 3,1% | 16,6ac | 6,2 | | | | | | | | | |
| 4.146 | 9 | MTGNPAIPV | 93,4 | Pr55(250-258) | 2,1% | | 3,1 | | | | | | 33,3 | | | |
| 4.147 | 9 | KMVKMYSPV | 69,8 | Pr55(272-280) | 2,1% | | | | | | | | | | | 100 |
| 4.148 | 9 | KMYSPVSIL | 72,8 | Pr55(275-283) | 2,1% | | | | | | | | | | | 100 |
| 4.149 | 9 | VLAEAMSQV | 40,3 | Pr55(362-370) | 49,0% | 87,5 | 71,8 | 12,5 | 20 | | | | 100 | 100 | 100 | |
| 4.150 | 9 | ILAEAMSQV | 26,7 | Pr55(362-370) | 4,2% | | 9,4 | | | | 16,6 | | | | | |
| 4.151 | 8 | ILGQLQPS | 94,9 | Pr55(60-67) | 5,6% | | 46,8 | | | | | | | | | |
| 4.152 | 8 | ILGQLQPA | 95,2 | Pr55(60-67) | 7,3% | 12,5 | 15,6 | | | | | | 33,3 | | | |
| 4.153 | 8 | ILGQLQPA | 90,8 | Pr55(60-67) | 4,2% | 12,5 | | | 40 | | | | | | | |
| 4.154 | 8 | TSNPPVPV | 74,5 | Pr55(251-258) | 8,3% | 25agi | | 25 | | | 50 | 33,3 | | | | |
| 4.155 | 8 | TNNPPVPV | 79,9 | Pr55(251-258) | 3,1% | | | 25 | | | 16,6 | | | | | |
| 4.156 | 8 | THNPPIPV | 75,8 | Pr55(251-258) | 3,1% | 16,6ac | 6,2 | | | | | | | | | |
| 4.157 | 8 | YALGPAAT | 70 | Pr55(336-343) | 27,1% | | 75 | | 20 | | | | | | | |
| 4.158 | 8 | ELGKIWPS | 38,6 | Pr55(433-440) | 84,4% | 75 | 93,7 | 87,5 | 80 | 100 | 100 | 100 | 100 | 100 | | |
| 4.159 | 8 | ELGRIWPS | 75 | Pr55(433-440) | 2,1% | 12,5 | | | 20 | | | | | | | |
| 4.160 | 9 | YQQWWIWGV | 26 | gp160(7-15) | 1,9% | | 18,2 | | | | | | | | | |
| 4.161 | 9 | MLQWGTMLL | 34,4 | gp160(14-22) | 2,3% | | 5 | | | | | | | | | |

EP 1 250 351 B1

| NAME | LENGTH | PEPTIDE | Kd nM | HXB2 MAPPING | GLOBAL CONSERVATION | A | B | C | D | AE | F | G | H | J | N | O |
|---|---|---|---|---|---|---|---|---|---|---|---|---|---|---|---|---|
| 4.162 | 9 | ALFYRLDVV | 64,1 | gp160(174-182) | 8,5% | 6,2 | 3 | 9,1 | 21,4 | | | 25 | 66,6 | | | |
| 4.163 | 9 | ALFYRLDIV | 71,1 | gp160(174-182) | 7,5% | 25agi | 2. | 36,4 | 14,3 | 11,1 | | | | | | |
| 4.164 | 9 | SLFYRLDIV | 62 | gp160(174-182) | 2,3% | 18,7 | | | | | | | | | | |
| 4.165 | 9 | SLFYRLDVV | 54,4 | gp160(174-182) | 1,9% | 12,5 | | | | | | | | | | |
| 4.166 | 9 | ALFYNLDVV | 68,8 | gp160(174-182) | 1,4% | | 3 | | | | | | | | | |
| 4.167 | 9 | FCAPAGFAI | 88,1 | gp160(217-225) | 2,8% | | 5 | | 7,1 | | | | | | | |
| 4.168 | 9 | SLAEEEVVL | 90,7 | gp160(264-272) | 1,9% | | 4 | | | | | | | | | |
| 4.169 | 9 | KLAEHFPNK | 72,9 | gp160(348-356) | 3,8% | | | 36,4 | | | | | | | | |
| 4.170 | 9 | MYAPPIQGV | 34,3 | gp160(434-441) | 2,8% | 25 | | | | | | | | | | |
| 4.171 | 9 | NLASGIQKV | 24,1 | gp160(434-441) | 1,4% | | | | | | | | | | | 37,5 |
| 4.172 | 9 | IYAPPIQGV | 44,1 | gp160(434-441) | 1,4% | 18,7 | | | | | | | | | | |
| 4.173 | 9 | SLGVAPTRA | 98,3 | gp160(493-501) | 1,4% | | | | | | | 25 | | | | |
| 4.174 | 9 | FLSAAGSTM | 75,5 | gp160(522-530) | 1,4% | | | | | | | 75 | | | | |
| 4.175 | 9 | TMGAASMTL | 41,4 | gp160(529-537) | 15,0% | 6,2 | 27 | | 14,3 | | | | | | | |
| 4.176 | 9 | TMGAAATAL | 57,8 | gp160(529-537) | 2,3% | | | | | | | | | | | 62,5 |
| 4.177 | 9 | TMGAAAVTL | 94 | gp160(529-537) | 2,3% | | 4 | | | | | | | | | |
| 4.178 | 9 | TMGARSMTL | 50,1 | gp160(529-537) | 1,9% | | 4 | | | | | | | | | |
| 4.179 | 9 | AIQAQQQLL | 71,9 | gp160(558-566) | 2,8% | | | | | | | | | | | 75 |
| 4.180 | 9 | LLQLTVWGI | 38 | gp160(565-573) | 58,2% | | 80 | 27,3 | 92,8 | 88,9 | 75 | 100 | | | | |
| 4.181 | 9 | MLQLTVWGI | 43,7 | gp160(565-573) | 16,0% | | 12 | 72,7 | 7,1 | 11,1 | 25 | | 66,6 | | | |
| 4.182 | 9 | SLQGFLPLL | 70,6 | gp160(708-716) | 1,4% | | | | | | | | | | | 37,5 |
| 4.183 | 9 | LIAARIVEL | 86,4 | gp160(776-784) | 6,6% | | 7 | | 35,7 | | | | | | | |
| 4.184 | 9 | LLGRRGWEA | 37,1 | gp160(784-792) | 24,9% | | 37 | | 64,3 | | | | 33,3 | | | |
| 4.185 | 9 | LLGRRGWEV | 13,5 | gp160(784-792) | 9,4% | | 19 | | 7,4 | | | | | | | |
| 4.186 | 9 | LLGRRGWEI | 47,6 | gp160(784-792) | 5,6% | | 12 | | | | | | | | | |
| 4.187 | 9 | ILGRRGWEA | 54,3 | gp160(784-792) | 2,8% | | 6 | | | | | | | | | |
| 4.188 | 9 | LLGRRGWEL | 22,9 | gp160(784-792) | 1,9% | | 4 | | | | | | | | | |
| 4.189 | 9 | LLLYWGQEL | 47,1 | gp160(799-807) | 7,5% | 12,5 | | | 66,6 | | | | | | 25 | 33,3 |

| NAME | LENGTH | PEPTIDE | Kd nM | HXB2 MAPPING | GLOBAL CONSERVATION | % OF INTRA-SUBTYPES CONSERVATION | | | | | | | | | | |
|---|---|---|---|---|---|---|---|---|---|---|---|---|---|---|---|---|
| | | | | | | A | B | C | D | AE | F | G | H | J | N | O |
| 4.190 | 9 | LLQYWIQEL | 18,1 | gp160(799-807) | 7,0% | | 7 | | 42,8 | | | | | | | |
| 4.191 | 9 | LLQYWGQEL | 30,1 | gp160(799-807) | 5,2% | 100adi | 7 | | | | | | 66,6 | | | |
| 4.192 | 9 | LLQYWGQEI | 66,5 | gp160(799-807) | 1,4% | | 3 | | | | | | | | | |
| 4.193 | 9 | SLLDTIAIA | 88,1 | gp160(813-821) | 3,8% | 6,2 | | 9,1 | 21,4 | | | | | | | |
| 4.194 | 9 | SLFDTIAIA | 49,4 | gp160(813-821) | 1,9% | | 1 | 9,1 | | | | | | | | |
| 4.195 | 9 | LLDATAIAV | 69,1 | gp160(814-822) | 4,7% | | 4 | 4,5 | | 33,3 | | | | | | |
| 4.196 | 9 | LLNTTAIAV | 94,1 | gp160(814-822) | 4,7% | adi,ag | 4 | | | | | | 67 | 100 | | |
| 4.197 | 9 | LLNTTAIVV | 90 | gp160(814-822) | 2,8% | | 1 | 4,5 | | | | 25 | 33,3 | | 100 | |
| 4.198 | 9 | LLNAIAIAV | 34,8 | gp160(814-822) | 1,4% | | 3 | | | | | | | | | |
| 4.199 | 9 | RIIEVVQRV | 69,9 | gp160(828-835) | 1,4% | | 3 | | | | | | | | | |
| 4.200 | 8 | HIGRGQAL | | gp160(310-317) | 1,4% | | 2 | | | | | 25 | | | | |
| 4.201 | 8 | IGDIRKA | | gp160(322-329) | 7,0% | | 5 | 4,5 | 7,1 | 11,1 | 100 | | | 50 | | |
| 4.202 | 8 | LGGDREV | | gp160(365-372) | | | 4 | | | | | | | | | |
| 4.203 | 8 | IINYWQEV | 95 | gp160(423-430) | 21,7% | | 39 | 50 | 35,7 | | | | | | | |
| 4.204 | 8 | IINMWQKV | 166,8 | gp160(423-430) | 3,8% | | | 4,5 | | | | | | | | |
| 4.205 | 8 | FLSELSAA | | gp160(519-526) | | | | | | | 75 | | | | | |
| 4.206 | 8 | YLGQQL | 16,7 | gp160(586-593) | 1,9% | | 3 | | 7,1 | | | | | | | |
| 4.207 | 8 | MSDLNK | 85,4 | gp160(586-593) | 1,9% | | | | | | | | | | | 37,5 |
| 4.208 | 8 | YTGLIYTL | | gp160(638-645) | 5,6% | | 11 | | 7,1 | | | | | | | |
| 4.209 | 8 | YTGLIYN | | gp160(638-645) | | | 7 | | 7,1 | | | | | | | |
| 4.210 | 8 | YTGLIYSL | | gp160(638-645) | | | | | 42,8 | | | | | | | |
| 4.211 | 8 | YTGIYSL | | gp160(638-645) | | 20ad | | | | | | | | 100 | | |
| 4.212 | 8 | YTGLIYNL | 80 | gp160(638-645) | 1,4% | 100adi | 2 | | | | | | | | | |
| 4.213 | 8 | GLKMFAV | | gp160(697-704) | 1,4% | | 2 | | | | | 25 | | | | |

*Table 5B:*

9-mers representing one best new improved binder for each natural epitope with a binding affinity $IC_{50}$ between 50nM and 500nM and with a global conservation above 8%. 100 HLA-A2 9-mer-epitopes were found. These 100 HLA-A2 epitopes were regarded as natural intermediate binders whose binding affinity could be increased to obtain an improved binding affinity of $IC_{50}$ below 100nM by modifying one or two of the primary anchor positions. 100 optimally (best) improved HLA-A2 restricted HIV epitopes were identified.

## Table 5B

| NAME | IMPROVED | Kd (nM) | NATURAL | Kd (nM) | HXB2 MAPPING | CONSERVATION in % GLOBAL | INTRA-SUBTYPES A | B | C | D | AE | F | G | H | J | N | O |
|------|----------|---------|---------|---------|--------------|--------|---|---|---|---|----|---|---|---|---|---|---|
| 8.1 | VLAAIIAIV | 19.1 | VVAAIIAIV | 140,7 | vpu(13-21) | 9,6 | | 22 | | 6 | | | | | | | |
| 8.2 | ILAIVVWTV | 12.1 | I(I/L)AIVVWTI | 373,1/ 39,9 | Vpu(17-25) | 41,8 | 74 | 35 | 54 | 87,4 | | | | | | | |
| 8.3 | ALVEMGHHV | 35.5 | ALVEMGHHA | 123,7 | Vpu(66-74) | 17,3 | 8 | 35 | | 25 | | | | | | | |
| 8.4 | FLRPWLHGV | 10.2 | FPRPWLHGL | 499,7 | Vpr(34-42) | 17,3 | 62,5 | | | | | | | | | | |
| 8.5 | SLGQHIYEV | 17.8 | SLGQHIYET | 161 | Vpr(41-49) | 19,7 | | 29 | | 40 | | | | | | | |
| 8.6 | SLGQYIYEV | 28.9 | SLGQYIYET | 314,4 | Vpr(41-49) | 13,3 | | 16,5 | | 60 | | | | | | | |
| 8.7 | LLITTYWGL | 24 | LVITTYWGL | 189,1 | Vif(64-72) | 38,5 | | 39 | 12,5 | | | | | | | | |
| 8.8 | LLVRTYWGV | 11.7 | LVVRTYWGL | 146,3 | Vif(64-72) | 9,8 | 75 | | | 8,7 | | | | | | | |
| 8.9 | LLVTTYWGV | 20.1 | LVVTTYWGL | 328,2 | Vif(64-72) | 9,8 | | 12 | | | | | | | | | |
| 8.10 | KLKPPLPSV | 48.8 | K(I/T)KPPLPSV | 444,4/ 231,1 | Vif(158-166) | 56,3 | 35 | 12 | 25 | 87 | | | | | | | |
| 8.11 | GLADQLIHV | 47.0 | GLADQLIH(L/M) | 90,6/ 279,4 | Vif(101-109) | 23,4 | | 25 | 87,5 | 91 | | | | | | | |
| 8.12 | ALAALITPV | 11.9 | ALAALITPK | 137,6 | Vif(149-157) | 18,5 | | 29,6 | | | | | | | | | |
| 8.13 | ALTALITPV | 27.5 | ALTALITPK | 440,5 | Vif(149-157) | 17 | 5 | 20,6 | | 39,5 | | | | | | | |
| 8.14 | LLLPPIERV | 19.5 | LQLPPIERL | 422,6 | Rev(73-81) | 15,2 | 36 | | 42 | | | | | | | | |
| 8.15 | GLGSPQILV | 37.7 | G(V/M)GSPQILV | 347,6/ 55,9 | Rev(96-104) | 18,1 | | 52,8 | | | | | | | | | |
| 8.16 | ILVESPAVV | 82.6 | ILVESPAVL | 169,7 | Rev(102-110) | 8,6 | | 26.4 | | | | | | | | | |
| 8.17 | ALVEICTEV | 31.8 | ALVEICTEM | 183,4 | p51(33-41) | 26,7 | | 78,5 | | | | | | | | | |
| 8.28 | ALTEICTEV | 32,8 | ALTEICTEM | 194,7 | p51(33-41) | 16,3 | | | | | | | | | | | |
| 8.18 | LLIPHPAGV | 6.6 | LGIPHPAGL | 317,9 | p51(92-100) | 88,4 | 75 | 90 | 100 | 100 | 100 | 100 | 66,6 | 100 | 100 | 100 | |
| 8.19 | YLAFTIPSV | 53,1 | YTAFTIPSV | 266,2 | p51(127-135) | 12,8 | | 10,7 | 12,5 | | | 50 | | | | | 100 |
| 8.20 | HLLRWGFTV | 33.4 | HLLRWGFTT | 351,8 | p51(208-216) | 36 | | 61 | 37,5 | 40 | | | | 66,6 | 66,6 | | |
| 8.21 | FLWMGYELV | 29 | FLWMGYELH | 288,8 | p51(227-235) | 97,7 | 87,5 | 100 | 100 | 100 | 100 | 100 | 100 | 100 | 100 | 100 | 100 |
| 8.22 | KLNWASQIV | 30 | KLNWASQIY | 256,7 | p51(263-271) | 94,2 | 100 | 92,8 | 100 | 80 | 100 | 50 | 100 | 100 | 100 | 100 | 100 |

EP 1 250 351 B1

| NAME | IMPROVED | Kd (nM) | NATURAL | Kd (nM) | HXB2 MAPPING | GLOBAL | A | B | C | D | AE | F | G | H | J | N | O |
|------|----------|---------|---------|---------|--------------|--------|---|---|---|---|----|----|---|---|---|---|---|
| 8.23 | SLIYAGIKV | 27.9 | SQIYAGIKV | 268 | p51(268-276) | 43 | 75 | 71 | | 20 | 100 | | | | | | |
| 8.24 | SLIYPGIKV | 21.3 | SQIYPGIKV | 205,1 | p51(268-276) | 39,5 | 12,5 | 21,4 | 100 | 80 | | 50 | 100 | 66,6 | 50 | | |
| 8.25 | ALTEVIPLV | 29.2 | ALTEVIPLT | 319,4 | p51(288-296) | 24,4 | | 61 | | 80 | | | | | | | |
| 8.29 | ALTDIVPLV | 31,8 | ALTDIVPLT | 348,8 | p51(288-296) | 26,7 | | | | | | | | | | | |
| 8.26 | ALTDIVTLV | 22.7 | ALTDIVTLT | 231,8 | p51(288-296) | 10,5 | 75 | | 12,5 | | 33,3 | | | | | | |
| 8.27 | ALTEVVPLV | 26.4 | ALTEVVPLT | 281 | p51(288-296) | 10,5 | | 25 | | 20 | | | | | | | |
| 8.30 | KLWYQLEKV | 17.7 | KLWYQLEK(E/D) | 445,3/ 397,8 | p51(424-432) | 71 | 88 | 87,5 | 50 | 100 | 100 | | | | 100 | | |
| 8.31 | LLGRWPVKV | 8.0 | LAGRWPVKV | 91,9 | p31(104-112) | 40,7 | 75 | | 75 | 80 | 66,6 | | 33,3 | | 50 | 100 | |
| 8.32 | ALKAACWWV | 17.6 | A(V/M)KAACWWA | 483,1/ 77,7 | p31(125-133) | 50 | 50 | | 87,5 | 60 | 100 | 50 | 100 | 100 | 100 | | |
| 8.33 | LLTAVQMAV | 7 | LKTAVQMAV | 140,2 | P31(172-180) | 90,7 | 100 | 96 | 87,5 | 100 | 100 | 100 | 100 | 33,3 | 100 | 100 | 50 |
| 8.34 | KLMAGADCV | 35.2 | KQMAGADCV | 450,7 | p31(273-281) | 9,3 | | | 87,5 | | | | | | | | |
| 8.35 | NLAFPQGEV | 83,3 | NLAFPQGEA | 330,7 | Pol(5-13) | 15,1 | | 17,8 | 87,5 | | | | | | | | |
| 8.36 | LLQRPLVTV | 13.3 | LWQRPLVTV | 227,5 | Pol(61-69) | 29,1 | 87,5 | | | | | | 66,6 | 66,6 | | | |
| 8.37 | ILLWQRPLV | 74,1 | ITLWQRPLV | 400,8 | Pol(59-67) | 73,3 | 87,5 | 75 | 100 | 60 | 100 | 100 | 66,6 | 66,6 | 100 | | |
| 8.38 | ILLWQRPIV | 70.7 | ITLWQRPIV | 378,6 | Pol(59-67) | 8,1 | | 14,3 | | | | | 33,3 | 33,3 | | | |
| 8.39 | LLGPTPVNV | 13.1 | LVGPTPVNI | 468,4 | Pol(132-140) | 86 | 100 | 100 | 100 | 100 | 100 | 100 | | 100 | | 100 | 100 |
| 8.40 | FLISPIETV | 13.6 | FPISPIETV | 467,6 | Pol(155-163) | 84,9 | | | | | | | | | | | |
| 8.41 | KLGKAGYVV | 26 | KLGKAGYVT | 245,2 | Pol(606-614) | 57 | 87,5 | 78,5 | | 80 | 66,6 | | 100 | 33,3 | | | 100 |
| 8.42 | YLAWVPAHV | 15.4 | YLAWVPAHK | 220,1 | Pol(687-695) | 38,4 | 12,5 | 96,4 | | 80 | | | | | | | |
| 8.43 | ALNADCAWV | 44.1 | ATNADCAWL | 458 | Nef(50-58) | 20,3 | | 28 | 15 | | | | | | | | |
| 8.44 | FLVRPQVPV | 6 | FPVRPQVPL | 222 | Nef(68-76) | 74,9 | 62 | 73 | 90 | | | | | | | | |
| 8.45 | LLFGWCFKL | 10 | L(T/C)FGWCFKL | 29,6/ 115,2 | Nef(137-145) | 79,2 | 100 | 78 | 100 | | 95,4 | 50 | 100 | 100 | 50 | | |
| 8.46 | LLWKFDSRV | 66,5 | LMWKFDSRL | 218,3 | Nef(181-189) | 10,4 | 12,5 | 13,2 | | | | | | 100 | | | |
| 8.47 | RLAFHHMAV | 16,2 | RLAFHHMAR | 223,6 | Nef(188-196) | 26,3 | | 40 | | | | | | | | | |
| 8.48 | SLYNTVATV | 33,4 | SLYNTVATL | 63,8 | Pr55(77-85) | 32,3 | 50 | 50 | 12,5 | 20 | | | | 100 | | | |
| 8.49 | NLVATLYCV | 36 | NTVATLYCV | 169,7 | Pr55(80-88) | 57,3 | 62,5 | 69 | 87 | 80 | | | 66,6 | 33,3 | 100 | | |
| 8.50 | NLVAVLYCV | 38.4 | NTVAVLYCV | 179 | Pr55(80-88) | 8,3 | 25 | 19 | | | | | | | | | |

CONSERVATION in %

INTRA-SUBTYPES

| NAME | IMPROVED | Kd (nM) | NATURAL | Kd (nM) | HXB2 MAPPING | CONSERVATION In % | | | | | | | | | | | |
|---|---|---|---|---|---|---|---|---|---|---|---|---|---|---|---|---|---|
| | | | | | | GLOBAL | INTRA-SUBTYPES | | | | | | | | | | |
| | | | | | | | A | B | C | D | AE | F | G | H | J | N | O |
| 8.51 | TLYCVHQKV | 72,6 | TLYCVHQKI | 385,6 | Pr55(84-92) | 15,6 | 12,5 | 25 | 12,5 | | | | 66,6 | | | | |
| 8.52 | TLWCVHQRV | 71.6 | TLWCVHQRI | 388,8 | Pr55(84-92) | 9,4 | 12,5 | | | | 66,6 | | | | | | |
| 8.53 | LLGQMVHQV | 14 | LQGQMVHQA | 481 | Pr55(138-146) | 28,1 | | 40 | 62,5 | 60 | | 50 | | | | | |
| 8.54 | RLLNAWVKV | 73,2 | RTLNAWVKV | 366,8 | pr55(150-158) | 94,8 | 100 | 100 | 100 | 100 | 100 | 100 | 100 | 100 | 100 | 100 | |
| 8.55 | RLHPVQAGV | 18.1 | RLHPVQAGP | 359,6 | Pr55(214-222) | 10,4 | | 15,6 | 12,5 | 20 | | 16,6 | | | | | |
| 8.56 | TLQEQIGWV | 42,3 | TLQEQIGWM | 335,9 | Pr55(242-250) | 43,8 | | 87,5 | | 20 | 66,6 | | | | 50 | | |
| 8.57 | TLQEQIAWV | 32.6 | TLQEQIAWM | 189,6 | Pr55(242-250) | 14,6 | | 6 | 62 | 40 | | | | 66,6 | | | |
| 8.58 | MLNNPPIPV | 18.5 | MTNNPPIPV | 71,3 | Pr55(250-258) | 29,2 | | 78 | | | 66,6 | | | | | | |
| 8.59 | MLSNPPIPV | 21.7 | MTSNPPIPV | 87,8 | Pr55(250-258) | 29,2 | 38 | 9 | | 80 | 33,3 | 16,6 | 33,3 | | | | |
| 8.60 | MLSNPPVPV | 19.9 | MTSNPPVPV | 78,1 | Pr55(250-258) | 8,3 | | | 25 | | | 50 | 33,3 | | | | |
| 8.61 | KLVRMYSPV | 20 | KIVRMYSPV | 157,1 | Pr55(272-280) | 59,4 | 100 | | 100 | 80 | 66,6 | 83,3 | 100 | 100 | 100 | | |
| 8.62 | RLYSPVSIV | 34 | RMYSPVSIL | 104,6 | Pr55(275-283) | 58,3 | 100 | | 100 | 80 | 66,6 | 83,3 | 100 | 100 | 100 | 100 | |
| 8.63 | RLYSPTSIV | 81 | RMYSPTSIL | 272,9 | Pr55(275-283) | 22,9 | | 65 | | | | | | | | | |
| 8.64 | ALGPAATLV | 14.9 | ALGPAATLE | 392,9 | Pr55(336-344) | 26 | | 72 | | | | | | | | | |
| 8.65 | ALLEEMMTV | 26.6 | ATLEEMMTA | 431 | Pr55(341-349) | 75 | 87 | 97 | | 80 | 100 | 83,3 | 66,6 | | 100 | 100 | |
| 8.142 | SLEEMMTAV | 32.4 | SLEEMMTAC | 295,8 | Pr55(342-350) | | | | | | | | | | | | |
| 8.66 | ELMTACQGV | 85 | EMMTACQGV | 134,2 | pr55(345-353) | 91,7 | 87,5 | 100 | 100 | 80 | 100 | 83,3 | 66,6 | | 100 | 100 | |
| 8.67 | MLQRGNFRV | 16.7 | MMQRGNFR(N/G) | 347,4/ 439,4 | Pr55(377-385) | 30,2 | 37,5 | 62,5 | | | | | | | | | |
| 8.68 | MLQRGNFKV | 12.3 | MMQRGNFKG | 247,4 | Pr55(377-385) | 13,5 | 37,5 | | | 60 | 66,6 | 16,6 | | | | | |
| 8.69 | FLQSRPEPV | 13 | FLQSRPEPT | 96,5 | Pr55(448-456) | 43,8 | | 72 | 62 | 60 | | 50 | | 66,6 | 100 | | |
| 8.70 | FLQNRPEPV | 14.2 | FLQNRPEPT | 114,9 | Pr55(448-456) | 13,5 | | | 12,5 | | | 16,6 | 66,6 | | | | |
| 8.71 | HLWRWGTMV | 56 | HLWRWGTML | 111,5 | gp160(9-21) | 14,1 | | 25 | | 28,6 | | | | | | | |
| 8.72 | MLLGMLMIV | 19.2 | MLLGMLMIC | 146,4 | gp160(19-27) | 23,5 | | 45 | | | | | | | | | |
| 8.73 | KLWVTVYYV | 18.3 | KLWVTVYYG | 297,7 | gp160(33-41) | 18,3 | 18,75 | 30 | | 7,1 | 11,1 | | 33,3 | | | | |
| 8.74 | VLVYYGVPV | 60 | VTVYYGVPV | 299,2 | gp160(35-44) | 90,1 | 87,5 | 98 | 91 | 85,7 | 100 | 75,0 | 100 | 100 | 100 | 100 | |
| 8.75 | NLWATHACV | 50 | N(V/I)WATHACV | 490,6/ 486,1 | gp160(67-75) | 90,7 | 100 | 94 | 95,4 | 85,5 | 88,/ | 100 | 100 | 100 | 100 | | |
| 8.76 | KLTPLCVTV | 57 | KLTPLCVTL | 114,7 | gp160(121-129) | 82,2 | 87,5 | 87 | 95,4 | 85,7 | 77,7 | 100 | 100 | 100 | | | |

EP 1 250 351 B1

EP 1 250 351 B1

**CONSERVATION in %**

| NAME | IMPROVED | Kd (nM) | NATURAL | Kd (nM) | HXB2 MAPPING | GLOBAL | A | B | C | D | AE | F | G | H | J | N | O |
|---|---|---|---|---|---|---|---|---|---|---|---|---|---|---|---|---|---|
| | | | | | | | | | | | INTRA-SUBTYPES | | | | | | |
| 8.143 | YLAPAGFAV | 5,9 | YCAPAGFAI | 204,5 | gp160(217-225) | 52,1 | | | | | | | | | | | |
| 8.77 | YLAPAGYAV | 8.3 | YCAPAGYAI | 420,5 | gp160(217-225) | 16,9 | 12,5 | 1 | 77,2 | 7 | 11,1 | 100 | | | | | 75 |
| 8.78 | SLAEEEVVV | 46 | SLAEEEVV(I/L) | 218,3/ 90,7 | gp160(264-272) | 31 | | 63 | | | | | | | | | |
| 8.79 | SLAEEEIIV | 57.3 | SLAEEEIII | 281 | gp160(264-272) | 9,9 | | | 18,2 | 64,3 | 66,6 | | | | | | |
| 8.80 | ALYAPPIRV | 13.0 | AMYAPPIRG | 276,8 | gp160(433-441) | 22,1 | 6 | 45 | | | | | | 33,3 | | | |
| 8.81 | ALYAPPIEV | 15 | AMYAPPIEG | 354,1 | gp160(433-441) | 12,2 | 6 | 7 | 22,7 | 65 | | | | | | | |
| 8.82 | AMYAPPIKV | 12.1 | AMYAPPIKG | 154,6 | gp160(433-441) | 8,9 | 12,5 | 8 | 27,0 | | | | | 33,3 | | | |
| 8.83 | AMYAPPIAV | 18.6 | AMYAPPIAG | 298 | gp160(433-441) | 8,5 | | 7 | 22,7 | 7 | | | 75 | | | | |
| 8.84 | PLGIAPTKV | 68,6 | PLGIAPTKA | 264,4 | gp160(493-501) | 11,3 | | 15 | 27,3 | | 22,2 | | | | | | |
| 8.85 | TLGAASITV | 41 | TMGAASITL | 126,8 | gp160(529-537) | 46,9 | 81 | 25 | 100 | | 100 | 75 | 75 | 100 | 100 | 100 | |
| 8.86 | TLGAASLTV | 81 | TMGAASLTL | 273,2 | gp160(529-537) | 13,1 | | 17 | | 42,8 | | 25 | | | | | |
| 8.87 | RLIEAQQHV | 13.5 | RAIEAQQHL | 489,9 | gp160(557-565) | 65,3 | 62,5 | 77 | 27,2 | 71,4 | 88,8 | 75 | 100 | | | | 100 |
| 8.88 | ALEAQQHLV | 14 | AIEAQQHLL | 200 | gp160(558-566) | 70 | 81,2 | 77 | 27,3 | 78,5 | 88,8 | 75 | 100 | | 50 | | 100 |
| 8.89 | ALEAQQHMV | 19.4 | AIEAQQHML | 310,2 | gp160(558-566) | 14,1 | | 12 | 63,6 | | | | | | | | |
| 8.90 | LLKLTVWGV | 27 | LLKLTVWGI | 120,4 | gp160(565-573) | 14,1 | 93,7 | | | | | | | 100 | | | |
| 8.91 | CLTAVPWNV | 15.3 | CTTAVPWNA | 202,5 | gp160(604-612) | 17,4 | | 37 | | | | | | | | | |
| 8.92 | SLWNWFSIV | 40.0 | SLWNWFSIT | 442,3 | gp160(668-676) | 11,7 | | 13 | | 50 | 11,1 | | | 33,3 | | | |
| 8.93 | YLKIFIMIV | 33 | YIKIFIMIV | 286,3 | gp160(681-689) | 60,1 | 37,5 | 68 | 72,7 | 57,1 | 66,6 | 100 | 75 | 33,3 | | | |
| 8.94 | YLRIFIMIV | 42.3 | YIRIFIMIV | 378,9 | gp160(681-689) | 9,9 | 31,2 | | | | | | | | | | |
| 8.95 | FLMIVGGLV | 41.3 | FIMIVGGLV | 391,4 | gp160(685-693) | 22,5 | | 47 | | | | | | | | | |
| 8.96 | ILFAVLSIV | 24.9 | I(V/I)FAVLSIV | 203,1/ 200,6 | gp160(697-705) | 40,3 | | 41 | 72,7 | 7 | 100 | 75 | 100 | 100 | | | |
| 8.97 | LLAARTVEV | 14.6 | LIAARTVEL | 317,2 | gp160(776-784) | 26,3 | 62,5 | | 9 | | 55,5 | | 50 | | | | |
| 8.98 | LLVARIVEV | 17.9 | LIVARIVEL | 240,5 | gp160(776-784) | 8,9 | | 18 | | | | | | | | | |

*Table 5C:*

8-mers representing one best new improved binder for each natural epitope with a binding affinity $IC_{50}$ between 50nM and 500nM and with a global conservation above 8%. 47 HLA-A2 8-mer-epitopes were found. These 47 HLA-A2 epitopes were regarded as natural intermediate binders whose binding affinity could be increased to obtain an improved binding affinity of $IC_{50}$ below 100nM by modifying one or two of the primary anchor positions. 45 optimally (best) improved HLA-A2 restricted HIV epitopes were identified.

# Table 5C

| NAME | IMPROVED | Kd nM | NATURAL | Kd nM | HBX2 MAPPING | % CONSERVATION | | | | | | | | | | | |
|---|---|---|---|---|---|---|---|---|---|---|---|---|---|---|---|---|---|
| | | | | | | GLOBAL | A | B | C | D | AE | F | G | H | J | N | O |
| 8.99 | LLFIHFRV | 92.5 | LLFIHFRI | 495,2 | vpr(67-74) | 69,9 | 12,5 | 87,4 | 62,5 | 100 | | 100 | 100 | 66,6 | 100 | | |
| 8.100 | LLFVHFRV | 67,1 | LLFVHFRI | 359 | Vpr(67-74) | 11 | 87,5 | | 12,5 | 100 | | | | | | | |
| 8.101 | ILGHIVSV | 20.6 | ILGHIVSP | 268,1 | Vif(124-131) | 20,4 | 5 | 27,8 | | 17,4 | | | | | | | |
| 8.102 | KLGSLQYV | 29 | KVGSLQYL | 151,6 | Vif(141-148) | 82,3 | 95 | 89,7 | 75 | 100 | 100 | 100 | 66,6 | 33,3 | 100 | | |
| 8.103 | RLAEPVPV | 36.8 | RSAEPVPL | 340,5 | Rev(66-73) | 19 | 9 | 23,5 | 25 | 12,5 | | | 33,3 | | | | |
| 8.144 | YLSNPYPV | 12,2 | YQSNPYPK | 241,2 | Rev(23-30) | 15,2 | 36,4 | | 25 | | | 75,0 | | 50,0 | | | |
| 8.104 | GLGSPQIV | 31.3 | GVGSPQIL | 168,8 | Rev(96-103) | 16,2 | | 47 | | | | | | | | | |
| 8.105 | KLGPENPV | 38.4 | KIGPENPY | 497 | p51(49-56) | 76,7 | 100 | 89,3 | 87,5 | 20 | 100 | 50 | 100 | 66,6 | | | |
| 8.106 | QLGIPHPV | 16 | QLGIPHPA | 161,7 | p51(91-98) | 88,4 | 75 | 89 | 100 | 100 | 100 | 100 | 66,6 | 100 | 100 | 100 | |
| 8.107 | TLNDIQKV | 15 | TVNDIQKL | 66,5 | p51(253-260) | 97,7 | 100 | 100 | 100 | 80 | 100 | 50 | 100 | 100 | 100 | 100 | 100 |
| 8.108 | ALTDIVPV | 57,8 | ALTDIVPL | 78,9 | p51(288-295) | 26,7 | | | 50 | | 66,6 | 50 | 33,0 | | 100 | | |
| 8.109 | LLAEIQKV | 20.4 | LIAEIQKQ | 311,5 | p51(325-332) | 53,5 | 75 | 50 | 100 | 60 | | 50 | | 66,6 | | | |
| 8.110 | LLEVVQKV | 62,2 | LAEVVQKV | 408,2 | p51(368-375) | 10,5 | 62,5 | | | 33,3 | | | | | | | 50 |
| 8.111 | FLNTPPLV | 26 | FVNTPPLV | 97,6 | p51(416-423) | 86 | 100 | 89 | 100 | 80 | 100 | 50 | 100 | | | 100 | 100 |
| 8.112 | FLFPQITV | 37.8 | FSFPQITL | 318 | Pol(54-61) | 27,9 | 62,5 | 42,8 | | 20 | 100 | | | | | | |
| 8.113 | FLFPQITV | 37.8 | FNFPQITL | 316,8 | Pol(54-61) | 14 | | 14,2 | 12,5 | 20 | | | | 33,3 | | | 100 |
| 8.114 | FLKVKQYV | 28.8 | FIKVKQYD | 477,2 | Pol(109-116) | 20,9 | 87,5 | 3,5 | | | 33 | 100 | | | | | |
| 8.115 | LLFPISPV | 15 | LNFPISPI | 369,8 | Pol(153-160) | 93 | 100 | 96,4 | 100 | 100 | 100 | 50 | 100 | 66,6 | 100 | 100 | 100 |
| 8.116 | ALGIIQAV | 13 | ALGIIQAQ | 131,3 | Pol(657-664) | 84,9 | 87,5 | 93 | 87,5 | 100 | 100 | 100 | 100 | 100 | 100 | | |
| 8.117 | GLGVRYPV | 20.7 | GPGVRYPL | 417 | Nef(130-137) | 17,5 | 25 | 17,3 | 35 | 9 | | | | | | | |
| 8.118 | GLGTRFPV | 16 | GPGTRFPL | 281,8 | Nef(130-137) | 13,9 | 25 | 11 | 25 | | | | 100 | | | | 50 |
| 8.119 | TLGWCFKV | 27 | TFGWCFKL | 254,3 | Nef(138-145) | 66,5 | 87,5 | 74 | 100 | | | 50 | 100 | 100 | 50 | 100 | |

EP 1 250 351 B1

| NAME | IMPROVED | Kd nM | NATURAL | Kd nM | HBX2 MAPPING | GLOBAL | % CONSERVATION | | | | | | | | | | |
| | | | | | | | INTRA-SUBTYPES | | | | | | | | | | |
| | | | | | | | A | B | C | D | AE | F | G | H | J | N | O |
| 8.120 | CLGWCFKV | 19.5 | CFGWCFKL | 168,6 | Nef(138-145) | 13,5 | | 5,4 | | | 100 | | | | | | |
| 8.121 | WLFKLVPV | 49.9 | WCFKLVPV | 433,6 | Nef(141-148) | 82,5 | 87,5 | 87,4 | 100 | | 100 | 100 | | 66,6 | 50 | | |
| 8.145 | FLHVAREV | 84,4 | FHHVAREL | 381,9 | Nef(191-198) | 13,5 | nd | nd | nd | nd | nd | nd | nd | nd | nd | nd | nd |
| 8.146 | FLHMAREV | 89,1 | FHHMAREL | 394,7 | Nef(191-198) | 19,9 | nd | nd | nd | nd | nd | nd | nd | nd | nd | nd | nd |
| 8.122 | ILGQLQPV | 6,7 | ILGQLQPS | 94.9 | Pr55(60-67) | 15,6 | | 47 | | | | | | | | | |
| 8.123 | TLNPPIPV | 26.4 | T(N/S/G)NPPIPV | 164,6/160,9/380,3 | Pr55(251-258) | 68,8 | 100 | 87,4 | | 80 | 100 | 17 | 66 | | | | |
| 8.124 | PLGEIYKV | 11.3 | PVGEIYKR | 457,5 | Pr55(257-264) | 61,5 | | 93,8 | 25 | 80 | | 50 | 100 | | 100 | | |
| 8.125 | PLGDIYKV | 7.9 | PVGDIYKR | 211,2 | Pr55(257-264) | 30,2 | 87,5 | 6 | 75 | | 66,6 | 33,3 | | 100 | | | |
| 8.126 | ALGPAATV | 51,3 | ALGPAATL | 70 | Pr55(336-343) | 27,1 | | 75 | | 20 | | | | | | | |
| 8.127 | FLGKIWPV | 4 | FLGKIWPS | 38,6 | Pr55(433-440) | 84,4 | 75 | 93,8 | 87,5 | 80 | 100 | 100 | 100 | 100 | 100 | | |
| 8.128 | LLNRPEPV | 29.4 | LQNRPEPT | 492,9 | Pr55(449-456 | 13,5 | | 3,1 | 12,5 | | | 16,6 | 66,6 | | | | |
| 8.129 | ILGDIRQV | 15.1 | IIGDIRQA | 190,2 | gp160(322-329) | 47,4 | 43,7 | 58 | 86,3 | | | | 50 | 33,3 | | | |
| 8.130 | SLGDPEIV | 16.8 | SGGDPEIV | 200,5 | gp160(365-372) | 37,6 | | 79 | | | | | | | | | |
| 8.131 | CLGEFFYV | 9.1 | CRGEFFYC | 490,8 | gp160(378-385) | 22,5 | | 1 | 86,3 | 7 | 100 | 75 | 75 | 66,6 | | 100 | |
| 8.132 | ILNMWQEV | 62 | IINMWQEV | 95,1 | gp160(423-430) | 27,7 | | 39 | 50 | 35,7 | | | | | | | |
| 8.133 | ILNMWQGV | 95,6 | IINMWQGV | 146,4 | gp160(423-430) | 11,7 | | 9 | 40,9 | 21,4 | 33,3 | | | | | | |
| 8.134 | FLGFLGAV | 15 | FLGFLGAA | 156,1 | gp160(519-526) | 73,2 | 43,7 | 89 | 86,3 | 78,6 | | 25 | 75 | 100 | | 100 | |
| 8.135 | FLGAAGSV | 30 | FLGAAGST | 178,7 | gp160(522-529) | 88,3 | 94 | 96 | 95,4 | 78,6 | 100 | 25 | 75 | 100 | | 100 | |
| 8.136 | LLARILAV | 96,5 | LQARILAV | 281,4 | gp160(576-583) | 10,3 | 6 | 8 | | 50 | | | | | | | |
| 8.137 | YLKDQQLV | 56 | YLKDQQLL | 75,8 | gp160(586-593) | 50,2 | 50 | 54 | 68 | 57 | | 50 | 50 | 66,6 | 100 | | |
| 8.138 | YLRDQQLV | 96,4 | YLRDQQLL | 132,8 | gp160(586-593) | 22,5 | 37,5 | 26 | 9 | 21 | | | 25 | 33 | | | |
| 8.139 | ILGGLVGV | 84 | IVGGLVGL | 497,5 | gp160(688-695) | 24,9 | | 52 | | | | | | | | | |
| 8.140 | ILFAVLSV | 57 | IIFAVLSI | 449,3 | gp160(697-704) | 17,8 | 6 | 4 | 82 | | 77,7 | | 25 | 66,6 | | | |
| 8.141 | LLNGFLAV | 74,7 | LVNGFLAL | 447,2 | gp160(748-755) | 13,1 | 6 | 15 | 27 | | | 50 | | | 100 | | |

*Table 5D:*

8-mers and 9-mers representing other improved binders for each natural epitope with a binding affinity IC$_{50}$ between 50nM and 500nM and with a global conservation above 8%. 812 HLA-A2 epitopes were found. These 812 HLA-A2 epitopes were regarded as natural intermediate binders whose binding affinity could be increased to obtain an improved binding affinity of IC$_{50}$ below 100nM by modifying one or two of the primary anchor positions.

Table 5D

| Peptide | Kd nM | Peptide | Kd nM |
|---------|-------|---------|-------|
| AIEAQQHLV | 95,4 | AMEAQQHLI | 81,4 |
| AIGIIQAI | 76,2 | AMEAQQHLL | 36,1 |
| AIGIIQAL | 23,5 | AMEAQQHLV | 20,0 |
| AIGIIQAV | 18,3 | AMEAQQHMA | 90,9 |
| AIYAPPIEV | 98,4 | AMEAQQHML | 51,3 |
| AIYAPPIRV | 77,0 | AMEAQQHMV | 27,5 |
| AIAALITPV | 67,8 | AMGIIQAI | 78,0 |
| ALEAQQHLA | 39,9 | AMGIIQAL | 23,8 |
| ALEAQQHLI | 52,6 | AMGIIQAV | 18,5 |
| ALEAQQHLL | 24,6 | AMGPAATLA | 65,9 |
| ALEAQQHMA | 58,4 | AMGPAATLI | 87,5 |
| ALEAQQHMI | 78,9 | AMGPAATLL | 38,3 |
| ALEAQQHML | 34,4 | AMGPAATLV | 20,9 |
| ALGIIQAI | 51,3 | AMKAACWVVA | 77,7 |
| ALGIIQAL | 16,9 | AMKAACWWL | 45,8 |
| ALGPAATLA | 42,9 | AMKAACWWV | 24,6 |
| ALGPAATLI | 56,0 | AMLEEMMTL | 74,6 |
| ALGPAATLL | 25,9 | AMLEEMMTV | 38,3 |
| ALKAACWWA | 50,9 | AMNADCAWV | 66,6 |
| ALKAACWWI | 66,8 | AMTALITPL | 75,4 |
| ALKAACWWL | 31,0 | AMTALITPV | 39,6 |
| ALLEEMMTA | 84,2 | AMTDIVPLL | 90,4 |
| ALLEEMMTL | 49,8 | AMTDIVPLV | 45,9 |
| ALNADCAWL | 86,8 | AMTDIVTLL | 62,4 |
| ALTALITPA | 87,9 | AMTDIVTLV | 32,5 |
| ALTALITPL | 49,7 | AMTEICTEL | 99,4 |
| ALTDIVPLL | 59,4 | AMTEICTEV | 49,3 |
| ALTDIVTLA | 73,1 | AMTEVIPLL | 84,0 |
| ALTDIVTLI | 98,0 | AMTEVIPLV | 42,8 |
| ALTDIVTLL | 41,3 | AMTEVVPLL | 74,7 |
| ALTEICTEL | 63,3 | AMTEVVPLV | 38,2 |
| ALTEVIPLA | 97,5 | AMVEICTEL | 94,3 |
| ALTEVIPLL | 54,8 | AMVEICTEV | 47,4 |
| ALTEWPLA | 86,9 | AMVEMGHHV | 52,4 |
| ALTEWPLL | 49,0 | AMYAPPIEA | 61,9 |
| ALVEICTEL | 60,5 | AMYAPPIEI | 83,3 |
| ALVEMGHHL | 68,3 | AMYAPPIEL | 36,1 |
| ALYAPPIEA | 41,8 | AMYAPPIEV | 20,6 |
| ALYAPPIEI | 55,3 | AMYAPPIRA | 49,5 |
| ALYAPPIEL | 25,4 | AMYAPPIRI | 66,2 |
| ALYAPPIRA | 34,0 | AMYAPPIRL | 29,5 |
| ALYAPPIRI | 44,5 | AMYAPPIRV | 17,4 |
| ALYAPPIRL | 21,0 | AMAALITPA | 44,0 |

Table 5D   (continued)

| Peptide | Kd nM | Peptide | Kd nM |
|---|---|---|---|
| ALAALITPA | 30,2 | AMAALITPI | 59,0 |
| ALAALITPI | 39,5 | AMAALITPL | 27,0 |
| ALAAUTPL | 19,1 | AMAALITPV | 15.9 |
| AMEAQQHLA | 60,9 | AQGIIQAL | 38,3 |
| AQGIIQAV | 28,9 | FIQNRPEPV | 93,5 |
| AQAALITPV | 91,7 | FIQSRPEPV | 79,4 |
| CIGEFFYA | 76,4 | FIRPWLHGL | 99,8 |
| CIGEFFYC | 70,2 | FIRPWLHGV | 52,4 |
| CIGEFFYI | 46,5 | FIVRPQVPA | 82,0 |
| CIGEFFYL | 16,3 | FIVRPQVPL | 46,9 |
| CIGEFFYV | 13,1 | FIVRPQVPV | 26,1 |
| CIGWCFKL | 36,3 | FLFPQITL | 50,2 |
| CIGWCFKV | 27,7 | FLFPQITL | 50,2 |
| CLGEFFYA | 50,7 | FLGFLGAI | 61,2 |
| CLGEFFYC | 45,8 | FLGFLGAL | 19,2 |
| CLGEFFYI | 29,8 | FLGAAGSL | 40,8 |
| CLGEFFYL | 11,2 | FLISPIETA | 36,7 |
| CLGWCFKI | 84,1 | FLISPIETI | 48,3 |
| CLGWCFKL | 25,1 | FLISPIETL | 22,6 |
| CLTAVPWNA | 42,4 | FLKVKQYL | 38,0 |
| CLTAVPWNI | 55,8 | FLMIVGGLL | 81,0 |
| CLTAVPWNL | 26,6 | FLNTPPLL | 34,6 |
| CMGEFFYA | 77,8 | FLQNRPEPA | 39,2 |
| CMGEFFYC | 71,8 | FLQNRPEPI | 50,9 |
| CMGEFFYI | 46,5 | FLQNRPEPL | 23,8 |
| CMGEFFYL | 16,2 | FLQSRPEPA | 34,1 |
| CMGEFFYV | 13,0 | FLOSRPEPI | 43,8 |
| CMGWCFKL | 37,1 | FLQSRPEPL | 21,1 |
| CMGWCFKV | 28,1 | FLRPWLHGA | 24,3 |
| CMTAVPWNA | 64,5 | FLRPWLHGI | 31,3 |
| CMTAVPWNI | 86,2 | FLRPWLHGL | 15,6 |
| CMTAVPWNL | 39,1 | FLVRPQVPA | 13,3 |
| CMTAVPWNV | 21,4 | FLVRPQVPI | 16,4 |
| CQGEFFYI | 68,4 | FLVRPQVPL | 9,3 |
| CQGEFFYL | 21,8 | FLWMGYELA | 97,2 |
| CQGEFFYV | 17,1 | FLWMGYELL | 56,0 |
| CQGWCFKL | 61,9 | FMFPQITL | 82,5 |
| CQGWCFKV | 45,7 | FMFPQITL | 82,5 |
| CRGEFFYL | 61,7 | FMFPQITV | 61,5 |
| CRGEFFYV | 46,0 | FMFPQITV | 61,5 |
| CTTAVPWNV | 54,9 | FMGFLGAI | 96,1 |
| EMQKQITKV | 92,3 | FMGFLGAL | 27,9 |
| FIFPQITL | 80,8 | FMGFLGAV | 21,8 |
| FIFPQITL | 80,8 | FMGAAGSL | 61,7 |
| FIFPQITV | 60,4 | FMGAAGSV | 45,4 |
| FIFPQITV | 60,4 | FMISPIETA | 54,8 |
| FIGFLGAI | 91,7 | FMISPIETI | 73,3 |
| FIGFLGAL | 26,7 | FMISPIETL | 32,3 |
| FIGFLGAV | 20,9 | FMISPIETV | 18,5 |
| FIGAAGSL | 58,0 | FMKVKQYL | 60,9 |

Table 5D   (continued)

| Peptide | Kd nM | Peptide | Kd nM |
|---|---|---|---|
| FIGAAGSV | 42,7 | FMKVKQYV | 45,8 |
| FIISPIETV | 86,2 | FMMIVGGLV | 62,4 |
| FIKVKQYL | 59,2 | FMNTPPLL | 53,4 |
| FIKVKQYV | 44,7 | FMNTPPLV | 40,2 |
| FINTPPLL | 52,2 | FMQNRPEPA | 59,2 |
| FINTPPLV | 39,6 | FMQNRPEPI | 78,5 |
| FMQNRPEPL | 34,6 | GQGTRFPV | 35,9 |
| FMQNRPEPV | 19,5 | GQGVRYPL | 68,4 |
| FMQSRPEPA | 51,0 | GQGVRYPV | 49,7 |
| FMQSRPEPI | 66,9 | HLLRWGFTL | 62,7 |
| FMQSRPEPL | 30,5 | HMLRWGFTL | 97,1 |
| FMQSRPEPV | 17,3 | HMLRWGFTV | 49,2 |
| FMRPWLHGA | 34,2 | HMWRWGTMV | 85,4 |
| FMRPWLHGI | 44,9 | IIAIVVWTV | 71,3 |
| FMRPWLHGL | 21,3 | IIFAVLSV | 88,7 |
| FMRPWLHGV | 13,3 | IIGAETFYV | 51,8 |
| FMVRPQVPA | 18,2 | IIGAETFYV | 51,8 |
| FMVRPQVPI | 22,9 | IIGDIRQI | 94,1 |
| FMVRPQVPL | 12,2 | IIGDIRQL | 28,6 |
| FMVRPQVPV | 8,2 | IIGDIRQV | 22,1 |
| FMWMGYELL | 86,6 | IIGHIVSL | 39,1 |
| FMWMGYELV | 44,0 | IIGHIVSV | 29,7 |
| FQFPQITV | 95,2 | IIGQLQPA | 50,8 |
| FQFPQITV | 95,2 | IIGQLQPI | 29,9 |
| FQGFLGAL | 45,0 | IIGQLQPL | 11,2 |
| FQGFLGAV | 34,2 | IIGQLQPV | 9,2 |
| FQGAAGSV | 75,6 | ILAIVVWTA | 31,0 |
| FQKVKQYL | 93,6 | ILAIVVWTI | 39,9 |
| FQKVKQYV | 69,0 | ILAIVVWTL | 19,8 |
| FQNTPPLL | 86,8 | ILFAVLSIA | 79,5 |
| FQNTPPLV | 63,4 | ILFAVLSIA | 79,5 |
| FQRPWLHGV | 71,3 | ILFAVLSIL | 45,1 |
| FQVRPQVPL | 61,4 | ILFAVLSIL | 45,1 |
| FQVRPQVPV | 33,4 | ILFAVLSL | 77,6 |
| GIGSPQIL | 62,1 | ILGAETFYA | 23,6 |
| GIGSPQIV | 46,4 | ILGAETFYA | 23,6 |
| GIGTRFPI | 97,3 | ILGAETFYA | 23,6 |
| GIGTRFPL | 28,5 | ILGAETFYI | 30,4 |
| GIGTRFPV | 22,3 | ILGAETFYI | 30,4 |
| GIGVRYPL | 37,9 | ILGAETFYI | 30,4 |
| GIGVRYPV | 28,6 | ILGAETFYL | 15,4 |
| GLGSPQIL | 41,7 | ILGAETFYL | 15,4 |
| GLGSPQILL | 71,7 | ILGAETFYL | 15,4 |
| GLGTRFPI | 64,3 | ILGDIRQI | 60,6 |
| GLGTRFPL | 20,2 | ILGDIRQL | 19,4 |
| GLGVRYPI | 92,7 | ILGHIVSI | 90,8 |
| GLGVRYPL | 27,2 | ILGHIVSL | 26,7 |
| GMADQLIHV | 69,9 | ILGQLQPA | 35,2 |
| GMGSPQIL | 63,3 | ILGQLQPI | 19,8 |
| GMGSPQILV | 55,9 | ILGQLQPL | 8,0 |

Table 5D   (continued)

| Peptide | Kd nM | Peptide | Kd nM |
|---------|-------|---------|-------|
| GMGSPQIV | 46,9 | ILNMWQEL | 85,4 |
| GMGTRFPI | 99,8 | IMAIWWTA | 46,4 |
| GMGTRFPL | 29,1 | IMAIWWTI | 60,8 |
| GMGTRFPV | 22,6 | IMAIVVWTL | 28,5 |
| GMGVRYPL | 38,7 | IMAIVVWTV | 16,4 |
| GMGVRYPV | 28,9 | IMFAVLSIL | 68,5 |
| GQGSPQIV | 79,9 | IMFAVLSIL | 68,5 |
| GQGTRFPL | 47,5 | IMFAVLSIV | 35,8 |
| IMFAVLSIV | 35,8 | KLWYQLEKA | 51,5 |
| IMFAVLSV | 91,8 | KLWYQLEKI | 67,2 |
| IMGAETFYA | 33,9 | KLWYQLEKI | 67,2 |
| IMGAETFYA | 33,9 | KLWYQLEKL | 31,3 |
| IMGAETFYA | 33,9 | KLWYQLEKL | 31,3 |
| IMGAETFYI | 44,7 | KMGKAGYVL | 74,0 |
| IMGAETFYI | 44,7 | KMGKAGYVV | 38,2 |
| IMGAETFYI | 44,7 | KMGPENPL | 75,6 |
| IMGAETFYL | 21,3 | KMGPENPV | 55,8 |
| IMGAETFYL | 21,3 | KMGSLQYL | 57,5 |
| IMGAETFYL | 21,3 | KMGSLQYV | 42,6 |
| IMGAETFYV | 12,9 | KMKPPLPSV | 72,4 |
| IMGAETFYV | 12,9 | KMKPPLPSV | 72,4 |
| IMGAETFYV | 12,9 | KMNWASQIL | 86,3 |
| IMGDIRQI | 97,4 | KMNWASQIV | 44,5 |
| IMGDIRQL | 29,3 | KMTPLCVTV | 87,5 |
| IMGDIRQV | 22,4 | KMVRMYSPA | 99,5 |
| IMGHIVSL | 40,4 | KMVRMYSPL | 56,5 |
| IMGHIVSV | 30,6 | KMVRMYSPV | 29,6 |
| IMGQLQPA | 52,0 | KMWVTVYYA | 82,8 |
| IMGQLQPI | 30,1 | KMWVTVYYL | 48,0 |
| IMGQLQPL | 11,1 | KMWVTVYYV | 25,9 |
| IMGQLQPV | 9,1 | KMWYQLEKA | 79,3 |
| IQAIVVWTV | 94,3 | KMWYQLEKA | 79,3 |
| IQGAETFYV | 69,9 | KMWYQLEKL | 46,4 |
| IQGDIRQL | 44,2 | KMWYQLEKL | 46,4 |
| IQGDIRQV | 33,1 | KMWYQLEKV | 25,1 |
| IQGHIVSL | 66,1 | KMWYQLEKV | 25,1 |
| IQGHIVSV | 48,7 | KQGSLQYV | 72,5 |
| IQGQLQPA | 92,6 | LCFGWCFKV | 57,4 |
| IQGQLQPI | 44,7 | LIAEIQKL | 40,0 |
| IQGQLQPL | 15,0 | LIAEIQKV | 30,6 |
| IQGQLQPV | 12,0 | LIEVVQKV | 95,2 |
| KIGPENPL | 73,9 | LIFGWCFKA | 88,1 |
| KIGPENPV | 54,8 | LIFGWCFKL | 50,7 |
| KIGSLQYL | 56,2 | LIFGWCFKV | 27,1 |
| KIGSLQYV | 42,3 | LIFPISPI | 94,7 |
| KLGKAGYVA | 82,1 | LIFPISPL | 28,9 |
| KLGKAGYVL | 48,3 | LIFPISPV | 22,7 |
| KLGPENPL | 51,5 | LIGPTPVNV | 84,2 |
| KLGSLQYL | 38,3 | LIGQMVHQV | 89,7 |
| KLKPPLPSL | 93,3 | LIGRWPVKL | 71,3 |

Table 5D   (continued)

| Peptide | Kd nM | Peptide | Kd nM |
|---|---|---|---|
| KLKPPLPSL | 93,3 | LIGRWPVKV | 37,2 |
| KLNWASQIA | 94,6 | LIIPHPAGA | 93,0 |
| KLNWASQIL | 56,7 | LIIPHPAGL | 51,6 |
| KLVRMYSPA | 63,7 | LIIPHPAGV | 27,7 |
| KLVRMYSPI | 83,4 | LIITTYWGV | 88,7 |
| KLVRMYSPL | 37,5 | LINRPEPL | 59,1 |
| KLWVTVYYA | 54,3 | LINRPEPV | 44,1 |
| KLWVTVYYI | 71,9 | LIQRPLVTV | 80,5 |
| KLWVTVYYL | 32,5 | LITAVQMAL | 52,0 |
| KLWYQLEKA | 51,5 | LITAVQMAV | 28,0 |
| LITAVQMAA | 91,4 | LMGPTPVNI | 71,2 |
| LIAARTVEV | 95,2 | LMGPTPVNL | 31,5 |
| LLAEIQKI | 89,4 | LMGPTPVNV | 17,9 |
| LLAEIQKL | 26,4 | LMGQMVHQA | 56,6 |
| LLEVVQKL | 85,6 | LMGQMVHQI | 75,2 |
| LLFGWCFKA | 13,9 | LMGQMVHQL | 33,3 |
| LLFGWCFKI | 17,1 | LMGQMVHQV | 18,8 |
| LLFGWCFKV | 6,7 | LMGRWPVKA | 25,0 |
| LLFPISPI | 57,9 | LMGRWPVKI | 32,3 |
| LLFPISPL | 18,8 | LMGRWPVKL | 16,3 |
| LLFVHFRL | 91,2 | LMGRWPVKV | 10,2 |
| LLGPTPVNA | 35,6 | LMIPHPAGA | 19,1 |
| LLGPTPVNI | 46,6 | LMIPHPAGI | 23,8 |
| LLGPTPVNL | 21,8 | LMIPHPAGL | 12,7 |
| LLGQMVHQA | 38,4 | LMIPHPAGV | 8,3 |
| LLGQMVHQI | 50,1 | LMITTYWGA | 58,2 |
| LLGQMVHQL | 23,4 | LMITTYWGI | 75,1 |
| LLGRWPVKA | 17,8 | LMITTYWGL | 34,3 |
| LLGRWPVKI | 22,5 | LMITTYWGV | 19,2 |
| LLGRWPVKL | 12,1 | LMKLTVWGL | 77,0 |
| LLIPHPAGA | 13,9 | LMKLTVWGV | 39,3 |
| LLIPHPAGI | 17,0 | LMLPPIERA | 90,4 |
| LLIPHPAGL | 9,6 | LMLPPIERL | 50,4 |
| LLITTYWGA | 38,6 | LMLPPIERV | 27,5 |
| LLITTYWGI | 49,0 | LMNRPEPL | 60,7 |
| LLITTYWGV | 14,0 | LMNRPEPV | 44,9 |
| LLKLTVWGA | 90,2 | LMQRPLVTA | 51,0 |
| LLKLTVWGL | 50,3 | LMQRPLVTI | 68,7 |
| LLLPPIERA | 59,0 | LMQRPLVTL | 30,4 |
| LLLPPIERI | 78,6 | LMQRPLVTV | 17,7 |
| LLLPPIERL | 33,9 | LMTAVQMAI | 24,7 |
| LLNRPEPL | 39,1 | LMTAVQMAL | 13,0 |
| LLQRPLVTA | 34,9 | LMTAVQMAV | 8,5 |
| LLQRPLVTI | 46,0 | LMTAVQMAA | 19,2 |
| LLQRPLVTL | 21,6 | LMAARTVEA | 60,5 |
| LLTAVQMAI | 17,5 | LMAARTVEI | 80,9 |
| LLTAVQMAL | 9,8 | LMAARTVEL | 36,4 |
| LLTAVQMAA | 14,0 | LMAARTVEV | 20,4 |
| LLAARTVEA | 39,7 | LNFPISPL | 94,9 |
| LLAARTVEI | 52,2 | LNFPISPV | 70,4 |

Table 5D   (continued)

| Peptide | Kd nM | Peptide | Kd nM |
|---|---|---|---|
| LLAARTVEL | 24,7 | LQAEIQKL | 63,9 |
| LMAEIQKL | 40,5 | LQAEIQKV | 47,5 |
| LMAEIQKV | 30,7 | LQFGWCFKL | 66,1 |
| LMEVVQKV | 97,7 | LQFGWCFKV | 34,5 |
| LMFGWCFKA | 19,1 | LQFPISPL | 41,8 |
| LMFGWCFKI | 24,1 | LQFPISPV | 32,0 |
| LMFGWCFKL | 13,1 | LQGRWPVKV | 49,0 |
| LMFGWCFKV | 8,4 | LQIPHPAGL | 68,7 |
| LMFPISPI | 94,6 | LQIPHPAGV | 35,9 |
| LMFPISPL | 28,4 | LQNRPEPV | 73,0 |
| LMFPISPV | 22,2 | LQTAVQMAL | 68,8 |
| LMGPTPVNA | 53,8 | LQTAVQMAV | 36,2 |
| LVGPTPVNV | 84,1 | PMGDIYKA | 67,8 |
| LVITTYWGV | 90,9 | PMGDIYKI | 38,2 |
| MIQRGNFKV | 71,5 | PMGDIYKL | 13,8 |
| MLLGMLMIA | 56,8 | PMGDIYKR | 77,4 |
| MLLGMLMII | 73,8 | PMGDIYKV | 10,9 |
| MLLGMLMIL | 34,4 | PMGEIYKI | 62,4 |
| MLNNPPIPA | 54,9 | PMGEIYKL | 20,3 |
| MLNNPPIPL | 32,2 | PMGEIYKV | 15,7 |
| MLQRGNFKA | 31,4 | PQGDIYKI | 57,5 |
| MLQRGNFKI | 40,8 | PQGDIYKL | 18,8 |
| MLQRGNFKL | 20,4 | PQGDIYKV | 14,6 |
| MLQRGNFRA | 48,0 | PQGEIYKL | 30,5 |
| MLQRGNFRI | 63,8 | PQGEIYKV | 22,9 |
| MLQRGNFRL | 29,0 | PVGDIYKI | 88,0 |
| MLSNPPIPA | 66,8 | PVGDIYKL | 26,9 |
| MLSNPPIPL | 38,4 | PVGDIYKV | 20,5 |
| MMLGMLMIA | 86,2 | PVGEIYKL | 44,1 |
| MMLGMLMIL | 50,7 | PVGEIYKV | 32,7 |
| MMLGMLMIV | 27,0 | QIGIPHPI | 96,8 |
| MMNNPPIPL | 47,8 | QIGIPHPL | 28,2 |
| MMNNPPIPV | 25,8 | QIGIPHPV | 21,9 |
| MMQRGNFKA | 46,4 | OLGIPHPI | 63,5 |
| MMQRGNFKI | 61,4 | QLGIPHPL | 20,0 |
| MMQRGNFKL | 29,1 | QLTEAVQKL | 92,7 |
| MMQRGNFKV | 16,6 | QMGIPHPI | 99,5 |
| MMQRGNFRA | 73,4 | QMGIPHPL | 28,9 |
| MMQRGNFRI | 99,8 | QMGIPHPV | 22,4 |
| MMQRGNFRL | 42,7 | QMTEAVQKV | 71,0 |
| MMQRGNFRV | 23,3 | QQGIPHPL | 47,0 |
| MMSNPPIPL | 57,5 | QQGIPHPV | 35,5 |
| MMSNPPIPV | 30,9 | RIAEPVPL | 72,0 |
| MQQRGNFKV | 94,7 | RIAEPVPV | 54,0 |
| NLVATLYCL | 69,6 | RIIEAQQHV | 84,1 |
| NLWATHACL | 99,6 | RLAEPVPL | 48,4 |
| NMADQLIHV | 60,8 | RLAFHHMAI | 61,4 |
| NMVATLYCV | 54,4 | RLAFHHMAL | 28,4 |
| NMWATHACV | 77,3 | RLAFHHMAA | 45,9 |
| PIGDIYKA | 66,0 | RLHPVQAGA | 53,6 |

Table 5D   (continued)

| Peptide | Kd nM | Peptide | Kd nM |
|---|---|---|---|
| PIGDIYKI | 37,9 | RLHPVQAGI | 71,3 |
| PIGDIYKL | 13,7 | RLHPVQAGL | 31,2 |
| PIGDIYKR | 75,2 | RLIEAQQHA | 36,1 |
| PIGDIYKV | 11,0 | RLIEAQQHI | 46,5 |
| PIGEIYKI | 61,4 | RLIEAQQHL | 22,9 |
| PIGEIYKL | 20,2 | RLYSPVSIL | 67,1 |
| PIGEIYKV | 15,7 | RMAEPVPL | 73,4 |
| PLGDIYKA | 45,2 | RMAEPVPV | 54,8 |
| PLGDIYKI | 24,9 | RMAFHHMAI | 96,4 |
| PLGDIYKL | 9,8 | RMAFHHMAL | 42,3 |
| PLGDIYKR | 50,7 | RMAFHHMAV | 23,1 |
| PLGEIYKA | 96,1 | RMAFHHMAA | 70,9 |
| PLGEIYKI | 40,9 | RMHPVQAGA | 82,5 |
| PLGEIYKL | 14,3 | RMHPVQAGL | 46,5 |
| RMHPVQAGV | 25,6 | TINPPIPL | 52,2 |
| RMIEAQQHA | 54,8 | TINPPIPV | 39,1 |
| RMIEAQQHI | 72,0 | TINPPIPV | 39,1 |
| RMIEAQQHL | 33,5 | TLGWCFKL | 35,5 |
| RMIEAQQHV | 18,8 | TLGAASITL | 80,6 |
| RMYSPVSIV | 51,5 | TLNDIQKI | 61,2 |
| RQAEPVPV | 95,1 | TLNDIQKL | 19,8 |
| SIGDPEIL | 31,9 | TLNPPIPL | 34,9 |
| SIGDPEIV | 24,7 | TLNPPIPL | 34,9 |
| SLAEEEVVL | 90,7 | TLQEQIAWL | 62,2 |
| SLEEMMTAL | 61,9 | TMGWCFKL | 51,7 |
| SLGDPEII | 69,4 | TMGWCFKV | 38,6 |
| SLGDPEIL | 21,4 | TMGAASITV | 62,6 |
| SLGQHIYEA | 53,8 | TMNDIQKL | 29,7 |
| SLGQHIYEI | 70,5 | TMNDIQKV | 22,7 |
| SLGQHIYEL | 31,7 | TMNPPIPL | 53,0 |
| SLGQYIYEA | 97,8 | TMNPPIPL | 53,0 |
| SLGQYIYEL | 54,7 | TMNPPIPV | 39,6 |
| SLIYAGIKA | 85,6 | TMNPPIPV | 39,6 |
| SLIYAGIKL | 51,5 | TMQEQIAWL | 96,7 |
| SLIYPGIKA | 62,6 | TMQEQIAWV | 48,2 |
| SLIYPGIKI | 81,9 | TMQEQIGWV | 80,1 |
| SLIYPGIKL | 37,7 | TQGWCFKL | 91,6 |
| SLVKHHMYA | 77,5 | TQGWCFKV | 66,4 |
| SLVKHHMYL | 48,0 | TQNDIQKL | 44,4 |
| SLWNWFSIL | 77,8 | TQNDIQKV | 33,0 |
| SMAEEEVVV | 70,5 | TQNPPIPL | 89,8 |
| SMEEMMTAL | 96,9 | TQNPPIPL | 89,8 |
| SMEEMMTAV | 49,4 | TQNPPIPV | 65,2 |
| SMGDPEIL | 32,7 | TQNPPIPV | 65,2 |
| SMGDPEIV | 25,1 | TVNDIQKV | 49,2 |
| SMGQHIYEA | 83,4 | VLAEAMSQL | 78,1 |
| SMGQHIYEL | 47,6 | VMAEAMSQV | 60,7 |
| SMGQHIYEV | 25,3 | VMVYYGVPV | 92,0 |
| SMGQYIYEL | 84,9 | WIFKLVPV | 78,9 |
| SMGQYIYEV | 42,7 | WLFKLVPL | 65,9 |

Table 5D   (continued)

| Peptide | Kd nM | Peptide | Kd nM |
|---------|-------|---------|-------|
| SMIYAGIKL | 78,3 | WMFKLVPV | 79,9 |
| SMIYAGIKV | 40,9 | YCAPAGFAL | 83,0 |
| SMIYPGIKA | 96,8 | YCAPAGFAV | 42,5 |
| SMIYPGIKL | 56,4 | YCAPAGYAV | 80,7 |
| SMIYPGIKV | 30,3 | YIAPAGFAI | 88,0 |
| SMVKHHMYL | 72,0 | YIAPAGFAL | 39,9 |
| SMVKHHMYV | 38,4 | YIAPAGFAV | 22,1 |
| SMWNWFSIV | 60,7 | YIAPAGFAA | 66,8 |
| SMYNTVATV | 49,7 | YIAPAGYAL | 75,5 |
| SQGDPEIL | 49,8 | YIAPAGYAV | 38,9 |
| SQGDPEIV | 37,4 | YISNPYPI | 71,5 |
| TIGWCFKL | 50,9 | YISNPYPL | 22,4 |
| TIGWCFKV | 38,2 | YISNPYPV | 17,6 |
| TINDIQKL | 29,7 | YLAPAGFAI | 14,0 |
| TINDIQKV | 22,7 | YLAPAGFAL | 8,5 |
| TINPPIPL | 52,2 | YLAPAGFAA | 11,6 |
| YLAPAGYAI | 23,4 | AMYAPPIKL | 19,7 |
| YLAPAGYAL | 13,0 | AQYAPPIKV | 62,4 |
| YLAPAGYAA | 18,8 | KLMAGADCL | 68,4 |
| YLAWVPAHA | 43,4 | KMMAGADCV | 53,3 |
| YLAWVPAHI | 57,0 | LIVRTYWGV | 69,2 |
| YLAWVPAHL | 26,3 | LLVARIVEA | 52,0 |
| YLKIFIMIL | 62,8 | LLVARIVEI | 68,7 |
| YLSNPYPI | 45,8 | LLVARIVEL | 31,0 |
| YLSNPYPK | 83,8 | LLVRTYWGA | 30,9 |
| YLSNPYPL | 15,3 | LLVRTYWGI | 38,9 |
| YMAFTIPSV | 81,0 | LLVRTYWGL | 19,3 |
| YMALQDSGV | 69,5 | LLVTTYWGA | 62,5 |
| YMAPAGFAI | 19,9 | LLVTTYWGI | 80,9 |
| YMAPAGFAL | 11,4 | LLVTTYWGL | 36,1 |
| YMAPAGFAV | 7,5 | LMVARIVEA | 79,6 |
| YMAPAGFAA | 16,1 | LMVARIVEL | 45,9 |
| YMAPAGYAI | 34,9 | LMVARIVEV | 25,3 |
| YMAPAGYAL | 18,2 | LMVRTYWGA | 45,9 |
| YMAPAGYAV | 11,1 | LMVRTYWGI | 58,9 |
| YMAPAGYAA | 27,4 | LMVRTYWGL | 27,6 |
| YMAWVPAHA | 65,7 | LMVRTYWGV | 15,8 |
| YMAWVPAHI | 88,5 | LMVTTYWGA | 96,4 |
| YMAWVPAHL | 38,5 | LMVTTYWGL | 54,3 |
| YMAWVPAHV | 21,4 | LMVTTYWGV | 28,8 |
| YMKIFIMIL | 96,9 | LQVRTYWGV | 90,7 |
| YMKIFIMIV | 48,9 | LWRTYWGV | 71,3 |
| YMSNPYPI | 71,4 | MLSNPPVPA | 60,0 |
| YMSNPYPL | 22,2 | MLSNPPVPL | 35,1 |
| YMSNPYPV | 17,3 | MMSNPPVPL | 52,5 |
| YQAPAGFAL | 48,1 | MMSNPPVPV | 28,4 |
| YQAPAGFAV | 25,9 | NLVAVLYCL | 73,8 |
| YQAPAGFAA | 84,4 | NMVAVLYCV | 57,7 |
| YQAPAGYAL | 93,2 | QMTEWQKV | 85,3 |
| YQAPAGYAV | 46,9 | SMAEEEIIV | 89,3 |

Table 5D   (continued)

| Peptide | Kd nM | Peptide | Kd nM |
|---------|-------|---------|-------|
| YQSNPYPL | 33,2 | VLAAIIAIA | 56,4 |
| YQSNPYPV | 25,3 | VLAAIIAII | 75,3 |
| AIYAPPIAV | 84,5 | VLAAIIAIL | 33,4 |
| AIYAPPIKL | 89,8 | VMAAIIAIA | 87,2 |
| AIYAPPIKV | 46,3 | VMAAIIAIL | 49,8 |
| ALYAPPIAA | 36,7 | VMAAIIAIV | 27,2 |
| ALYAPPIAI | 48,7 | YLRIFIMIL | 82,2 |
| ALYAPPIAL | 22,6 | YMRIFIMIV | 64,0 |
| ALYAPPIAV | 13,9 | | |
| ALYAPPIKA | 21,9 | | |
| ALYAPPIKI | 27,9 | | |
| ALYAPPIKL | 14,6 | | |
| ALYAPPIKV | 9,4 | | |
| AMYAPPIAA | 53,9 | | |
| AMYAPPIAI | 72,7 | | |
| AMYAPPIAL | 32,0 | | |
| AMYAPPIKA | 30,8 | | |
| AMYAPPIKI | 40,0 | | |

**Table 5E:**

8-mers and 9-mers representing new natural intermediate binders (predicted binding $IC_{50}$ = 50 - 500 nM) with a global conservation among HIV strains above 8% and which cannot readily be improved by changing of anchor position amino acids.

Table 5E

| Natural epitope | predicted Kd (nM) | HXB2 mapping | % of global conservation |
|---------|---------|---------|---------|
| IIRILQQL | 480,5 | Vpr(60-67) | 60,7 |
| DLADQLIHL | 322,5 | Vif(101-109) | 28,3 |
| SLFGNDPL | 451,6 | Pr55(491-498) | 19,8 |
| SLFGSDPL | 272,2 | Pr55(491-498) | 12,5 |
| ALGTGATL | 386,8 | Pr55(336-343) | 9,4 |
| ALQDSGSEV | 189,3 | Pol(640-648) | 37,2 |
| ELQAIQLAL | 268,4 | Pol(633-641) | 8,1 |
| ELQAIQLAL | 268,4 | Pol(633-641) | 8,1 |
| NLAFQQGEA | 407,8 | Pol(5-13) | 23,3 |
| KLVDFREL | 477,7 | p51(73-80) | 98,8 |
| ALTDIVTL | 278,2 | p51(288-295) | 10,5 |
| ALTEVVPL | 277,1 | p51(288-295) | 10,5 |
| ELHPDKWTV | 290,4 | P51(233-241) | 91,9 |
| VILVAVHV | 272,7 | p31(72-79) | 44,2 |
| IILVAVHV | 175,3 | p31(72-79) | 39,5 |
| PLWKGPAKL | 462 | p31(233-241) | 32,6 |
| RQGFERAL | 420,1 | gp160(848-855) | 13,6 |
| RQGFERAL | 420,1 | gp160(848-855) | 13,6 |
| SLLNATAIA | 434,5 | gp160(813-821) | 24,9 |
| SLLNATAIA | 434,5 | gp160(813-821) | 24,9 |
| ALKYWWNLL | 374,4 | gp160(792-800) | 17,8 |
| LIVARIVEL | 268,1 | gp160(776-784) | 8,5 |

Table 5E   (continued)

| Natural epitope | predicted Kd (nM) | HXB2 mapping | % of global conservation |
|---|---|---|---|
| RLRDFILIA | 437 | gp160(770-778) | 9,4 |
| RLRDFILIA | 437 | gp160(770-778) | 9,4 |
| FLALAWDDL | 317,2 | gp160(752-760) | 26,3 |
| FLAIFWVDL | 240,5 | gp160(752-760) | 8,9 |
| RLVSGFLAL | 479,9 | gp160(747-755) | 22,5 |
| LQARVLAV | 487,6 | gp16O(576-583) | 57,3 |
| QLQARVLAV | 129,4 | gp160(575-583) | 57,3 |
| RLISCNTSV | 103,5 | gp160(192-200) | 20,7 |
| RLINCNTSV | 123,6 | gp160(192-200) | 9,4 |
| ALFYKLDW | 180,4 | gp160(174-182) | 23 |

*Table 6:*

Complete 8-mer, 9-mer and 10-mer sets of PSCPL were synthesised as described in the text.

## Table 6: An example of a PSCPL generated matrix (HLA-A2- 9-mer)

|  | Position 1 | Position 2 | Position 3 | Position 4 | Position 5 | Position 6 | Position 7 | Position 8 | Position 9 |
|---|---|---|---|---|---|---|---|---|---|
| A | 0.755 | 0.377 | 1.081 | 0.391 | 0.592 | 0.203 | 0.799 | 1.593 | 2.498 |
| C | 0.318 | 0.018 | 0.086 | 0.291 | 0.586 | 0.234 | 0.350 | 0.185 | 0.097 |
| D | 0.024 | 0.012 | 0.428 | 2.711 | 0.779 | 0.279 | 0.428 | 0.508 | 0.085 |
| E | 0.057 | 0.011 | 0.178 | 1.335 | 0.609 | 0.371 | 0.748 | 1.425 | 0.192 |
| F | 4.617 | 0.151 | 1.786 | 1.627 | 1.799 | 2.457 | 3.846 | 2.232 | 0.614 |
| G | 0.578 | 0.096 | 0.514 | 0.723 | 0.566 | 0.274 | 0.266 | 0.677 | 0.287 |
| H | 0.428 | 0.025 | 0.622 | 1.132 | 0.905 | 0.802 | 0.654 | 1.098 | 0.081 |
| I | 0.953 | 1.458 | 1.040 | 0.723 | 1.267 | 2.649 | 1.253 | 0.478 | 3.843 |
| K | 0.697 | 0.014 | 0.197 | 0.469 | 0.396 | 0.130 | 0.230 | 0.328 | 0.092 |
| etc | etc | etc | etc | etc | etc | etc | etc | etc | etc |
| Y | 3.439 | 0.051 | 2.267 | 0.840 | 0.931 | 0.516 | 1.103 | 1.288 | 0.097 |

*Table 7:*

HIV-1 protein sequences from which HLA-A2 epitopes were predicted and their distribution within the genetic subtypes composing group M (subtypes A, AB, AC, AD, ADI, AE, AG, AGI, AGJ, B, BF, C, CD, D, F, G, H, J) or within the groups N or group O. HIV-1-related sequences such as SIVcpz were included since SIVcpz viruses share a high genetic homology with HIV-1 group N in Env.

## Table 7: Number of available HIV-1 sequences in Los Alamos 1998-1999 HIV protein sequences Database according to genetic groupes and subtypes

| HIV-1 Groups | | | | | | | | | | | | | | | | | | | | | M | N | O | CPZ |
|---|---|---|---|---|---|---|---|---|---|---|---|---|---|---|---|---|---|---|---|---|---|---|---|---|
| subtypes | in all DB | A | AB | AC | AD | ADI | AE | AG | AGI | AGJ | B | BF | C | CD | D | F | G | H | J | | | O | CPZ |
| GAG | 96 | 8 | 0 | 6 | 2 | 1 | 3 | 5 | 4 | 1 | 32 | 1 | 8 | 0 | 5 | 6 | 3 | 3 | 2 | 1 | | | 2 | 3 |
| POL | 86 | 8 | 0 | 5 | 1 | 1 | 3 | 5 | 4 | 1 | 28 | 1 | 8 | 0 | 5 | 2 | 3 | 3 | 2 | 1 | | | 2 | 3 |
| VIF | 265 | 20 | 0 | 4 | 1 | 1 | 3 | 5 | 4 | 1 | 165 | 1 | 8 | 0 | 23 | 2 | 3 | 3 | 2 | 1 | | | 15 | 3 |
| VPR | 173 | 8 | 0 | 4 | 1 | 1 | 3 | 5 | 4 | 1 | 103 | 1 | 8 | 0 | 5 | 2 | 3 | 3 | 2 | 1 | | | 15 | 3 |
| VPU | 156 | 12 | 2 | 6 | 1 | 1 | 3 | 6 | 4 | 1 | 63 | 1 | 13 | 0 | 16 | 4 | 3 | 3 | 2 | 1 | | | 11 | 3 |
| TAT | 101 | 10 | 0 | 5 | 1 | 1 | 3 | 5 | 4 | 1 | 35 | 1 | 10 | 0 | 7 | 4 | 3 | 3 | 2 | 1 | | | 2 | 3 |
| REV | 105 | 11 | 1 | 5 | 1 | 1 | 3 | 5 | 4 | 1 | 34 | 1 | 12 | 0 | 8 | 4 | 3 | 3 | 2 | 1 | | | 2 | 3 |
| ENV | 213 | 16 | 1 | 5 | 5 | 1 | 9 | 8 | 4 | 1 | 100 | 1 | 22 | 1 | 14 | 4 | 4 | 3 | 2 | 1 | | | 8 | 3 |
| NEF | 251 | 8 | 0 | 3 | 2 | 1 | 22 | 5 | 2 | 1 | 167 | 1 | 20 | 0 | 4 | 2 | 3 | 3 | 2 | 1 | | | 2 | 3 |

EP 1 250 351 B1

*Table 8:*

Shows that the HIV-1 subtype B sequences often dominated in the database that was used for predicting HLA-A2 epitopes.

## Table 8: Percentage of available HIV-1 sequences in The Los Alamos 1998-1999 HIV sequences Database, according to genetic subtypes and viral proteins.

| | HIV-1/M | | | | | | | | | | | | | | | | | HIV-1/N | HIV-1/O |
|------|-------|------|------|------|------|------|------|-------|------|-------|-------|-------|----|-------|------|------|------|------|------|------|
| | A | AB | AC | AD | ADI | AE | AG | AGI | AGJ | B | BF | C | CD | D | F | G | H | J | | |
| GAG | 8,33 | 0 | 6,25 | 2,08 | 1,04 | 3,12 | 5,2 | 4,165 | 1,04 | 33,3 | 1,045 | 8,33 | 0 | 5,2 | 6,25 | 3,12 | 3,12 | 2.08 | 1,04 | 2,08 |
| POL | 9,3 | 0 | 5,81 | 1,16 | 1,16 | 3,5 | 5,81 | 4,65 | 1,16 | 32,55 | 1,16 | 9,3 | 0 | 5,81 | 2,3 | 3,49 | 3,49 | 2,3 | 1,16 | 2,3 |
| VIF | 7,55 | 0 | 1,5 | 0,3 | 0,3 | 1,13 | 1,88 | 1,5 | 0,3 | 62,26 | 0,3 | 3 | 0 | 8.68 | 0,75 | 1,13 | 1,13 | 0,75 | 0,38 | 5,66 |
| VPR | 4,62 | 0 | 2,3 | 0,5 | 0,5 | 1,7 | 2,89 | 2,3 | 0,5 | 59,54 | 0,5 | 4,62 | 0 | 2,89 | 1,15 | 1,73 | 1,73 | 1,15 | 0,57 | 8,67 |
| VPU | 7,69 | 1,28 | 3,84 | 0,64 | 0,64 | 1,92 | 3,84 | 2,56 | 0,64 | 40,38 | 0,64 | 8,33 | 0 | 10,25 | 2,56 | 1,92 | 1,92 | 1,28 | 0,64 | 7,05 |
| TAT | 9,9 | 0 | 4,95 | 0,99 | 0,99 | 2,97 | 5,95 | 3,96 | 0,99 | 34,65 | 0,99 | 9,9 | 0 | 6,93 | 3,96 | 2,97 | 2,97 | 1,98 | 0,99 | 1,98 |
| REV | 10,47 | 0,95 | 4,76 | 0,95 | 0,95 | 2,85 | 4,76 | 3,8 | 0,95 | 32,38 | 0,95 | 11,42 | 0 | 7,6 | 3,8 | 2,85 | 2,85 | 1,9 | 0,95 | 1,9 |
| ENV | 7,5 | 0,46 | 2,35 | 2,35 | 0,46 | 4,22 | 3,75 | 1,87 | 0,46 | 46,95 | 0,46 | 10,33 | 0,46 | 6,57 | 1,88 | 1,88 | 1,4 | 0,93 | 0,4 | 3,75 |

EP 1 250 351 B1

*Tables 9 and 10:*

Based on the criteria described in the text, 53 out of 354 epitopes were selected to be incorporated in a synthetic polytope vaccine.
They are divided in 8 sets:

Set 1: 4 epitopes located in Vpu
Set 2: 4 epitopes located in Vpr
Set 3: 6 epitopes located in Vif
Set 4: 4 epitopes located in Rev
Set 5: 5 epitopes located in Nef
Set 6: 10 epitopes located in Pol from which 4 can be located in the Reverse Transcriptase polypeptide (p51) and one epitope in the integrase polypeptide (p31).
Set 7: 10 epitopes located in Gag
Set 8: 10 epitopes located in Env

For each epitope the improved epitope or the related natural epitope with measured good binding ($IC_{50}$<100nM) were be chosen.

# Table 9: example of epitopes candidates for a "cover-all" vaccine

| NAME | EPITOPE | Kd (nM) | HXB2 MAPPING | RELATED NATURAL | GLOBAL | A | B | C | D | AE | F | G | H | J | N | O |
|---|---|---|---|---|---|---|---|---|---|---|---|---|---|---|---|---|
| 4.8 | IVGLIVAL | 67,8 | Vpu(9-16) | | 3,2 | 16,6 | | | | 66,6 | | | | | | |
| 8.1 | VLAAIIAIV | 19.1 | vpu(13-21 | VVAAIIAIV | 9,6 | | 22 | | 6 | | | | | | | |
| 8.2 | ILAIVVWTV | 12.1 | Vpu(17-25) | I(I/L)AIVVWTI | 41,8 | 74 | 35 | 54 | 87,4 | | | | | | | |
| 8.3 | ALVEMGHHV | 35.5 | Vpu(66-74) | ALVEMGHHA | 17,3 | 8 | 35 | | 25 | | | | | | | |
| 4.13 | LHGLGQYV | 96,4 | Vpr(39-46) | | 4,0 | | | 62,5 | | | | | | 50 | | |
| 8.4 | FLRPWLHGV | 10.2 | Vpr(34-42) | FPRPWLHGL | 17,3 | 62,5 | | | | | | | | | | |
| 8.5 | SLGQHIYEV | 17.8 | Vpr(41-49) | SLGQHIYET | 19,7 | | 29 | | 40 | | | | | | | |
| 8.99 | LLFIHFRV | 92.5 | vpr(67-74 | LLFIHFRI | 69,9 | 12,5 | 87,4 | 62,5 | 100 | | 100 | 100 | 66,6 | 100 | | |
| 4.14 | SLVKHHMYV | 26,6 | Vif(23-31) | SLVKHHMYI | 61,8 | 90 | 64 | 75,0 | 69,5 | 66,6 | | 33,3 | 33,3 | 100 | | |
| 8.7 | LLITTYWGL | 24 | Vif(64-72) | LVITTYWGL | 38,5 | | 39 | 12,5 | | | | | | | | |
| 8.10 | KLKPPLPSV | 48.8 | Vif(158-166) | K(I/T)KPPLPSV | 56,3 | 35 | 12 | 25 | 87 | | | | | | | |
| 8.11 | GLADQLIHV | 47.0 | Vif(101-109) | GLADQLIH(L/M) | 23,4 | | 25 | 87,5 | 91 | | | | | | | |
| 8.12 | ALAALITPV | 11.9 | Vif(149-157) | ALAALITPK | 18,5 | | 29,6 | | | | | | | | | |
| 8.102 | KLGSLQYV | 29 | Vif(141-148) | KVGSLQYL | 82,3 | 95 | 89,7 | 75 | 100 | 100 | 100 | 66,6 | 33,3 | 100 | | |
| 8.103 | RLAEPVPV | 36.8 | Rev(66-73) | RSAEPVPL | 19 | 9 | 23,5 | 25 | 12,5 | | | 33,3 | | | | |
| 8.14 | LLLPPIERV | 19.5 | Rev(73-81) | LQLPPIERL | 15,2 | 36 | | 42 | | | | | | | | |
| 8.15 | GLGSPQILV | 37.7 | Rev(96-104) | G(V/M)GSPQILV | 18,1 | | 52,8 | | | | | | | | | |

%of COVERAGE — GLOBAL — INTRA-SUBTYPES

| NAME | EPITOPE | Kd (nM) | HXB2 MAPPING | RELATED NATURAL | GLOBAL | A | B | C | D | AE | F | G | H | J | N | O |
|---|---|---|---|---|---|---|---|---|---|---|---|---|---|---|---|---|
| 8.16 | ILVESPAVV | 82.6 | Rev(102-110) | ILVESPAVL | 8,6 | | 26.4 | | | | | | | | | |
| 8.43 | ALNADCAWV | 44.1 | Nef(50-58) | ATNADCAWL | 20,3 | | 28 | 15 | | | | | | | | |
| 8.44 | FLVRPQVPV | 6 | Nef(68-76) | FPVRPQVPL | 74,9 | 62 | 73 | 90 | | | | | | | | |
| 8.45 | LLFGWCFKL | 10 | Nef(137-145) | L(T/C)FGWCFKL | 79,2 | 100 | 78 | 100 | | 95,4 | 50 | 100 | 100 | 50 | | |
| 8.121 | WLFKLVPV | 49.9 | Nef(141-148) | WCFKLVPV | 82,5 | 87,5 | 87,4 | 100 | | 100 | 100 | | 66,6 | 50 | | |
| 8.47 | RLAFHHMAV | 16,2 | Nef(188-196) | RLAFHHMAR | 26,3 | | 40 | | | | | | | | | |

%of COVERAGE — INTRA-SUBTYPES

## Table 10: example of epitopes candidates for a "cover-all" vaccine

| | | | | | | | | | | %of COVERAGE | | | | | | |
|---|---|---|---|---|---|---|---|---|---|---|---|---|---|---|---|---|
| NAME | EPITOPE | Kd (nM) | HXB2 MAPPING | RELATED NATURAL | GLOBAL | | | | | INTRA-SUBTYPES | | | | | | |
| | | | | | | A | B | C | D | AE | F | G | H | J | N | O |
| 8.17 | ALVEICTEV | 31.8 | p51(33-41) | ALVEICTEM | 26,7 | | 78,5 | | | | | | | | | |
| 8.21 | FLWMGYELV | 29 | p51(227-235) | FLWMGYELH | 97,7 | 87,5 | 100 | 100 | 100 | 100 | 100 | 100 | 100 | 100 | 100 | 100 |
| 8.111 | FLNTPPLV | 26 | p51(416-423) | FVNTPPLV | 86 | 100 | 89 | 100 | 80 | 100 | 50 | 100 | | 100 | 100 | |
| 8.30 | KLWYQLEKV | 17.7 | p51(424-432) | KLWYQLEK(E/D) | 71 | 88 | 87,5 | 50 | 100 | 100 | | | | 100 | | |
| 8.33 | LLTAVQMAV | 7 | P31(172-180) | LKTAVQMAV | 90,7 | 100 | 96 | 87,5 | 100 | 100 | 100 | 100 | 33,3 | 100 | 100 | 50 |
| 8.37 | ILLWQRPLV | 74,1 | Pol(59-67) | ITLWQRPLV | 73,3 | 87,5 | 75 | 100 | 60 | 100 | 100 | 66,6 | 66,6 | 100 | | |
| 8.115 | LLFPISPV | 15 | Pol(153-160) | LNFPISPI | 93 | 100 | 96,4 | 100 | 100 | 100 | 50 | 100 | 66,6 | 100 | 100 | 100 |
| 8.41 | KLGKAGYVV | 26 | Pol(606-614) | KLGKAGYVT | 57 | 87,5 | 78,5 | | 80 | 66,6 | | 100 | 33,3 | | | 100 |
| 8.116 | ALGIIQAV | 13 | Pol(657-664) | ALGIIQAQ | 84,9 | 87,5 | 93 | 87,5 | 100 | 100 | 100 | 100 | 100 | 100 | | |
| 8.42 | YLAWVPAHV | 15.4 | Pol(687-695) | YLAWVPAHK | 38,4 | 12,5 | 96,4 | | 80 | | | | | | | |
| 8.49 | NLVATLYCV | 36 | Pr55(80-88) | NTVATLYCV | 57,3 | 62,5 | 69 | 87 | 80 | | | 66,6 | 33,3 | 100 | | |
| 8.54 | RLLNAWVKV | 73,2 | pr55(150-158) | RTLNAWVKV | 94,8 | 100 | 100 | 100 | 100 | 100 | 100 | 100 | 100 | 100 | 100 | |
| 8.56 | TLQEQIGWV | 42,3 | Pr55(242-250) | TLQEQIGWM | 43,8 | | 87,5 | | 20 | 66,6 | | | | 50 | | |
| 8.123 | TLNPPIPV | 26.4 | Pr55(251-258) | T(N/S/G)NPPIPV | 68,8 | 100 | 87,4 | | 80 | 100 | 17 | 66 | | | | |
| 8.124 | PLGEIYKV | 11.3 | Pr55(257-264) | PVGEIYKR | 61,5 | | 93,8 | 25 | 80 | | 50 | 100 | | 100 | | |
| 8.64 | ALGPAATLV | 14.9 | Pr55(336-344) | ALGPAATLE | 26 | | 72 | | | | | | | | | |
| 8.65 | ALLEEMMTV | 26.6 | Pr55(341-349) | ATLEEMMTA | 75 | 87 | 97 | | 80 | 100 | 83,3 | 66,6 | | 100 | 100 | |

EP 1 250 351 B1

| NAME | EPITOPE | Kd (nM) | HXB2 MAPPING | RELATED NATURAL | GLOBAL | %of COVERAGE INTRA-SUBTYPES | | | | | | | | | | | |
|---|---|---|---|---|---|---|---|---|---|---|---|---|---|---|---|---|---|
| | | | | | | A | B | C | D | AE | F | G | H | J | N | O |
| 8.66 | ELMTACQGV | 85 | pr55(345-353) | EMMTACQGV | 91,7 | 87,5 | 100 | 100 | 80 | 100 | 83,3 | 66,6 | | 100 | 100 | |
| 4.149 | VLAEAMSQV | 40,3 | Pr55(362-370) | VLAEAMSQA | 49 | 87,5 | 71,8 | 12,5 | 20 | | | | 100 | 100 | 100 | |
| 8.127 | FLGKIWPV | 4 | Pr55(433-440) | FLGKIWPS | 84,4 | 75 | 93,8 | 87,5 | 80 | 100 | 100 | 100 | 100 | 100 | | |
| 8.74 | VLVYYGVPV | 60 | gp160(35-44) | VTVYYGVPV | 90,1 | 87,5 | 98 | 91 | 85,7 | 100 | 75,0 | 100 | 100 | 100 | 100 | |
| 8.75 | NLWATHACV | 50 | gp160(67-75) | N(V/I)WATHACV | 90,7 | 100 | 94 | 95,4 | 85,5 | 88 | 100 | 100 | 100 | 100 | | |
| 8.76 | KLTPLCVTV | 57 | gp160(121-129) | KLTPLCVTL | 82,2 | 87,5 | 87 | 95,4 | 85,7 | 77,7 | 100 | 100 | 100 | | | |
| 8.85 | TLGAASITV | 41 | gp160(529-537) | TMGAASITL | 46,9 | 81 | 25 | 100 | | 100 | 75 | 75 | 100 | 100 | 100 | |
| 8.88 | ALEAQQHLV | 14 | gp160(558-566) | AIEAQQHLL | 70 | 81,2 | 77 | 27,3 | 78,5 | 88,8 | 75 | 100 | | 50 | 100 | |
| 4.180 | LLQLTVWGI | 38 | gp160(565-573) | | 58,2 | | 80 | 27,3 | 92,8 | 88,9 | 75 | 100 | | | | |
| 8.93 | YLKIFIMIV | 33 | gp160(681-689) | YIKIFIMIV | 60,1 | 37,5 | 68 | 72,7 | 57,1 | 66,6 | 100 | 75 | 33,3 | | | |
| 8.129 | ILGDIRQV | 15.1 | gp160(322-329) | IIGDIRQA | 47,4 | 43,7 | 58 | 86,3 | | | | 50 | 33,3 | | | |
| 8.130 | SLGDPEIV | 16.8 | gp160(365-372) | SGGDPEIV | 37,6 | | 79 | | | | | | | | | |
| 8.135 | FLGAAGSV | 30 | gp160(522-529) | FLGAAGST | 88,3 | 94 | 96 | 95,4 | 78,6 | 100 | 25 | 75 | 100 | | 100 | |

*Table 11:*

Table 11 shows an example of predicted and measured Kd of epitopes from tables 5 and 6 "cover all" epitopes. Peptides corresponding to epitopes from tables 5 and 6 including related natural versions were synthezised and binding to HLA-A2 MHC-I measured *in vitro* as described in example 1. Results are sorted by increasing Kd values for Related natural epitopes. Measured binding values from Related Natural Epitopes (right side of the table) can be improved by anchor optimizations (Epitopes, left site of the table) as predicted.

## Table 11

| Name | Epitope | Predicted Kd (nM) | Measured Kd(nM) | Name | Related Natural | Predicted Kd (nM) | Measured Kd(nM) | Gene |
|---|---|---|---|---|---|---|---|---|
| 8.11mod | GLADQLIHV | 47.0 | 32 | 8.11natL (4.20) | GLADQLIHL | 90,6 | 3 | vif |
| 8.5mod | SLGQHIYEV | 17.8 | 6 | 8.5nat | SLGQHIYET | 161 | 5 | vpr |
| 8.14mod | LLLPPIERV | 19.5 | 4,4 | 8.14nat | LQLPPIERL | 422,6 | 7,3 | rev |
| 8.127mod | FLGKIWPV | 4 | 3,3 | 8.127nat (4.158) | FLGKIWPS | 38,6 | 10 | gag |
| 4.180 | LLQLTVWGI | 38 | 12 | | | | | env |
| 8.16mod | ILVESPAVV | 82.6 | 23 | 8.16nat | ILVESPAVL | 169,7 | 13 | rev |
| 8.74mod | VLVYYGVPV | 60 | 4,1 | 8.74nat | VTVYYGVPV | 299,2 | 15 | env |
| 8.45mod | LLFGWCFKL | 10 | 25 | 8.45natT | LTFGWCFKL | 29,6 | 17 | nef |
| 8.93mod | YLKIFIMIV | 33 | 13 | 8.93nat | YIKIFIMIV | 286,3 | 24 | env |
| 4.149nat | VLAEAMSQV | 40,3 | 2 | 4.149 variant | VLAEAMSQA | 135,4 | 27 | gag |
| 8.115mod | LLFPISPV | 15 | 16,2 | 8.115nat | LNFPISPI | 369,8 | 29 | pol |
| 8.111mod | FLNTPPLV | 26 | 5,6 | 8.111nat (4.119) | FVNTPPLV | 97,6 | 32 | pol-RT |
| 8.10mod | KLKPPLPSV | 48.8 | 6,4 | 8.10natI | KIKPPLPSV | 444,4 | 25 | vif |
| 8.10mod | KLKPPLPSV | 48.8 | 6,4 | 8.10natT | KTKPPLPSV | 231,1 | 39 | vif |
| 8.75mod | NLWATHACV | 50 | 23 | 8.75natI | NVWATHACV | 490,6 | 47 | env |
| 8.66mod | ELMTACQGV | 85 | 3970 | 8.66nat | EMMTACQGV | 134,2 | 51 | gag |
| 8.75mod | NLWATHACV | 50 | 23 | 8.75natV | NIWATHACV | 486,1 | 57 | env |
| 8.76mod | KLTPLCVTV | 57 | 43 | 8.76nat | KLTPLCVTL | 114,7 | 56 | env |
| 8.4mod | FLRPWLHGV | 10.2 | 148,3 | 8.4nat | FPRPWLHGL | 499,7 | 60 | vpr |
| 8.12mod | ALAALITPV | 11.9 | 3,5 | 8.12nat | ALAALITPK | 137,6 | 62 | vif |
| 8.65mod | ALLEEMMTV | 26.6 | 1,1 | 8.65nat | ATLEEMMTA | 431 | 77 | gag |
| 8.116mod | ALGIIQAV | 13 | 26 | 8.116nat | ALGIIQAQ | 131,3 | 97 | pol |
| 8.37mod | ILLWQRPLV | 74,1 | 76 | 8.37nat | ITLWQRPLV | 400,8 | 110 | pol |
| 8.15mod | GLGSPQILV | 37.7 | 13,5 | 8.15natM (4.40 ) | GMGSPQILV | 55,9 | 120 | rev |
| 4.14 | SLVKHHMYV | 26,6 | 21 | 4.14 variant | SLVKHHMYI | 101,2 | 123 | vif |
| 8.11mod | GLADQLIHV | 47.0 | 32 | 8.11natM | GLADQLIHM | 279,4 | 135 | vif |
| 8.3mod | ALVEMGHHV | 35.5 | 2,5 | 8.3nat | ALVEMGHHA | 123,7 | 160 | vpu |
| 8.49mod | NLVATLYCV | 36 | 25 | 8.49nat | NTVATLYCV | 169,7 | 170 | gag |
| 8.54mod | RLLNAWVKV | 73,2 | 42 | 8.54nat | RTLNAWVKV | 366,8 | 170 | gag |
| 8.41mod | KLGKAGYVV | 26 | 7,3 | 8.41nat | KLGKAGYVT | 245,2 | 170 | pol |
| 8.121mod | WLFKLVPV | 49.9 | 183,3 | 8.121nat | WCFKLVPV | 433,6 | 200 | nef |

EP 1 250 351 B1

| | | | | | | | | |
|---|---|---|---|---|---|---|---|---|
| 8.42mod | YLAWVPAHV | 15,4 | 143,3 | 8.42nat | YLAWVPAHK | 220,1 | 200 | pol |
| 8.56mod | TLQEQIGWV | 42,3 | 42 | 8.56nat | TLQEQIGWM | 335,9 | 245 | gag |
| 8.85mod | TLGAASITV | 41 | 385 | 8.85nat | TMGAASITL | 126,8 | 280 | env |
| 8.1mod | VLAAIIAIV | 19,1 | ND | 8.1nat | VVAAIIAIV | 140,7 | 260 | vpu |
| 8.21mod | FLWMGYELV | 29 | ND | 8.21nat | FLWMGYELH | 288,8 | 445 | pol(RT) |
| 8.45mod | LLFGWCFKL | 10 | 25 | 8.45natC | LCFGWCFKL | 115,2 | 580 | nef |
| 8.47mod | RLAFHHMAV | 16,2 | 36,5 | 8.47nat | RLAFHHMAR | 223,6 | 670 | nef |
| 8.123mod | TLNPPIPV | 26,4 | 11 | 8.123natG | TGNPPIPV | 380,3 | 700 | gag |
| 8.64mod | ALGPAATLV | 14,9 | 1,3 | 8.64nat | ALGPAATLE | 392,9 | 830 | gag |
| 8.88mod | ALEAQQHLV | 14 | 1,2 | 8.88nat | AIEAQQHLL | 200 | 850 | env |
| 8.135mod | FLGAAGSV | 30 | 5 | 8.135nat | FLGAAGST | 178,7 | 870 | env |
| 8.130mod | SLGDPEIV | 16,8 | 260 | 8.130nat | SGGDPEIV | 200,5 | 950 | env |
| 8.33mod | LLTAVQMAV | 7 | 80 | 8.33nat | LKTAVQMAV | 140,2 | 1330 | pol (P31) |

**Figure legends**

*Figure 1:*

Figure 1 demonstrates the induction of CTL after immunisation of HLA-A2 transgenicC57BU6 mice with a peptide representing a predicted HLA-A2 CTL epitope.

Figure 1A: three HLA-A2 transgenic mice (4-4, 5-1, 5-2) were immunised with H3 peptide in Freunds incomplete adjuvans at day 0. Splenocytes from day 10 were in vitro stimulated for 5 days with H3 peptide loaded LPS-blast cells and assayed in $^{51}$Cr release assay for immune reactivity against EL4-A2 or EL4 target cell lines loaded with H3 peptide or not. Different Effector to Target cell (E: T) ratios were tested.
Figure 1B: control experiment showing no lysis of the EL4-A2 or EL4 target cells loaded with another HLA-A2 restricted epitope peptide LV9 (sequence LLGRNSFEV) or not by effector splenocytes from non immunised HLA-A2 mice even after 5 days of in vitro stimulation with LV9 loaded LPS-blast cells.
Figure 1C: control experiment showing no lysis of EL4-A2 or EL4 target cells loaded with H3 or not by effector splenocytes from non immunised HLA-A2 mice even after 5 days of in vitro stimulation with H3 peptide loaded LPS-blast cells.

*Figure 2:*

Figure 2 shows the cytotoxic activity of splenocytes obtained from peptide-immunised C57BI6/A2 transgenic mice after 5 days of *in vitro* restimulation. Open symbols represent specific lysis of peptide-pulsed EL4A2, whereas filled symbols represent specific lysis of peptide-pulsed EL4(Kb) target cells.

Figure 2A: CTL response raised against the natural peptide. The spenocytes were obtained from two mice (m9-1 and m9-3) immunised with the natural epitope (LTFGWCFKL). No specific lysis could be observed against EL4A2 targets pulsed with the natural peptide (less than 10% of the targets were lysed).
Figure 2B: CTL responses raised against the improved peptide. One mouse (m9-4) was immunised with the anchor position improved peptide (LLFGWCFKL). The splenocytes were restimulated *in vitro* with either the improved peptide or the natural peptide. After five days of restimulation, the splenocytes harbour a high cytotoxic activity (90% of specific lysis at E:T ratio of 50) against EL4A2 targets pulsed with the improved peptide. In addition, the splenocytes induced against the improved epitope are able to lyse EL4A2 targets pulsed with the natural peptide(14% of specific lysis at E:T ratio of 50).

*Figure 3:*

Figure 3 shows the cytotoxic activity of splenocytes obtained from C57BL6/A2 transgenic mice immunised with anchor optimized peptide 8.54 RLLNAWVKV (from tables 5B and 10) after 5 days of in vitro restimulation with the same peptide and assayed against EL4-A2 target cells pulsed with the same peptide 8.54 (squares) or the related natural 8.54Nat epitope peptide (triangles). Right panels (figures 3B and 3D) show negative controls where the effector splenocytes do not react against peptide-pulsed EL4 cells that do not have the HLA-A2 MHC-I on the surface. A clear response to the peptide used for immunisation ant with cross-reaction to the related natural epitope is seen in one of 4 mice (mouse 1, upper panels, figure 3A) and a less response in mouse 2 (lower panel, figure 3C). It is known that not all mice in a group responds to such peptide vaccination which may be due to both technical and biological variations of the A2 heterologous mice.

**References**

Sette A, Vitiello A, Reherman B, Fowler P, Nayersina R, Kast WM, Melief CJM, Oseroff C, Yuan L, Ruppert J, Sidney J, del Guercio M-F, Southwood S, Kubo RT, Chesnut RW, Grey HM, Chisari FV. The relationship between class I binding affinity and immunogenicity of potential cytotoxic T cell epitopes. J. Immunol 1994; 153: 5586-5592.

Brunak S., Engelbrecht J., and Knudsen S., Prediction of human mRNA donor and acceptor sites from the DNA sequence, J. Mol. Biol., 220, 49-65, 1991.

Mathews B.W., Comparison of the Predicted and Observed Secondary Structure of {T4} Phage Lysozyme, Biochim. Biophys. Acta, 405, 442-451,1975.

Hobohm U., Scharf M., Schneider R. and Sander C., Selection of representative protein data sets, Protein Science, 1, 409-417, 1992

Potter, H..1993, Application of electroporation in recombinant DNA technology. Methods Enzymol., 217,461-478

Fomsgaard A, Nielsen HV, Kirkby N, Bryder K, Corbet S, Nielsen C, Hinkula J, Buus S. Induction of cytotoxic T-cell responses by gene gun DNA vaccination with minigenes encoding influenza A virus HA and NP CTL-epitopes. Vaccine 1999; 18: 681-691.

Marker O & Volkert M. Studies on cell-mediated immunity to lymphocyte choriomeningitis virus in mice. J Exp Med 1973; 137: 1511-1513.

Houghten RA. Combinatorial libraries: Finding the needle in the haystack. Curr Biol 1994; 4: 564-7 Review.

Milich DR, McLachlan A, Thornton GB, Hughes JL. Antibody production to the nucleocapsid and envelope of the hepatitis B virus primed by a single synthetic T cell site. Nature 1987; 6139: 547-9.

Schmitz JE, Kuroda MJ, Santra S, Sasseville VG, Simon MA, Lifton MA, Racz P, Tenner-Racz K, Dalesandro M, Scallon BJ, Ghrayeb J, Forman MA, Montefiori DC, Rieber EP, Letvin NL, Reimann KA. Control of viremia in Simian immunodeficiency virus infection by CD8+ lymphocytes. Science 1999; 282: 857860.

Gallimore A, Cranage M, Cook N, Almond N, Bootman J, Rud E, Silvera P, Dennis M, Corcoran T, Stott J et al. Early suppression of SIV replication by CD8+ nef-specific T cells in vaccinated macaques. Nature Med 1995; 351: 290-296

Shibata R et al. Neutralizing antibody directed against the HIV-1 envelope glycoprotein can completely block HIV-1/SIV chimeric virus infection of macaque monkeys. Nature Med 5(2):204-210,1999.

Koup RA et al. Temporal association of cellular immune responses with the initial control of viremia in Primary HIV-1 syndrome. J Virol 68(7):4650-5,1994

Harrer E et al. HIV-1-specific cytotoxic T lymphocyte response in healthy, long-term nonprogressing seropositive persons. AIDS Res Hum Retrovir 10(supp.2):77-78,1994

Falk K, Rotzschke O, Stevanovic S et al. Allele-specific motifs revealed by sequencing of self-peptides eluted from MHC molecules. Nature 1991; 351:290-6.

Ruppert J, Sidney J, Celis E et al. Prominent role of secondary anchor residues in peptide binding to HLA-A2.1 molecules. Cell 1993; 74:929-37.

Madden, D. R. 1995. The three-dimensional structure of peptide-MHC complexes. Annual Review of Immunology 13:587.

Sette, A., S. Buus, S. M. Colon, J. A. Smith, C. Miles, and H. M. Grey. 1987. Structural characteristics of an antigen required for its interaction with la and recognition by T cells. Nature 328:395.

Rammensee, H.-G., T. Friede, and S. Stevanonic. 1995. MHC ligands and peptide motifs: first listing. Immunogenetics 41:178.

Fremont, D. H., M. Matsumura, E. A. Stura, P. A. Peterson, and I. A. Wilson. 1992. Crystal structure of two viral peptides in complex with murine MHC class I H-2Kb. Science 257:919.

Garrett, T. P. J., M. A. Saper, P. J. Björkman, J. L. Strominger, and D. C. Wiley. 1989. Specificity pockets for the side chains of peptide antigens in HLA-Aw68. Nature 342:692.

Matsumura, M., D. H. Fremont, P. A. Peterson, and I. A. Wilson. 1992. Emerging principles for the recognition of peptide antigens by MHC class I molecules. Science 257:927.

Schafer JR et al. Vaccine 16(19):1828-35, 1998

Stryhn A, Pedersen LØ, Romme T et al. Peptide binding specificity of major histocompatibility complex class I resolved into an array of apparently independent sub-specificities: quantitation by peptide libraries and improved prediction of binding. Eur J Immunol 1996; 26:1911-18.

Sette A, Buus S, Apella E et al. Prediction of major histocompatibility complex binding regions of protein antigens by sequence pattern analysis. Proc Natl Acad Sci U S A 1989; 86:3296-300.

Meister GE, Roberts CG, Berzofsky JA et al. Two novel T cell epitope prediction algorithms based on MHC-binding motifs; comparison of predicted and published epitopes from Mycobacterium tuberculosis and HIV protein sequences. Vaccine 1995; 13:581-91.

Rognan D, Scapozza L, Folkers G et al. Molecular dynamics simulation of MHC-peptide complexes as a tool for predicting potential T cell epitopes. Biochemistry 1994; 33:11476-85.

Mata M, Travers PJ, Liu Q et al. The MHC class I-restricted immune response to HIV-gag in BALB/c mice selects a single epitope that does not have a predictable MHC-binding motif and binds to Kd through interactions between a glutamine at P3 and pocket D. J Immunol 1998; 161:2985-93.

Altuvia Y, Schueler O, Margalit H. Ranking potential binding peptides to MHC molecules by a computational threading approach. J Mol Biol 1995; 249:244-50.

Altuvia Y, Sette A, Sidney J et al. A structure-based algorithm to predict potential binding peptides to MHC molecules with hydrophobic binding pockets. Hum Immunol 1997; 58:1-11.

Vasmatzis G, Zhang C, Cornette JL et al. Computational determination of side chain specificity for pockets in class I MHC molecules. Mol Immunol 1996; 33:1231-9.

Zhang C, Anderson A, De Lisi C. Structural principles that govern the peptide-binding motifs of class I MHC molecules. J Mol Biol 1998; 281:929-47.

U.S. Patents 4,608,251; 4,601,903; 4,599,231; 4,599,230; 4,596,792; and 4,578,770

Buus, S., A. Sette, S. M. Colon, D. M. Jenis, and H. M. Grey. 1986. Isolation and characterization of antigen-la complexes involved in T cell recognition. Cell 47:1071.

Holm A, Meldal M. Peptides, E.Bayer and G Jung. Walter de Gruyter & Co.: Berlin-New York. 1989.

Parker KC, Bednarek MA, Coligan JE. Scheme for ranking potential HLA-A2 binding peptides based on independent binding of individual peptide side-chains. J Immunol 1994; 152:163-75.

Milik M., Sauer D., Brunmark A. P., Yuan L., Vitiello A., Jackson M. R., Peterson P. A., Skolnick J., Glass C. A. Application of an artificial neural network to predict specific class I MHC binding peptide sequences. Nature Biotechnology 1998; 16: 753-756.

**List of preferred CTL eptiopes**

SEQ ID NOs:1-213 are epitopes with high affinity binding and an intermediate global conservation, meaning with a global conservation of more than 1% and a cut off value for the weighted average of the MHC class I binding for the query peptide of less than 100 nM. SEQ ID NOs: 214-313 are 9-mer-epitopes representing one best new improved binder for each natural epitope with a binding affinity $IC_{50}$ between 50nM and 500nM and with a global conservation above 8%. SEQ ID NOs: 314-359 are 8-mer-epitopes representing one best new improved binder for each natural epitope with a binding affinity $IC_{50}$ between 50nM and 500nM and with a global conservation above 8%.

| SEQ ID: | SEQ Name | PEPTIDE | Kd nM | GLOBAL CONSERVATION |
|---------|----------|---------|-------|---------------------|
| 1 | 4.1 | LLAGVDYRI | 83,9 | 1,3% |
| 2 | 4.2 | LLAKVDYRL | 44,6 | 1,3% |
| 3 | 4.3 | ILAIVVWTI | 39,9 | 9,6% |
| 4 | 4.4 | KLVEMGHHA | 71,5 | 2,6% |
| 5 | 4.5 | ALMEMGHHA | 47,9 | 1,9% |
| 6 | 4.6 | ALVEMGHLA | 76,4 | 1,3% |
| 7 | 4.7 | ALGEMGPFI | 73,8 | 1,3% |
| 8 | 4.8 | IVGLIVAL | 67,8 | 3,2% |
| 9 | 4.9 | IVGLIVAV | 50,2 | 1,3% |
| 10 | 4.10 | ALIRTLQQL | 38,6 | 3,5% |
| 11 | 4.11 | ALIRILQQL | 52,8 | 3,5% |
| 12 | 4.12 | ALIRMLQQL | 26,9 | 2,3% |
| 13 | 4.13 | LHGLGQYV | 96,4 | 4,0% |
| 14 | 4.14 | SLVKHHMYV | 26,6 | 26,0% |
| 15 | 4.15 | SLVKHHMHV | 51,2 | 1,5% |
| 16 | 4.16 | SLVKHHIYV | 67,7 | 1,5% |
| 17 | 4.17 | LVIRTYWGL | 88 | 1,5% |
| 18 | 4.18 | RLRRYSTQV | 76,7 | 1,5% |
| 19 | 4.19 | RLKRYSTQV | 60,1 | 1,1% |
| 20 | 4.20 | GLADQLIHL | 90,6 | 14,3% |
| 21 | 4.21 | NLADQLIHL | 78,3 | 10,2% |
| 22 | 4.22 | PLGDARLV | 45,4 | 26.8% |
| 23 | 4.23 | PLGDAKLV | 63,9 | 19,2% |
| 24 | 4.24 | PLGEARLV | 92,6 | 17,0% |
| 25 | 4.25 | PLGDATLV | 35,9 | 1,9% |
| 26 | 4.26 | KIGSLQYL | 56,2 | 1,5% |
| 27 | 4.27 | TLIPKQPL | 42,2 | 2,0% |
| 28 | 4.28 | KLLYQSNPL | 46,9 | 3,8% |
| 29 | 4.29 | CLGRPAEPV | 35,1 | 15,2% |
| 30 | 4.30 | YLGRPAEPV | 22,4 | 10,5% |
| 31 | 4.31 | FLGRPAEPV | 12,7 | 3,8% |
| 32 | 4.32 | CLGRPTEPV | 37,7 | 3,8% |
| 33 | 4.33 | CLGRPEEPV | 59,8 | 3,8% |
| 34 | 4.34 | FLGRPEEPV | 18,7 | 2,9% |
| 35 | 4.35 | YLGRPEEPV | 36 | 2,9% |
| 36 | 4.36 | CLGRPPEPV | 18,3 | 1,9% |
| 37 | 4.37 | YLGRPTEPV | 23,8 | 1,9% |
| 38 | 4.38 | FLGRSAEPV | 46,2 | 1,9% |
| 39 | 4.39 | HLGRPAEPV | 96,6 | 1,9% |
| 40 | 4.40 | GMGSPQILV | 55,9 | 2,9% |
| 41 | 4.41 | ILVESPTVL | 74,4 | 9,5% |
| 42 | 4.42 | VLVEPPWL | 47,5 | 1,9% |
| 43 | 4.43 | ILVESPTIL | 84,7 | 1,9% |
| 44 | 4.44 | GMGSPQIL | 63,3 | 2,9% |
| 45 | 4.45 | ISGEPCMV | 86,8 | 1,9% |
| 46 | 4.46 | ISGKPCAV | 95,1 | 1,9% |

(continued)

| SEQ ID: | SEQ Name | PEPTIDE | Kd nM | GLOBAL CONSERVATION |
|---------|----------|---------|-------|---------------------|
| 47 | 4.47 | LLGRWKPKM | 65,1 | 1,2% |
| 48 | 4.48 | YMEAEVIPA | 98 | 4,7% |
| 49 | 4.49 | YLEAEVIPA | 63,4 | 3,5% |
| 50 | 4.50 | LAGRWPVKV | 91,9 | 40,7% |
| 51 | 4.51 | LAARWPVKV | 60,9 | 2,3% |
| 52 | 4.52 | LTLAGRWPV | 70,2 | 1,2% |
| 53 | 4.53 | AMKAACWWA | 77,7 | 1,2% |
| 54 | 4.54 | ALQKQITKI | 55,2 | 1,2% |
| 55 | 4.55 | ELQKQITKV | 60 | 1,2% |
| 56 | 4.56 | NLQTQILKV | 85,7 | 1,2% |
| 57 | 4.57 | ILKIQNFRV | 74,6 | 1,2% |
| 58 | 4.58 | DLGDAYFSV | 83,4 | 1,2% |
| 59 | 4.59 | KLHPEQARA | 62,8 | 1,2% |
| 60 | 4.60 | ILASQIQTT | 71,4 | 2,3% |
| 61 | 4.61 | LTAEAEMEL | 71,4 | 2,3% |
| 62 | 4.62 | MTAEAEMEL | 84,8 | 1,2% |
| 63 | 4.63 | ILKEPVHGA | 40,9 | 7,0% |
| 64 | 4.64 | ILKDPVHGV | 15 | 5,8% |
| 65 | 4.65 | ILREPVHGV | 17,7 | 3,5% |
| 66 | 4.66 | ILKTPVHGV | 35,1 | 2,3% |
| 67 | 4.67 | ILKDPVHGA | 40,2 | 2,3% |
| 68 | 4.68 | KLKEPVHGV | 11,1 | 1,2% |
| 69 | 4.69 | ILKAPVHGV | 12,3 | 1,2% |
| 70 | 4.70 | ILKEPIHGV | 16,6 | 1,2% |
| 71 | 4.71 | ILRDPVHGV | 17,4 | 1,2% |
| 72 | 4.72 | ILKDPVHWV | 18,8 | 1,2% |
| 73 | 4.73 | ILREPIHGV | 19,6 | 1,2% |
| 74 | 4.74 | ILKEPVHEV | 20,2 | 1,2% |
| 75 | 4.75 | ILKEPLHGV | 20,6 | 1,2% |
| 76 | 4.76 | ILKEPMHGV | 27,6 | 1,2% |
| 77 | 4.77 | RLKQPVHGV | 44,8 | 1,2% |
| 78 | 4.78 | ILRIPVHGV | 49,4 | 1,2% |
| 79 | 4.79 | ILKESVHGV | 58,4 | 1,2% |
| 80 | 4.80 | ILRKPVHEV | 67,8 | 1,2% |
| 81 | 4.81 | ILKVPVHGV | 68,1 | 1,2% |
| 82 | 4.82 | QLAEVVQKV | 20,6 | 10,5% |
| 83 | 4.83 | QLTEVVQKV | 55,5 | 5,8% |
| 84 | 4.84 | QLTEAVQKV | 46,5 | 3,5% |
| 85 | 4.85 | QLAEVVQKI | 84,4 | 3,5% |
| 86 | 4.86 | QLAEAVQKI | 70,5 | 2,3% |
| 87 | 4.87 | QLAEMVQKV | 14,9 | 1,2% |
| 88 | 4.88 | QLAEVIQKV | 22,7 | 1,2% |
| 89 | 4.89 | QLTAWQKV | 40 | 1,2% |
| 90 | 4.90 | QLVEVVQKV | 52,3 | 1,2% |
| 91 | 4.91 | YLLEEDPIV | 45,1 | 1,2% |
| 92 | 4.92 | NLAFPQWKA | 31,7 | 1,2% |
| 93 | 4.93 | KLSSEQTRA | 66,6 | 2,3% |
| 94 | 4.94 | SLSFPQITL | 99,5 | 9,3% |
| 95 | 4.95 | SLSLPQITL | 78,9 | 4,7% |

(continued)

| SEQ ID: | SEQ Name | PEPTIDE | Kd nM | GLOBAL CONSERVATION |
|---|---|---|---|---|
| 96 | 4.96 | SLNFPQITL | 81 | 3,5% |
| 97 | 4.97 | ALNFPQITL | 96 | 2,3% |
| 98 | 4.98 | SLSFPQTTL | 48 | 1,2% |
| 99 | 4.99 | SLCFPQITL | 63,6 | 1,2% |
| 100 | 4.100 | TLNCPQITL | 98,2 | 1,2% |
| 101 | 4.101 | IIGAETFYV | 51,8 | 15,1% |
| 102 | 4.102 | IMGAETFYV | 12,9 | 3,5% |
| 103 | 4.103 | ILGAETFYV | 9,9 | 1,2% |
| 104 | 4.104 | IMGAETYYV | 20,7 | 1,2% |
| 105 | 4.105 | ITGAETFYV | 29,5 | 1,2% |
| 106 | 4.106 | IVGADSFFV | 92,8 | 1,2% |
| 107 | 4.107 | ITLWQPPLV | 71,3 | 1,2% |
| 108 | 4.108 | ELQAILMAL | 80,8 | 2,3% |
| 109 | 4.109 | YLALQDSGV | 44,9 | 1,2% |
| 110 | 4.110 | ALQDSGPEV | 86,5 | 3,5% |
| 111 | 4.111 | ALQDSQSEV | 64,8 | 1,2% |
| 112 | 4.112 | ALQESGPEV | 92,4 | 1,2% |
| 113 | 4.113 | KIGGQLKV | 95,4 | 1,2% |
| 114 | 4.114 | VLIGPTPV | 41,8 | 2,3% |
| 115 | 4.115 | ILVGPTPV | 64,5 | 1,2% |
| 116 | 4.116 | ALTDIVPL | 78,9 | 26,7% |
| 117 | 4.117 | VLTDIVPL | 74,5 | 1,2% |
| 118 | 4.118 | TLTDIVPL | 93,1 | 1,2% |
| 119 | 4.119 | FVNTPPLV | 97,6 | 86,0% |
| 120 | 4.120 | FVNTPHLV | 68,8 | 3,5% |
| 121 | 4.121 | FVNTPLLV | 68,6 | 1,2% |
| 122 | 4.122 | LQGKARKL | 69,4 | 1,2% |
| 123 | 4.123 | KLGKAGYV | 96,7 | 57,0% |
| 124 | 4.124 | LTFGWCFKL | 29,6 | 65,7% |
| 125 | 4.125 | LTLGWCFKL | 50,5 | 4.8% |
| 126 | 4.126 | LTPGWCFKL | 94,9 | 1,2% |
| 127 | 4.127 | RLAYHHMAR | 74,8 | 1,2% |
| 128 | 4.128 | TLGWCFKL | 35,5 | 4.8% |
| 129 | 4.129 | YMMKHLVWA | 80,7 | 4,2% |
| 130 | 4.130 | SLYNTVAVL | 71,3 | 7,3% |
| 131 | 4.131 | SLYNTIATL | 71,8 | 4,2% |
| 132 | 4.132 | SLFNTVAVL | 51,1 | 3,1% |
| 133 | 4.133 | SLFNTIATL | 52,4 | 2,1% |
| 134 | 4.134 | SLYNAVATL | 56,6 | 2,1% |
| 135 | 4.135 | NTIATLWCV | 65,8 | 5,2% |
| 136 | 4.136 | DLNAMLNTV | 91,1 | 3,1% |
| 137 | 4.137 | TLQEQITWM | 84,3 | 3,1% |
| 138 | 4.138 | SLQEQIAWM | 72,5 | 2,1% |
| 139 | 4.139 | MTNNPPIPV | 71,3 | 29,2% |
| 140 | 4.140 | MTSNPPIPV | 87,8 | 29,2% |
| 141 | 4.141 | MTGNPPIPV | 42,8 | 10,4% |
| 142 | 4.142 | MTSNPPVPV | 78,1 | 8,3% |
| 143 | 4.143 | MTGNPPVPV | 38,4 | 6,2% |
| 144 | 4.144 | MTNNPPVPV | 64,2 | 3,1% |

(continued)

| SEQ ID: | SEQ Name | PEPTIDE | Kd nM | GLOBAL CONSERVATION |
|---------|----------|---------|-------|---------------------|
| 145 | 4.145 | MTHNPPIPV | 95,4 | 3,1% |
| 146 | 4.146 | MTGNPAIPV | 93,4 | 2,1% |
| 147 | 4.147 | KMVKMYSPV | 69,8 | 2,1% |
| 148 | 4.148 | KMYSPVSIL | 72,8 | 2,1% |
| 149 | 4.149 | VLAEAMSQV | 40,3 | 49,0% |
| 150 | 4.150 | ILAEAMSQV | 26,7 | 4,2% |
| 151 | 4.151 | ILGQLQPS | 94,9 | 15.6% |
| 152 | 4.152 | ILGQLQPA | 35,2 | 7,3% |
| 153 | 4.153 | IIGQLQPA | 50,8 | 4,2% |
| 154 | 4.154 | TSNPPVPV | 71,5 | 8,3% |
| 155 | 4.155 | TNNPPVPV | 73,9 | 3,1% |
| 156 | 4.156 | THNPPIPV | 75,8 | 3,1% |
| 157 | 4.157 | ALGPAATL | 70 | 27,1% |
| 158 | 4.158 | FLGKIWPS | 38,6 | 84,4% |
| 159 | 4.159 | FLGRIWPS | 75 | 2,1% |
| 160 | 4.160 | YQQWWIWGV | 26 | 1,9% |
| 161 | 4.161 | MLQWGTMLL | 34,4 | 2,3% |
| 162 | 4.162 | ALFYRLDW | 64,1 | 8,5% |
| 163 | 4.163 | ALFYRLDIV | 71,1 | 7,5% |
| 164 | 4.164 | SLFYRLDIV | 62 | 2,3% |
| 165 | 4.165 | SLFYRLDVV | 54,4 | 1,9% |
| 166 | 4.166 | ALFYNLDW | 68,8 | 1,4% |
| 167 | 4.167 | FCAPAGFAI | 88,1 | 2,8% |
| 168 | 4.168 | SLAEEEVVL | 90,7 | 1,9% |
| 169 | 4.169 | KLAEHFPNK | 72,9 | 3,8% |
| 170 | 4.170 | MYAPPIQGV | 34,3 | 2,8% |
| 171 | 4.171 | NLASGIQKV | 24,1 | 1,4% |
| 172 | 4.172 | IYAPPIQGV | 44,1 | 1,4% |
| 173 | 4.173 | SLGVAPTRA | 98,3 | 1,4% |
| 174 | 4.174 | FLSAAGSTM | 75,5 | 1.4% |
| 175 | 4.175 | TMGAASMTL | 41,4 | 15,0% |
| 176 | 4.176 | TMGAAATAL | 57,8 | 2,3% |
| 177 | 4.177 | TMGAAAVTL | 94 | 2,3% |
| 178 | 4.178 | TMGARSMTL | 50,1 | 1,9% |
| 179 | 4.179 | AIQAQQQLL | 71,9 | 2,8% |
| 180 | 4.180 | LLQLTVWGI | 38 | 58,2% |
| 181 | 4.181 | MLQLTVWGI | 43,7 | 16,0% |
| 182 | 4.182 | SLQGFLPLL | 70,6 | 1,4% |
| 183 | 4.183 | LIAARIVEL | 86,4 | 6.6% |
| 184 | 4.184 | LLGRRGWEA | 37,1 | 24,9% |
| 185 | 4.185 | LLGRRGWEV | 13,5 | 9,4% |
| 186 | 4.186 | LLGRRGWEI | 47,6 | 5,6% |
| 187 | 4.187 | ILGRRGWEA | 54,3 | 2.8% |
| 188 | 4.188 | LLGRRGWEL | 22,9 | 1,9% |
| 189 | 4.189 | LLLYWGQEL | 47,1 | 7,5% |
| 190 | 4.190 | LLQYWIQEL | 18,1 | 7,0% |
| 191 | 4.191 | LLQYWGQEL | 30,1 | 5,2% |
| 192 | 4.192 | LLQYWGQEI | 66,5 | 1,4% |
| 193 | 4.193 | SLLDTIAIA | 88,1 | 3,8% |

(continued)

| SEQ ID: | SEQ Name | PEPTIDE | Kd nM | GLOBAL CONSERVATION |
|---|---|---|---|---|
| 194 | 4.194 | SLFDTIAIA | 49,4 | 1,9% |
| 195 | 4.195 | LLDATAIAV | 69,1 | 4,7% |
| 196 | 4.196 | LLNTTAIAV | 94,1 | 4,7% |
| 197 | 4.197 | LLNTTAIVV | 90 | 2.8% |
| 198 | 4.198 | LLNAIAIAV | 34,8 | 1,4% |
| 199 | 4.199 | RIIEVVQRV | 69,9 | 1,4% |
| 200 | 4.200 | HIGPGQAL | 81,5 | 1,4% |
| 201 | 4.201 | IIGDIRKA | 85,7 | 7,0% |
| 202 | 4.202 | LGGDPEIV | 90,2 | 1,9% |
| 203 | 4.203 | IINMWQEV | 95,1 | 27,7% |
| 204 | 4.204 | IINMWQKV | 56,8 | 3,8% |
| 205 | 4.205 | FLGFLSAA | 79,2 | 1,4% |
| 206 | 4.206 | YLGDQQLL | 16,7 | 1,9% |
| 207 | 4.207 | YLSDLMKL | 85,4 | 1,4% |
| 208 | 4.208 | YTGLIYTL | 59,7 | 5,6% |
| 209 | 4.209 | YTGLIYNL | 84,4 | 4,2% |
| 210 | 4.210 | YTGLIYSL | 68,3 | 3,3% |
| 211 | 4.211 | YTGIIYSL | 63,5 | 1,4% |
| 212 | 4.212 | YTGIIYNL | 80 | 1,4% |
| 213 | 4.213 | GLKIVFAV | 96,6 | 1,4% |
| 214 | 8.1 | VLAAIIAIV | 19.1 | 9,6% |
| 215 | 8.2 | ILAIVVWTV | 12.1 | 41,8% |
| 216 | 8.3 | ALVEMGHHV | 35.5 | 17,3% |
| 217 | 8.4 | FLRPWLHGV | 10.2 | 17,3% |
| 218 | 8.5 | SLGQHIYEV | 17.8 | 19,7% |
| 219 | 8.6 | SLGQYIYEV | 28.9 | 13,3% |
| 220 | 8.7 | LLITTYWGL | 24 | 38,5% |
| 221 | 8.8 | LLVRTYWGV | 11.7 | 9,8% |
| 222 | 8.9 | LLVTTYWGV | 20.1 | 9,8% |
| 223 | 8.10 | KLKPPLPSV | 48.8 | 56,3% |
| 224 | 8.11 | GLADQLIHV | 47.0 | 23,4% |
| 225 | 8.12 | ALAALITPV | 11.9 | 18,5% |
| 226 | 8.13 | ALTALITPV | 27.5 | 17% |
| 227 | 8.14 | LLLPPIERV | 19.5 | 15.2% |
| 228 | 8.15 | GLGSPQILV | 37.7 | 18,1% |
| 229 | 8.16 | ILVESPAVV | 82.6 | 8,6% |
| 230 | 8.17 | ALVEICTEV | 31.8 | 26,7% |
| 231 | 8.28 | ALTEICTEV | 32.8 | 16,3% |
| 232 | 8.18 | LLIPHPAGV | 6.6 | 88,4% |
| 233 | 8.19 | YLAFTIPSV | 53.1 | 12,8% |
| 234 | 8.20 | HLLRWGFTV | 33.4 | 36% |
| 235 | 8.21 | FLWMGYELV | 29 | 97,7% |
| 236 | 8.22 | KLNWASQIV | 30 | 94,2% |
| 237 | 8.23 | SLIYAGIKV | 27.9 | 43% |
| 238 | 8.24 | SLIYPGIKV | 21.3 | 39,5% |
| 239 | 8.25 | ALTEVIPLV | 29.2 | 24,4% |
| 240 | 8.29 | ALTDIVPLV | 31,8 | 26,7% |
| 241 | 8.26 | ALTDIVTLV | 22.7 | 10,5% |
| 242 | 8.27 | ALTEWPLV | 26.4 | 10,5% |

(continued)

| SEQ ID: | SEQ Name | PEPTIDE | Kd nM | GLOBAL CONSERVATION |
|---|---|---|---|---|
| 243 | 8.30 | KLWYQLEKV | 17.7 | 71% |
| 244 | 8.31 | LLGRWPVKV | 8.0 | 40,7% |
| 245 | 8.32 | ALKAACWWV | 17.6 | 50% |
| 246 | 8.33 | LLTAVQMAV | 7 | 90,7% |
| 247 | 8.34 | KLMAGADCV | 35.2 | 9,3% |
| 248 | 8.35 | NLAFPQGEV | 83,3 | 15,1% |
| 249 | 8.36 | LLQRPLVTV | 13.3 | 29,1% |
| 250 | 8.37 | ILLWQRPLV | 74,1 | 73,3% |
| 251 | 8.38 | ILLWQRPIV | 70.7 | 8,1% |
| 252 | 8.39 | LLGPTPVNV | 13.1 | 86% |
| 253 | 8.40 | FLISPIETV | 13.6 | 84,9% |
| 254 | 8.41 | KLGKAGYVV | 26 | 57% |
| 255 | 8.42 | YLAWVPAHV | 15.4 | 38,4% |
| 256 | 8.43 | ALNADCAWV | 44.1 | 20,3% |
| 257 | 8.44 | FLVRPQVPV | 6 | 74,9% |
| 258 | 8.45 | LLFGWCFKL | 10 | 79,2% |
| 259 | 8.46 | LLWKFDSRV | 66,5 | 10,4% |
| 260 | 8.47 | RLAFHHMAV | 16,2 | 26,3% |
| 261 | 8.48 | SLYNTVATV | 33,4 | 32,3% |
| 262 | 8.49 | NLVATLYCV | 36 | 57,3% |
| 263 | 8.50 | NLVAVLYCV | 38.4 | 8,3% |
| 264 | 8.51 | TLYCVHQKV | 72,6 | 15,6% |
| 265 | 8.52 | TLWCVHQRV | 71.6 | 9,4% |
| 266 | 8.53 | LLGQMVHQV | 14 | 28,1% |
| 267 | 8.54 | RLLNAWVKV | 73,2 | 94,8% |
| 268 | 8.55 | RLHPVQAGV | 18.1 | 10,4% |
| 269 | 8.56 | TLQEQIGWV | 42,3 | 43,8% |
| 270 | 8.57 | TLQEQIAWV | 32.6 | 14,6% |
| 271 | 8.58 | MLNNPPIPV | 18.5 | 29,2% |
| 272 | 8.59 | MLSNPPIPV | 21.7 | 29,2% |
| 273 | 8.60 | MLSNPPVPV | 19.9 | 8,3% |
| 274 | 8.61 | KLVRMYSPV | 20 | 59,4% |
| 275 | 8.62 | RLYSPVSIV | 34 | 58,3% |
| 276 | 8.63 | RLYSPTSIV | 81 | 22,9% |
| 277 | 8.64 | ALGPAATLV | 14.9 | 26% |
| 278 | 8.65 | ALLEEMMTV | 26.6 | 75% |
| 279 | 8.142 | SLEEMMTAV | 32.4 | |
| 280 | 8.66 | ELMTACQGV | 85 | 91,7% |
| 281 | 8.67 | MLQRGNFRV | 16.7 | 30,2% |
| 282 | 8.68 | MLQRGNFKV | 12.3 | 13,5% |
| 283 | 8.69 | FLQSRPEPV | 13 | 43,8% |
| 284 | 8.70 | FLQNRPEPV | 14.2 | 13,5% |
| 285 | 8.71 | HLWRWGTMV | 56 | 14,1% |
| 286 | 8.72 | MLLGMLMIV | 19.2 | 23,5% |
| 287 | 8.73 | KLWVTVYYV | 18.3 | 18,3% |
| 288 | 8.74 | VLVYYGVPV | 60 | 90,1% |
| 289 | 8.75 | NLWATHACV | 50 | 90,7% |
| 290 | 8.76 | KLTPLCVTV | 57 | 82,2% |
| 291 | 8.143 | YLAPAGFAV | 5,9 | 52,1% |

(continued)

| SEQ ID: | SEQ Name | PEPTIDE | Kd nM | GLOBAL CONSERVATION |
|---------|----------|---------|-------|---------------------|
| 292 | 8.77 | YLAPAGYAV | 8.3 | 16,9% |
| 293 | 8.78 | SLAEEEVVV | 46 | 31% |
| 294 | 8.79 | SLAEEEIIV | 57.3 | 9,9% |
| 295 | 8.80 | ALYAPPIRV | 13.0 | 22,1% |
| 296 | 8.81 | ALYAPPIEV | 15 | 12,2% |
| 297 | 8.82 | AMYAPPIKV | 12.1 | 8,9% |
| 298 | 8.83 | AMYAPPIAV | 18.6 | 8,5% |
| 299 | 8.84 | PLGIAPTKV | 68,6 | 11,3% |
| 300 | 8.85 | TLGAASITV | 41 | 46,9% |
| 301 | 8.86 | TLGAASLTV | 81 | 13,1% |
| 302 | 8.87 | RLIEAQQHV | 13.5 | 65,3% |
| 303 | 8.88 | ALEAQQHLV | 14 | 70% |
| 304 | 8.89 | ALEAQQHMV | 19.4 | 14,1% |
| 305 | 8.90 | LLKLTVWGV | 27 | 14,1% |
| 306 | 8.91 | CLTAVPWNV | 15.3 | 17,4% |
| 307 | 8.92 | SLWNWFSIV | 40.0 | 11,7% |
| 308 | 8.93 | YLKIFIMIV | 33 | 60,1% |
| 309 | 8.94 | YLRIFIMIV | 42.3 | 9,9% |
| 310 | 8.95 | FLMIVGGLV | 41.3 | 22,5% |
| 311 | 8.96 | ILFAVLSIV | 24.9 | 40,3% |
| 312 | 8.97 | LLAARTVEV | 14.6 | 26,3v% |
| 313 | 8.98 | LLVARIVEV | 17,9 | 8,9% |
| 314 | 8.99 | LLFIHFRV | 92.5 | 69,9% |
| 315 | 8.100 | LLFVHFRV | 67,1 | 11% |
| 316 | 8.101 | ILGHIVSV | 20.6 | 20,4% |
| 317 | 8.102 | KLGSLQYV | 29 | 82,3% |
| 318 | 8.103 | RLAEPVPV | 36.8 | 19% |
| 319 | 8.144 | YLSNPYPV | 12,2 | 15,2% |
| 320 | 8.104 | GLGSPQIV | 31.3 | 16,2% |
| 321 | 8.105 | KLGPENPV | 38.4 | 76,7% |
| 322 | 8.106 | QLGIPHPV | 16 | 88,4% |
| 323 | 8.107 | TLNDIQKV | 15 | 97,7% |
| 324 | 8.108 | ALTDIVPV | 57,8 | 26,7% |
| 325 | 8.109 | LLAEIQKV | 20.4 | 53,5% |
| 326 | 8.110 | LLEVVQKV | 62,2 | 10,5% |
| 327 | 8.111 | FLNTPPLV | 26 | 86% |
| 328 | 8.112 | FLFPQITV | 37.8 | 27,9% |
| 329 | 8.113 | FLFPQITV | 37.8 | 14% |
| 330 | 8.114 | FLKVKQYV | 28.8 | 20,9% |
| 331 | 8.115 | LLFPISPV | 15 | 93% |
| 332 | 8.116 | ALGIIQAV | 13 | 84,9% |
| 333 | 8.117 | GLGVRYPV | 20.7 | 17,5% |
| 334 | 8.118 | GLGTRFPV | 16 | 13,9% |
| 335 | 8.119 | TLGWCFKV | 27 | 66,5% |
| 336 | 8.120 | CLGWCFKV | 19.5 | 13.5% |
| 337 | 8.121 | WLFKLVPV | 49.9 | 82,5% |
| 338 | 8.145 | FLHVAREV | 84,4 | 13,5% |
| 339 | 8.146 | FLHMAREV | 89,1 | 19,9% |
| 340 | 8.122 | ILGQLQPV | 6,7 | 15,6% |

(continued)

| SEQ ID: | SEQ Name | PEPTIDE | Kd nM | GLOBAL CONSERVATION |
|---|---|---|---|---|
| 341 | 8.123 | TLNPPIPV | 26.4 | 68,8% |
| 342 | 8.124 | PLGEIYKV | 11.3 | 61,5% |
| 343 | 8.125 | PLGDIYKV | 7.9 | 30,2% |
| 344 | 8.126 | ALGPAATV | 51,3 | 27,1% |
| 345 | 8.127 | FLGKIWPV | 4 | 84,4% |
| 346 | 8.128 | LLNRPEPV | 29.4 | 13,5% |
| 347 | 8.129 | ILGDIRQV | 15.1 | 47,4% |
| 348 | 8.130 | SLGDPEIV | 16.8 | 37,6% |
| 349 | 8.131 | CLGEFFYV | 9.1 | 22.5% |
| 350 | 8.132 | ILNMWQEV | 62 | 27,7% |
| 351 | 8.133 | ILNMWQGV | 95,6 | 11,7% |
| 352 | 8.134 | FLGFLGAV | 15 | 73,2% |
| 353 | 8.135 | FLGAAGSV | 30 | 88,3% |
| 354 | 8.136 | LLARILAV | 96,5 | 10,3% |
| 355 | 8.137 | YLKDQQLV | 56 | 50,2% |
| 356 | 8.138 | YLRDQQLV | 96,4 | 22,5% |
| 357 | 8.139 | ILGGLVGV | 84 | 24,9% |
| 358 | 8.140 | ILFAVLSV | 57 | 17,8% |
| 359 | 8.141 | LLNGFLAV | 74,7 | 13,1% |

**Claims**

1. A method to identify a CTL epitope comprising the steps of:

   (a) generating primary position specific prediction means from experimental MHC class I structural or peptide binding data;
   (b) identifying potentially high affinity binding epitopes by scanning a set of protein sequences and calculating the binding affinity according to the primary prediction means obtained in step (a);

(c) optionally reducing the high number of peptides identified in step (b) by exclusion means based on sequence similarity;

(d) generating experimental binding data for the peptides identified in step (c) or (b);

(e) training one or more artificial neural networks to predict binding affinities to MHC class I, using the experimental binding data from step (d) such that the individual peptide binding data examples, weighted according to their frequency in sub-intervals in the binding affinity range of 1nM to 50,000 nM, are equally presented; and

(f) estimating the binding affinity of a query peptide by testing said query peptide in each of the artificial neural networks trained in step (e) obtaining an approximate binding affinity of the query peptide from each of the artificial neural networks, and calculating the weighted average of the approximate bindings thereby obtaining the estimated binding affinity of the query peptide;

the CTL epitope having a weighted average of the MHC class I binding affinity of less than 500 nM.

**2.** A method according to claim 1, wherein a PSCPL is used to generate primary position specific prediction means in step (a).

**3.** A method according to claim 1 or 2, wherein the MHC class I structural or peptide binding data in step (a) are HLA structural or peptide binding data.

**4.** A method according to any of the preceding claims, wherein the query peptide originates from a protein database, the protein database being a HIV-1 or HIV-2 protein sequence database, or a part thereof.

**5.** A method according to any of the preceding claims, further comprising the step of:

(k) determining the global conservation of the query peptide across a set of HIV protein sequences;

the CTL epitope of step (k) having an MHC class I binding of less than 500 nM.

**6.** A method according to claim 5, wherein the percent global conservation being representative for how often the CTI epitope , among the 9 HIV-1 proteins Gag, Pol, Env, Vif, Vpr, Vpu, Tat, Rev and Nef, is found in all subtypes and groups of HIV-1 is more than 1%.

**7.** A method according to any of the preceding claims, wherein the weighted average MHC class I binding is less than 100 nM.

**8.** A method according to any of claims 5-7, wherein the global conservation is more than 8% across HIV protein sequences.

**9.** A method according to claims 5-8, wherein the weighted average of the MHC class I binding is more than 50 nM.

**10.** A method according to any of the preceding claims, wherein the CTL epitope is a HIV virus epitope binding to the HLA complex.

**11.** A method according to any of the preceding claims, further comprising the steps of:

(I) modifying the CTL epitope by computationally replacing amino acids in the anchor positions; and

(m) estimating the binding affinity of the modified CTL epitope of step (I) by testing said modified CTL epitope in each of the artificial neural networks trained in step (e) obtaining an approximate binding affinity of the CTL epitope from each of the artificial neural networks, and calculating the weighted average of the approximate bindings thereby obtaining the estimated binding affinity of the CTL epitope;

the modified CTL epitope having a predicted MHC class I binding of less than 100 nM and being able to bind MHC class I with a lower predicted $EC_{50}$ value than that of the unmodified CTL epitope.

**12.** A CTL epitope having the sequence SEQ ID NO: 1.

**13.** A CTL epitope having the sequence SEQ ID NO: 1 for use in medicine.

**14.** Use of a CTL epitope having the sequence SEQ ID NO: 1 for the manufacture of a vaccine.

**15.** Use of a CTL epitope having the sequence SEQ ID NO: 1 for the manufacture of a diagnostic agent.

**Patentansprüche**

**1.** Verfahren zur Identifizierung eines CTL-Epitops, umfassend die Schritte:

(a) Erzeugen primärer positionsspezifischer Voraussagemöglichkeiten von experimentellen MHC-Klasse I-Struktur- oder Peptidbindungsdaten;
(b) Identifizieren von Epitopen mit einer potentiell hohen Bindungsaffinität durch Scannen eines Satzes von Proteinsequenzen und Kalkulieren der Bindungsaffinität gemäß den primären Voraussagemöglichkeiten erhalten in Schritt (a);
(c) gegebenenfalls Reduzieren der hohen Anzahl von Peptiden identifiziert in Schritt (b) durch Ausschlussmöglichkeiten basierend auf Sequenzähnlichkeiten;
(d) Erzeugen experimenteller Bindungsdaten für die Peptide identifiziert in Schritt (c) oder (b);
(e) Erproben eines oder weiterer künstlicher neuraler Netzwerke, um Bindungsaffinitäten zu MHC-Klasse I vorauszusagen, unter Verwendung der experimentellen Bindungsdaten aus Schritt (d) in der Form, dass die einzelnen Peptidbindungsdatenbeispiele, gewichtet entsprechend ihrer Häufigkeit in Sub-Intervallen im Bindungsaffinitätsbereich von 1 nM bis 50.000 nM, gleichmäßig dargelegt werden; und
(f) Abschätzen der Bindungsaffinität eines fraglichen Peptids durch Testen des fraglichen Peptids in jedem der künstlichen neuralen Netzwerke erprobt in Schritt (e), dabei eine ungefähre Bindungsaffinität des fraglichen Peptids von jedem der künstlichen neuralen Netzwerke erhaltend, und Kalkulieren des gewichteten Mittelwerts der ungefähren Bindungen, dabei die abgeschätzte Bindungsaffinität des fraglichen Peptids erhaltend;

wobei das CTL-Epitop einen gewichteten MHC-Klasse I-Bindungsaffinitäts-Mittelwert kleiner als 500 nM hat.

**2.** Verfahren nach Anspruch 1, wobei ein PSCPL verwendet wird, um primäre positionsspezifische Voraussagemöglichkeiten in Schritt (a) zu eszeugen.

**3.** Verfahren nach Anspruch 1 oder 2, wobei die MHC-Klasse 1-Struktur- oder Peptidbindungsdaten in Schritt (a) HLA-Struktur- oder Peptidbindungsdaten sind.

**4.** Verfahren nach einem der vorangehenden Ansprüche, wobei das fragliche Peptid aus einer Proteindatenbank stammt, wobei die Proteindatenbank eine HIV-1- oder HIV-2-Proteinsequenzdatenbank, oder ein Teil davon, ist.

**5.** Verfahren nach einem der vorangehenden Ansprüche, weiter umfassend den Schritt:

(k) Bestimmen der globalen Konservierung des fraglichen Peptids über einen Satz von HIV-Proteinsequenzen;

wobei das CTL-Epitop in Schritt (k) eine MHC-Klasse 1-Bindung kleiner als 500 nM hat.

**6.** Verfahren nach Anspruch 5, wobei die prozentuale globale Konservierung, die kennzeichnend dafür ist, wie oft das CTL-Epitop unter den 9 HIV-1 Proteinen Gag, Pol, Env, Vif, Vpr, Vpu, Tat, Rev und Nef, in allen Subtypen und HIV-1-Gruppen gefunden wird, größer als 1 % ist.

**7.** Verfahren nach einem der vorangehenden Ansprüche, wobei der gewichtete MHC-Klasse 1-Bindungs-Mittelwert kleiner als 100 nM ist.

**8.** Verfahren nach einem der Ansprüche 5 bis 7, wobei die globale Konservierung über HIV-Proteinsequenzen größer als 8 % ist.

**9.** Verfahren nach einem der Ansprüche 5 bis 8, wobei der gewichtete MHC-Klasse I-Bindungs-Mittelwert größer als 50 nM ist.

**10.** Verfahren nach einem der vorangehenden Ansprüche, wobei das CTL-Epitop ein HIV-Virusepitop ist, das den HLA-Komplex bindet.

**11.** Verfahren nach einem der vorangehenden Ansprüche, weiter umfassend die Schritte:

(l) Modifizieren des CTL-Epitops durch computerunterstütztes Ersetzen von Aminosäuren in den Ankerpositionen; und

(m) Abschätzen der Bindungsaffinität des modifizierten CTL-Epitops von Schritt (l) durch Testen des modifizierten CTL-Epitops in jedem der künstlichen neuralen Netzwerke erprobt in Schritt (e), dabei eine ungefähre Bindungsaffinität des CTL-Epitops von jedem der künstlichen neuralen Netzwerke erhaltend, und Kalkulieren des gewichteten Mittelwerts der ungefähren Bindungen, dabei erhaltend die abgeschätzten Bindungsaffinitäten des CTL-Epitops;

wobei das modifizierte CTL-Epitop eine vorausgesagte MHC-Klasse 1-Bindung kleiner als 100 nM hat und in der Lage ist, MHC-Klasse I mit einem kleiner vorausgesagten $EC_{50}$-Wert als dem des nicht-modifizierten CTL-Epitops zu binden.

**12.** CTL-Epitop mit der Sequenz von SEQ ID NO:1.

**13.** CTL-Epitop mit der Sequenz von SEQ ID NO:1 zur Verwendung in der Medizin.

**14.** Verwendung eines CTL-Epitops mit der Sequenz von SEQ ID NO:1 zur Herstellung eines Impfstoffs.

**15.** Verwendung eines CTL-Epitops mit der Sequenz von SEQ ID NO:1 zur Herstellung eines Diagnostikums.

**Revendications**

**1.** Procédé pour identifier un épitope CTL comprenant les étapes consistant à :

(a) produire des moyens de prévision spécifiques de position primaire à partir de données structurales ou de liaison peptidique MHC classe 1 expérimentales ;

(b) identifier des épitopes de liaison d'affinité potentiellement élevée en balayant un ensemble de séquences de protéine et en calculant l'affinité de liaison selon les moyens de prévision primaire obtenus à l'étape (a) ;

(c) réduire éventuellement le nombre élevé de peptides identifiés à l'étape (b) par des moyens d'exclusion basés sur une similitude de séquence ;

(d) produire des données de liaison expérimentales pour les peptides identifiés à l'étape (c) ou (b) ;

(e) former un ou plusieurs réseaux neuronaux artificiels pour prévoir des affinités de liaison avec un MHC classe I, en utilisant les données de liaison expérimentales obtenues à l'étape (d) de telle sorte que des exemples de données de liaison de peptide individuelles, pondérés selon leur fréquence dans des sous-intervalles dans la gamme d'affinité de liaison comprise entre 1 nM et 50.000 nM, soient présentés de manière égale ; et

(f) estimer l'affinité de liaison d'un peptide demandeur en testant ledit peptide demandeur dans chacun des réseaux neuronaux artificiels formés à l'étape (e), obtenir une affinité de liaison approximative du peptide demandeur à partir de chacun des réseaux neuronaux artificiels, et calculer la moyenne pondérée des liaisons approximatives en obtenant de ce fait l'affinité de liaison estimée du peptide demandeur ;

l'épitope CTL ayant une moyenne pondérée de l'affinité de liaison de MHC classe I inférieure à 500 nM.

**2.** Procédé selon la revendication 1, dans lequel un PSCPL est utilisé pour produire les moyens de prévision spécifiques de position primaire de l'étape (a).

**3.** Procédé selon l'une quelconque des revendications 1 ou 2, dans lequel les données structurales ou de liaison peptidique MHC classe 1 de l'étape (a) sont des données structurales ou de liaison peptidique HLA.

**4.** Procédé selon l'une quelconque des revendications précédentes, dans lequel le peptide demandeur provient d'une base de données de protéines, la base de données de protéines étant une base de données de séquences de protéines d'HIV - 1 ou d'HIV - 2, ou une partie de celle-ci.

**5.** Procédé selon l'une quelconque des revendications précédentes, comprenant de plus l'étape consistant à :

(k) déterminer la conservation globale du peptide demandeur à travers un ensemble de séquences de protéines d'HIV ;

l'épitope CTL de l'étape (k) ayant une liaison de MHC classe I inférieure à 500 nM.

**6.** Procédé selon la revendication 5, dans lequel la conservation globale en pour cent, représentative de la fréquence de rencontre de l'épitope CTL parmi les 9 protéines d'HIV - 1, Gag, Pol, Env, Vif, Vpr, Vpu, Tat, Rev et Nef, dans tous les sous-types et les groupes du HIV - 1, est inférieure à 1%.

**7.** Procédé selon l'une quelconque des revendications précédentes, dans lequel la moyenne pondérée de la liaison de MHC classe I est inférieure à 100 nM.

**8.** Procédé selon l'une quelconque des revendications 5 à 7, dans lequel la conservation globale est supérieure à 8 % dans les séquences de protéines d'HIV.

**9.** Procédé selon l'une quelconque des revendications 5 à 8, dans lequel la moyenne pondérée de la liaison de MHC classe I est supérieure à 50 nM.

**10.** Procédé selon l'une quelconque des revendications précédentes, dans lequel l'épitope CTL est un épitope de virus HIV lié au complexe HLA.

**11.** Procédé selon l'une quelconque des revendications précédentes, comprenant de plus les étapes consistant à :

(l) modifier l'épitope CTL en remplaçant de manière informatique des acides aminés dans les positions d'ancrage ;
et
(m) estimer l'affinité de liaison de l'épitope CTL modifié de l'étape (l) en testant ledit épitope CTL modifié dans chacun des réseaux neuronaux artificiels formés à l'étape (e), obtenir une affinité de liaison approximative de l'épitope CTL à partir de chacun des réseaux neuronaux artificiels, et calculer la moyenne pondérée des liaisons approximatives en obtenant de ce fait l'affinité de liaison estimée de l'épitope CTL ;

l'épitope CTL modifié ayant une liaison à un MHC classe 1 prévue inférieure à 100 nM et pouvant se lier à un MHC classe I avec une valeur prévue $EC_{50}$ inférieure à celle de l'épitope CTL non modifié.

**12.** Épitope CTL ayant la séquence SEQ ID NO : 1.

**13.** Épitope CTL ayant la séquence SEQ ID NO : 1, destiné à une utilisation en médecine.

**14.** Épitope CTL ayant la séquence SEQ ID NO : 1, destiné à la fabrication d'un vaccin.

**15.** Épitope CTL ayant la séquence SEQ ID NO : 1, destiné à la fabrication d'un agent de diagnostic.

H3 immunized (peptide ILKEPVHGV)

Fig.1A

**Not immunized re-stimulated w. LV9**

Fig. 1B

**Not immunized re-stimulated w. H3**

Fig.1C

A/ CTL response induced by the natural epitope (LTFGWCFKL)

Fig. 2A

B/ CTL response induced by the improved epitope (LLFGWCFKL)

Fig.2B

**8.54Mod=RLLNAWVKV (42nM) / 8.54Nat=RTLNAWVKV (170nM)**

**Mouse 1**

Fig. 3A

Fig. 3B

—◆— Unpulsed target
—⊠— Target pulsed with 8.56Nat
—▲— Target pulsed with 8.56Mod

**Mouse 2**

Fig. 3C

Fig. 3D

Fig. 3

SEQUENCE LISTING

<110> Statens Serum Institut

<120> HIV peptide and nucleic acids encoding them for diagnosis and control of HIV infections

<130> 21922xx1

<160> 1369

<170> FastSEQ for Windows Version 3.0

<210> 1
<211> 9
<212> PRT
<213> Artificial Sequence

<220>
<223> HIV peptide

<400> 1

```
            Leu Leu Ala Gly Val Asp Tyr Arg Ile
             1                   5
```

<210> 2
<211> 9
<212> PRT
<213> Artificial Sequence

<220>
<223> HIV peptide

<400> 2

```
                          Leu Leu Ala Lys Val Asp Tyr Arg Leu
                           1                   5
```

<210> 3
<211> 9
<212> PRT
<213> Artificial Sequence

<220>
<223> HIV peptide

<400> 3

```
                          Ile Leu Ala Ile Val Val Trp Thr Ile
                           1                   5
```

<210> 4
<211> 9
<212> PRT
<213> Artificial Sequence

<220>
<223> HIV peptide

<400> 4

```
                          Lys Leu Val Glu Met Gly His His Ala
                           1                   5
```

<210> 5
<211> 9
<212> PRT
<213> Artificial Sequence

<220>
<223> HIV peptide

<400> 5

```
                          Ala Leu Met Glu Met Gly His His Ala
                           1                   5
```

<210> 6
<211> 9
<212> PRT
<213> Artificial Sequence

<220>
<223> HIV peptide

<400> 6

```
Ala Leu Val Glu Met Gly His Leu Ala
 1               5
```

<210> 7
<211> 9
<212> PRT
<213> Artificial Sequence

<220>
<223> HIV peptide

<400> 7

```
Ala Leu Gly Glu Met Gly Pro Phe Ile
 1               5
```

<210> 8
<211> 8
<212> PRT
<213> Artificial Sequence

<220>
<223> HIV peptide

<400> 8

```
Ile Val Gly Leu Ile Val Ala Leu
 1               5
```

<210> 9
<211> 8
<212> PRT
<213> Artificial Sequence

<220>
<223> HIV peptide

<400> 9

```
Ile Val Gly Leu Ile Val Ala Val
 1               5
```

<210> 10
<211> 9
<212> PRT
<213> Artificial Sequence

<220>
<223> HIV peptide

<400> 10

```
Ala Leu Ile Arg Thr Leu Gln Gln Leu
 1               5
```

<210> 11
<211> 9
<212> PRT
<213> Artificial Sequence

<220>
<223> HIV peptide

<400> 11

```
Ala Leu Ile Arg Ile Leu Gln Gln Leu
 1               5
```

<210> 12
<211> 9
<212> PRT
<213> Artificial Sequence

<220>
<223> HIV peptide

<400> 12

```
Ala Leu Ile Arg Met Leu Gln Gln Leu
 1               5
```

<210> 13
<211> 8
<212> PRT
<213> Artificial Sequence

<220>
<223> HIV peptide

<400> 13

```
Leu His Gly Leu Gly Gln Tyr Val
 1               5
```

<210> 14
<211> 9
<212> PRT
<213> Artificial Sequence

<220>
<223> HIV peptide

<400> 14

```
                        Ser Leu Val Lys His His Met Tyr Val
                         1               5
```

<210> 15
<211> 9
<212> PRT
<213> Artificial Sequence

<220>
<223> HIV peptide

<400> 15.

```
                        Ser Leu Val Lys His His Met His Val
                         1               5
```

<210> 16
<211> 9
<212> PRT
<213> Artificial Sequence

<220>
<223> HIV peptide

<400> 16

```
                        Ser Leu Val Lys His His Ile Tyr Val
                         1               5
```

<210> 17
<211> 9
<212> PRT
<213> Artificial Sequence

<220>
<223> HIV peptide

<400> 17

```
                        Leu Val Ile Arg Thr Tyr Trp Gly Leu
                         1               5
```

<210> 18
<211> 9
<212> PRT
<213> Artificial Sequence

<220>
<223> HIV peptide

<400> 18

```
Arg Leu Arg Arg Tyr Ser Thr Gln Val
 1               5
```

<210> 19
<211> 9
<212> PRT
<213> Artificial Sequence

<220>
<223> HIV peptide

<400> 19

```
Arg Leu Lys Arg Tyr Ser Thr Gln Val
 1               5
```

<210> 20
<211> 9
<212> PRT
<213> Artificial Sequence

<220>
<223> HIV peptide

<400> 20

```
Gly Leu Ala Asp Gln Leu Ile His Leu
 1               5
```

<210> 21
<211> 9
<212> PRT
<213> Artificial Sequence

<220>
<223> HIV peptide

<400> 21

```
Asn Leu Ala Asp Gln Leu Ile His Leu
 1               5
```

<210> 22
<211> 8
<212> PRT
<213>.Artificial Sequence

<220>
<223> HIV peptide

<400> 22

```
                    Pro Leu Gly Asp Ala Arg Leu Val
                     1                   5
```

<210> 23
<211> 8
<212> PRT
<213> Artificial Sequence

<220>
<223> HIV peptide

<400> 23

```
                    Pro Leu Gly Asp Ala Lys Leu Val
                     1                   5
```

<210> 24
<211> 8
<212> PRT
<213> Artificial Sequence

<220>
<223> HIV peptide

<400> 24

```
                    Pro Leu Gly Glu Ala Arg Leu Val
                     1                   5
```

<210> 25
<211> 8
<212> PRT
<213> Artificial Sequence

<220>
<223> HIV peptide

<400> 25

```
                    Pro Leu Gly Asp Ala Thr Leu Val
                     1                   5
```

<210> 26
<211> 8
<212> PRT
<213> Artificial Sequence

<220>
<223> HIV peptide

<400> 26

```
                    Pro Leu Gly Asp Ala Arg Leu Val
                     1                   5
```

Lys Ile Gly Ser Leu Gln Tyr Leu
1               5

<210> 27
<211> 8
<212> PRT
<213> Artificial Sequence

<220>
<223> HIV peptide

<400> 27

Thr Leu Ile Pro Lys Gln Pro Leu
1               5

<210> 28
<211> 9
<212> PRT
<213> Artificial Sequence

<220>
<223> HIV peptide

<400> 28

Lys Leu Leu Tyr Gln Ser Asn Pro Leu
1               5

<210> 29
<211> 9
<212> PRT
<213> Artificial Sequence

<220>
<223> HIV peptide

<400> 29

Cys Leu Gly Arg Pro Ala Glu Pro Val
1               5

<210> 30
<211> 9
<212> PRT
<213> Artificial Sequence

<220>
<223> HIV peptide

<400> 30

```
                    Tyr Leu Gly Arg Pro Ala Glu Pro Val
                     1                   5
```

<210> 31
<211> 9
<212> PRT
<213> Artificial Sequence

<220>
<223> HIV peptide

<400> 31

```
                    Phe Leu Gly Arg Pro Ala Glu Pro Val
                     1                   5
```

<210> 32
<211> 9
<212> PRT
<213> Artificial Sequence

<220>
<223> HIV peptide

<400> 32

```
                    Cys Leu Gly Arg Pro Thr Glu Pro Val
                     1                   5
```

<210> 33
<211> 9
<212> PRT
<213> Artificial Sequence

<220>
<223> HIV peptide

<400> 33

```
                    Cys Leu Gly Arg Pro Glu Glu Pro Val
                     1                   5
```

<210> 34
<211> 9
<212> PRT
<213> Artificial Sequence

<220>
<223> HIV peptide

<400> 34

```
                          Phe Leu Gly Arg Pro Glu Glu Pro Val
                           1               5
```

<210> 35
<211> 9
<212> PRT
<213> Artificial Sequence

<220>
<223> HIV peptide

<400> 35

```
                          Tyr Leu Gly Arg Pro Glu Glu Pro Val
                           1               5
```

<210> 36
<211> 9
<212> PRT
<213> Artificial Sequence

<220>
<223> HIV peptide

<400> 36

```
                          Cys Leu Gly Arg Pro Pro Glu Pro Val
                           1               5
```

<210> 37
<211> 9
<212> PRT
<213> Artificial Sequence

<220>
<223> HIV peptide

<400> 37

```
                          Tyr Leu Gly Arg Pro Thr Glu Pro Val
                           1               5
```

<210> 38
<211> 9
<212> PRT
<213> Artificial Sequence

<220>
<223> HIV peptide

<400> 38

```
            Phe Leu Gly Arg Ser Ala Glu Pro Val
             1                   5
```

<210> 39
<211> 9
<212> PRT
<213> Artificial Sequence

<220>
<223> HIV peptide

<400> 39

```
            His Leu Gly Arg Pro Ala Glu Pro Val
             1                   5
```

<210> 40
<211> 9
<212> PRT
<213> Artificial Sequence

<220>
<223> HIV peptide

<400> 40

```
            Gly Met Gly Ser Pro Gln Ile Leu Val
             1                   5
```

<210> 41
<211> 9
<212> PRT
<213> Artificial Sequence

<220>
<223> HIV peptide

<400> 41

```
            Ile Leu Val Glu Ser Pro Thr Val Leu
             1                   5
```

<210> 42
<211> 9
<212> PRT
<213> Artificial Sequence

<220>
<223> HIV peptide

<400> 42

```
                    Val Leu Val Glu Pro Pro Val Val Leu
                     1               5
```

<210> 43
<211> 9
<212> PRT
<213> Artificial Sequence

<220>
<223> HIV peptide

<400> 43

```
                    Ile Leu Val Glu Ser Pro Thr Ile Leu
                     1               5
```

<210> 44
<211> 8
<212> PRT
<213> Artificial Sequence

<220>
<223> HIV peptide

<400> 44

```
                    Gly Met Gly Ser Pro Gln Ile Leu
                     1               5
```

<210> 45
<211> 8
<212> PRT
<213> Artificial Sequence

<220>
<223> HIV peptide

<400> 45

```
                    Ile Ser Gly Glu Pro Cys Met Val
                     1               5
```

<210> 46
<211> 8
<212> PRT
<213> Artificial Sequence

<220>
<223> HIV peptide

<400> 46

```
                    Ile Ser Gly Lys Pro Cys Ala Val
                     1               5
```

<210> 47
<211> 9
<212> PRT
<213> Artificial Sequence

<220>
<223> HIV peptide

<400> 47

```
                  Leu Leu Gly Arg Trp Lys Pro Lys Met
                   1               5
```

<210> 48
<211> 9
<212> PRT
<213> Artificial Sequence

<220>
<223> HIV peptide

<400> 48

```
                  Tyr Met Glu Ala Glu Val Ile Pro Ala
                   1               5
```

<210> 49
<211> 9
<212> PRT
<213> Artificial Sequence

<220>
<223> HIV peptide

<400> 49

```
                  Tyr Leu Glu Ala Glu Val Ile Pro Ala
                   1               5
```

<210> 50
<211> 9
<212> PRT
<213> Artificial Sequence

<220>
<223> HIV peptide

<400> 50

```
                              Leu Ala Gly Arg Trp Pro Val Lys Val
                               1               5
```

<210> 51
<211> 9
<212> PRT
<213> Artificial Sequence

<220>
<223> HIV peptide

<400> 51

```
                              Leu Ala Ala Arg Trp Pro Val Lys Val
                               1               5
```

<210> 52
<211> 9
<212> PRT
<213> Artificial Sequence

<220>
<223> HIV peptide

<400> 52

```
                              Leu Thr Leu Ala Gly Arg Trp Pro Val
                               1               5
```

<210> 53
<211> 9
<212> PRT
<213> Artificial Sequence

<220>
<223> HIV peptide

<400> 53

```
                              Ala Met Lys Ala Ala Cys Trp Trp Ala
                               1               5
```

<210> 54
<211> 9
<212> PRT
<213> Artificial Sequence

<220>
<223> HIV peptide

<400> 54

```
Ala Leu Gln Lys Gln Ile Thr Lys Ile
 1               5
```

<210> 55
<211> 9
<212> PRT
<213> Artificial Sequence

<220>
<223> HIV peptide

<400> 55

```
Glu Leu Gln Lys Gln Ile Thr Lys Val
 1               5
```

<210> 56
<211> 9
<212> PRT
<213> Artificial Sequence

<220>
<223> HIV peptide

<400> 56

```
Asn Leu Gln Thr Gln Ile Leu Lys Val
 1               5
```

<210> 57
<211> 9
<212> PRT
<213> Artificial Sequence

<220>
<223> HIV peptide

<400> 57

```
Ile Leu Lys Ile Gln Asn Phe Arg Val
 1               5
```

<210> 58
<211> 9
<212> PRT
<213> Artificial Sequence

<220>
<223> HIV peptide

<400> 58

```
                              Asp Leu Gly Asp Ala Tyr Phe Ser Val
                               1               5
```

<210> 59
<211> 9
<212> PRT
<213> Artificial Sequence

<220>
<223> HIV peptide

<400> 59

```
                              Lys Leu His Pro Glu Gln Ala Arg Ala
                               1               5
```

<210> 60
<211> 9
<212> PRT
<213> Artificial Sequence

<220>
<223> HIV peptide

<400> 60

```
                              Ile Leu Ala Ser Gln Ile Gln Thr Thr
                               1               5
```

<210> 61
<211> 9
<212> PRT
<213> Artificial Sequence

<220>
<223> HIV peptide

<400> 61

```
                              Leu Thr Ala Glu Ala Glu Met Glu Leu
                               1               5
```

<210> 62
<211> 9
<212> PRT
<213> Artificial Sequence

<220>
<223> HIV peptide

<400> 62

```
Met Thr Ala Glu Ala Glu Met Glu Leu
 1               5
```

<210> 63
<211> 9
<212> PRT
<213> Artificial Sequence

<220>
<223> HIV peptide

<400> 63

```
Ile Leu Lys Glu Pro Val His Gly Ala
 1               5
```

<210> 64
<211> 9
<212> PRT
<213> Artificial Sequence

<220>
<223> HIV peptide

<400> 64

```
Ile Leu Lys Asp Pro Val His Gly Val
 1               5
```

<210> 65
<211> 9
<212> PRT
<213> Artificial Sequence

<220>
<223> HIV peptide

<400> 65

```
Ile Leu Arg Glu Pro Val His Gly Val
 1               5
```

<210> 66
<211> 9
<212> PRT
<213> Artificial Sequence

<220>
<223> HIV peptide

<400> 66

```
Ile Leu Lys Thr Pro Val His Gly Val
 1               5
```

<210> 67
<211> 9
<212> PRT
<213> Artificial Sequence

<220>
<223> HIV peptide

<400> 67

```
Ile Leu Lys Asp Pro Val His Gly Ala
 1               5
```

<210> 68
<211> 9
<212> PRT
<213> Artificial Sequence

<220>
<223> HIV peptide

<400> 68

```
Lys Leu Lys Glu Pro Val His Gly Val
 1               5
```

<210> 69
<211> 9
<212> PRT
<213> Artificial Sequence

<220>
<223> HIV peptide

<400> 69

```
Ile Leu Lys Ala Pro Val His Gly Val
 1               5
```

<210> 70
<211> 9
<212> PRT
<213> Artificial Sequence

<220>
<223> HIV peptide

<400> 70

```
Ile Leu Lys Glu Pro Ile His Gly Val
 1                   5
```

<210> 71
<211> 9
<212> PRT
<213> Artificial Sequence

<220>
<223> HIV peptide

<400> 71

```
Ile Leu Arg Asp Pro Val His Gly Val
 1                   5
```

<210> 72
<211> 9
<212> PRT
<213> Artificial Sequence

<220>
<223> HIV peptide

<400> 72

```
Ile Leu Lys Asp Pro Val His Trp Val
 1                   5
```

<210> 73
<211> 9
<212> PRT
<213> Artificial Sequence

<220>
<223> HIV peptide

<400> 73

```
Ile Leu Arg Glu Pro Ile His Gly Val
 1                   5
```

<210> 74
<211> 9
<212> PRT
<213> Artificial Sequence

<220>
<223> HIV peptide

<400> 74

```
Ile Leu Lys Glu Pro Val His Glu Val
 1                   5
```

<210> 75
<211> 9
<212> PRT
<213> Artificial Sequence

<220>
<223> HIV peptide

<400> 75

```
Ile Leu Lys Glu Pro Leu His Gly Val
 1                   5
```

<210> 76
<211> 9
<212> PRT
<213> Artificial Sequence

<220>
<223> HIV peptide

<400> 76

```
Ile Leu Lys Glu Pro Met His Gly Val
 1                   5
```

<210> 77
<211> 9
<212> PRT
<213> Artificial Sequence

<220>
<223> HIV peptide

<400> 77

```
Arg Leu Lys Gln Pro Val His Gly Val
 1                   5
```

<210> 78
<211> 9
<212> PRT
<213> Artificial Sequence

<220>
<223> HIV peptide

<400> 78

```
Ile Leu Arg Ile Pro Val His Gly Val
 1               5
```

<210> 79
<211> 9
<212> PRT
<213> Artificial Sequence

<220>
<223> HIV peptide

<400> 79

```
Ile Leu Lys Glu Ser Val His Gly Val
 1               5
```

<210> 80
<211> 9
<212> PRT
<213> Artificial Sequence

<220>
<223> HIV peptide

<400> 80

```
Ile Leu Arg Lys Pro Val His Glu Val
 1               5
```

<210> 81
<211> 9
<212> PRT
<213> Artificial Sequence

<220>
<223> HIV peptide

<400> 81

```
Ile Leu Lys Val Pro Val His Gly Val
 1               5
```

<210> 82
<211> 9
<212> PRT
<213> Artificial Sequence

<220>
<223> HIV peptide

<400> 82

```
Gln Leu Ala Glu Val Val Gln Lys Val
1               5
```

<210> 83
<211> 9
<212> PRT
<213> Artificial Sequence

<220>
<223> HIV peptide

<400> 83

```
Gln Leu Thr Glu Val Val Gln Lys Val
1               5
```

<210> 84
<211> 9
<212> PRT
<213> Artificial Sequence

<220>
<223> HIV peptide

<400> 84

```
Gln Leu Thr Glu Ala Val Gln Lys Val
1               5
```

<210> 85
<211> 9
<212> PRT
<213> Artificial Sequence

<220>
<223> HIV peptide

<400> 85

```
Gln Leu Ala Glu Val Val Gln Lys Ile
1               5
```

<210> 86
<211> 9
<212> PRT
<213> Artificial Sequence

<220>
<223> HIV peptide

<400> 86

```
Gln Leu Ala Glu Ala Val Gln Lys Ile
1               5
```

<210> 87
<211> 9
<212> PRT
<213> Artificial Sequence

<220>
<223> HIV peptide

<400> 87

```
Gln Leu Ala Glu Met Val Gln Lys Val
1               5
```

<210> 88
<211> 9
<212> PRT
<213> Artificial Sequence

<220>
<223> HIV peptide

<400> 88

```
Gln Leu Ala Glu Val Ile Gln Lys Val
1               5
```

<210> 89
<211> 9
<212> PRT
<213> Artificial Sequence

<220>
<223> HIV peptide

<400> 89

```
Gln Leu Thr Ala Val Val Gln Lys Val
1               5
```

<210> 90
<211> 9
<212> PRT
<213> Artificial Sequence

<220>
<223> HIV peptide

<400> 90

```
                    Gln Leu Val Glu Val Val Gln Lys Val
                     1               5
```

<210> 91
<211> 9
<212> PRT
<213> Artificial Sequence

<220>
<223> HIV peptide

<400> 91

```
                    Tyr Leu Leu Glu Glu Asp Pro Ile Val
                     1               5
```

<210> 92
<211> 9
<212> PRT
<213> Artificial Sequence

<220>
<223> HIV peptide

<400> 92

```
                    Asn Leu Ala Phe Pro Gln Trp Lys Ala
                     1               5
```

<210> 93
<211> 9
<212> PRT
<213> Artificial Sequence

<220>
<223> HIV peptide

<400> 93

```
                    Lys Leu Ser Ser Glu Gln Thr Arg Ala
                     1               5
```

<210> 94
<211> 9
<212> PRT
<213> Artificial Sequence

<220>
<223> HIV peptide

<400> 94

```
Ser Leu Ser Phe Pro Gln Ile Thr Leu
 1               5
```

<210> 95
<211> 9
<212> PRT
<213> Artificial Sequence

<220>
<223> HIV peptide

<400> 95

```
Ser Leu Ser Leu Pro Gln Ile Thr Leu
 1               5
```

<210> 96
<211> 9
<212> PRT
<213> Artificial Sequence

<220>
<223> HIV peptide

<400> 96

```
Ser Leu Asn Phe Pro Gln Ile Thr Leu
 1               5
```

<210> 97
<211> 9
<212> PRT
<213> Artificial Sequence

<220>
<223> HIV peptide

<400> 97

```
Ala Leu Asn Phe Pro Gln Ile Thr Leu
 1               5
```

<210> 98
<211> 9
<212> PRT
<213> Artificial Sequence

<220>
<223> HIV peptide

<400> 98

```
Ser Leu Ser Phe Pro Gln Thr Thr Leu
 1               5
```

<210> 99
<211> 9
<212> PRT
<213> Artificial Sequence

<220>
<223> HIV peptide

<400> 99

```
Ser Leu Cys Phe Pro Gln Ile Thr Leu
 1               5
```

<210> 100
<211> 9
<212> PRT
<213> Artificial Sequence

<220>
<223> HIV peptide

<400> 100

```
Thr Leu Asn Cys Pro Gln Ile Thr Leu
 1               5
```

<210> 101
<211> 9
<212> PRT
<213> Artificial Sequence

<220>
<223> HIV peptide

<400> 101

```
Ile Ile Gly Ala Glu Thr Phe Tyr Val
 1               5
```

<210> 102
<211> 9
<212> PRT
<213> Artificial Sequence

<220>
<223> HIV peptide

<400> 102

```
                    Ile Met Gly Ala Glu Thr Phe Tyr Val
                     1               5
```

<210> 103
<211> 9
<212> PRT
<213> Artificial Sequence

<220>
<223> HIV peptide

<400> 103

```
                    Ile Leu Gly Ala Glu Thr Phe Tyr Val
                     1               5
```

<210> 104
<211> 9
<212> PRT
<213> Artificial Sequence

<220>
<223> HIV peptide

<400> 104

```
                    Ile Met Gly Ala Glu Thr Tyr Tyr Val
                     1               5
```

<210> 105
<211> 9
<212> PRT
<213> Artificial Sequence

<220>
<223> HIV peptide

<400> 105

```
                    Ile Thr Gly Ala Glu Thr Phe Tyr Val
                     1               5
```

<210> 106
<211> 9
<212> PRT
<213> Artificial Sequence

<220>
<223> HIV peptide

<400> 106

```
                    Ile Met Gly Ala Glu Thr Phe Tyr Val
                     1               5
```

```
Ile Val Gly Ala Asp Ser Phe Phe Val
1               5
```

<210> 107
<211> 9
<212> PRT
<213> Artificial Sequence

<220>
<223> HIV peptide

<400> 107

```
Ile Thr Leu Trp Gln Pro Pro Leu Val
1               5
```

<210> 108
<211> 9
<212> PRT
<213> Artificial Sequence

<220>
<223> HIV peptide

<400> 108

```
Glu Leu Gln Ala Ile Leu Met Ala Leu
1               5
```

<210> 109
<211> 9
<212> PRT
<213> Artificial Sequence

<220>
<223> HIV peptide

<400> 109

```
Tyr Leu Ala Leu Gln Asp Ser Gly Val
1               5
```

<210> 110
<211> 9
<212> PRT
<213> Artificial Sequence

<220>
<223> HIV peptide

<400> 110

```
Ala Leu Gln Asp Ser Gly Pro Glu Val
 1               5
```

<210> 111
<211> 9
<212> PRT
<213> Artificial Sequence

<220>
<223> HIV peptide

<400> 111

```
Ala Leu Gln Asp Ser Gln Ser Glu Val
 1               5
```

<210> 112
<211> 9
<212> PRT
<213> Artificial Sequence

<220>
<223> HIV peptide

<400> 112

```
Ala Leu Gln Glu Ser Gly Pro Glu Val
 1               5
```

<210> 113
<211> 8
<212> PRT
<213> Artificial Sequence

<220>
<223> HIV peptide

<400> 113

```
Lys Ile Gly Gly Gln Leu Lys Val
 1               5
```

<210> 114
<211> 8
<212> PRT
<213> Artificial Sequence

<220>
<223> HIV peptide

<400> 114

```
Val Leu Ile Gly Pro Thr Pro Val
 1               5
```

<210> 115
<211> 8
<212> PRT
<213> Artificial Sequence

<220>
<223> HIV peptide

<400> 115

```
Ile Leu Val Gly Pro Thr Pro Val
 1               5
```

<210> 116
<211> 8
<212> PRT
<213> Artificial Sequence

<220>
<223> HIV peptide

<400> 116

```
Ala Leu Thr Asp Ile Val Pro Leu
 1               5
```

<210> 117
<211> 8
<212> PRT
<213> Artificial Sequence

<220>
<223> HIV peptide

<400> 117

```
Val Leu Thr Asp Ile Val Pro Leu
 1               5
```

<210> 118
<211> 8
<212> PRT
<213> Artificial Sequence

<220>
<223> HIV peptide

<400> 118

```
                    Thr Leu Thr Asp Ile Val Pro Leu
                     1               5
```

<210> 119
<211> 8
<212> PRT
<213> Artificial Sequence

<220>
<223> HIV peptide

<400> 119

```
                    Phe Val Asn Thr Pro Pro Leu Val
                     1               5
```

<210> 120
<211> 8
<212> PRT
<213> Artificial Sequence

<220>
<223> HIV peptide

<400> 120

```
                    Phe Val Asn Thr Pro His Leu Val
                     1               5
```

<210> 121
<211> 8
<212> PRT
<213> Artificial Sequence

<220>
<223> HIV peptide

<400> 121

```
                    Phe Val Asn Thr Pro Leu Leu Val
                     1               5
```

<210> 122
<211> 8
<212> PRT
<213> Artificial Sequence

<220>
<223> HIV peptide

<400> 122

```
Leu Gln Gly Lys Ala Arg Lys Leu
 1               5
```

<210> 123
<211> 8
<212> PRT
<213> Artificial Sequence

<220>
<223> HIV peptide

<400> 123

```
Lys Leu Gly Lys Ala Gly Tyr Val
 1               5
```

<210> 124
<211> 9
<212> PRT
<213> Artificial Sequence

<220>
<223> HIV peptide

<400> 124

```
Leu Thr Phe Gly Trp Cys Phe Lys Leu
 1               5
```

<210> 125
<211> 9
<212> PRT
<213> Artificial Sequence

<220>
<223> HIV peptide

<400> 125

```
Leu Thr Leu Gly Trp Cys Phe Lys Leu
 1               5
```

<210> 126
<211> 9
<212> PRT
<213> Artificial Sequence

<220>
<223> HIV peptide

<400> 126

```
Leu Thr Pro Gly Trp Cys Phe Lys Leu
 1               5
```

<210> 127
<211> 9
<212> PRT
<213> Artificial Sequence

<220>
<223> HIV peptide

<400> 127

```
Arg Leu Ala Tyr His His Met Ala Arg
 1               5
```

<210> 128
<211> 8
<212> PRT
<213> Artificial Sequence

<220>
<223> HIV peptide

<400> 128

```
Thr Leu Gly Trp Cys Phe Lys Leu
 1               5
```

<210> 129
<211> 9
<212> PRT
<213> Artificial Sequence

<220>
<223> HIV peptide

<400> 129

```
Tyr Met Met Lys His Leu Val Trp Ala
 1               5
```

<210> 130
<211> 9
<212> PRT
<213> Artificial Sequence

<220>
<223> HIV peptide

<400> 130

```
                              Ser Leu Tyr Asn Thr Val Ala Val Leu
                               1               5
```

<210> 131
<211> 9
<212> PRT
<213> Artificial Sequence

<220>
<223> HIV peptide

<400> 131

```
                              Ser Leu Tyr Asn Thr Ile Ala Thr Leu
                               1               5
```

<210> 132
<211> 9
<212> PRT
<213> Artificial Sequence

<220>
<223> HIV peptide

<400> 132

```
                              Ser Leu Phe Asn Thr Val Ala Val Leu
                               1               5
```

<210> 133
<211> 9
<212> PRT
<213> Artificial Sequence

<220>
<223> HIV peptide

<400> 133

```
                              Ser Leu Phe Asn Thr Ile Ala Thr Leu
                               1               5
```

<210> 134
<211> 9
<212> PRT
<213> Artificial Sequence

<220>
<223> HIV peptide

<400> 134

```
                        Ser Leu Tyr Asn Ala Val Ala Thr Leu
                         1               5
```

<210> 135
<211> 9
<212> PRT
<213> Artificial Sequence

<220>
<223> HIV peptide

<400> 135

```
                        Asn Thr Ile Ala Thr Leu Trp Cys Val
                         1               5
```

<210> 136
<211> 9
<212> PRT
<213> Artificial Sequence

<220>
<223> HIV peptide

<400> 136

```
                        Asp Leu Asn Ala Met Leu Asn Thr Val
                         1               5
```

<210> 137
<211> 9
<212> PRT
<213> Artificial Sequence

<220>
<223> HIV peptide

<400> 137

```
                        Thr Leu Gln Glu Gln Ile Thr Trp Met
                         1               5
```

<210> 138
<211> 9
<212> PRT
<213> Artificial Sequence

<220>
<223> HIV peptide

<400> 138

```
                    Ser Leu Gln Glu Gln Ile Ala Trp Met
                     1               5
```

<210> 139
<211> 9
<212> PRT
<213> Artificial Sequence

<220>
<223> HIV peptide

<400> 139

```
                    Met Thr Asn Asn Pro Pro Ile Pro Val
                     1               5
```

<210> 140
<211> 9
<212> PRT
<213> Artificial Sequence

<220>
<223> HIV peptide

<400> 140

```
                    Met Thr Ser Asn Pro Pro Ile Pro Val
                     1               5
```

<210> 141
<211> 9
<212> PRT
<213> Artificial Sequence

<220>
<223> HIV peptide

<400> 141

```
                    Met Thr Gly Asn Pro Pro Ile Pro Val
                     1               5
```

<210> 142
<211> 9
<212> PRT
<213> Artificial Sequence

<220>
<223> HIV peptide

<400> 142

```
Met Thr Ser Asn Pro Pro Val Pro Val
 1               5
```

<210> 143
<211> 9
<212> PRT
<213> Artificial Sequence

<220>
<223> HIV peptide

<400> 143

```
Met Thr Gly Asn Pro Pro Val Pro Val
 1               5
```

<210> 144
<211> 9
<212> PRT
<213> Artificial Sequence

<220>
<223> HIV peptide

<400> 144

```
Met Thr Asn Asn Pro Pro Val Pro Val
 1               5
```

<210> 145
<211> 9
<212> PRT
<213> Artificial Sequence

<220>
<223> HIV peptide

<400> 145

```
Met Thr His Asn Pro Pro Ile Pro Val
 1               5
```

<210> 146
<211> 9
<212> PRT
<213> Artificial Sequence

<220>
<223> HIV peptide

<400> 146

```
Met Thr Gly Asn Pro Ala Ile Pro Val
 1               5
```

<210> 147
<211> 9
<212> PRT
<213> Artificial Sequence

<220>
<223> HIV peptide

<400> 147

```
Lys Met Val Lys Met Tyr Ser Pro Val
 1               5
```

<210> 148
<211> 9
<212> PRT
<213> Artificial Sequence

<220>
<223> HIV peptide

<400> 148

```
Lys Met Tyr Ser Pro Val Ser Ile Leu
 1               5
```

<210> 149
<211> 9
<212> PRT
<213> Artificial Sequence

<220>
<223> HIV peptide

<400> 149

```
Val Leu Ala Glu Ala Met Ser Gln Val
 1               5
```

<210> 150
<211> 9
<212> PRT
<213> Artificial Sequence

<220>
<223> HIV peptide

<400> 150

```
Ile Leu Ala Glu Ala Met Ser Gln Val
 1               5
```

<210> 151
<211> 8
<212> PRT
<213> Artificial Sequence

<220>
<223> HIV peptide

<400> 151

```
Ile Leu Gly Gln Leu Gln Pro Ser
 1               5
```

<210> 152
<211> 8
<212> PRT
<213> Artificial Sequence

<220>
<223> HIV peptide

<400> 152

```
Ile Leu Gly Gln Leu Gln Pro Ala
 1               5
```

<210> 153
<211> 8
<212> PRT
<213> Artificial Sequence

<220>
<223> HIV peptide

<400> 153

```
Ile Ile Gly Gln Leu Gln Pro Ala
 1               5
```

<210> 154
<211> 8
<212> PRT
<213> Artificial Sequence

<220>
<223> HIV peptide

<400> 154

```
Ile Leu Ala Glu Ala Met Ser Gln Val
 1               5
```

```
                        Thr Ser Asn Pro Pro Val Pro Val
                         1                   5
```

<210> 155
<211> 8
<212> PRT
<213> Artificial Sequence

<220>
<223> HIV peptide

<400> 155

```
                        Thr Asn Asn Pro Pro Val Pro Val
                         1                   5
```

<210> 156
<211> 8
<212> PRT
<213> Artificial Sequence

<220>
<223> HIV peptide

<400> 156

```
                        Thr His Asn Pro Pro Ile Pro Val
                         1                   5
```

<210> 157
<211> 8
<212> PRT
<213> Artificial Sequence

<220>
<223> HIV peptide

<400> 157

```
                        Ala Leu Gly Pro Ala Ala Thr Leu
                         1                   5
```

<210> 158
<211> 8
<212> PRT
<213> Artificial Sequence

<220>
<223> HIV peptide

<400> 158

```
                              Phe Leu Gly Lys Ile Trp Pro Ser
                               1               5
```

<210> 159
<211> 8
<212> PRT
<213> Artificial Sequence

<220>
<223> HIV peptide

<400> 159

```
                              Phe Leu Gly Arg Ile Trp Pro Ser
                               1               5
```

<210> 160
<211> 9
<212> PRT
<213> Artificial Sequence

<220>
<223> HIV peptide

<400> 160

```
                           Tyr Gln Gln Trp Trp Ile Trp Gly Val
                            1               5
```

<210> 161
<211> 9
<212> PRT
<213> Artificial Sequence

<220>
<223> HIV peptide

<400> 161

```
                           Met Leu Gln Trp Gly Thr Met Leu Leu
                            1               5
```

<210> 162
<211> 9
<212> PRT
<213> Artificial Sequence

<220>
<223> HIV peptide

<400> 162

**EP 1 250 351 B1**

```
                    Ala Leu Phe Tyr Arg Leu Asp Val Val
                     1                   5
```

<210> 163
<211> 9
<212> PRT
<213> Artificial Sequence

<220>
<223> HIV peptide

<400> 163

```
                    Ala Leu Phe Tyr Arg Leu Asp Ile Val
                     1                   5
```

<210> 164
<211> 9
<212> PRT
<213> Artificial Sequence

<220>
<223> HIV peptide

<400> 164

```
                    Ser Leu Phe Tyr Arg Leu Asp Ile Val
                     1                   5
```

<210> 165
<211> 9
<212> PRT
<213> Artificial Sequence

<220>
<223> HIV peptide

<400> 165

```
                    Ser Leu Phe Tyr Arg Leu Asp Val Val
                     1                   5
```

<210> 166
<211> 9
<212> PRT
<213> Artificial Sequence

<220>
<223> HIV peptide

<400> 166

108

```
Ala Leu Phe Tyr Asn Leu Asp Val Val
 1               5
```

<210> 167
<211> 9
<212> PRT
<213> Artificial Sequence

<220>
<223> HIV peptide

<400> 167

```
Phe Cys Ala Pro Ala Gly Phe Ala Ile
 1               5
```

<210> 168
<211> 9
<212> PRT
<213> Artificial Sequence

<220>
<223> HIV peptide

<400> 168

```
Ser Leu Ala Glu Glu Glu Val Val Leu
 1               5
```

<210> 169
<211> 9
<212> PRT
<213> Artificial Sequence

<220>
<223> HIV peptide

<400> 169

```
Lys Leu Ala Glu His Phe Pro Asn Lys
 1               5
```

<210> 170
<211> 9
<212> PRT
<213> Artificial Sequence

<220>
<223> HIV peptide

<400> 170

```
                    Met Tyr Ala Pro Pro Ile Gln Gly Val
                     1               5
```

<210> 171
<211> 9
<212> PRT
<213> Artificial Sequence

<220>
<223> HIV peptide

<400> 171

```
                    Asn Leu Ala Ser Gly Ile Gln Lys Val
                     1               5
```

<210> 172
<211> 9
<212> PRT
<213> Artificial Sequence

<220>
<223> HIV peptide

<400> 172

```
                    Ile Tyr Ala Pro Pro Ile Gln Gly Val
                     1               5
```

<210> 173
<211> 9
<212> PRT
<213> Artificial Sequence

<220>
<223> HIV peptide

<400> 173

```
                    Ser Leu Gly Val Ala Pro Thr Arg Ala
                     1               5
```

<210> 174
<211> 9
<212> PRT
<213> Artificial Sequence

<220>
<223> HIV peptide

<400> 174

```
                    Met Tyr Ala Pro Pro Ile Gln Gly Val
                     1               5
```

```
                    Phe Leu Ser Ala Ala Gly Ser Thr Met
                      1               5
```

<210> 175
<211> 9
<212> PRT
<213> Artificial Sequence

<220>
<223> HIV peptide

<400> 175

```
                    Thr Met Gly Ala Ala Ser Met Thr Leu
                      1               5
```

<210> 176
<211> 9
<212> PRT
<213> Artificial Sequence

<220>
<223> HIV peptide

<400> 176

```
                    Thr Met Gly Ala Ala Ala Thr Ala Leu
                      1               5
```

<210> 177
<211> 9
<212> PRT
<213> Artificial Sequence

<220>
<223> HIV peptide

<400> 177

```
                    Thr Met Gly Ala Ala Ala Val Thr Leu
                      1               5
```

<210> 178
<211> 9
<212> PRT
<213> Artificial Sequence

<220>
<223> HIV peptide

<400> 178

```
                    Thr Met Gly Ala Arg Ser Met Thr Leu
                     1               5
```

<210> 179
<211> 9
<212> PRT
<213> Artificial Sequence

<220>
<223> HIV peptide

<400> 179

```
                    Ala Ile Gln Ala Gln Gln Gln Leu Leu
                     1               5
```

<210> 180
<211> 9
<212> PRT
<213> Artificial Sequence

<220>
<223> HIV peptide

<400> 180

```
                    Leu Leu Gln Leu Thr Val Trp Gly Ile
                     1               5
```

<210> 181
<211> 9
<212> PRT
<213> Artificial Sequence

<220>
<223> HIV peptide

<400> 181

```
                    Met Leu Gln Leu Thr Val Trp Gly Ile
                     1               5
```

<210> 182
<211> 9
<212> PRT
<213> Artificial Sequence

<220>
<223> HIV peptide

<400> 182

```
                    Ser Leu Gln Gly Phe Leu Pro Leu Leu
                     1               5
```

<210> 183
<211> 9
<212> PRT
<213> Artificial Sequence

<220>
<223> HIV peptide

<400> 183

```
                    Leu Ile Ala Ala Arg Ile Val Glu Leu
                     1               5
```

<210> 184
<211> 9
<212> PRT
<213> Artificial Sequence

<220>
<223> HIV peptide

<400> 184

```
                    Leu Leu Gly Arg Arg Gly Trp Glu Ala
                     1               5
```

<210> 185
<211> 9
<212> PRT
<213> Artificial Sequence

<220>
<223> HIV peptide

<400> 185

```
                    Leu Leu Gly Arg Arg Gly Trp Glu Val
                     1               5
```

<210> 186
<211> 9
<212> PRT
<213> Artificial Sequence

<220>
<223> HIV peptide

<400> 186

```
Leu Leu Gly Arg Arg Gly Trp Glu Ile
 1                   5
```

<210> 187
<211> 9
<212> PRT
<213> Artificial Sequence

<220>
<223> HIV peptide

<400> 187

```
Ile Leu Gly Arg Arg Gly Trp Glu Ala
 1                   5
```

<210> 188
<211> 9
<212> PRT
<213> Artificial Sequence

<220>
<223> HIV peptide

<400> 188

```
Leu Leu Gly Arg Arg Gly Trp Glu Leu
 1                   5
```

<210> 189
<211> 9
<212> PRT
<213> Artificial Sequence

<220>
<223> HIV peptide

<400> 189

```
Leu Leu Leu Tyr Trp Gly Gln Glu Leu
 1                   5
```

<210> 190
<211> 9
<212> PRT
<213> Artificial Sequence

<220>
<223> HIV peptide

<400> 190

```
Leu Leu Gln Tyr Trp Ile Gln Glu Leu
 1               5
```

<210> 191
<211> 9
<212> PRT
<213> Artificial Sequence

<220>
<223> HIV peptide

<400> 191

```
Leu Leu Gln Tyr Trp Gly Gln Glu Leu
 1               5
```

<210> 192
<211> 9
<212> PRT
<213> Artificial Sequence

<220>
<223> HIV peptide

<400> 192

```
Leu Leu Gln Tyr Trp Gly Gln Glu Ile
 1               5
```

<210> 193
<211> 9
<212> PRT
<213> Artificial Sequence

<220>
<223> HIV peptide

<400> 193

```
Ser Leu Leu Asp Thr Ile Ala Ile Ala
 1               5
```

<210> 194
<211> 9
<212> PRT
<213> Artificial Sequence

<220>
<223> HIV peptide

<400> 194

```
                        Ser Leu Phe Asp Thr Ile Ala Ile Ala
                         1               5
```

<210> 195
<211> 9
<212> PRT
<213> Artificial Sequence

<220>
<223> HIV peptide

<400> 195

```
                        Leu Leu Asp Ala Thr Ala Ile Ala Val
                         1               5
```

<210> 196
<211> 9
<212> PRT
<213> Artificial Sequence

<220>
<223> HIV peptide

<400> 196

```
                        Leu Leu Asn Thr Thr Ala Ile Ala Val
                         1               5
```

<210> 197
<211> 9
<212> PRT
<213> Artificial Sequence

<220>
<223> HIV peptide

<400> 197

```
                        Leu Leu Asn Thr Thr Ala Ile Val Val
                         1               5
```

<210> 198
<211> 9
<212> PRT
<213> Artificial Sequence

<220>
<223> HIV peptide

<400> 198

```
                    Leu Leu Asn Ala Ile Ala Ile Ala Val
                     1                   5
```

<210> 199
<211> 9
<212> PRT
<213> Artificial Sequence

<220>
<223> HIV peptide

<400> 199

```
                    Arg Ile Ile Glu Val Val Gln Arg Val
                     1                   5
```

<210> 200
<211> 8
<212> PRT
<213> Artificial Sequence

<220>
<223> HIV peptide

<400> 200

```
                    His Ile Gly Pro Gly Gln Ala Leu
                     1                   5
```

<210> 201
<211> 8
<212> PRT
<213> Artificial Sequence

<220>
<223> HIV peptide

<400> 201

```
                    Ile Ile Gly Asp Ile Arg Lys Ala
                     1                   5
```

<210> 202
<211> 8
<212> PRT
<213> Artificial Sequence

<220>
<223> HIV peptide

<400> 202

```
                         Leu Gly Gly Asp Pro Glu Ile Val
                          1               5
```

<210> 203
<211> 8
<212> PRT
<213> Artificial Sequence

<220>
<223> HIV peptide

<400> 203

```
                         Ile Ile Asn Met Trp Gln Glu Val
                          1               5
```

<210> 204
<211> 8
<212> PRT
<213> Artificial Sequence

<220>
<223> HIV peptide

<400> 204

```
                         Ile Ile Asn Met Trp Gln Lys Val
                          1               5
```

<210> 205
<211> 8
<212> PRT
<213> Artificial Sequence

<220>
<223> HIV peptide

<400> 205

```
                         Phe Leu Gly Phe Leu Ser Ala Ala
                          1               5
```

<210> 206
<211> 8
<212> PRT
<213> Artificial Sequence

<220>
<223> HIV peptide

<400> 206

```
                         Tyr Leu Gly Asp Gln Gln Leu Leu
                          1               5
```

<210> 207
<211> 8
<212> PRT
<213> Artificial Sequence

<220>
<223> HIV peptide

<400> 207

```
                         Tyr Leu Ser Asp Leu Met Lys Leu
                          1               5
```

<210> 208
<211> 8
<212> PRT
<213> Artificial Sequence

<220>
<223> HIV peptide

<400> 208

```
                         Tyr Thr Gly Leu Ile Tyr Thr Leu
                          1               5
```

<210> 209
<211> 8
<212> PRT
<213> Artificial Sequence

<220>
<223> HIV peptide

<400> 209

```
                         Tyr Thr Gly Leu Ile Tyr Asn Leu
                          1               5
```

<210> 210
<211> 8
<212> PRT
<213> Artificial Sequence

<220>
<223> HIV peptide

<400> 210

```
                    Tyr Thr Gly Leu Ile Tyr Ser Leu
                     1               5
```

<210> 211
<211> 8
<212> PRT
<213> Artificial Sequence

<220>
<223> HIV peptide

<400> 211

```
                    Tyr Thr Gly Ile Ile Tyr Ser Leu
                     1               5
```

<210> 212
<211> 8
<212> PRT
<213> Artificial Sequence

<220>
<223> HIV peptide

<400> 212

```
                    Tyr Thr Gly Ile Ile Tyr Asn Leu
                     1               5
```

<210> 213
<211> 8
<212> PRT
<213> Artificial Sequence

<220>
<223> HIV peptide

<400> 213

```
                    Gly Leu Lys Ile Val Phe Ala Val
                     1               5
```

<210> 214
<211> 9
<212> PRT
<213> Artificial Sequence

<220>
<223> HIV peptide

<400> 214

```
Val Leu Ala Ala Ile Ile Ala Ile Val
 1                   5
```

<210> 215
<211> 9
<212> PRT
<213> Artificial Sequence

<220>
<223> HIV peptide

<400> 215

```
Ile Leu Ala Ile Val Val Trp Thr Val
 1                   5
```

<210> 216
<211> 9
<212> PRT
<213> Artificial Sequence

<220>
<223> HIV peptide

<400> 216

```
Ala Leu Val Glu Met Gly His His Val
 1                   5
```

<210> 217
<211> 9
<212> PRT
<213> Artificial Sequence

<220>
<223> HIV peptide

<400> 217

```
Phe Leu Arg Pro Trp Leu His Gly Val
 1                   5
```

<210> 218
<211> 9
<212> PRT
<213> Artificial Sequence

<220>
<223> HIV peptide

<400> 218

```
Ser Leu Gly Gln His Ile Tyr Glu Val
 1               5
```

<210> 219
<211> 9
<212> PRT
<213> Artificial Sequence

<220>
<223> HIV peptide

<400> 219

```
Ser Leu Gly Gln Tyr Ile Tyr Glu Val
 1               5
```

<210> 220
<211> 9
<212> PRT
<213> Artificial Sequence

<220>
<223> HIV peptide

<400> 220

```
Leu Leu Ile Thr Thr Tyr Trp Gly Leu
 1               5
```

<210> 221
<211> 9
<212> PRT
<213> Artificial Sequence

<220>
<223> HIV peptide

<400> 221

```
Leu Leu Val Arg Thr Tyr Trp Gly Val
 1               5
```

<210> 222
<211> 9
<212> PRT
<213> Artificial Sequence

<220>
<223> HIV peptide

<400> 222

```
        Leu Leu Val Thr Thr Tyr Trp Gly Val
         1               5
```

<210> 223
<211> 9
<212> PRT
<213> Artificial Sequence

<220>
<223> HIV peptide

<400> 223

```
        Lys Leu Lys Pro Pro Leu Pro Ser Val
         1               5
```

<210> 224
<211> 9
<212> PRT
<213> Artificial Sequence

<220>
<223> HIV peptide

<400> 224

```
        Gly Leu Ala Asp Gln Leu Ile His Val
         1               5
```

<210> 225
<211> 9
<212> PRT
<213> Artificial Sequence

<220>
<223> HIV peptide

<400> 225

```
        Ala Leu Ala Ala Leu Ile Thr Pro Val
         1               5
```

<210> 226
<211> 9
<212> PRT
<213> Artificial Sequence

<220>
<223> HIV peptide

<400> 226

```
Ala Leu Thr Ala Leu Ile Thr Pro Val
 1               5
```

<210> 227
<211> 9
<212> PRT
<213> Artificial Sequence

<220>
<223> HIV peptide

<400> 227

```
Leu Leu Leu Pro Pro Ile Glu Arg Val
 1               5
```

<210> 228
<211> 9
<212> PRT
<213> Artificial Sequence

<220>
<223> HIV peptide

<400> 228

```
Gly Leu Gly Ser Pro Gln Ile Leu Val
 1               5
```

<210> 229
<211> 9
<212> PRT
<213> Artificial Sequence

<220>
<223> HIV peptide

<400> 229

```
Ile Leu Val Glu Ser Pro Ala Val Val
 1               5
```

<210> 230
<211> 9
<212> PRT
<213> Artificial Sequence

<220>
<223> HIV peptide

<400> 230

```
Ala Leu Val Glu Ile Cys Thr Glu Val
 1               5
```

<210> 231
<211> 9
<212> PRT
<213> Artificial Sequence

<220>
<223> HIV peptide

<400> 231

```
Ala Leu Thr Glu Ile Cys Thr Glu Val
 1               5
```

<210> 232
<211> 9
<212> PRT
<213> Artificial Sequence

<220>
<223> HIV peptide

<400> 232

```
Leu Leu Ile Pro His Pro Ala Gly Val
 1               5
```

<210> 233
<211> 9
<212> PRT
<213> Artificial Sequence

<220>
<223> HIV peptide

<400> 233

```
Tyr Leu Ala Phe Thr Ile Pro Ser Val
 1               5
```

<210> 234
<211> 9
<212> PRT
<213> Artificial Sequence

<220>
<223> HIV peptide

<400> 234

```
                              His Leu Leu Arg Trp Gly Phe Thr Val
                               1               5
```

<210> 235
<211> 9
<212> PRT
<213> Artificial Sequence

<220>
<223> HIV peptide

<400> 235

```
                              Phe Leu Trp Met Gly Tyr Glu Leu Val
                               1               5
```

<210> 236
<211> 9
<212> PRT
<213> Artificial Sequence

<220>
<223> HIV peptide

<400> 236

```
                              Lys Leu Asn Trp Ala Ser Gln Ile Val
                               1               5
```

<210> 237
<211> 9
<212> PRT
<213> Artificial Sequence

<220>
<223> HIV peptide

<400> 237

```
                              Ser Leu Ile Tyr Ala Gly Ile Lys Val
                               1               5
```

<210> 238
<211> 9
<212> PRT
<213> Artificial Sequence

<220>
<223> HIV peptide

<400> 238

```
Ser Leu Ile Tyr Pro Gly Ile Lys Val
 1               5
```

<210> 239
<211> 9
<212> PRT
<213> Artificial Sequence

<220>
<223> HIV peptide

<400> 239

```
Ala Leu Thr Glu Val Ile Pro Leu Val
 1               5
```

<210> 240
<211> 9
<212> PRT
<213> Artificial Sequence

<220>
<223> HIV peptide

<400> 240

```
Ala Leu Thr Asp Ile Val Pro Leu Val
 1               5
```

<210> 241
<211> 9
<212> PRT
<213> Artificial Sequence

<220>
<223> HIV peptide

<400> 241

```
Ala Leu Thr Asp Ile Val Thr Leu Val
 1               5
```

<210> 242
<211> 9
<212> PRT
<213> Artificial Sequence

<220>
<223> HIV peptide

<400> 242

```
Ala Leu Thr Glu Val Val Pro Leu Val
 1               5
```

<210> 243
<211> 9
<212> PRT
<213> Artificial Sequence

<220>
<223> HIV peptide

<400> 243

```
Lys Leu Trp Tyr Gln Leu Glu Lys Val
 1               5
```

<210> 244
<211> 9
<212> PRT
<213> Artificial Sequence

<220>
<223> HIV peptide

<400> 244

```
Leu Leu Gly Arg Trp Pro Val Lys Val
 1               5
```

<210> 245
<211> 9
<212> PRT
<213> Artificial Sequence

<220>
<223> HIV peptide

<400> 245

```
Ala Leu Lys Ala Ala Cys Trp Trp Val
 1               5
```

<210> 246
<211> 9
<212> PRT
<213> Artificial Sequence

<220>
<223> HIV peptide

<400> 246

```
Leu Leu Thr Ala Val Gln Met Ala Val
 1               5
```

<210> 247
<211> 9
<212> PRT
<213> Artificial Sequence

<220>
<223> HIV peptide

<400> 247

```
Lys Leu Met Ala Gly Ala Asp Cys Val
 1               5
```

<210> 248
<211> 9
<212> PRT
<213> Artificial Sequence

<220>
<223> HIV peptide

<400> 248

```
Asn Leu Ala Phe Pro Gln Gly Glu Val
 1               5
```

<210> 249
<211> 9
<212> PRT
<213> Artificial Sequence

<220>
<223> HIV peptide

<400> 249

```
Leu Leu Gln Arg Pro Leu Val Thr Val
 1               5
```

<210> 250
<211> 9
<212> PRT
<213> Artificial Sequence

<220>
<223> HIV peptide

<400> 250

```
Ile Leu Leu Trp Gln Arg Pro Leu Val
 1               5
```

<210> 251
<211> 9
<212> PRT
<213> Artificial Sequence

<220>
<223> HIV peptide

<400> 251

```
Ile Leu Leu Trp Gln Arg Pro Ile Val
 1               5
```

<210> 252
<211> 9
<212> PRT
<213> Artificial Sequence

<220>
<223> HIV peptide

<400> 252

```
Leu Leu Gly Pro Thr Pro Val Asn Val
 1               5
```

<210> 253
<211> 9
<212> PRT
<213> Artificial Sequence

<220>
<223> HIV peptide

<400> 253

```
Phe Leu Ile Ser Pro Ile Glu Thr Val
 1               5
```

<210> 254
<211> 9
<212> PRT
<213> Artificial Sequence

<220>
<223> HIV peptide

<400> 254

```
Lys Leu Gly Lys Ala Gly Tyr Val Val
 1               5
```

<210> 255
<211> 9
<212> PRT
<213> Artificial Sequence

<220>
<223> HIV peptide

<400> 255

```
Tyr Leu Ala Trp Val Pro Ala His Val
 1               5
```

<210> 256
<211> 9
<212> PRT
<213> Artificial Sequence

<220>
<223> HIV peptide

<400> 256

```
Ala Leu Asn Ala Asp Cys Ala Trp Val
 1               5
```

<210> 257
<211> 9
<212> PRT
<213> Artificial Sequence

<220>
<223> HIV peptide

<400> 257

```
Phe Leu Val Arg Pro Gln Val Pro Val
 1               5
```

<210> 258
<211> 9
<212> PRT
<213> Artificial Sequence

<220>
<223> HIV peptide

<400> 258

```
                         Leu Leu Phe Gly Trp Cys Phe Lys Leu
                          1               5
```

<210> 259
<211> 9
<212> PRT
<213> Artificial Sequence

<220>
<223> HIV peptide

<400> 259

```
                         Leu Leu Trp Lys Phe Asp Ser Arg Val
                          1               5
```

<210> 260
<211> 9
<212> PRT
<213> Artificial Sequence

<220>
<223> HIV peptide

<400> 260

```
                         Arg Leu Ala Phe His His Met Ala Val
                          1               5
```

<210> 261
<211> 9
<212> PRT
<213> Artificial Sequence

<220>
<223> HIV peptide

<400> 261

```
                         Ser Leu Tyr Asn Thr Val Ala Thr Val
                          1               5
```

<210> 262
<211> 9
<212> PRT
<213> Artificial Sequence

<220>
<223> HIV peptide

<400> 262

```
                              Asn Leu Val Ala Thr Leu Tyr Cys Val
                               1                   5
```

<210> 263
<211> 9
<212> PRT
<213> Artificial Sequence

<220>
<223> HIV peptide

<400> 263

```
                              Asn Leu Val Ala Val Leu Tyr Cys Val
                               1                   5
```

<210> 264
<211> 9
<212> PRT
<213> Artificial Sequence

<220>
<223> HIV peptide

<400> 264

```
                              Thr Leu Tyr Cys Val His Gln Lys Val
                               1                   5
```

<210> 265
<211> 9
<212> PRT
<213> Artificial Sequence

<220>
<223> HIV peptide

<400> 265

```
                              Thr Leu Trp Cys Val His Gln Arg Val
                               1                   5
```

<210> 266
<211> 9
<212> PRT
<213> Artificial Sequence

<220>
<223> HIV peptide

<400> 266

```
                    Leu Leu Gly Gln Met Val His Gln Val
                     1               5
```

<210> 267
<211> 9
<212> PRT
<213> Artificial Sequence

<220>
<223> HIV peptide

<400> 267

```
                    Arg Leu Leu Asn Ala Trp Val Lys Val
                     1               5
```

<210> 268
<211> 9
<212> PRT
<213> Artificial Sequence

<220>
<223> HIV peptide

<400> 268

```
                    Arg Leu His Pro Val Gln Ala Gly Val
                     1               5
```

<210> 269
<211> 9
<212> PRT
<213> Artificial Sequence

<220>
<223> HIV peptide

<400> 269

```
                    Thr Leu Gln Glu Gln Ile Gly Trp Val
                     1               5
```

<210> 270
<211> 9
<212> PRT
<213> Artificial Sequence

<220>
<223> HIV peptide

<400> 270

```
                    Thr Leu Gln Glu Gln Ile Ala Trp Val
                     1               5
```

<210> 271
<211> 9
<212> PRT
<213> Artificial Sequence

<220>
<223> HIV peptide

<400> 271

```
                    Met Leu Asn Asn Pro Pro Ile Pro Val
                     1               5
```

<210> 272
<211> 9
<212> PRT
<213> Artificial Sequence

<220>
<223> HIV peptide

<400> 272

```
                    Met Leu Ser Asn Pro Pro Ile Pro Val
                     1               5
```

<210> 273
<211> 9
<212> PRT
<213> Artificial Sequence

<220>
<223> HIV peptide

<400> 273

```
                    Met Leu Ser Asn Pro Pro Val Pro Val
                     1               5
```

<210> 274
<211> 9
<212> PRT
<213> Artificial Sequence

<220>
<223> HIV peptide

<400> 274

```
Lys Leu Val Arg Met Tyr Ser Pro Val
 1               5
```

<210> 275
<211> 9
<212> PRT
<213> Artificial Sequence

<220>
<223> HIV peptide

<400> 275

```
Arg Leu Tyr Ser Pro Val Ser Ile Val
 1               5
```

<210> 276
<211> 9
<212> PRT
<213> Artificial Sequence

<220>
<223> HIV peptide

<400> 276

```
Arg Leu Tyr Ser Pro Thr Ser Ile Val
 1               5
```

<210> 277
<211> 9
<212> PRT
<213> Artificial Sequence

<220>
<223> HIV peptide

<400> 277

```
Ala Leu Gly Pro Ala Ala Thr Leu Val
 1               5
```

<210> 278
<211> 9
<212> PRT
<213> Artificial Sequence

<220>
<223> HIV peptide

<400> 278

```
Ala Leu Leu Glu Glu Met Met Thr Val
 1               5
```

<210> 279
<211> 9
<212> PRT
<213> Artificial Sequence

<220>
<223> HIV peptide

<400> 279

```
Ser Leu Glu Glu Met Met Thr Ala Val
 1               5
```

<210> 280
<211> 9
<212> PRT
<213> Artificial Sequence

<220>
<223> HIV peptide

<400> 280

```
Glu Leu Met Thr Ala Cys Gln Gly Val
 1               5
```

<210> 281
<211> 9
<212> PRT
<213> Artificial Sequence

<220>
<223> HIV peptide

<400> 281

```
Met Leu Gln Arg Gly Asn Phe Arg Val
 1               5
```

<210> 282
<211> 9
<212> PRT
<213> Artificial Sequence

<220>
<223> HIV peptide

<400> 282

```
                    Met Leu Gln Arg Gly Asn Phe Lys Val
                     1               5
```

<210> 283
<211> 9
<212> PRT
<213> Artificial Sequence

<220>
<223> HIV peptide

<400> 283

```
                    Phe Leu Gln Ser Arg Pro Glu Pro Val
                     1               5
```

<210> 284
<211> 9
<212> PRT
<213> Artificial Sequence

<220>
<223> HIV peptide

<400> 284

```
                    Phe Leu Gln Asn Arg Pro Glu Pro Val
                     1               5
```

<210> 285
<211> 9
<212> PRT
<213> Artificial Sequence

<220>
<223> HIV peptide

<400> 285

```
                    His Leu Trp Arg Trp Gly Thr Met Val
                     1               5
```

<210> 286
<211> 9
<212> PRT
<213> Artificial Sequence

<220>
<223> HIV peptide

<400> 286

```
Met Leu Leu Gly Met Leu Met Ile Val
 1               5
```

<210> 287
<211> 9
<212> PRT
<213> Artificial Sequence

<220>
<223> HIV peptide

<400> 287

```
Lys Leu Trp Val Thr Val Tyr Tyr Val
 1               5
```

<210> 288
<211> 9
<212> PRT
<213> Artificial Sequence

<220>
<223> HIV peptide

<400> 288

```
Val Leu Val Tyr Tyr Gly Val Pro Val
 1               5
```

<210> 289
<211> 9
<212> PRT
<213> Artificial Sequence

<220>
<223> HIV peptide

<400> 289

```
Asn Leu Trp Ala Thr His Ala Cys Val
 1               5
```

<210> 290
<211> 9
<212> PRT
<213> Artificial Sequence

<220>
<223> HIV peptide

<400> 290

```
Lys Leu Thr Pro Leu Cys Val Thr Val
 1               5
```

<210> 291
<211> 9
<212> PRT
<213> Artificial Sequence

<220>
<223> HIV peptide

<400> 291

```
Tyr Leu Ala Pro Ala Gly Phe Ala Val
 1               5
```

<210> 292
<211> 9
<212> PRT
<213> Artificial Sequence

<220>
<223> HIV peptide

<400> 292

```
Tyr Leu Ala Pro Ala Gly Tyr Ala Val
 1               5
```

<210> 293
<211> 9
<212> PRT
<213> Artificial Sequence

<220>
<223> HIV peptide

<400> 293

```
Ser Leu Ala Glu Glu Glu Val Val Val
 1               5
```

<210> 294
<211> 9
<212> PRT
<213> Artificial Sequence

<220>
<223> HIV peptide

<400> 294

```
                          Ser Leu Ala Glu Glu Glu Ile Ile Val
                           1               5
```

<210> 295
<211> 9
<212> PRT
<213> Artificial Sequence

<220>
<223> HIV peptide

<400> 295

```
                          Ala Leu Tyr Ala Pro Pro Ile Arg Val
                           1               5
```

<210> 296
<211> 9
<212> PRT
<213> Artificial Sequence

<220>
<223> HIV peptide

<400> 296

```
                          Ala Leu Tyr Ala Pro Pro Ile Glu Val
                           1               5
```

<210> 297
<211> 9
<212> PRT
<213> Artificial Sequence

<220>
<223> HIV peptide

<400> 297

```
                          Ala Met Tyr Ala Pro Pro Ile Lys Val
                           1               5
```

<210> 298
<211> 9
<212> PRT
<213> Artificial Sequence

<220>
<223> HIV peptide

<400> 298

```
                              Ala Met Tyr Ala Pro Pro Ile Ala Val
                               1               5
```

<210> 299
<211> 9
<212> PRT
<213> Artificial Sequence

<220>
<223> HIV peptide

<400> 299

```
                              Pro Leu Gly Ile Ala Pro Thr Lys Val
                               1               5
```

<210> 300
<211> 9
<212> PRT
<213> Artificial Sequence

<220>
<223> HIV peptide

<400> 300

```
                              Thr Leu Gly Ala Ala Ser Ile Thr Val
                               1               5
```

<210> 301
<211> 9
<212> PRT
<213> Artificial Sequence

<220>
<223> HIV peptide

<400> 301

```
                              Thr Leu Gly Ala Ala Ser Leu Thr Val
                               1               5
```

<210> 302
<211> 9
<212> PRT
<213> Artificial Sequence

<220>
<223> HIV peptide

<400> 302

```
                              Ala Met Tyr Ala Pro Pro Ile Ala Val
                               1               5
```

```
                    Arg Leu Ile Glu Ala Gln Gln His Val
                     1               5
```

<210> 303
<211> 9
<212> PRT
<213> Artificial Sequence

<220>
<223> HIV peptide

<400> 303

```
                    Ala Leu Glu Ala Gln Gln His Leu Val
                     1               5
```

<210> 304
<211> 9
<212> PRT
<213> Artificial Sequence

<220>
<223> HIV peptide

<400> 304

```
                    Ala Leu Glu Ala Gln Gln His Met Val
                     1               5
```

<210> 305
<211> 9
<212> PRT
<213> Artificial Sequence

<220>
<223> HIV peptide

<400> 305

```
                    Leu Leu Lys Leu Thr Val Trp Gly Val
                     1               5
```

<210> 306
<211> 9
<212> PRT
<213> Artificial Sequence

<220>
<223> HIV peptide

<400> 306

```
                         Cys Leu Thr Ala Val Pro Trp Asn Val
                          1               5
```

<210> 307
<211> 9
<212> PRT
<213> Artificial Sequence

<220>
<223> HIV peptide

<400> 307

```
                         Ser Leu Trp Asn Trp Phe Ser Ile Val
                          1               5
```

<210> 308
<211> 9
<212> PRT
<213> Artificial Sequence

<220>
<223> HIV peptide

<400> 308

```
                         Tyr Leu Lys Ile Phe Ile Met Ile Val
                          1               5
```

<210> 309
<211> 9
<212> PRT
<213> Artificial Sequence

<220>
<223> HIV peptide

<400> 309

```
                         Tyr Leu Arg Ile Phe Ile Met Ile Val
                          1               5
```

<210> 310
<211> 9
<212> PRT
<213> Artificial Sequence

<220>
<223> HIV peptide

<400> 310

```
                              Phe Leu Met Ile Val Gly Gly Leu Val
                               1                   5
```

<210> 311
<211> 9
<212> PRT
<213> Artificial Sequence

<220>
<223> HIV peptide

<400> 311

```
                              Ile Leu Phe Ala Val Leu Ser Ile Val
                               1                   5
```

<210> 312
<211> 9
<212> PRT
<213> Artificial Sequence

<220>
<223> HIV peptide

<400> 312

```
                              Leu Leu Ala Ala Arg Thr Val Glu Val
                               1                   5
```

<210> 313
<211> 9
<212> PRT
<213> Artificial Sequence

<220>
<223> HIV peptide

<400> 313

```
                              Leu Leu Val Ala Arg Ile Val Glu Val
                               1                   5
```

<210> 314
<211> 8
<212> PRT
<213> Artificial Sequence

<220>
<223> HIV peptide

<400> 314

```
Leu Leu Phe Ile His Phe Arg Val
 1               5
```

<210> 315
<211> 8
<212> PRT
<213> Artificial Sequence

<220>
<223> HIV peptide

<400> 315

```
Leu Leu Phe Val His Phe Arg Val
 1               5
```

<210> 316
<211> 8
<212> PRT
<213> Artificial Sequence

<220>
<223> HIV peptide

<400> 316

```
Ile Leu Gly His Ile Val Ser Val
 1               5
```

<210> 317
<211> 8
<212> PRT
<213> Artificial Sequence

<220>
<223> HIV peptide

<400> 317

```
Lys Leu Gly Ser Leu Gln Tyr Val
 1               5
```

<210> 318
<211> 8
<212> PRT
<213> Artificial Sequence

<220>
<223> HIV peptide

<400> 318

Arg Leu Ala Glu Pro Val Pro Val
1               5

<210> 319
<211> 8
<212> PRT
<213> Artificial Sequence

<220>
<223> HIV peptide

<400> 319

Tyr Leu Ser Asn Pro Tyr Pro Val
1               5

<210> 320
<211> 8
<212> PRT
<213> Artificial Sequence

<220>
<223> HIV peptide

<400> 320

Gly Leu Gly Ser Pro Gln Ile Val
1               5

<210> 321
<211> 8
<212> PRT
<213> Artificial Sequence

<220>
<223> HIV peptide

<400> 321

Lys Leu Gly Pro Glu Asn Pro Val
1               5

<210> 322
<211> 8
<212> PRT
<213> Artificial Sequence

<220>
<223> HIV peptide

<400> 322

```
Gln Leu Gly Ile Pro His Pro Val
 1               5
```

<210> 323
<211> 8
<212> PRT
<213> Artificial Sequence

<220>
<223> HIV peptide

<400> 323

```
Thr Leu Asn Asp Ile Gln Lys Val
 1               5
```

<210> 324
<211> 8
<212> PRT
<213> Artificial Sequence

<220>
<223> HIV peptide

<400> 324

```
Ala Leu Thr Asp Ile Val Pro Val
 1               5
```

<210> 325
<211> 8
<212> PRT
<213> Artificial Sequence

<220>
<223> HIV peptide

<400> 325

```
Leu Leu Ala Glu Ile Gln Lys Val
 1               5
```

<210> 326
<211> 8
<212> PRT
<213> Artificial Sequence

<220>
<223> HIV peptide

<400> 326

Leu Leu Glu Val Val Gln Lys Val
1               5

<210> 327
<211> 8
<212> PRT
<213> Artificial Sequence

<220>
<223> HIV peptide

<400> 327

Phe Leu Asn Thr Pro Pro Leu Val
1               5

<210> 328
<211> 8
<212> PRT
<213> Artificial Sequence

<220>
<223> HIV peptide

<400> 328

Phe Leu Phe Pro Gln Ile Thr Val
1               5

<210> 329
<211> 8
<212> PRT
<213> Artificial Sequence

<220>
<223> HIV peptide

<400> 329

Phe Leu Phe Pro Gln Ile Thr Val
1               5

<210> 330
<211> 8
<212> PRT
<213> Artificial Sequence

<220>
<223> HIV peptide,

<400> 330

Phe Leu Lys Val Lys Gln Tyr Val
1               5

<210> 331
<211> 8
<212> PRT
<213> Artificial Sequence

<220>
<223> HIV peptide

<400> 331

```
Leu Leu Phe Pro Ile Ser Pro Val
1               5
```

<210> 332
<211> 8
<212> PRT
<213> Artificial Sequence

<220>
<223> HIV peptide

<400> 332

```
Ala Leu Gly Ile Ile Gln Ala Val
1               5
```

<210> 333
<211> 8
<212> PRT
<213> Artificial Sequence

<220>
<223> HIV peptide

<400> 333

```
Gly Leu Gly Val Arg Tyr Pro Val
1               5
```

<210> 334
<211> 8
<212> PRT
<213> Artificial Sequence

<220>
<223> HIV peptide

<400> 334

```
Gly Leu Gly Thr Arg Phe Pro Val
1               5
```

<210> 335
<211> 8

<212> PRT
<213> Artificial Sequence

<220>
<223> HIV peptide

<400> 335

```
                    Thr Leu Gly Trp Cys Phe Lys Val
                     1               5
```

<210> 336
<211> 8
<212> PRT
<213> Artificial Sequence

<220>
<223> HIV peptide

<400> 336

```
                    Cys Leu Gly Trp Cys Phe Lys Val
                     1               5
```

<210> 337
<211> 8
<212> PRT
<213> Artificial Sequence

<220>
<223> HIV peptide

<400> 337

```
                    Trp Leu Phe Lys Leu Val Pro Val
                     1               5
```

<210> 338
<211> 8
<212> PRT
<213> Artificial Sequence

<220>
<223> HIV peptide

<400> 338

```
                    Phe Leu His Val Ala Arg Glu Val
                     1               5
```

<210> 339
<211> 8
<212> PRT

<213> Artificial Sequence

<220>
<223> HIV peptide

<400> 339

```
Phe Leu His Met Ala Arg Glu Val
 1               5
```

<210> 340
<211> 8
<212> PRT
<213> Artificial Sequence

<220>
<223> HIV peptide

<400> 340

```
Ile Leu Gly Gln Leu Gln Pro Val
 1               5
```

<210> 341
<211> 8
<212> PRT
<213> Artificial Sequence

<220>
<223> HIV peptide

<400> 341

```
Thr Leu Asn Pro Pro Ile Pro Val
 1               5
```

<210> 342
<211> 8
<212> PRT
<213> Artificial Sequence

<220>
<223> HIV peptide

<400> 342

```
Pro Leu Gly Glu Ile Tyr Lys Val
 1               5
```

<210> 343
<211> 8
<212> PRT
<213> Artificial Sequence

<220>
<223> HIV peptide

<400> 343

```
Pro Leu Gly Asp Ile Tyr Lys Val
 1               5
```

<210> 344
<211> 8
<212> PRT
<213> Artificial Sequence

<220>
<223> HIV peptide

<400> 344

```
Ala Leu Gly Pro Ala Ala Thr Val
 1               5
```

<210> 345
<211> 8
<212> PRT
<213> Artificial Sequence

<220>
<223> HIV peptide

<400> 345

```
Phe Leu Gly Lys Ile Trp Pro Val
 1               5
```

<210> 346
<211> 8
<212> PRT
<213> Artificial Sequence

<220>
<223> HIV peptide

<400> 346

```
Leu Leu Asn Arg Pro Glu Pro Val
 1               5
```

<210> 347
<211> 8
<212> PRT
<213> Artificial Sequence

<220>
<223> HIV peptide

<400> 347

```
Ile Leu Gly Asp Ile Arg Gln Val
 1               5
```

<210> 348
<211> 8
<212> PRT
<213> Artificial Sequence

<220>
<223> HIV peptide

<400> 348

```
Ser Leu Gly Asp Pro Glu Ile Val
 1               5
```

<210> 349
<211> 8
<212> PRT
<213> Artificial Sequence

<220>
<223> HIV peptide

<400> 349

```
Cys Leu Gly Glu Phe Phe Tyr Val
 1               5
```

<210> 350
<211> 8
<212> PRT
<213> Artificial Sequence

<220>
<223> HIV peptide

<400> 350

```
Ile Leu Asn Met Trp Gln Glu Val
 1               5
```

<210> 351
<211> 8
<212> PRT
<213> Artificial Sequence

<220>
<223> HIV peptide

<400> 351

```
                                    Ile Leu Asn Met Trp Gln Gly Val
                                     1               5
```

<210> 352
<211> 8
<212> PRT
<213> Artificial Sequence

<220>
<223> HIV peptide

<400> 352

```
                                    Phe Leu Gly Phe Leu Gly Ala Val
                                     1               5
```

<210> 353
<211> 8
<212> PRT
<213> Artificial Sequence

<220>
<223> HIV peptide

<400> 353

```
                                    Phe Leu Gly Ala Ala Gly Ser Val
                                     1               5
```

<210> 354
<211> 8
<212> PRT
<213> Artificial Sequence

<220>
<223> HIV peptide

<400> 354

```
                                    Leu Leu Ala Arg Ile Leu Ala Val
                                     1               5
```

<210> 355
<211> 8
<212> PRT
<213> Artificial Sequence

<220>
<223> HIV peptide

<400> 355

```
Tyr Leu Lys Asp Gln Gln Leu Val
1               5
```

<210> 356
<211> 8
<212> PRT
<213> Artificial Sequence

<220>
<223> HIV peptide

<400> 356

```
Tyr Leu Arg Asp Gln Gln Leu Val
1               5
```

<210> 357
<211> 8
<212> PRT
<213> Artificial Sequence

<220>
<223> HIV peptide

<400> 357

```
Ile Leu Gly Gly Leu Val Gly Val
1               5       .
```

<210> 358
<211> 8
<212> PRT
<213> Artificial Sequence

<220>
<223> HIV peptide

<400> 358

```
Ile Leu Phe Ala Val Leu Ser Val
1               5
```

<210> 359
<211> 8
<212> PRT
<213> Artificial Sequence

<220>
<223> HIV peptide

<400> 359

```
Leu Leu Asn Gly Phe Leu Ala Val
 1               5
```

<210> 360
<211> 9
<212> PRT
<213> Artificial Sequence

<220>
<223> HIV peptide

<400> 360

```
Ala Ile Glu Ala Gln Gln His Leu Val
 1               5
```

<210> 361
<211> 8
<212> PRT
<213> Artificial Sequence

<220>
<223> HIV peptide

<400> 361

```
Ala Ile Gly Ile Ile Gln Ala Ile
 1               5
```

<210> 362
<211> 8
<212> PRT
<213> Artificial Sequence

<220>
<223> HIV peptide

<400> 362

```
Ala Ile Gly Ile Ile Gln Ala Leu
 1               5
```

<210> 363
<211> 8
<212> PRT
<213> Artificial Sequence

<220>
<223> HIV peptide

<400> 363

```
                          Ala Ile Gly Ile Ile Gln Ala Val
                           1                5
```

<210> 364
<211> 9
<212> PRT
<213> Artificial Sequence

<220>
<223> HIV peptide

<400> 364

```
                          Ala Ile Tyr Ala Pro Pro Ile Glu Val
                           1                5
```

<210> 365
<211> 9
<212> PRT
<213> Artificial Sequence

<220>
<223> HIV peptide

<400> 365

```
                          Ala Ile Tyr Ala Pro Pro Ile Arg Val
                           1                5
```

<210> 366
<211> 9
<212> PRT
<213> Artificial Sequence

<220>
<223> HIV peptide

<400> 366

```
                          Ala Ile Ala Ala Leu Ile Thr Pro Val
                           1                5
```

<210> 367
<211> 9
<212> PRT
<213> Artificial Sequence

<220>
<223> HIV peptide

<400> 367

```
                              Ala Leu Glu Ala Gln Gln His Leu Ala
                               1               5
```

<210> 368
<211> 9
<212> PRT
<213> Artificial Sequence

<220>
<223> HIV peptide

<400> 368

```
                              Ala Leu Glu Ala Gln Gln His Leu Ile
                               1               5
```

<210> 369
<211> 9
<212> PRT
<213> Artificial Sequence

<220>
<223> HIV peptide

<400> 369

```
                              Ala Leu Glu Ala Gln Gln His Leu Leu
                               1               5
```

<210> 370
<211> 9
<212> PRT
<213> Artificial Sequence

<220>
<223> HIV peptide

<400> 370

```
                              Ala Leu Glu Ala Gln Gln His Met Ala
                               1               5
```

<210> 371
<211> 9
<212> PRT
<213> Artificial Sequence

<220>
<223> HIV peptide

<400> 371

```
Ala Leu Glu Ala Gln Gln His Met Ile
 1               5
```

<210> 372
<211> 9
<212> PRT
<213> Artificial Sequence

<220>
<223> HIV peptide

<400> 372

```
Ala Leu Glu Ala Gln Gln His Met Leu
 1               5
```

<210> 373
<211> 8
<212> PRT
<213> Artificial Sequence

<220>
<223> HIV peptide

<400> 373

```
Ala Leu Gly Ile Ile Gln Ala Ile
 1               5
```

<210> 374
<211> 8
<212> PRT
<213> Artificial Sequence

<220>
<223> HIV peptide

<400> 374

```
Ala Leu Gly Ile Ile Gln Ala Leu
 1               5
```

<210> 375
<211> 9
<212> PRT
<213> Artificial Sequence

<220>
<223> HIV peptide

<400> 375

```
Ala Leu Gly Pro Ala Ala Thr Leu Ala
 1                   5
```

<210> 376
<211> 9
<212> PRT
<213> Artificial Sequence

<220>
<223> HIV peptide

<400> 376

```
Ala Leu Gly Pro Ala Ala Thr Leu Ile
 1                   5
```

<210> 377
<211> 9
<212> PRT
<213> Artificial Sequence

<220>
<223> HIV peptide

<400> 377

```
Ala Leu Gly Pro Ala Ala Thr Leu Leu
 1                   5
```

<210> 378
<211> 9
<212> PRT
<213> Artificial Sequence

<220>
<223> HIV peptide

<400> 378

```
Ala Leu Lys Ala Ala Cys Trp Trp Ala
 1                   5
```

<210> 379
<211> 9
<212> PRT
<213> Artificial Sequence

<220>
<223> HIV peptide

<400> 379

```
                    Ala Leu Lys Ala Ala Cys Trp Trp Ile
                     1               5
```

<210> 380
<211> 9
<212> PRT
<213> Artificial Sequence

<220>
<223> HIV peptide

<400> 380

```
                    Ala Leu Lys Ala Ala Cys Trp Trp Leu
                     1               5
```

<210> 381
<211> 9
<212> PRT
<213> Artificial Sequence

<220>
<223> HIV peptide

<400> 381

```
                    Ala Leu Leu Glu Glu Met Met Thr Ala
                     1               5
```

<210> 382
<211> 9
<212> PRT
<213> Artificial Sequence

<220>
<223> HIV peptide

<400> 382

```
                    Ala Leu Leu Glu Glu Met Met Thr Leu
                     1               5
```

<210> 383
<211> 9
<212> PRT
<213> Artificial Sequence

<220>
<223> HIV peptide

<400> 383

```
Ala Leu Asn Ala Asp Cys Ala Trp Leu
 1               5
```

<210> 384
<211> 9
<212> PRT
<213> Artificial Sequence

<220>
<223> HIV peptide

<400> 384

```
Ala Leu Thr Ala Leu Ile Thr Pro Ala
 1               5
```

<210> 385
<211> 9
<212> PRT
<213> Artificial Sequence

<220>
<223> HIV peptide

<400> 385

```
Ala Leu Thr Ala Leu Ile Thr Pro Leu
 1               5
```

<210> 386
<211> 9
<212> PRT
<213> Artificial Sequence

<220>
<223> HIV peptide

<400> 386

```
Ala Leu Thr Asp Ile Val Pro Leu Leu
 1               5
```

<210> 387
<211> 9
<212> PRT
<213> Artificial Sequence

<220>
<223> HIV peptide

<400> 387

```
Ala Leu Thr Asp Ile Val Thr Leu Ala
 1                   5
```

<210> 388
<211> 9
<212> PRT
<213> Artificial Sequence

<220>
<223> HIV peptide

<400> 388

```
Ala Leu Thr Asp Ile Val Thr Leu Ile
 1                   5
```

<210> 389
<211> 9
<212> PRT
<213> Artificial Sequence

<220>
<223> HIV peptide

<400> 389

```
Ala Leu Thr Asp Ile Val Thr Leu Leu
 1                   5
```

<210> 390
<211> 9
<212> PRT
<213> Artificial Sequence

<220>
<223> HIV peptide

<400> 390

```
Ala Leu Thr Glu Ile Cys Thr Glu Leu
 1                   5
```

<210> 391
<211> 9
<212> PRT
<213> Artificial Sequence

<220>
<223> HIV peptide

<400> 391

```
Ala Leu Thr Glu Val Ile Pro Leu Ala
1               5
```

<210> 392
<211> 9
<212> PRT
<213> Artificial Sequence

<220>
<223> HIV peptide

<400> 392

```
Ala Leu Thr Glu Val Ile Pro Leu Leu
1               5
```

<210> 393
<211> 9
<212> PRT
<213> Artificial Sequence

<220>
<223> HIV peptide

<400> 393

```
Ala Leu Thr Glu Val Val Pro Leu Ala
1               5
```

<210> 394
<211> 9
<212> PRT
<213> Artificial Sequence

<220>
<223> HIV peptide

<400> 394

```
Ala Leu Thr Glu Val Val Pro Leu Leu
1               5
```

<210> 395
<211> 9
<212> PRT
<213> Artificial Sequence

<220>
<223> HIV peptide

<400> 395

```
Ala Leu Thr Glu Val Ile Pro Leu Ala
1               5
```

```
Ala Leu Val Glu Ile Cys Thr Glu Leu
 1               5
```

<210> 396
<211> 9
<212> PRT
<213> Artificial Sequence

<220>
<223> HIV peptide

<400> 396

```
Ala Leu Val Glu Met Gly His His Leu
 1               5
```

<210> 397
<211> 9
<212> PRT
<213> Artificial Sequence

<220>
<223> HIV peptide

<400> 397

```
Ala Leu Tyr Ala Pro Pro Ile Glu Ala
 1               5
```

<210> 398
<211> 9
<212> PRT
<213> Artificial Sequence

<220>
<223> HIV peptide

<400> 398

```
Ala Leu Tyr Ala Pro Pro Ile Glu Ile
 1               5
```

<210> 399
<211> 9
<212> PRT
<213> Artificial Sequence

<220>
<223> HIV peptide

<400> 399

```
Ala Leu Tyr Ala Pro Pro Ile Glu Leu
 1                   5
```

<210> 400
<211> 9
<212> PRT
<213> Artificial Sequence

<220>
<223> HIV peptide

<400> 400

```
Ala Leu Tyr Ala Pro Pro Ile Arg Ala
 1                   5
```

<210> 401
<211> 9
<212> PRT
<213> Artificial Sequence

<220>
<223> HIV peptide

<400> 401

```
Ala Leu Tyr Ala Pro Pro Ile Arg Ile
 1                   5
```

<210> 402
<211> 9
<212> PRT
<213> Artificial Sequence

<220>
<223> HIV peptide

<400> 402

```
Ala Leu Tyr Ala Pro Pro Ile Arg Leu
 1                   5
```

<210> 403
<211> 9
<212> PRT
<213> Artificial Sequence

<220>
<223> HIV peptide

<400> 403

```
                        Ala Leu Ala Ala Leu Ile Thr Pro Ala
                          1                   5
```

<210> 404
<211> 9
<212> PRT
<213> Artificial Sequence

<220>
<223> HIV peptide

<400> 404

```
                        Ala Leu Ala Ala Leu Ile Thr Pro Ile
                          1                   5
```

<210> 405
<211> 9
<212> PRT
<213> Artificial Sequence

<220>
<223> HIV peptide

<400> 405

```
                        Ala Leu Ala Ala Leu Ile Thr Pro Leu
                          1                   5
```

<210> 406
<211> 9
<212> PRT
<213> Artificial Sequence

<220>
<223> HIV peptide

<400> 406

```
                        Ala Met Glu Ala Gln Gln His Leu Ala
                          1                   5
```

<210> 407
<211> 9
<212> PRT
<213> Artificial Sequence

<220>
<223> HIV peptide

<400> 407

```
                        Ala Met Glu Ala Gln Gln His Leu Ile
                          1                   5
```

<210> 408
<211> 9
<212> PRT
<213> Artificial Sequence

<220>
<223> HIV peptide

<400> 408

```
Ala Met Glu Ala Gln Gln His Leu Leu
1               5
```

<210> 409
<211> 9
<212> PRT
<213> Artificial Sequence

<220>
<223> HIV peptide

<400> 409

```
Ala Met Glu Ala Gln Gln His Leu Val
1               5
```

<210> 410
<211> 9
<212> PRT
<213> Artificial Sequence

<220>
<223> HIV peptide

<400> 410

```
Ala Met Glu Ala Gln Gln His Met Ala
1               5
```

<210> 411
<211> 9
<212> PRT
<213> Artificial Sequence

<220>
<223> HIV peptide

<400> 411

```
Ala Met Glu Ala Gln Gln His Met Leu
1               5
```

<210> 412
<211> 9

<212> PRT
<213> Artificial Sequence

<220>
<223> HIV peptide

<400> 412

```
                          Ala Met Glu Ala Gln Gln His Met Val
                          1                   5
```

<210> 413
<211> 8
<212> PRT
<213> Artificial Sequence

<220>
<223> HIV peptide

<400> 413

```
                          Ala Met Gly Ile Ile Gln Ala Ile
                          1                   5
```

<210> 414
<211> 8
<212> PRT
<213> Artificial Sequence

<220>
<223> HIV peptide

<400> 414

```
                          Ala Met Gly Ile Ile Gln Ala Leu
                          1                   5
```

<210> 415
<211> 8
<212> PRT
<213> Artificial Sequence

<220>
<223> HIV peptide

<400> 415

```
                          Ala Met Gly Ile Ile Gln Ala Val
                          1                   5
```

<210> 416
<211> 9
<212> PRT
<213> Artificial Sequence

<220>
<223> HIV peptide

<400> 416

```
Ala Met Gly Pro Ala Ala Thr Leu Ala
 1               5
```

<210> 417
<211> 9
<212> PRT
<213> Artificial Sequence

<220>
<223> HIV peptide

<400> 417

```
Ala Met Gly Pro Ala Ala Thr Leu Ile
 1               5
```

<210> 418
<211> 9
<212> PRT
<213> Artificial Sequence

<220>
<223> HIV peptide

<400> 418

```
Ala Met Gly Pro Ala Ala Thr Leu Leu
 1               5
```

<210> 419
<211> 9
<212> PRT
<213> Artificial Sequence

<220>
<223> HIV peptide

<400> 419

```
Ala Met Gly Pro Ala Ala Thr Leu Val
 1               5
```

<210> 420
<211> 9
<212> PRT
<213> Artificial Sequence

<220>
<223> HIV peptide

<400> 420

```
        Ala Met Lys Ala Ala Cys Trp Trp Ala
         1               5
```

<210> 421
<211> 9
<212> PRT
<213> Artificial Sequence

<220>
<223> HIV peptide

<400> 421

```
        Ala Met Lys Ala Ala Cys Trp Trp Leu
         1               5
```

<210> 422
<211> 9
<212> PRT
<213> Artificial Sequence

<220>
<223> HIV peptide

<400> 422

```
        Ala Met Lys Ala Ala Cys Trp Trp Val
         1               5
```

<210> 423
<211> 9
<212> PRT
<213> Artificial Sequence

<220>
<223> HIV peptide

<400> 423

```
        Ala Met Leu Glu Glu Met Met Thr Leu
         1               5
```

<210> 424
<211> 9
<212> PRT
<213> Artificial Sequence

<220>
<223> HIV peptide

<400> 424

```
Ala Met Leu Glu Glu Met Met Thr Val
 1               5
```

<210> 425
<211> 9
<212> PRT
<213> Artificial Sequence

<220>
<223> HIV peptide

<400> 425

```
Ala Met Asn Ala Asp Cys Ala Trp Val
 1               5
```

<210> 426
<211> 9
<212> PRT
<213> Artificial Sequence

<220>
<223> HIV peptide

<400> 426

```
Ala Met Thr Ala Leu Ile Thr Pro Leu
 1               5
```

<210> 427
<211> 9
<212> PRT
<213> Artificial Sequence

<220>
<223> HIV peptide

<400> 427

```
Ala Met Thr Ala Leu Ile Thr Pro Val
 1               5
```

<210> 428
<211> 9
<212> PRT
<213> Artificial Sequence

<220>
<223> HIV peptide

<400> 428

```
Ala Met Thr Asp Ile Val Pro Leu Leu
 1               5
```

<210> 429
<211> 9
<212> PRT
<213> Artificial Sequence

<220>
<223> HIV peptide

<400> 429

```
Ala Met Thr Asp Ile Val Pro Leu Val
 1               5
```

<210> 430
<211> 9
<212> PRT
<213> Artificial Sequence

<220>
<223> HIV peptide

<400> 430

```
Ala Met Thr Asp Ile Val Thr Leu Leu
 1               5
```

<210> 431
<211> 9
<212> PRT
<213> Artificial Sequence

<220>
<223> HIV peptide

<400> 431

```
Ala Met Thr Asp Ile Val Thr Leu Val
 1               5
```

<210> 432
<211> 9
<212> PRT
<213> Artificial Sequence

<220>
<223> HIV peptide

<400> 432

```
Ala Met Thr Glu Ile Cys Thr Glu Leu
 1                   5
```

<210> 433
<211> 9
<212> PRT
<213> Artificial Sequence

<220>
<223> HIV peptide

<400> 433

```
Ala Met Thr Glu Ile Cys Thr Glu Val
 1                   5
```

<210> 434
<211> 9
<212> PRT
<213> Artificial Sequence

<220>
<223> HIV peptide

<400> 434

```
Ala Met Thr Glu Val Ile Pro Leu Leu
 1                   5
```

<210> 435
<211> 9
<212> PRT
<213> Artificial Sequence

<220>
<223> HIV peptide

<400> 435

```
Ala Met Thr Glu Val Ile Pro Leu Val
 1                   5
```

<210> 436
<211> 9
<212> PRT
<213> Artificial Sequence

<220>
<223> HIV peptide

<400> 436

```
                Ala Met Thr Glu Val Val Pro Leu Leu
                 1                   5
```

<210> 437
<211> 9
<212> PRT
<213> Artificial Sequence

<220>
<223> HIV peptide

<400> 437

```
                        Ala Met Thr Glu Val Val Pro Leu Val
                         1                   5
```

<210> 438
<211> 9
<212> PRT
<213> Artificial Sequence

<220>
<223> HIV peptide

<400> 438

```
                        Ala Met Val Glu Ile Cys Thr Glu Leu
                         1                   5
```

<210> 439
<211> 9
<212> PRT
<213> Artificial Sequence

<220>
<223> HIV peptide

<400> 439

```
                        Ala Met Val Glu Ile Cys Thr Glu Val
                         1                   5
```

<210> 440
<211> 9
<212> PRT
<213> Artificial Sequence

<220>
<223> HIV peptide

<400> 440

```
                        Ala Met Val Glu Met Gly His His Val
                         1               5
```

<210> 441
<211> 9
<212> PRT
<213> Artificial Sequence

<220>
<223> HIV peptide

<400> 441

```
                        Ala Met Tyr Ala Pro Pro Ile Glu Ala
                         1               5
```

<210> 442
<211> 9
<212> PRT
<213> Artificial Sequence

<220>
<223> HIV peptide

<400> 442

```
                        Ala Met Tyr Ala Pro Pro Ile Glu Ile
                         1               5
```

<210> 443
<211> 9
<212> PRT
<213> Artificial Sequence

<220>
<223> HIV peptide

<400> 443

```
                        Ala Met Tyr Ala Pro Pro Ile Glu Leu
                         1               5
```

<210> 444
<211> 9
<212> PRT
<213> Artificial Sequence

<220>
<223> HIV peptide

<400> 444

```
Ala Met Tyr Ala Pro Pro Ile Glu Val
 1               5
```

<210> 445
<211> 9
<212> PRT
<213> Artificial Sequence

<220>
<223> HIV peptide

<400> 445

```
Ala Met Tyr Ala Pro Pro Ile Arg Ala
 1               5
```

<210> 446
<211> 9
<212> PRT
<213> Artificial Sequence

<220>
<223> HIV peptide

<400> 446

```
Ala Met Tyr Ala Pro Pro Ile Arg Ile
 1               5
```

<210> 447
<211> 9
<212> PRT
<213> Artificial Sequence

<220>
<223> HIV peptide

<400> 447

```
Ala Met Tyr Ala Pro Pro Ile Arg Leu
 1               5
```

<210> 448
<211> 9
<212> PRT
<213> Artificial Sequence

<220>
<223> HIV peptide

<400> 448

```
                        Ala Met Tyr Ala Pro Pro Ile Arg Val
                         1                   5
```

<210> 449
<211> 9
<212> PRT
<213> Artificial Sequence

<220>
<223> HIV peptide

<400> 449

```
                        Ala Met Ala Ala Leu Ile Thr Pro Ala
                         1                   5
```

<210> 450
<211> 9
<212> PRT
<213> Artificial Sequence

<220>
<223> HIV peptide

<400> 450

```
                        Ala Met Ala Ala Leu Ile Thr Pro Ile
                         1                   5
```

<210> 451
<211> 9
<212> PRT
<213> Artificial Sequence

<220>
<223> HIV peptide

<400> 451

```
                        Ala Met Ala Ala Leu Ile Thr Pro Leu
                         1                   5
```

<210> 452
<211> 9
<212> PRT
<213> Artificial Sequence

<220>
<223> HIV peptide

<400> 452

```
                          Ala Met Ala Ala Leu Ile Thr Pro Val
                           1                   5
```

<210> 453
<211> 8
<212> PRT
<213> Artificial Sequence

<220>
<223> HIV peptide

<400> 453

```
                              Ala Gln Gly Ile Ile Gln Ala Leu
                               1                   5
```

<210> 454
<211> 8
<212> PRT
<213> Artificial Sequence

<220>
<223> HIV peptide

<400> 454

```
                              Ala Gln Gly Ile Ile Gln Ala Val
                               1                   5
```

<210> 455
<211> 9
<212> PRT
<213> Artificial Sequence

<220>
<223> HIV peptide

<400> 455

```
                              Ala Gln Ala Ala Leu Ile Thr Pro Val
                               1                   5
```

<210> 456
<211> 8
<212> PRT
<213> Artificial Sequence

<220>
<223> HIV peptide

<400> 456

```
                    Cys Ile Gly Glu Phe Phe Tyr Ala
                     1                   5
```

<210> 457
<211> 8
<212> PRT
<213> Artificial Sequence

<220>
<223> HIV peptide

<400> 457

```
                    Cys Ile Gly Glu Phe Phe Tyr Cys
                     1                   5
```

<210> 458
<211> 8
<212> PRT
<213> Artificial Sequence

<220>
<223> HIV peptide

<400> 458

```
                    Cys Ile Gly Glu Phe Phe Tyr Ile
                     1                   5
```

<210> 459
<211> 8
<212> PRT
<213> Artificial Sequence

<220>
<223> HIV peptide

<400> 459

```
                    Cys Ile Gly Glu Phe Phe Tyr Leu
                     1                   5
```

<210> 460
<211> 8
<212> PRT
<213> Artificial Sequence

<220>
<223> HIV peptide

<400> 460

```
Cys Ile Gly Glu Phe Phe Tyr Val
 1               5
```

<210> 461
<211> 8
<212> PRT
<213> Artificial Sequence

<220>
<223> HIV peptide

<400> 461

```
Cys Ile Gly Trp Cys Phe Lys Leu
 1               5
```

<210> 462
<211> 8
<212> PRT
<213> Artificial Sequence

<220>
<223> HIV peptide

<400> 462

```
Cys Ile Gly Trp Cys Phe Lys Val
 1               5
```

<210> 463
<211> 8
<212> PRT
<213> Artificial Sequence

<220>
<223> HIV peptide

<400> 463

```
Cys Leu Gly Glu Phe Phe Tyr Ala
 1               5
```

<210> 464
<211> 8
<212> PRT
<213> Artificial Sequence

<220>
<223> HIV peptide

<400> 464

```
                          Cys Leu Gly Glu Phe Phe Tyr Cys
                           1                   5
```

<210> 465
<211> 8
<212> PRT
<213> Artificial Sequence

<220>
<223> HIV peptide

<400> 465

```
                          Cys Leu Gly Glu Phe Phe Tyr Ile
                           1                   5
```

<210> 466
<211> 8
<212> PRT
<213> Artificial Sequence

<220>
<223> HIV peptide

<400> 466

```
                          Cys Leu Gly Glu Phe Phe Tyr Leu
                           1                   5
```

<210> 467
<211> 8
<212> PRT
<213> Artificial Sequence

<220>
<223> HIV peptide

<400> 467

```
                          Cys Leu Gly Trp Cys Phe Lys Ile
                           1                   5
```

<210> 468
<211> 8
<212> PRT
<213> Artificial Sequence

<220>
<223> HIV peptide

<400> 468

```
Cys Leu Gly Trp Cys Phe Lys Leu
 1               5
```

<210> 469
<211> 9
<212> PRT
<213> Artificial Sequence

<220>
<223> HIV peptide

<400> 469

```
Cys Leu Thr Ala Val Pro Trp Asn Ala
 1               5
```

<210> 470
<211> 9
<212> PRT
<213> Artificial Sequence

<220>
<223> HIV peptide

<400> 470

```
Cys Leu Thr Ala Val Pro Trp Asn Ile
 1               5
```

<210> 471
<211> 9
<212> PRT
<213> Artificial Sequence

<220>
<223> HIV peptide

<400> 471

```
Cys Leu Thr Ala Val Pro Trp Asn Leu
 1               5
```

<210> 472
<211> 8
<212> PRT
<213> Artificial Sequence

<220>
<223> HIV peptide

<400> 472

```
                        Cys Met Gly Glu Phe Phe Tyr Ala
                         1               5
```

<210> 473
<211> 8
<212> PRT
<213> Artificial Sequence

<220>
<223> HIV peptide

<400> 473

```
                        Cys Met Gly Glu Phe Phe Tyr Cys
                         1               5
```

<210> 474
<211> 8
<212> PRT
<213> Artificial Sequence

<220>
<223> HIV peptide

<400> 474

```
                        Cys Met Gly Glu Phe Phe Tyr Ile
                         1               5
```

<210> 475
<211> 8
<212> PRT
<213> Artificial Sequence

<220>
<223> HIV peptide

<400> 475

```
                        Cys Met Gly Glu Phe Phe Tyr Leu
                         1               5
```

<210> 476
<211> 8
<212> PRT
<213> Artificial Sequence

<220>
<223> HIV peptide

<400> 476

```
                         Cys Met Gly Glu Phe Phe Tyr Val
                          1                   5
```

<210> 477
<211> 8
<212> PRT
<213> Artificial Sequence

<220>
<223> HIV peptide

<400> 477

```
                         Cys Met Gly Trp Cys Phe Lys Leu
                          1                   5
```

<210> 478
<211> 8
<212> PRT
<213> Artificial Sequence

<220>
<223> HIV peptide

<400> 478

```
                         Cys Met Gly Trp Cys Phe Lys Val
                          1                   5
```

<210> 479
<211> 9
<212> PRT
<213> Artificial Sequence

<220>
<223> HIV peptide

<400> 479

```
                         Cys Met Thr Ala Val Pro Trp Asn Ala
                          1                   5
```

<210> 480
<211> 9
<212> PRT
<213> Artificial Sequence

<220>
<223> HIV peptide

<400> 480

```
Cys Met Thr Ala Val Pro Trp Asn Ile
 1               5
```

<210> 481
<211> 9
<212> PRT
<213> Artificial Sequence

<220>
<223> HIV peptide

<400> 481

```
Cys Met Thr Ala Val Pro Trp Asn Leu
 1               5
```

<210> 482
<211> 9
<212> PRT
<213> Artificial Sequence

<220>
<223> HIV peptide

<400> 482

```
Cys Met Thr Ala Val Pro Trp Asn Val
 1               5
```

<210> 483
<211> 8
<212> PRT
<213> Artificial Sequence

<220>
<223> HIV peptide

<400> 483

```
Cys Gln Gly Glu Phe Phe Tyr Ile
 1               5
```

<210> 484
<211> 8
<212> PRT
<213> Artificial Sequence

<220>
<223> HIV peptide

<400> 484

```
Cys Gln Gly Glu Phe Phe Tyr Leu
 1               5
```

<210> 485
<211> 8
<212> PRT
<213> Artificial Sequence

<220>
<223> HIV peptide

<400> 485

```
Cys Gln Gly Glu Phe Phe Tyr Val
 1               5
```

<210> 486
<211> 8
<212> PRT
<213> Artificial Sequence

<220>
<223> HIV peptide

<400> 486

```
Cys Gln Gly Trp Cys Phe Lys Leu
 1               5
```

<210> 487
<211> 8
<212> PRT
<213> Artificial Sequence

<220>
<223> HIV peptide

<400> 487

```
Cys Gln Gly Trp Cys Phe Lys Val
 1               5
```

<210> 488
<211> 8
<212> PRT
<213> Artificial Sequence

<220>
<223> HIV peptide

<400> 488

Cys Arg Gly Glu Phe Phe Tyr Leu
1                   5

<210> 489
<211> 8
<212> PRT
<213> Artificial Sequence

<220>
<223> HIV peptide

<400> 489

Cys Arg Gly Glu Phe Phe Tyr Val
1                   5

<210> 490
<211> 9
<212> PRT
<213> Artificial Sequence

<220>
<223> HIV peptide

<400> 490

Cys Thr Thr Ala Val Pro Trp Asn Val
1                   5

<210> 491
<211> 9
<212> PRT
<213> Artificial Sequence

<220>
<223> HIV peptide

<400> 491

Glu Met Gln Lys Gln Ile Thr Lys Val
1                   5

<210> 492
<211> 8
<212> PRT
<213> Artificial Sequence

<220>
<223> HIV peptide

<400> 492

```
Phe Ile Phe Pro Gln Ile Thr Leu
 1               5
```

<210> 493
<211> 8
<212> PRT
<213> Artificial Sequence

<220>
<223> HIV peptide

<400> 493

```
Phe Ile Phe Pro Gln Ile Thr Leu
 1               5
```

<210> 494
<211> 8
<212> PRT
<213> Artificial Sequence

<220>
<223> HIV peptide

<400> 494

```
Phe Ile Phe Pro Gln Ile Thr Val
 1               5
```

<210> 495
<211> 8
<212> PRT
<213> Artificial Sequence

<220>
<223> HIV peptide

<400> 495

```
Phe Ile Phe Pro Gln Ile Thr Val
 1               5
```

<210> 496
<211> 8
<212> PRT
<213> Artificial Sequence

<220>
<223> HIV peptide

<400> 496

```
Phe Ile Gly Phe Leu Gly Ala Ile
 1               5
```

<210> 497
<211> 8
<212> PRT
<213> Artificial Sequence

<220>
<223> HIV peptide

<400> 497

```
Phe Ile Gly Phe Leu Gly Ala Leu
 1               5
```

<210> 498
<211> 8
<212> PRT
<213> Artificial Sequence

<220>
<223> HIV peptide

<400> 498

```
Phe Ile Gly Phe Leu Gly Ala Val
 1               5
```

<210> 499
<211> 8
<212> PRT
<213> Artificial Sequence

<220>
<223> HIV peptide

<400> 499

```
Phe Ile Gly Ala Ala Gly Ser Leu
 1               5
```

<210> 500
<211> 8
<212> PRT
<213> Artificial Sequence

<220>
<223> HIV peptide

<400> 500

```
Phe Ile Gly Ala Ala Gly Ser Val
 1               5
```

<210> 501
<211> 9
<212> PRT
<213> Artificial Sequence

<220>
<223> HIV peptide

<400> 501

```
Phe Ile Ile Ser Pro Ile Glu Thr Val
 1               5
```

<210> 502
<211> 8
<212> PRT
<213> Artificial Sequence

<220>
<223> HIV peptide

<400> 502

```
Phe Ile Lys Val Lys Gln Tyr Leu
 1               5
```

<210> 503
<211> 8
<212> PRT
<213> Artificial Sequence

<220>
<223> HIV peptide

<400> 503

```
Phe Ile Lys Val Lys Gln Tyr Val
 1               5
```

<210> 504
<211> 8
<212> PRT
<213> Artificial Sequence

<220>
<223> HIV peptide

<400> 504

```
Phe Ile Asn Thr Pro Pro Leu Leu
 1               5
```

<210> 505
<211> 8
<212> PRT
<213> Artificial Sequence

<220>
<223> HIV peptide

<400> 505

```
Phe Ile Asn Thr Pro Pro Leu Val
 1               5
```

<210> 506
<211> 9
<212> PRT
<213> Artificial Sequence

<220>
<223> HIV peptide

<400> 506

```
Phe Ile Gln Asn Arg Pro Glu Pro Val
 1               5
```

<210> 507
<211> 9
<212> PRT
<213> Artificial Sequence

<220>
<223> HIV peptide

<400> 507

```
Phe Ile Gln Ser Arg Pro Glu Pro Val
 1               5
```

<210> 508
<211> 9
<212> PRT
<213> Artificial Sequence

<220>
<223> HIV peptide

<400> 508

```
                    Phe Ile Arg Pro Trp Leu His Gly Leu
                     1               5
```

<210> 509
<211> 9
<212> PRT
<213> Artificial Sequence

<220>
<223> HIV peptide

<400> 509

```
                    Phe Ile Arg Pro Trp Leu His Gly Val
                     1               5
```

<210> 510
<211> 9
<212> PRT
<213> Artificial Sequence

<220>
<223> HIV peptide

<400> 510

```
                    Phe Ile Val Arg Pro Gln Val Pro Ala
                     1               5
```

<210> 511
<211> 9
<212> PRT
<213> Artificial Sequence

<220>
<223> HIV peptide

<400> 511

```
                    Phe Ile Val Arg Pro Gln Val Pro Leu
                     1               5
```

<210> 512
<211> 9
<212> PRT
<213> Artificial Sequence

<220>
<223> HIV peptide

<400> 512

```
Phe Ile Val Arg Pro Gln Val Pro Val
 1                   5
```

<210> 513
<211> 8
<212> PRT
<213> Artificial Sequence

<220>
<223> HIV peptide

<400> 513

```
Phe Leu Phe Pro Gln Ile Thr Leu
 1                   5
```

<210> 514
<211> 8
<212> PRT
<213> Artificial Sequence

<220>
<223> HIV peptide

<400> 514

```
Phe Leu Phe Pro Gln Ile Thr Leu
 1                   5
```

<210> 515
<211> 8
<212> PRT
<213> Artificial Sequence

<220>
<223> HIV peptide

<400> 515

```
Phe Leu Gly Phe Leu Gly Ala Ile
 1                   5
```

<210> 516
<211> 8
<212> PRT
<213> Artificial Sequence

<220>
<223> HIV peptide

<400> 516

```
Phe Leu Gly Phe Leu Gly Ala Leu
 1               5
```

<210> 517
<211> 8
<212> PRT
<213> Artificial Sequence

<220>
<223> HIV peptide

<400> 517

```
Phe Leu Gly Ala Ala Gly Ser Leu
 1               5
```

<210> 518
<211> 9
<212> PRT
<213> Artificial Sequence

<220>
<223> HIV peptide

<400> 518

```
Phe Leu Ile Ser Pro Ile Glu Thr Ala
 1               5
```

<210> 519
<211> 9
<212> PRT
<213> Artificial Sequence

<220>
<223> HIV peptide

<400> 519

```
Phe Leu Ile Ser Pro Ile Glu Thr Ile
 1               5
```

<210> 520
<211> 9
<212> PRT
<213> Artificial Sequence

<220>
<223> HIV peptide

<400> 520

```
Phe Leu Ile Ser Pro Ile Glu Thr Leu
 1                   5
```

<210> 521
<211> 8
<212> PRT
<213> Artificial Sequence

<220>
<223> HIV peptide

<400> 521

```
Phe Leu Lys Val Lys Gln Tyr Leu
 1                   5
```

<210> 522
<211> 9
<212> PRT
<213> Artificial Sequence

<220>
<223> HIV peptide

<400> 522

```
Phe Leu Met Ile Val Gly Gly Leu Leu
 1                   5
```

<210> 523
<211> 8
<212> PRT
<213> Artificial Sequence

<220>
<223> HIV peptide

<400> 523

```
Phe Leu Asn Thr Pro Pro Leu Leu
 1                   5
```

<210> 524
<211> 9
<212> PRT
<213> Artificial Sequence

<220>
<223> HIV peptide

<400> 524

```
Phe Leu Gln Asn Arg Pro Glu Pro Ala
 1               5
```

<210> 525
<211> 9
<212> PRT
<213> Artificial Sequence

<220>
<223> HIV peptide

<400> 525

```
Phe Leu Gln Asn Arg Pro Glu Pro Ile
 1               5
```

<210> 526
<211> 9
<212> PRT
<213> Artificial Sequence

<220>
<223> HIV peptide

<400> 526

```
Phe Leu Gln Asn Arg Pro Glu Pro Leu
 1               5
```

<210> 527
<211> 9
<212> PRT
<213> Artificial Sequence

<220>
<223> HIV peptide

<400> 527

```
Phe Leu Gln Ser Arg Pro Glu Pro Ala
 1               5
```

<210> 528
<211> 9
<212> PRT
<213> Artificial Sequence

<220>
<223> HIV peptide

<400> 528

Phe Leu Gln Ser Arg Pro Glu Pro Ile
1               5

<210> 529
<211> 9
<212> PRT
<213> Artificial Sequence

<220>
<223> HIV peptide

<400> 529

Phe Leu Gln Ser Arg Pro Glu Pro Leu
1               5

<210> 530
<211> 9
<212> PRT
<213> Artificial Sequence

<220>
<223> HIV peptide

<400> 530

Phe Leu Arg Pro Trp Leu His Gly Ala
1               5

<210> 531
<211> 9
<212> PRT
<213> Artificial Sequence

<220>
<223> HIV peptide

<400> 531

Phe Leu Arg Pro Trp Leu His Gly Ile
1               5

<210> 532
<211> 9
<212> PRT
<213> Artificial Sequence

<220>
<223> HIV peptide

<400> 532

Phe Leu Arg Pro Trp Leu His Gly Leu
1               5

<210> 533
<211> 9
<212> PRT
<213> Artificial Sequence

<220>
<223> HIV peptide

<400> 533

```
Phe Leu Val Arg Pro Gln Val Pro Ala
 1               5
```

<210> 534
<211> 9
<212> PRT
<213> Artificial Sequence

<220>
<223> HIV peptide

<400> 534

```
Phe Leu Val Arg Pro Gln Val Pro Ile
 1               5
```

<210> 535
<211> 9
<212> PRT
<213> Artificial Sequence

<220>
<223> HIV peptide

<400> 535

```
Phe Leu Val Arg Pro Gln Val Pro Leu
 1               5
```

<210> 536
<211> 9
<212> PRT
<213> Artificial Sequence

<220>
<223> HIV peptide

<400> 536

```
Phe Leu Trp Met Gly Tyr Glu Leu Ala
 1               5
```

<210> 537
<211> 9

<212> PRT
<213> Artificial Sequence

<220>
<223> HIV peptide

<400> 537

```
              Phe Leu Trp Met Gly Tyr Glu Leu Leu
              1               5
```

<210> 538
<211> 8
<212> PRT
<213> Artificial Sequence

<220>
<223> HIV peptide

<400> 538

```
              Phe Met Phe Pro Gln Ile Thr Leu
              1               5
```

<210> 539
<211> 8
<212> PRT
<213> Artificial Sequence

<220>
<223> HIV peptide

<400> 539

```
              Phe Met Phe Pro Gln Ile Thr Leu
              1               5
```

<210> 540
<211> 8
<212> PRT
<213> Artificial Sequence

<220>
<223> HIV peptide

<400> 540

```
              Phe Met Phe Pro Gln Ile Thr Val
              1               5
```

<210> 541
<211> 8
<212> PRT
<213> Artificial Sequence

<220>
<223> HIV peptide

<400> 541

```
Phe Met Phe Pro Gln Ile Thr Val
1               5
```

<210> 542
<211> 8
<212> PRT
<213> Artificial Sequence

<220>
<223> HIV peptide

<400> 542

```
Phe Met Gly Phe Leu Gly Ala Ile
1               5
```

<210> 543
<211> 8
<212> PRT
<213> Artificial Sequence

<220>
<223> HIV peptide

<400> 543

```
Phe Met Gly Phe Leu Gly Ala Leu
1               5
```

<210> 544
<211> 8
<212> PRT
<213> Artificial Sequence

<220>
<223> HIV peptide

<400> 544

```
Phe Met Gly Phe Leu Gly Ala Val
1               5
```

<210> 545
<211> 8
<212> PRT
<213> Artificial Sequence

<220>
<223> HIV peptide

<400> 545

```
                        Phe Met Gly Ala Ala Gly Ser Leu
                         1                   5
```

<210> 546
<211> 8
<212> PRT
<213> Artificial Sequence

<220>
<223> HIV peptide

<400> 546

```
                        Phe Met Gly Ala Ala Gly Ser Val
                         1                   5
```

<210> 547
<211> 9
<212> PRT
<213> Artificial Sequence

<220>
<223> HIV peptide

<400> 547

```
                        Phe Met Ile Ser Pro Ile Glu Thr Ala
                         1                   5
```

<210> 548
<211> 9
<212> PRT
<213> Artificial Sequence

<220>
<223> HIV peptide

<400> 548

```
                        Phe Met Ile Ser Pro Ile Glu Thr Ile
                         1                   5
```

<210> 549
<211> 9
<212> PRT
<213> Artificial Sequence

<220>
<223> HIV peptide

<400> 549

Phe Met Ile Ser Pro Ile Glu Thr Leu
1 5

<210> 550
<211> 9
<212> PRT
<213> Artificial Sequence

<220>
<223> HIV peptide

<400> 550

Phe Met Ile Ser Pro Ile Glu Thr Val
1 5

<210> 551
<211> 8
<212> PRT
<213> Artificial Sequence

<220>
<223> HIV peptide

<400> 551

Phe Met Lys Val Lys Gln Tyr Leu
1 5

<210> 552
<211> 8
<212> PRT
<213> Artificial Sequence

<220>
<223> HIV peptide

<400> 552

Phe Met Lys Val Lys Gln Tyr Val
1 5

<210> 553
<211> 9
<212> PRT
<213> Artificial Sequence

<220>
<223> HIV peptide

<400> 553

```
                    Phe Met Met Ile Val Gly Gly Leu Val
                     1               5
```

<210> 554
<211> 8
<212> PRT
<213> Artificial Sequence

<220>
<223> HIV peptide

<400> 554

```
                    Phe Met Asn Thr Pro Pro Leu Leu
                     1               5
```

<210> 555
<211> 8
<212> PRT
<213> Artificial Sequence

<220>
<223> HIV peptide

<400> 555

```
                    Phe Met Asn Thr Pro Pro Leu Val
                     1               5
```

<210> 556
<211> 9
<212> PRT
<213> Artificial Sequence

<220>
<223> HIV peptide

<400> 556

```
                    Phe Met Gln Asn Arg Pro Glu Pro Ala
                     1               5
```

<210> 557
<211> 9
<212> PRT
<213> Artificial Sequence

<220>
<223> HIV peptide

<400> 557

```
                    Phe Met Gln Asn Arg Pro Glu Pro Ile
                     1                   5
```

<210> 558
<211> 9
<212> PRT
<213> Artificial Sequence

<220>
<223> HIV peptide

<400> 558

```
                    Phe Met Gln Asn Arg Pro Glu Pro Leu
                     1                   5
```

<210> 559
<211> 9
<212> PRT
<213> Artificial Sequence

<220>
<223> HIV peptide

<400> 559

```
                    Phe Met Gln Asn Arg Pro Glu Pro Val
                     1                   5
```

<210> 560
<211> 9
<212> PRT
<213> Artificial Sequence

<220>
<223> HIV peptide

<400> 560

```
                    Phe Met Gln Ser Arg Pro Glu Pro Ala
                     1                   5
```

<210> 561
<211> 9
<212> PRT
<213> Artificial Sequence

<220>
<223> HIV peptide

<400> 561

```
Phe Met Gln Ser Arg Pro Glu Pro Ile
 1               5
```

<210> 562
<211> 9
<212> PRT
<213> Artificial Sequence

<220>
<223> HIV peptide

<400> 562

```
Phe Met Gln Ser Arg Pro Glu Pro Leu
 1               5
```

<210> 563
<211> 9
<212> PRT
<213> Artificial Sequence

<220>
<223> HIV peptide

<400> 563

```
Phe Met Gln Ser Arg Pro Glu Pro Val
 1               5
```

<210> 564
<211> 9
<212> PRT
<213> Artificial Sequence

<220>
<223> HIV peptide

<400> 564

```
Phe Met Arg Pro Trp Leu His Gly Ala
 1               5
```

<210> 565
<211> 9
<212> PRT
<213> Artificial Sequence

<220>
<223> HIV peptide

<400> 565

```
                    Phe Met Arg Pro Trp Leu His Gly Ile
                     1               5
```

<210> 566
<211> 9
<212> PRT
<213> Artificial Sequence

<220>
<223> HIV peptide

<400> 566

```
                    Phe Met Arg Pro Trp Leu His Gly Leu
                     1               5
```

<210> 567
<211> 9
<212> PRT
<213> Artificial Sequence

<220>
<223> HIV peptide

<400> 567

```
                    Phe Met Arg Pro Trp Leu His Gly Val
                     1               5
```

<210> 568
<211> 9
<212> PRT
<213> Artificial Sequence

<220>
<223> HIV peptide

<400> 568

```
                    Phe Met Val Arg Pro Gln Val Pro Ala
                     1               5
```

<210> 569
<211> 9
<212> PRT
<213> Artificial Sequence

<220>
<223> HIV peptide

<400> 569

```
                    Phe Met Val Arg Pro Gln Val Pro Ile
                     1               5
```

<210> 570
<211> 9
<212> PRT
<213> Artificial Sequence

<220>
<223> HIV peptide

<400> 570

```
                    Phe Met Val Arg Pro Gln Val Pro Leu
                     1               5
```

<210> 571
<211> 9
<212> PRT
<213> Artificial Sequence

<220>
<223> HIV peptide

<400> 571

```
                    Phe Met Val Arg Pro Gln Val Pro Val
                     1               5
```

<210> 572
<211> 9
<212> PRT
<213> Artificial Sequence

<220>
<223> HIV peptide

<400> 572

```
                    Phe Met Trp Met Gly Tyr Glu Leu Leu
                     1               5
```

<210> 573
<211> 9
<212> PRT
<213> Artificial Sequence

<220>
<223> HIV peptide

<400> 573

```
Phe Met Trp Met Gly Tyr Glu Leu Val
 1               5
```

<210> 574
<211> 8
<212> PRT
<213> Artificial Sequence

<220>
<223> HIV peptide

<400> 574

```
Phe Gln Phe Pro Gln Ile Thr Val
 1               5
```

<210> 575
<211> 8
<212> PRT
<213> Artificial Sequence

<220>
<223> HIV peptide

<400> 575

```
Phe Gln Phe Pro Gln Ile Thr Val
 1               5
```

<210> 576
<211> 8
<212> PRT
<213> Artificial Sequence

<220>
<223> HIV peptide

<400> 576

```
Phe Gln Gly Phe Leu Gly Ala Leu
 1               5
```

<210> 577
<211> 8
<212> PRT
<213> Artificial Sequence

<220>
<223> HIV peptide

<400> 577

```
Phe Gln Gly Phe Leu Gly Ala Val
 1               5
```

<210> 578
<211> 8
<212> PRT
<213> Artificial Sequence

<220>
<223> HIV peptide

<400> 578

```
Phe Gln Gly Ala Ala Gly Ser Val
 1               5
```

<210> 579
<211> 8
<212> PRT
<213> Artificial Sequence

<220>
<223> HIV peptide

<400> 579

```
Phe Gln Lys Val Lys Gln Tyr Leu
 1               5
```

<210> 580
<211> 8
<212> PRT
<213> Artificial Sequence

<220>
<223> HIV peptide

<400> 580

```
Phe Gln Lys Val Lys Gln Tyr Val
 1               5
```

<210> 581
<211> 8
<212> PRT
<213> Artificial Sequence

<220>
<223> HIV peptide

<400> 581

```
                    Phe Gln Asn Thr Pro Pro Leu Leu
                     1               5
```

<210> 582
<211> 8
<212> PRT
<213> Artificial Sequence

<220>
<223> HIV peptide

<400> 582

```
                    Phe Gln Asn Thr Pro Pro Leu Val
                     1               5
```

<210> 583
<211> 9
<212> PRT
<213> Artificial Sequence

<220>
<223> HIV peptide

<400> 583

```
                    Phe Gln Arg Pro Trp Leu His Gly Val
                     1               5
```

<210> 584
<211> 9
<212> PRT
<213> Artificial Sequence

<220>
<223> HIV peptide

<400> 584

```
                    Phe Gln Val Arg Pro Gln Val Pro Leu
                     1               5
```

<210> 585
<211> 9
<212> PRT
<213> Artificial Sequence

<220>
<223> HIV peptide

<400> 585

```
Phe Gln Val Arg Pro Gln Val Pro Val
 1               5
```

<210> 586
<211> 8
<212> PRT
<213> Artificial Sequence

<220>
<223> HIV peptide

<400> 586

```
Gly Ile Gly Ser Pro Gln Ile Leu
 1               5
```

<210> 587
<211> 8
<212> PRT
<213> Artificial Sequence

<220>
<223> HIV peptide

<400> 587

```
Gly Ile Gly Ser Pro Gln Ile Val
 1               5
```

<210> 588
<211> 8
<212> PRT
<213> Artificial Sequence

<220>
<223> HIV peptide

<400> 588

```
Gly Ile Gly Thr Arg Phe Pro Ile
 1               5
```

<210> 589
<211> 8
<212> PRT
<213> Artificial Sequence

<220>
<223> HIV peptide

<400> 589

```
Gly Ile Gly Thr Arg Phe Pro Leu
 1                   5
```

<210> 590
<211> 8
<212> PRT
<213> Artificial Sequence

<220>
<223> HIV peptide

<400> 590

```
Gly Ile Gly Thr Arg Phe Pro Val
 1                   5
```

<210> 591
<211> 8
<212> PRT
<213> Artificial Sequence

<220>
<223> HIV peptide

<400> 591

```
Gly Ile Gly Val Arg Tyr Pro Leu
 1                   5
```

<210> 592
<211> 8
<212> PRT
<213> Artificial Sequence

<220>
<223> HIV peptide

<400> 592

```
Gly Ile Gly Val Arg Tyr Pro Val
 1                   5
```

<210> 593
<211> 8
<212> PRT
<213> Artificial Sequence

<220>
<223> HIV peptide

<400> 593

```
Gly Leu Gly Ser Pro Gln Ile Leu
 1               5
```

<210> 594
<211> 9
<212> PRT
<213> Artificial Sequence

<220>
<223> HIV peptide

<400> 594

```
Gly Leu Gly Ser Pro Gln Ile Leu Leu
 1               5
```

<210> 595
<211> 8
<212> PRT
<213> Artificial Sequence

<220>
<223> HIV peptide

<400> 595

```
Gly Leu Gly Thr Arg Phe Pro Ile
 1               5
```

<210> 596
<211> 8
<212> PRT
<213> Artificial Sequence

<220>
<223> HIV peptide

<400> 596

```
Gly Leu Gly Thr Arg Phe Pro Leu
 1               5
```

<210> 597
<211> 8
<212> PRT
<213> Artificial Sequence

<220>
<223> HIV peptide

<400> 597

```
Gly Leu Gly Val Arg Tyr Pro Ile
 1               5
```

<210> 598
<211> 8
<212> PRT
<213> Artificial Sequence

<220>
<223> HIV peptide

<400> 598

```
Gly Leu Gly Val Arg Tyr Pro Leu
 1               5
```

<210> 599
<211> 9
<212> PRT
<213> Artificial Sequence

<220>
<223> HIV peptide

<400> 599

```
Gly Met Ala Asp Gln Leu Ile His Val
 1               5
```

<210> 600
<211> 8
<212> PRT
<213> Artificial Sequence

<220>
<223> HIV peptide

<400> 600

```
Gly Met Gly Ser Pro Gln Ile Leu
 1               5
```

<210> 601
<211> 9
<212> PRT
<213> Artificial Sequence

<220>
<223> HIV peptide

<400> 601

```
                    Gly Met Gly Ser Pro Gln Ile Leu Val
                     1               5
```

<210> 602
<211> 8
<212> PRT
<213> Artificial Sequence

<220>
<223> HIV peptide

<400> 602

```
                    Gly Met Gly Ser Pro Gln Ile Val
                     1               5
```

<210> 603
<211> 8
<212> PRT
<213> Artificial Sequence

<220>
<223> HIV peptide

<400> 603

```
                    Gly Met Gly Thr Arg Phe Pro Ile
                     1               5
```

<210> 604
<211> 8
<212> PRT
<213> Artificial Sequence

<220>
<223> HIV peptide

<400> 604

```
                    Gly Met Gly Thr Arg Phe Pro Leu
                     1               5
```

<210> 605
<211> 8
<212> PRT
<213> Artificial Sequence

<220>
<223> HIV peptide

<400> 605

```
Gly Met Gly Thr Arg Phe Pro Val
 1               5
```

<210> 606
<211> 8
<212> PRT
<213> Artificial Sequence

<220>
<223> HIV peptide

<400> 606

```
Gly Met Gly Val Arg Tyr Pro Leu
 1               5
```

<210> 607
<211> 8
<212> PRT
<213> Artificial Sequence

<220>
<223> HIV peptide

<400> 607

```
Gly Met Gly Val Arg Tyr Pro Val
 1               5
```

<210> 608
<211> 8
<212> PRT
<213> Artificial Sequence

<220>
<223> HIV peptide

<400> 608

```
Gly Gln Gly Ser Pro Gln Ile Val
 1               5
```

<210> 609
<211> 8
<212> PRT
<213> Artificial Sequence

<220>
<223> HIV peptide

<400> 609

```
                        Gly Gln Gly Thr Arg Phe Pro Leu
                         1                   5
```

<210> 610
<211> 8
<212> PRT
<213> Artificial Sequence

<220>
<223> HIV peptide

<400> 610

```
                        Gly Gln Gly Thr Arg Phe Pro Val
                         1                   5
```

<210> 611
<211> 8
<212> PRT
<213> Artificial Sequence

<220>
<223> HIV peptide

<400> 611

```
                        Gly Gln Gly Val Arg Tyr Pro Leu
                         1                   5
```

<210> 612
<211> 8
<212> PRT
<213> Artificial Sequence

<220>
<223> HIV peptide

<400> 612

```
                        Gly Gln Gly Val Arg Tyr Pro Val
                         1                   5
```

<210> 613
<211> 9
<212> PRT
<213> Artificial Sequence

<220>
<223> HIV peptide

<400> 613

```
His Leu Leu Arg Trp Gly Phe Thr Leu
 1               5
```

<210> 614
<211> 9
<212> PRT
<213> Artificial Sequence

<220>
<223> HIV peptide

<400> 614

```
His Met Leu Arg Trp Gly Phe Thr Leu
 1                5
```

<210> 615
<211> 9
<212> PRT
<213> Artificial Sequence

<220>
<223> HIV peptide

<400> 615

```
His Met Leu Arg Trp Gly Phe Thr Val
 1                5
```

<210> 616
<211> 9
<212> PRT
<213> Artificial Sequence

<220>
<223> HIV peptide

<400> 616

```
His Met Trp Arg Trp Gly Thr Met Val
 1                5
```

<210> 617
<211> 9
<212> PRT
<213> Artificial Sequence

<220>
<223> HIV peptide

<400> 617

```
Ile Ile Ala Ile Val Val Trp Thr Val
 1               5
```

<210> 618
<211> 8
<212> PRT
<213> Artificial Sequence

<220>
<223> HIV peptide

<400> 618

```
Ile Ile Phe Ala Val Leu Ser Val
 1               5
```

<210> 619
<211> 9
<212> PRT
<213> Artificial Sequence

<220>
<223> HIV peptide

<400> 619

```
Ile Ile Gly Ala Glu Thr Phe Tyr Val
 1               5
```

<210> 620
<211> 9
<212> PRT
<213> Artificial Sequence

<220>
<223> HIV peptide

<400> 620

```
Ile Ile Gly Ala Glu Thr Phe Tyr Val
 1               5
```

<210> 621
<211> 8
<212> PRT
<213> Artificial Sequence

<220>
<223> HIV peptide

<400> 621

```
                         Ile Ile Gly Asp Ile Arg Gln Ile
                          1               5
```

<210> 622
<211> 8
<212> PRT
<213> Artificial Sequence

<220>
<223> HIV peptide

<400> 622

```
                         Ile Ile Gly Asp Ile Arg Gln Leu
                          1               5
```

<210> 623
<211> 8
<212> PRT
<213> Artificial Sequence

<220>
<223> HIV peptide

<400> 623

```
                         Ile Ile Gly Asp Ile Arg Gln Val
                          1               5
```

<210> 624
<211> 8
<212> PRT
<213> Artificial Sequence

<220>
<223> HIV peptide

<400> 624

```
                         Ile Ile Gly His Ile Val Ser Leu
                          1               5
```

<210> 625
<211> 8
<212> PRT
<213> Artificial Sequence

<220>
<223> HIV peptide

<400> 625

```
                              Ile Ile Gly His Ile Val Ser Val
                               1               5
```

<210> 626
<211> 8
<212> PRT
<213> Artificial Sequence

<220>
<223> HIV peptide

<400> 626

```
                              Ile Ile Gly Gln Leu Gln Pro Ala
                               1               5
```

<210> 627
<211> 8
<212> PRT
<213> Artificial Sequence

<220>
<223> HIV peptide

<400> 627

```
                              Ile Ile Gly Gln Leu Gln Pro Ile
                               1               5
```

<210> 628
<211> 8
<212> PRT
<213> Artificial Sequence

<220>
<223> HIV peptide

<400> 628

```
                              Ile Ile Gly Gln Leu Gln Pro Leu
                               1               5
```

<210> 629
<211> 8
<212> PRT
<213> Artificial Sequence

<220>
<223> HIV peptide

<400> 629

```
                    Ile Ile Gly Gln Leu Gln Pro Val
                     1               5
```

<210> 630
<211> 9
<212> PRT
<213> Artificial Sequence

<220>
<223> HIV peptide

<400> 630

```
                    Ile Leu Ala Ile Val Val Trp Thr Ala
                     1               5
```

<210> 631
<211> 9
<212> PRT
<213> Artificial Sequence

<220>
<223> HIV peptide

<400> 631

```
                    Ile Leu Ala Ile Val Val Trp Thr Ile
                     1               5
```

<210> 632
<211> 9
<212> PRT
<213> Artificial Sequence

<220>
<223> HIV peptide

<400> 632

```
                    Ile Leu Ala Ile Val Val Trp Thr Leu
                     1               5
```

<210> 633
<211> 9
<212> PRT
<213> Artificial Sequence

<220>
<223> HIV peptide

<400> 633

```
                        Ile Leu Phe Ala Val Leu Ser Ile Ala
                          1               5
```

<210> 634
<211> 9
<212> PRT
<213> Artificial Sequence

<220>
<223> HIV peptide

<400> 634

```
                        Ile Leu Phe Ala Val Leu Ser Ile Ala
                          1               5
```

<210> 635
<211> 9
<212> PRT
<213> Artificial Sequence

<220>
<223> HIV peptide

<400> 635

```
                        Ile Leu Phe Ala Val Leu Ser Ile Leu
                          1               5
```

<210> 636
<211> 9
<212> PRT
<213> Artificial Sequence

<220>
<223> HIV peptide

<400> 636

```
                        Ile Leu Phe Ala Val Leu Ser Ile Leu
                          1               5
```

<210> 637
<211> 8
<212> PRT
<213> Artificial Sequence

<220>
<223> HIV peptide

<400> 637

```
Ile Leu Phe Ala Val Leu Ser Leu
 1               5
```

<210> 638
<211> 9
<212> PRT
<213> Artificial Sequence

<220>
<223> HIV peptide

<400> 638

```
Ile Leu Gly Ala Glu Thr Phe Tyr Ala
 1               5
```

<210> 639
<211> 9
<212> PRT
<213> Artificial Sequence

<220>
<223> HIV peptide

<400> 639

```
Ile Leu Gly Ala Glu Thr Phe Tyr Ala
 1               5
```

<210> 640
<211> 9
<212> PRT
<213> Artificial Sequence

<220>
<223> HIV peptide

<400> 640

```
Ile Leu Gly Ala Glu Thr Phe Tyr Ala
 1               5
```

<210> 641
<211> 9
<212> PRT
<213> Artificial Sequence

<220>
<223> HIV peptide

<400> 641

```
                    Ile Leu Gly Ala Glu Thr Phe Tyr Ile
                      1               5
```

<210> 642
<211> 9
<212> PRT
<213> Artificial Sequence

<220>
<223> HIV peptide

<400> 642

```
                    Ile Leu Gly Ala Glu Thr Phe Tyr Ile
                      1               5
```

<210> 643
<211> 9
<212> PRT
<213> Artificial Sequence

<220>
<223> HIV peptide

<400> 643

```
                    Ile Leu Gly Ala Glu Thr Phe Tyr Ile
                      1               5
```

<210> 644
<211> 9
<212> PRT
<213> Artificial Sequence

<220>
<223> HIV peptide

<400> 644

```
                    Ile Leu Gly Ala Glu Thr Phe Tyr Leu
                      1               5
```

<210> 645
<211> 9
<212> PRT
<213> Artificial Sequence

<220>
<223> HIV peptide

<400> 645

```
Ile Leu Gly Ala Glu Thr Phe Tyr Leu
 1               5
```

<210> 646
<211> 9
<212> PRT
<213> Artificial Sequence

<220>
<223> HIV peptide

<400> 646

```
Ile Leu Gly Ala Glu Thr Phe Tyr Leu
 1               5
```

<210> 647
<211> 8
<212> PRT
<213> Artificial Sequence

<220>
<223> HIV peptide

<400> 647

```
Ile Leu Gly Asp Ile Arg Gln Ile
 1               5
```

<210> 648
<211> 8
<212> PRT
<213> Artificial Sequence

<220>
<223> HIV peptide

<400> 648

```
Ile Leu Gly Asp Ile Arg Gln Leu
 1               5
```

<210> 649
<211> 8
<212> PRT
<213> Artificial Sequence

<220>
<223> HIV peptide

<400> 649

```
                        Ile Leu Gly His Ile Val Ser Ile
                         1               5
```

<210> 650
<211> 8
<212> PRT
<213> Artificial Sequence

<220>
<223> HIV peptide

<400> 650

```
                        Ile Leu Gly His Ile Val Ser Leu
                         1               5
```

<210> 651
<211> 8
<212> PRT
<213> Artificial Sequence

<220>
<223> HIV peptide

<400> 651

```
                        Ile Leu Gly Gln Leu Gln Pro Ala
                         1               5
```

<210> 652
<211> 8
<212> PRT
<213> Artificial Sequence

<220>
<223> HIV peptide

<400> 652

```
                        Ile Leu Gly Gln Leu Gln Pro Ile
                         1               5
```

<210> 653
<211> 8
<212> PRT
<213> Artificial Sequence

<220>
<223> HIV peptide

<400> 653

```
                    Ile Leu Gly Gln Leu Gln Pro Leu
                     1               5
```

<210> 654
<211> 8
<212> PRT
<213> Artificial Sequence

<220>
<223> HIV peptide

<400> 654

```
                    Ile Leu Asn Met Trp Gln Glu Leu
                     1               5
```

<210> 655
<211> 9
<212> PRT
<213> Artificial Sequence

<220>
<223> HIV peptide

<400> 655

```
                    Ile Met Ala Ile Val Val Trp Thr Ala
                     1               5
```

<210> 656
<211> 9
<212> PRT
<213> Artificial Sequence

<220>
<223> HIV peptide

<400> 656

```
                    Ile Met Ala Ile Val Val Trp Thr Ile
                     1               5
```

<210> 657
<211> 9
<212> PRT
<213> Artificial Sequence

<220>
<223> HIV peptide

<400> 657

```
Ile Met Ala Ile Val Val Trp Thr Leu
 1               5
```

<210> 658
<211> 9
<212> PRT
<213> Artificial Sequence

<220>
<223> HIV peptide

<400> 658

```
Ile Met Ala Ile Val Val Trp Thr Val
 1               5
```

<210> 659
<211> 9
<212> PRT
<213> Artificial Sequence

<220>
<223> HIV peptide

<400> 659

```
Ile Met Phe Ala Val Leu Ser Ile Leu
 1               5
```

<210> 660
<211> 9
<212> PRT
<213> Artificial Sequence

<220>
<223> HIV peptide

<400> 660

```
Ile Met Phe Ala Val Leu Ser Ile Leu
 1               5
```

<210> 661
<211> 9
<212> PRT
<213> Artificial Sequence

<220>
<223> HIV peptide

<400> 661

```
                              Ile Met Phe Ala Val Leu Ser Ile Val
                               1                   5
```

<210> 662
<211> 9
<212> PRT
<213> Artificial Sequence

<220>
<223> HIV peptide

<400> 662

```
                              Ile Met Phe Ala Val Leu Ser Ile Val
                               1                   5
```

<210> 663
<211> 8
<212> PRT
<213> Artificial Sequence

<220>
<223> HIV peptide

<400> 663

```
                              Ile Met Phe Ala Val Leu Ser Val
                               1                   5
```

<210> 664
<211> 9
<212> PRT
<213> Artificial Sequence

<220>
<223> HIV peptide

<400> 664

```
                              Ile Met Gly Ala Glu Thr Phe Tyr Ala
                               1                   5
```

<210> 665
<211> 9
<212> PRT
<213> Artificial Sequence

<220>
<223> HIV peptide

<400> 665

```
Ile Met Gly Ala Glu Thr Phe Tyr Ala
 1               5
```

<210> 666
<211> 9
<212> PRT
<213> Artificial Sequence

<220>
<223> HIV peptide

<400> 666

```
Ile Met Gly Ala Glu Thr Phe Tyr Ala
 1               5
```

<210> 667
<211> 9
<212> PRT
<213> Artificial Sequence

<220>
<223> HIV peptide

<400> 667

```
Ile Met Gly Ala Glu Thr Phe Tyr Ile
 1               5
```

<210> 668
<211> 9
<212> PRT
<213> Artificial Sequence

<220>
<223> HIV peptide

<400> 668

```
Ile Met Gly Ala Glu Thr Phe Tyr Ile
 1               5
```

<210> 669
<211> 9
<212> PRT
<213> Artificial Sequence

<220>
<223> HIV peptide

<400> 669

```
Ile Met Gly Ala Glu Thr Phe Tyr Ile
 1               5
```

<210> 670
<211> 9
<212> PRT
<213> Artificial Sequence

<220>
<223> HIV peptide

<400> 670

```
Ile Met Gly Ala Glu Thr Phe Tyr Leu
 1               5
```

<210> 671
<211> 9
<212> PRT
<213> Artificial Sequence

<220>
<223> HIV peptide

<400> 671

```
Ile Met Gly Ala Glu Thr Phe Tyr Leu
 1               5
```

<210> 672
<211> 9
<212> PRT
<213> Artificial Sequence

<220>
<223> HIV peptide

<400> 672

```
Ile Met Gly Ala Glu Thr Phe Tyr Leu
 1               5
```

<210> 673
<211> 9
<212> PRT
<213> Artificial Sequence

<220>
<223> HIV peptide

<400> 673

```
Ile Met Gly Ala Glu Thr Phe Tyr Val
 1               5
```

<210> 674
<211> 9
<212> PRT
<213> Artificial Sequence

<220>
<223> HIV peptide

<400> 674

```
Ile Met Gly Ala Glu Thr Phe Tyr Val
 1               5
```

<210> 675
<211> 9
<212> PRT
<213> Artificial Sequence

<220>
<223> HIV peptide

<400> 675

```
Ile Met Gly Ala Glu Thr Phe Tyr Val
 1               5
```

<210> 676
<211> 8
<212> PRT
<213> Artificial Sequence

<220>
<223> HIV peptide

<400> 676

```
Ile Met Gly Asp Ile Arg Gln Ile
 1               5
```

<210> 677
<211> 8
<212> PRT
<213> Artificial Sequence

<220>
<223> HIV peptide

<400> 677

```
                         Ile Met Gly Asp Ile Arg Gln Leu
                          1                   5
```

<210> 678
<211> 8
<212> PRT
<213> Artificial Sequence

<220>
<223> HIV peptide

<400> 678

```
                         Ile Met Gly Asp Ile Arg Gln Val
                          1                   5
```

<210> 679
<211> 8
<212> PRT
<213> Artificial Sequence

<220>
<223> HIV peptide

<400> 679

```
                         Ile Met Gly His Ile Val Ser Leu
                          1                   5
```

<210> 680
<211> 8
<212> PRT
<213> Artificial Sequence

<220>
<223> HIV peptide

<400> 680

```
                         Ile Met Gly His Ile Val Ser Val
                          1                   5
```

<210> 681
<211> 8
<212> PRT
<213> Artificial Sequence

<220>
<223> HIV peptide

<400> 681

```
                    Ile Met Gly Gln Leu Gln Pro Ala
                     1                   5
```

<210> 682
<211> 8
<212> PRT
<213> Artificial Sequence

<220>
<223> HIV peptide

<400> 682

```
                         Ile Met Gly Gln Leu Gln Pro Ile
                          1                   5
```

<210> 683
<211> 8
<212> PRT
<213> Artificial Sequence

<220>
<223> HIV peptide

<400> 683

```
                       Ile Met Gly Gln Leu Gln Pro Leu
                        1                   5
```

<210> 684
<211> 8
<212> PRT
<213> Artificial Sequence

<220>
<223> HIV peptide

<400> 684

```
                       Ile Met Gly Gln Leu Gln Pro Val
                        1                   5
```

<210> 685
<211> 9
<212> PRT
<213> Artificial Sequence

<220>
<223> HIV peptide

<400> 685

```
        Ile Gln Ala Ile Val Val Trp Thr Val
         1                   5
```

<210> 686
<211> 9
<212> PRT
<213> Artificial Sequence

<220>
<223> HIV peptide

<400> 686

```
        Ile Gln Gly Ala Glu Thr Phe Tyr Val
         1                   5
```

<210> 687
<211> 8
<212> PRT
<213> Artificial Sequence

<220>
<223> HIV peptide

<400> 687

```
        Ile Gln Gly Asp Ile Arg Gln Leu
         1                   5
```

<210> 688
<211> 8
<212> PRT
<213> Artificial Sequence

<220>
<223> HIV peptide

<400> 688

```
        Ile Gln Gly Asp Ile Arg Gln Val
         1                   5
```

<210> 689
<211> 8
<212> PRT
<213> Artificial Sequence

<220>
<223> HIV peptide

<400> 689

```
Ile Gln Gly His Ile Val Ser Leu
 1               5
```

<210> 690
<211> 8
<212> PRT
<213> Artificial Sequence

<220>
<223> HIV peptide

<400> 690

```
Ile Gln Gly His Ile Val Ser Val
 1               5
```

<210> 691
<211> 8
<212> PRT
<213> Artificial Sequence

<220>
<223> HIV peptide

<400> 691

```
Ile Gln Gly Gln Leu Gln Pro Ala
 1               5
```

<210> 692
<211> 8
<212> PRT
<213> Artificial Sequence

<220>
<223> HIV peptide

<400> 692

```
Ile Gln Gly Gln Leu Gln Pro Ile
 1               5
```

<210> 693
<211> 8
<212> PRT
<213> Artificial Sequence

<220>
<223> HIV peptide

<400> 693

```
Ile Gln Gly Gln Leu Gln Pro Leu
 1                   5
```

<210> 694
<211> 8
<212> PRT
<213> Artificial Sequence

<220>
<223> HIV peptide

<400> 694

```
Ile Gln Gly Gln Leu Gln Pro Val
 1                   5
```

<210> 695
<211> 8
<212> PRT
<213> Artificial Sequence

<220>
<223> HIV peptide

<400> 695

```
Lys Ile Gly Pro Glu Asn Pro Leu
 1                   5
```

<210> 696
<211> 8
<212> PRT
<213> Artificial Sequence

<220>
<223> HIV peptide

<400> 696

```
Lys Ile Gly Pro Glu Asn Pro Val
 1                   5
```

<210> 697
<211> 8
<212> PRT
<213> Artificial Sequence

<220>
<223> HIV peptide

<400> 697

```
                    Lys Ile Gly Ser Leu Gln Tyr Leu
                     1               5
```

<210> 698
<211> 8
<212> PRT
<213> Artificial Sequence

<220>
<223> HIV peptide

<400> 698

```
                    Lys Ile Gly Ser Leu Gln Tyr Val
                     1               5
```

<210> 699
<211> 9
<212> PRT
<213> Artificial Sequence

<220>
<223> HIV peptide

<400> 699

```
                    Lys Leu Gly Lys Ala Gly Tyr Val Ala
                     1               5
```

<210> 700
<211> 9
<212> PRT
<213> Artificial Sequence

<220>
<223> HIV peptide

<400> 700

```
                    Lys Leu Gly Lys Ala Gly Tyr Val Leu
                     1               5
```

<210> 701
<211> 8
<212> PRT
<213> Artificial Sequence

<220>
<223> HIV peptide

<400> 701

```
                    Lys Leu Gly Pro Glu Asn Pro Leu
                     1               5
```

<210> 702
<211> 8
<212> PRT
<213> Artificial Sequence

<220>
<223> HIV peptide

<400> 702

```
                    Lys Leu Gly Ser Leu Gln Tyr Leu
                     1               5
```

<210> 703
<211> 9
<212> PRT
<213> Artificial Sequence

<220>
<223> HIV peptide

<400> 703

```
                    Lys Leu Lys Pro Pro Leu Pro Ser Leu
                     1               5
```

<210> 704
<211> 9
<212> PRT
<213> Artificial Sequence

<220>
<223> HIV peptide

<400> 704

```
                    Lys Leu Lys Pro Pro Leu Pro Ser Leu
                     1               5
```

<210> 705
<211> 9
<212> PRT
<213> Artificial Sequence

<220>
<223> HIV peptide

<400> 705

Lys Leu Asn Trp Ala Ser Gln Ile Ala
1                   5

<210> 706
<211> 9
<212> PRT
<213> Artificial Sequence

<220>
<223> HIV peptide

<400> 706

Lys Leu Asn Trp Ala Ser Gln Ile Leu
1                   5

<210> 707
<211> 9
<212> PRT
<213> Artificial Sequence

<220>
<223> HIV peptide

<400> 707

Lys Leu Val Arg Met Tyr Ser Pro Ala
1                   5

<210> 708
<211> 9
<212> PRT
<213> Artificial Sequence

<220>
<223> HIV peptide

<400> 708

Lys Leu Val Arg Met Tyr Ser Pro Ile
1                   5

<210> 709
<211> 9
<212> PRT
<213> Artificial Sequence

<220>
<223> HIV peptide

<400> 709

```
                    Lys Leu Val Arg Met Tyr Ser Pro Leu
                     1               5
```

<210> 710
<211> 9
<212> PRT
<213> Artificial Sequence

<220>
<223> HIV peptide

<400> 710

```
                    Lys Leu Trp Val Thr Val Tyr Tyr Ala
                     1               5
```

<210> 711
<211> 9
<212> PRT
<213> Artificial Sequence

<220>
<223> HIV peptide

<400> 711

```
                    Lys Leu Trp Val Thr Val Tyr Tyr Ile
                     1               5
```

<210> 712
<211> 9
<212> PRT
<213> Artificial Sequence

<220>
<223> HIV peptide

<400> 712

```
                    Lys Leu Trp Val Thr Val Tyr Tyr Leu
                     1               5
```

<210> 713
<211> 9
<212> PRT
<213> Artificial Sequence

<220>
<223> HIV peptide

<400> 713

```
                    Lys Leu Trp Tyr Gln Leu Glu Lys Ala
                     1               5
```

<210> 714
<211> 9
<212> PRT
<213> Artificial Sequence

<220>
<223> HIV peptide

<400> 714

```
                    Lys Leu Trp Tyr Gln Leu Glu Lys Ala
                     1               5
```

<210> 715
<211> 9
<212> PRT
<213> Artificial Sequence

<220>
<223> HIV peptide

<400> 715

```
                    Lys Leu Trp Tyr Gln Leu Glu Lys Ile
                     1               5
```

<210> 716
<211> 9
<212> PRT
<213> Artificial Sequence

<220>
<223> HIV peptide

<400> 716

```
                    Lys Leu Trp Tyr Gln Leu Glu Lys Ile
                     1               5
```

<210> 717
<211> 9
<212> PRT
<213> Artificial Sequence

<220>
<223> HIV peptide

<400> 717

```
Lys Leu Trp Tyr Gln Leu Glu Lys Leu
 1               5
```

<210> 718
<211> 9
<212> PRT
<213> Artificial Sequence

<220>
<223> HIV peptide

<400> 718

```
Lys Leu Trp Tyr Gln Leu Glu Lys Leu
 1               5
```

<210> 719
<211> 9
<212> PRT
<213> Artificial Sequence

<220>
<223> HIV peptide

<400> 719

```
Lys Met Gly Lys Ala Gly Tyr Val Leu
 1               5
```

<210> 720
<211> 9
<212> PRT
<213> Artificial Sequence

<220>
<223> HIV peptide

<400> 720

```
Lys Met Gly Lys Ala Gly Tyr Val Val
 1               5
```

<210> 721
<211> 8
<212> PRT
<213> Artificial Sequence

<220>
<223> HIV peptide

<400> 721

```
                    Lys Met Gly Pro Glu Asn Pro Leu
                     1                   5
```

<210> 722
<211> 8
<212> PRT
<213> Artificial Sequence

<220>
<223> HIV peptide

<400> 722

```
                    Lys Met Gly Pro Glu Asn Pro Val
                     1                   5
```

<210> 723
<211> 8
<212> PRT
<213> Artificial Sequence

<220>
<223> HIV peptide

<400> 723

```
                    Lys Met Gly Ser Leu Gln Tyr Leu
                     1                   5
```

<210> 724
<211> 8
<212> PRT
<213> Artificial Sequence

<220>
<223> HIV peptide

<400> 724

```
                    Lys Met Gly Ser Leu Gln Tyr Val
                     1                   5
```

<210> 725
<211> 9
<212> PRT
<213> Artificial Sequence

<220>
<223> HIV peptide

<400> 725

```
                    Lys Met Lys Pro Pro Leu Pro Ser Val
                     1               5
```

<210> 726
<211> 9
<212> PRT
<213> Artificial Sequence

<220>
<223> HIV peptide

<400> 726

```
                         Lys Met Lys Pro Pro Leu Pro Ser Val
                          1               5
```

<210> 727
<211> 9
<212> PRT
<213> Artificial Sequence

<220>
<223> HIV peptide

<400> 727

```
                         Lys Met Asn Trp Ala Ser Gln Ile Leu
                          1               5
```

<210> 728
<211> 9
<212> PRT
<213> Artificial Sequence

<220>
<223> HIV peptide

<400> 728

```
                         Lys Met Asn Trp Ala Ser Gln Ile Val
                          1               5
```

<210> 729
<211> 9
<212> PRT
<213> Artificial Sequence

<220>
<223> HIV peptide

<400> 729

```
                        Lys Met Thr Pro Leu Cys Val Thr Val
                         1               5
```

<210> 730
<211> 9
<212> PRT
<213> Artificial Sequence

<220>
<223> HIV peptide

<400> 730

```
                        Lys Met Val Arg Met Tyr Ser Pro Ala
                         1               5
```

<210>731
<211> 9
<212> PRT
<213> Artificial Sequence

<220>
<223> HIV peptide

<400> 731

```
                        Lys Met Val Arg Met Tyr Ser Pro Leu
                         1               5
```

<210> 732
<211> 9
<212> PRT
<213> Artificial Sequence

<220>
<223> HIV peptide

<400> 732

```
                        Lys Met Val Arg Met Tyr Ser Pro Val
                         1               5
```

<210> 733
<211> 9
<212> PRT
<213> Artificial Sequence

<220>
<223> HIV peptide

<400> 733

```
                              Lys Met Trp Val Thr Val Tyr Tyr Ala
                               1               5
```

<210> 734
<211> 9
<212> PRT
<213> Artificial Sequence

<220>
<223> HIV peptide

<400> 734

```
                              Lys Met Trp Val Thr Val Tyr Tyr Leu
                               1                 5
```

<210> 735
<211> 9
<212> PRT
<213> Artificial Sequence

<220>
<223> HIV peptide

<400> 735

```
                              Lys Met Trp Val Thr Val Tyr Tyr Val
                               1                 5
```

<210> 736
<211> 9
<212> PRT
<213> Artificial Sequence

<220>
<223> HIV peptide

<400> 736

```
                              Lys Met Trp Tyr Gln Leu Glu Lys Ala
                               1               5
```

<210> 737
<211> 9
<212> PRT
<213> Artificial Sequence

<220>
<223> HIV peptide

<400> 737

```
                        Lys Met Trp Tyr Gln Leu Glu Lys Ala
                        1                   5
```

<210> 738
<211> 9
<212> PRT
<213> Artificial Sequence

<220>
<223> HIV peptide

<400> 738

```
                        Lys Met Trp Tyr Gln Leu Glu Lys Leu
                        1 .                 5
```

<210> 739
<211> 9
<212> PRT
<213> Artificial Sequence

<220>
<223> HIV peptide

<400> 739

```
                        Lys Met Trp Tyr Gln Leu Glu Lys Leu
                        1                   5
```

<210> 740
<211> 9
<212> PRT
<213> Artificial Sequence

<220>
<223> HIV peptide

<400> 740

```
                        Lys Met Trp Tyr Gln Leu Glu Lys Val
                        1                   5
```

<210> 741
<211> 9
<212> PRT
<213> Artificial Sequence

<220>
<223> HIV peptide

<400> 741

```
Lys Met Trp Tyr Gln Leu Glu Lys Val
 1                   5
```

<210> 742
<211> 8
<212> PRT
<213> Artificial Sequence

<220>
<223> HIV peptide

<400> 742

```
Lys Gln Gly Ser Leu Gln Tyr Val
 1                   5
```

<210> 743
<211> 9
<212> PRT
<213> Artificial Sequence

<220>
<223> HIV peptide

<400> 743

```
Leu Cys Phe Gly Trp Cys Phe Lys Val
 1                   5
```

<210> 744
<211> 8
<212> PRT
<213> Artificial Sequence

<220>
<223> HIV peptide

<400> 744

```
Leu Ile Ala Glu Ile Gln Lys Leu
 1                   5
```

<210> 745
<211> 8
<212> PRT
<213> Artificial Sequence

<220>
<223> HIV peptide

<400> 745

```
Leu Ile Ala Glu Ile Gln Lys Val
 1               5
```

<210> 746
<211> 8
<212> PRT
<213> Artificial Sequence

<220>
<223> HIV peptide

<400> 746

```
Leu Ile Glu Val Val Gln Lys Val
 1               5
```

<210> 747
<211> 9
<212> PRT
<213> Artificial Sequence

<220>
<223> HIV peptide

<400> 747

```
Leu Ile Phe Gly Trp Cys Phe Lys Ala
 1               5
```

<210> 748
<211> 9
<212> PRT
<213> Artificial Sequence

<220>
<223> HIV peptide

<400> 748

```
Leu Ile Phe Gly Trp Cys Phe Lys Leu
 1               5
```

<210> 749
<211> 9
<212> PRT
<213> Artificial Sequence

<220>
<223> HIV peptide

<400> 749

```
Leu Ile Phe Gly Trp Cys Phe Lys Val
 1               5
```

<210> 750
<211> 8
<212> PRT
<213> Artificial Sequence

<220>
<223> HIV peptide

<400> 750

```
Leu Ile Phe Pro Ile Ser Pro Ile
 1               5
```

<210> 751
<211> 8
<212> PRT
<213> Artificial Sequence

<220>
<223> HIV peptide

<400> 751

```
Leu Ile Phe Pro Ile Ser Pro Leu
 1               5
```

<210> 752
<211> 8
<212> PRT
<213> Artificial Sequence

<220>
<223> HIV peptide

<400> 752

```
Leu Ile Phe Pro Ile Ser Pro Val
 1               5
```

<210> 753
<211> 9
<212> PRT
<213> Artificial Sequence

<220>
<223> HIV peptide

<400> 753

```
Leu Ile Gly Pro Thr Pro Val Asn Val
 1               5
```

<210> 754
<211> 9
<212> PRT
<213> Artificial Sequence

<220>
<223> HIV peptide

<400> 754

```
Leu Ile Gly Gln Met Val His Gln Val
 1               5
```

<210> 755
<211> 9
<212> PRT
<213> Artificial Sequence

<220>
<223> HIV peptide

<400> 755

```
Leu Ile Gly Arg Trp Pro Val Lys Leu
 1               5
```

<210> 756
<211> 9
<212> PRT
<213> Artificial Sequence

<220>
<223> HIV peptide

<400> 756

```
Leu Ile Gly Arg Trp Pro Val Lys Val
 1               5
```

<210> 757
<211> 9
<212> PRT
<213> Artificial Sequence

<220>
<223> HIV peptide

<400> 757

```
Leu Ile Ile Pro His Pro Ala Gly Ala
1                   5
```

<210> 758
<211> 9
<212> PRT
<213> Artificial Sequence

<220>
<223> HIV peptide

<400> 758

```
Leu Ile Ile Pro His Pro Ala Gly Leu
1                   5                    .
```

<210> 759
<211> 9
<212> PRT
<213> Artificial Sequence

<220>
<223> HIV peptide

<400> 759

```
Leu Ile Ile Pro His Pro Ala Gly Val
1                   5
```

<210> 760
<211> 9
<212> PRT
<213> Artificial Sequence

<220>
<223> HIV peptide

<400> 760

```
Leu Ile Ile Thr Thr Tyr Trp Gly Val
1                   5
```

<210> 761
<211> 8
<212> PRT
<213> Artificial Sequence

<220>
<223> HIV peptide

<400> 761

```
                    Leu Ile Asn Arg Pro Glu Pro Leu
                     1               5
```

<210> 762
<211> 8
<212> PRT
<213> Artificial Sequence

<220>
<223> HIV peptide

<400> 762

```
                    Leu Ile Asn Arg Pro Glu Pro Val
                     1               5
```

<210> 763
<211> 9
<212> PRT
<213> Artificial Sequence

<220>
<223> HIV peptide

<400> 763

```
                    Leu Ile Gln Arg Pro Leu Val Thr Val
                     1               5
```

<210> 764
<211> 9
<212> PRT
<213> Artificial Sequence

<220>
<223> HIV peptide

<400> 764

```
                    Leu Ile Thr Ala Val Gln Met Ala Leu
                     1               5
```

<210> 765
<211> 9
<212> PRT
<213> Artificial Sequence

<220>
<223> HIV peptide

<400> 765

```
Leu Ile Thr Ala Val Gln Met Ala Val
 1               5
```

<210> 766
<211> 9
<212> PRT
<213> Artificial Sequence

<220>
<223> HIV peptide

<400> 766

```
Leu Ile Thr Ala Val Gln Met Ala Ala
 1               5
```

<210> 767
<211> 9
<212> PRT
<213> Artificial Sequence

<220>
<223> HIV peptide

<400> 767

```
Leu Ile Ala Ala Arg Thr Val Glu Val
 1               5
```

<210> 768
<211> 8
<212> PRT
<213> Artificial Sequence

<220>
<223> HIV peptide

<400> 768

```
Leu Leu Ala Glu Ile Gln Lys Ile
 1               5
```

<210> 769
<211> 8
<212> PRT
<213> Artificial Sequence

<220>
<223> HIV peptide

<400> 769

```
                    Leu Leu Ala Glu Ile Gln Lys Leu
                     1               5
```

<210> 770
<211> 8
<212> PRT
<213> Artificial Sequence

<220>
<223> HIV peptide

<400> 770

```
                    Leu Leu Glu Val Val Gln Lys Leu
                     1               5
```

<210> 771
<211> 9
<212> PRT
<213> Artificial Sequence

<220>
<223> HIV peptide

<400> 771

```
                    Leu Leu Phe Gly Trp Cys Phe Lys Ala
                     1               5
```

<210> 772
<211> 9
<212> PRT
<213> Artificial Sequence

<220>
<223> HIV peptide

<400> 772

```
                   .Leu Leu Phe Gly Trp Cys Phe Lys Ile
                     1               5
```

<210> 773
<211> 9
<212> PRT
<213> Artificial Sequence

<220>
<223> HIV peptide

<400> 773

```
Leu Leu Phe Gly Trp Cys Phe Lys Val
1               5
```

<210> 774
<211> 8
<212> PRT
<213> Artificial Sequence

<220>
<223> HIV peptide

<400> 774

```
Leu Leu Phe Pro Ile Ser Pro Ile
1               5
```

<210> 775
<211> 8
<212> PRT
<213> Artificial Sequence

<220>
<223> HIV peptide

<400> 775

```
Leu Leu Phe Pro Ile Ser Pro Leu
1               5
```

<210> 776
<211> 8
<212> PRT
<213> Artificial Sequence

<220>
<223> HIV peptide

<400> 776

```
Leu Leu Phe Val His Phe Arg Leu
1               5
```

<210> 777
<211> 9
<212> PRT
<213> Artificial Sequence

<220>
<223> HIV peptide

<400> 777

```
                    Leu Leu Gly Pro Thr Pro Val Asn Ala
                     1               5
```

<210> 778
<211> 9
<212> PRT
<213> Artificial Sequence

<220>
<223> HIV peptide

<400> 778

```
                    Leu Leu Gly Pro Thr Pro Val Asn Ile
                     1               5
```

<210> 779
<211> 9
<212> PRT
<213> Artificial Sequence

<220>
<223> HIV peptide

<400> 779

```
                    Leu Leu Gly Pro Thr Pro Val Asn Leu
                     1               5
```

<210> 780
<211> 9
<212> PRT
<213> Artificial Sequence

<220>
<223> HIV peptide

<400> 780

```
                    Leu Leu Gly Gln Met Val His Gln Ala
                     1               5
```

<210> 781
<211> 9
<212> PRT
<213> Artificial Sequence

<220>
<223> HIV peptide

<400> 781

```
                    Leu Leu Gly Gln Met Val His Gln Ile
                     1               5
```

<210> 782
<211> 9
<212> PRT
<213> Artificial Sequence

<220>
<223> HIV peptide

<400> 782

```
                    Leu Leu Gly Gln Met Val His Gln Leu
                     1               5
```

<210> 783
<211> 9
<212> PRT
<213> Artificial Sequence

<220>
<223> HIV peptide

<400> 783

```
                    Leu Leu Gly Arg Trp Pro Val Lys Ala
                     1               5
```

<210> 784
<211> 9
<212> PRT
<213> Artificial Sequence

<220>
<223> HIV peptide

<400> 784

```
                    Leu Leu Gly Arg Trp Pro Val Lys Ile
                     1               5
```

<210> 785
<211> 9
<212> PRT
<213> Artificial Sequence

<220>
<223> HIV peptide

<400> 785

```
                    Leu Leu Gly Arg Trp Pro Val Lys Leu
                     1               5
```

<210> 786
<211> 9
<212> PRT
<213> Artificial Sequence

<220>
<223> HIV peptide

<400> 786

```
                    Leu Leu Ile Pro His Pro Ala Gly Ala
                     1               5
```

<210> 787
<211> 9
<212> PRT
<213> Artificial Sequence

<220>
<223> HIV peptide

<400> 787

```
                    Leu Leu Ile Pro His Pro Ala Gly Ile
                     1               5
```

<210> 788
<211> 9
<212> PRT
<213> Artificial Sequence

<220>
<223> HIV peptide

<400> 788

```
                    Leu Leu Ile Pro His Pro Ala Gly Leu
                     1             .  5
```

<210> 789
<211> 9
<212> PRT
<213> Artificial Sequence

<220>
<223> HIV peptide

<400> 789

```
                    Leu Leu Gly Arg Trp Pro Val Lys Leu
                     1               5
```

```
                         Leu Leu Ile Thr Thr Tyr Trp Gly Ala
                           1               5
```

<210> 790
<211> 9
<212> PRT
<213> Artificial Sequence

<220>
<223> HIV peptide

<400> 790

```
                         Leu Leu Ile Thr Thr Tyr Trp Gly Ile
                           1               5
```

<210> 791
<211> 9
<212> PRT
<213> Artificial Sequence

<220>
<223> HIV peptide

<400> 791

```
                         Leu Leu Ile Thr Thr Tyr Trp Gly Val
                           1               5
```

<210> 792
<211> 9
<212> PRT
<213> Artificial Sequence

<220>
<223> HIV peptide

<400> 792

```
                         Leu Leu Lys Leu Thr Val Trp Gly Ala
                           1               5
```

<210> 793
<211> 9
<212> PRT
<213> Artificial Sequence

<220>
<223> HIV peptide

<400> 793

```
                    Leu Leu Lys Leu Thr Val Trp Gly Leu
                    1                   5
```

<210> 794
<211> 9
<212> PRT
<213> Artificial Sequence

<220>
<223> HIV peptide

<400> 794

```
                            Leu Leu Leu Pro Pro Ile Glu Arg Ala
                            1                   5
```

<210> 795
<211> 9
<212> PRT
<213> Artificial Sequence

<220>
<223> HIV peptide

<400> 795

```
                            Leu Leu Leu Pro Pro Ile Glu Arg Ile
                            1                   5
```

<210> 796
<211> 9
<212> PRT
<213> Artificial Sequence

<220>
<223> HIV peptide

<400> 796

```
                        Leu Leu Leu Pro Pro Ile Glu Arg Leu
                        1                   5
```

<210> 797
<211> 8
<212> PRT
<213> Artificial Sequence

<220>
<223> HIV peptide

<400> 797

```
                    Leu Leu Asn Arg Pro Glu Pro Leu
                     1               5
```

<210> 798
<211> 9
<212> PRT
<213> Artificial Sequence

<220>
<223> HIV peptide

<400> 798

```
                    Leu Leu Gln Arg Pro Leu Val Thr Ala
                    . 1               5
```

<210> 799
<211> 9
<212> PRT
<213> Artificial Sequence

<220>
<223> HIV peptide

<400> 799

```
                    Leu Leu Gln Arg Pro Leu Val Thr Ile
                     1               5
```

<210> 800
<211> 9
<212> PRT
<213> Artificial Sequence

<220>
<223> HIV peptide

<400> 800

```
                    Leu Leu Gln Arg Pro Leu Val Thr Leu
                     1               5
```

<210> 801
<211> 9
<212> PRT
<213> Artificial Sequence

<220>
<223> HIV peptide

<400> 801

```
                         Leu Leu Thr Ala Val Gln Met Ala Ile
                          1               5
```

<210> 802
<211> 9
<212> PRT
<213> Artificial Sequence

<220>
<223> HIV peptide

<400> 802

```
                         Leu Leu Thr Ala Val Gln Met Ala Leu
                          1               5
```

<210> 803
<211> 9
<212> PRT
<213> Artificial Sequence

<220>
<223> HIV peptide

<400> 803

```
                         Leu Leu Thr Ala Val Gln Met Ala Ala
                          1               5
```

<210> 804
<211> 9
<212> PRT
<213> Artificial Sequence

<220>
<223> HIV peptide

<400> 804

```
                         Leu Leu Ala Ala Arg Thr Val Glu Ala
                          1               5
```

<210> 805
<211> 9
<212> PRT
<213> Artificial Sequence

<220>
<223> HIV peptide

<400> 805

```
                            Leu Leu Ala Ala Arg Thr Val Glu Ile
                             1               5
```

<210> 806
<211> 9
<212> PRT
<213> Artificial Sequence

<220>
<223> HIV peptide

<400> 806

```
                        Leu Leu Ala Ala Arg Thr Val Glu Leu
                         1               5
```

<210> 807
<211> 8
<212> PRT
<213> Artificial Sequence

<220>
<223> HIV peptide

<400> 807

```
                        Leu Met Ala Glu Ile Gln Lys Leu
                         1               5
```

<210> 808
<211> 8
<212> PRT
<213> Artificial Sequence

<220>
<223> HIV peptide

<400> 808

```
                        Leu Met Ala Glu Ile Gln Lys Val
                         1               5
```

<210> 809
<211> 8
<212> PRT
<213> Artificial Sequence

<220>
<223> HIV peptide

<400> 809

```
                    Leu Met Glu Val Val Gln Lys Val
                     1               5
```

<210> 810
<211> 9
<212> PRT
<213> Artificial Sequence

<220>
<223> HIV peptide

<400> 810

```
                    Leu Met Phe Gly Trp Cys Phe Lys Ala
                     1               5
```

<210> 811
<211> 9
<212> PRT
<213> Artificial Sequence

<220>
<223> HIV peptide

<400> 811

```
                    Leu Met Phe Gly Trp Cys Phe Lys Ile
                     1               5
```

<210> 812
<211> 9
<212> PRT
<213> Artificial Sequence

<220>
<223> HIV peptide

<400> 812

```
                    Leu Met Phe Gly Trp Cys Phe Lys Leu
                     1               5
```

<210> 813
<211> 9
<212> PRT
<213> Artificial Sequence

<220>
<223> HIV peptide

<400> 813

```
Leu Met Phe Gly Trp Cys Phe Lys Val
1                   5
```

<210> 814
<211> 8
<212> PRT
<213> Artificial Sequence

<220>
<223> HIV peptide

<400> 814

```
Leu Met Phe Pro Ile Ser Pro Ile
1                   5
```

<210> 815
<211> 8
<212> PRT
<213> Artificial Sequence

<220>
<223> HIV peptide

<400> 815

```
Leu Met Phe Pro Ile Ser Pro Leu
1                   5
```

<210> 816
<211> 8
<212> PRT
<213> Artificial Sequence

<220>
<223> HIV peptide

<400> 816

```
Leu Met Phe Pro Ile Ser Pro Val
1                   5
```

<210> 817
<211> 9
<212> PRT
<213> Artificial Sequence

<220>
<223> HIV peptide

<400> 817

```
Leu Met Gly Pro Thr Pro Val Asn Ala
1               5
```

<210> 818
<211> 9
<212> PRT
<213> Artificial Sequence

<220>
<223> HIV peptide

<400> 818

```
Leu Met Gly Pro Thr Pro Val Asn Ile
1               5
```

<210> 819
<211> 9
<212> PRT
<213> Artificial Sequence

<220>
<223> HIV peptide

<400> 819

```
Leu Met Gly Pro Thr Pro Val Asn Leu
1               5
```

<210> 820
<211> 9
<212> PRT
<213> Artificial Sequence

<220>
<223> HIV peptide

<400> 820

```
Leu Met Gly Pro Thr Pro Val Asn Val
1               5
```

<210> 821
<211> 9
<212> PRT
<213> Artificial Sequence

<220>
<223> HIV peptide

<400> 821

```
                    Leu Met Gly Gln Met Val His Gln Ala
                     1               5
```

<210> 822
<211> 9
<212> PRT
<213> Artificial Sequence

<220>
<223> HIV peptide

<400> 822

```
                    Leu Met Gly Gln Met Val His Gln Ile
                     1               5
```

<210> 823
<211> 9
<212> PRT
<213> Artificial Sequence

<220>
<223> HIV peptide

<400> 823

```
                    Leu Met Gly Gln Met Val His Gln Leu
                     1               5
```

<210> 824
<211> 9
<212> PRT
<213> Artificial Sequence

<220>
<223> HIV peptide

<400> 824

```
                    Leu Met Gly Gln Met Val His Gln Val
                     1               5
```

<210> 825
<211> 9
<212> PRT
<213> Artificial Sequence

<220>
<223> HIV peptide

<400> 825

```
                    Leu Met Gly Arg Trp Pro Val Lys Ala  .
                     1               5
```

<210> 826
<211> 9
<212> PRT
<213> Artificial Sequence

<220>
<223> HIV peptide

<400> 826

```
                    Leu Met Gly Arg Trp Pro Val Lys Ile
                     1               5
```

<210> 827
<211> 9
<212> PRT
<213> Artificial Sequence

<220>
<223> HIV peptide

<400> 827

```
                    Leu Met Gly Arg Trp Pro Val Lys Leu
                     1   .           5
```

<210> 828
<211> 9
<212> PRT
<213> Artificial Sequence

<220>
<223> HIV peptide

<400> 828

```
                    Leu Met Gly Arg Trp Pro Val Lys Val
                 .   1               5
```

<210> 829
<211> 9
<212> PRT
<213> Artificial Sequence

<220>
<223> HIV peptide

<400> 829

```
                              Leu Met Ile Pro His Pro Ala Gly Ala
                               1                   5
```

<210> 830
<211> 9
<212> PRT
<213> Artificial Sequence

<220>
<223> HIV peptide

<400> 830

```
                              Leu Met Ile Pro His Pro Ala Gly Ile
                               1                   5
```

<210> 831
<211> 9
<212> PRT
<213> Artificial Sequence

<220>
<223> HIV peptide

<400> 831

```
                              Leu Met Ile Pro His Pro Ala Gly Leu
                               1                   5
```

<210> 832
<211> 9
<212> PRT
<213> Artificial Sequence

<220>
<223> HIV peptide

<400> 832

```
                              Leu Met Ile Pro His Pro Ala Gly Val
                               1                   5
```

<210> 833
<211> 9
<212> PRT
<213> Artificial Sequence

<220>
<223> HIV peptide

<400> 833

```
Leu Met Ile Thr Thr Tyr Trp Gly Ala
 1                   5
```

<210> 834
<211> 9
<212> PRT
<213> Artificial Sequence

<220>
<223> HIV peptide

<400> 834

```
Leu Met Ile Thr Thr Tyr Trp Gly Ile
 1                   5
```

<210> 835
<211> 9
<212> PRT
<213> Artificial Sequence

<220>
<223> HIV peptide

<400> 835

```
Leu Met Ile Thr Thr Tyr Trp Gly Leu
 1                   5
```

<210> 836
<211> 9
<212> PRT
<213> Artificial Sequence

<220>
<223> HIV peptide

<400> 836

```
Leu Met Ile Thr Thr Tyr Trp Gly Val
 1                   5
```

<210> 837
<211> 9
<212> PRT
<213> Artificial Sequence

<220>
<223> HIV peptide

<400> 837

```
                    Leu Met Lys Leu Thr Val Trp Gly Leu
                     1                   5
```

<210> 838
<211> 9
<212> PRT
<213> Artificial Sequence

<220>
<223> HIV peptide

<400> 838

```
                    Leu Met Lys Leu Thr Val Trp Gly Val
                     1                   5
```

<210> 839
<211> 9
<212> PRT
<213> Artificial Sequence

<220>
<223> HIV peptide

<400> 839

```
                    Leu Met Leu Pro Pro Ile Glu Arg Ala
                     1                   5
```

<210> 840
<211> 9
<212> PRT
<213> Artificial Sequence

<220>
<223> HIV peptide

<400> 840

```
                    Leu Met Leu Pro Pro Ile Glu Arg Leu
                     1                   5
```

<210> 841
<211> 9
<212> PRT
<213> Artificial Sequence

<220>
<223> HIV peptide

<400> 841

```
Leu Met Leu Pro Pro Ile Glu Arg Val
 1               5
```

<210> 842
<211> 8
<212> PRT
<213> Artificial Sequence

<220>
<223> HIV peptide

<400> 842

```
Leu Met Asn Arg Pro Glu Pro Leu
 1               5
```

<210> 843
<211> 8
<212> PRT
<213> Artificial Sequence

<220>
<223> HIV peptide

<400> 843

```
Leu Met Asn Arg Pro Glu Pro Val
 1               5
```

<210> 844
<211> 9
<212> PRT
<213> Artificial Sequence

<220>
<223> HIV peptide

<400> 844

```
Leu Met Gln Arg Pro Leu Val Thr Ala
 1               5
```

<210> 845
<211> 9
<212> PRT
<213> Artificial Sequence

<220>
<223> HIV peptide

<400> 845

```
                        Leu Met Gln Arg Pro Leu Val Thr Ile
                         1               5
```

<210> 846
<211> 9
<212> PRT
<213> Artificial Sequence

<220>
<223> HIV peptide

<400> 846

```
                        Leu Met Gln Arg Pro Leu Val Thr Leu
                         1               5
```

<210> 847
<211> 9
<212> PRT
<213> Artificial Sequence

<220>
<223> HIV peptide

<400> 847

```
                        Leu Met Gln Arg Pro Leu Val Thr Val
                         1               5
```

<210> 848
<211> 9
<212> PRT
<213> Artificial Sequence

<220>
<223> HIV peptide

<400> 848

```
                        Leu Met Thr Ala Val Gln Met Ala Ile
                         1               5
```

<210> 849
<211> 9
<212> PRT
<213> Artificial Sequence

<220>
<223> HIV peptide

<400> 849

```
Leu Met Thr Ala Val Gln Met Ala Leu
 1               5
```

<210> 850
<211> 9
<212> PRT
<213> Artificial Sequence

<220>
<223> HIV peptide

<400> 850

```
Leu Met Thr Ala Val Gln Met Ala Val
 1           .   5
```

<210> 851
<211> 9
<212> PRT
<213> Artificial Sequence

<220>
<223> HIV peptide

<400> 851

```
Leu Met Thr Ala Val Gln Met Ala Ala
 1               5
```

<210> 852
<211> 9
<212> PRT
<213> Artificial Sequence

<220>
<223> HIV peptide

<400> 852

```
Leu Met Ala Ala Arg Thr Val Glu Ala     .
 1               5
```

<210> 853
<211> 9
<212> PRT
<213> Artificial Sequence

<220>
<223> HIV peptide

<400> 853

```
                      Leu Met Ala Ala Arg Thr Val Glu Ile
                       1               5
```

<210> 854
<211> 9
<212> PRT
<213> Artificial Sequence

<220>
<223> HIV peptide

<400> 854

```
                      Leu Met Ala Ala Arg Thr Val Glu Leu
                       1               5
```

<210> 855
<211> 9
<212> PRT
<213> Artificial Sequence

<220>
<223> HIV peptide

<400> 855

```
                      Leu Met Ala Ala Arg Thr Val Glu Val
                       1               5
```

<210> 856
<211> 8
<212> PRT
<213> Artificial Sequence

<220>
<223> HIV peptide

<400> 856

```
                      Leu Asn Phe Pro Ile Ser Pro Leu
                       1               5
```

<210> 857
<211> 8
<212> PRT
<213> Artificial Sequence

<220>
<223> HIV peptide

<400> 857

```
                         Leu Asn Phe Pro Ile Ser Pro Val
                          1               5
```

<210> 858
<211> 8
<212> PRT
<213> Artificial Sequence

<220>
<223> HIV peptide

<400> 858

```
                         Leu Gln Ala Glu Ile Gln Lys Leu
                          1               5
```

<210> 859
<211> 8
<212> PRT
<213> Artificial Sequence

<220>
<223> HIV peptide

<400> 859

```
                         Leu Gln Ala Glu Ile Gln Lys Val
                          1               5
```

<210> 860
<211> 9
<212> PRT
<213> Artificial Sequence

<220>
<223> HIV peptide

<400> 860

```
                         Leu Gln Phe Gly Trp Cys Phe Lys Leu
                          1               5
```

<210> 861
<211> 9
<212> PRT
<213> Artificial Sequence

<220>
<223> HIV peptide

<400> 861

```
Leu Gln Phe Gly Trp Cys Phe Lys Val
 1               5
```

<210> 862
<211> 8
<212> PRT
<213> Artificial Sequence

<220>
<223> HIV peptide

<400> 862

```
Leu Gln Phe Pro Ile Ser Pro Leu
 1               5
```

<210> 863
<211> 8
<212> PRT
<213> Artificial Sequence

<220>
<223> HIV peptide

<400> 863

```
Leu Gln Phe Pro Ile Ser Pro Val
 1               5
```

<210> 864
<211> 9
<212> PRT
<213> Artificial Sequence

<220>
<223> HIV peptide

<400> 864

```
Leu Gln Gly Arg Trp Pro Val Lys Val
 1               5
```

<210> 865
<211> 9
<212> PRT
<213> Artificial Sequence

<220>
<223> HIV peptide

<400> 865

```
Leu Gln Ile Pro His Pro Ala Gly Leu
1                   5
```

<210> 866
<211> 9
<212> PRT
<213> Artificial Sequence

<220>
<223> HIV peptide

<400> 866

```
Leu Gln Ile Pro His Pro Ala Gly Val
1                   5
```

<210> 867
<211> 8
<212> PRT
<213> Artificial Sequence

<220>
<223> HIV peptide

<400> 867

```
Leu Gln Asn Arg Pro Glu Pro Val
1                   5
```

<210> 868
<211> 9
<212> PRT
<213> Artificial Sequence

<220>
<223> HIV peptide

<400> 868

```
Leu Gln Thr Ala Val Gln Met Ala Leu
1                   5
```

<210> 869
<211> 9
<212> PRT
<213> Artificial Sequence

<220>
<223> HIV peptide

<400> 869

```
          Leu Gln Thr Ala Val Gln Met Ala Val
            1               5
```

<210> 870
<211> 9
<212> PRT
<213> Artificial Sequence

<220>
<223> HIV peptide

<400> 870

```
          Leu Val Gly Pro Thr Pro Val Asn Val
            1               5
```

<210> 871
<211> 9
<212> PRT
<213> Artificial Sequence

<220>
<223> HIV peptide

<400> 871

```
          Leu Val Ile Thr Thr Tyr Trp Gly Val
            1               5
```

<210> 872
<211> 9
<212> PRT
<213> Artificial Sequence

<220>
<223> HIV peptide

<400> 872

```
          Met Ile Gln Arg Gly Asn Phe Lys Val
            1               5
```

<210> 873
<211> 9
<212> PRT
<213> Artificial Sequence

<220>
<223> HIV peptide

<400> 873

```
Met Leu Leu Gly Met Leu Met Ile Ala
 1               5
```

<210> 874
<211> 9
<212> PRT
<213> Artificial Sequence

<220>
<223> HIV peptide

<400> 874

```
Met Leu Leu Gly Met Leu Met Ile Ile
 1               5
```

<210> 875
<211> 9
<212> PRT
<213> Artificial Sequence

<220>
<223> HIV peptide

<400> 875

```
Met Leu Leu Gly Met Leu Met Ile Leu
 1               5
```

<210> 876
<211> 9
<212> PRT
<213> Artificial Sequence

<220>
<223> HIV peptide

<400> 876

```
Met Leu Asn Asn Pro Pro Ile Pro Ala
 1               5
```

<210> 877
<211> 9
<212> PRT
<213> Artificial Sequence

<220>
<223> HIV peptide

<400> 877

Met Leu Asn Asn Pro Pro Ile Pro Leu
1                5

<210> 878
<211> 9
<212> PRT
<213> Artificial Sequence

<220>
<223> HIV peptide

<400> 878

Met Leu Gln Arg Gly Asn Phe Lys Ala
1                5

<210> 879
<211> 9
<212> PRT
<213> Artificial Sequence

<220>
<223> HIV peptide

<400> 879

Met Leu Gln Arg Gly Asn Phe Lys Ile
1                5

<210> 880
<211> 9
<212> PRT
<213> Artificial Sequence

<220>
<223> HIV peptide

<400> 880

Met Leu Gln Arg Gly Asn Phe Lys Leu
1                5

<210> 881
<211> 9
<212> PRT
<213> Artificial Sequence

<220>
<223> HIV peptide

<400> 881

```
Met Leu Gln Arg Gly Asn Phe Arg Ala
 1               5
```

<210> 882
<211> 9
<212> PRT
<213> Artificial Sequence

<220>
<223> HIV peptide

<400> 882

```
Met Leu Gln Arg Gly Asn Phe Arg Ile
 1               5
```

<210> 883
<211> 9
<212> PRT
<213> Artificial Sequence

<220>
<223> HIV peptide

<400> 883

```
Met Leu Gln Arg Gly Asn Phe Arg Leu
 1               5
```

<210> 884
<211> 9
<212> PRT
<213> Artificial Sequence

<220>
<223> HIV peptide

<400> 884

```
Met Leu Ser Asn Pro Pro Ile Pro Ala
 1               5
```

<210> 885
<211> 9
<212> PRT
<213> Artificial Sequence

<220>
<223> HIV peptide

<400> 885

```
                              Met Leu Ser Asn Pro Pro Ile Pro Leu
                                1               5
```

<210> 886
<211> 9
<212> PRT
<213> Artificial Sequence

<220>
<223> HIV peptide

<900> 886

```
                              Met Met Leu Gly Met Leu Met Ile Ala
                                1               5
```

<210> 887
<211> 9
<212> PRT
<213> Artificial Sequence

<220>
<223> HIV peptide

<400> 887

```
                              Met Met Leu Gly Met Leu Met Ile Leu
                                1               5
```

<210> 888
<211> 9
<212> PRT
<213> Artificial Sequence

<220>
<223> HIV peptide

<400> 888

```
                              Met Met Leu Gly Met Leu Met Ile Val
                                1               5
```

<210> 889
<211> 9
<212> PRT
<213> Artificial Sequence

<220>
<223> HIV peptide

<400> 889

```
                    Met Met Asn Asn Pro Pro Ile Pro Leu
                    1               5
```

<210> 890
<211> 9
<212> PRT
<213> Artificial Sequence

<220>
<223> HIV peptide

<400> 890

```
                    Met Met Asn Asn Pro Pro Ile Pro Val
                    1               5
```

<210> 891
<211> 9
<212> PRT
<213> Artificial Sequence

<220>
<223> HIV peptide

<400> 891

```
                    Met Met Gln Arg Gly Asn Phe Lys Ala
                    1               5
```

<210> 892
<211> 9
<212> PRT
<213> Artificial Sequence

<220>
<223> HIV peptide

<400> 892

```
                    Met Met Gln Arg Gly Asn Phe Lys Ile
                    1               5
```

<210> 893
<211> 9
<212> PRT
<213> Artificial Sequence

<220>
<223> HIV peptide

<400> 893

```
                        Met Met Gln Arg Gly Asn Phe Lys Leu
                         1               5
```

<210> 894
<211> 9
<212> PRT
<213> Artificial Sequence

<220>
<223> HIV peptide

<400> 894

```
                        Met Met Gln Arg Gly Asn Phe Lys Val
                         1               5
```

<210> 895
<211> 9
<212> PRT
<213> Artificial Sequence

<220>
<223> HIV peptide

<400> 895

```
                        Met Met Gln Arg Gly Asn Phe Arg Ala
                         1               5
```

<210> 896
<211> 9
<212> PRT
<213> Artificial Sequence

<220>
<223> HIV peptide

<400> 896

```
                        Met Met Gln Arg Gly Asn Phe Arg Ile
                         1               5
```

<210> 897
<211> 9
<212> PRT
<213> Artificial Sequence

<220>
<223> HIV peptide

<400> 897

```
        Met Met Gln Arg Gly Asn Phe Arg Leu
          1               5
```

<210> 898
<211> 9
<212> PRT
<213> Artificial Sequence

<220>
<223> HIV peptide

<400> 898

```
        Met Met Gln Arg Gly Asn Phe Arg Val
          1               5
```

<210> 899
<211> 9
<212> PRT
<213> Artificial Sequence

<220>
<223> HIV peptide

<400> 899

```
        Met Met Ser Asn Pro Pro Ile Pro Leu
          1               5
```

<210> 900
<211> 9
<212> PRT
<213> Artificial Sequence

<220>
<223> HIV peptide

<400> 900

```
        Met Met Ser Asn Pro Pro Ile Pro Val
          1               5
```

<210> 901
<211> 9
<212> PRT
<213> Artificial Sequence

<220>
<223> HIV peptide

<400> 901

```
                    Met Gln Gln Arg Gly Asn Phe Lys Val
                     1                   5
```

<210> 902
<211> 9
<212> PRT
<213> Artificial Sequence

<220>
<223> HIV peptide

<400> 902

```
                    Asn Leu Val Ala Thr Leu Tyr Cys Leu
                     1                   5
```

<210> 903
<211> 9
<212> PRT
<213> Artificial Sequence

<220>
<223> HIV peptide

<400> 903

```
                    Asn Leu Trp Ala Thr His Ala Cys Leu
                     1                   5
```

<210> 904
<211> 9
<212> PRT
<213> Artificial Sequence

<220>
<223> HIV peptide

<400> 904

```
                    Asn Met Ala Asp Gln Leu Ile His Val
                     1                   5
```

<210> 905
<211> 9
<212> PRT
<213> Artificial Sequence

<220>
<223> HIV peptide

<400> 905

```
                    Asn Met Val Ala Thr Leu Tyr Cys Val
                     1               5
```

<210> 906
<211> 9
<212> PRT
<213> Artificial Sequence

<220>
<223> HIV peptide

<400> 906

```
                    Asn Met Trp Ala Thr His Ala Cys Val
                     1               5
```

<210> 907
<211> 8
<212> PRT
<213> Artificial Sequence

<220>
<223> HIV peptide

<400> 907

```
                    Pro Ile Gly Asp Ile Tyr Lys Ala
                     1               5
```

<210> 908
<211> 8
<212> PRT
<213> Artificial Sequence

<220>
<223> HIV peptide

<400> 908

```
                    Pro Ile Gly Asp Ile Tyr Lys Ile
                     1               5
```

<210> 909
<211> 8
<212> PRT
<213> Artificial Sequence

<220>
<223> HIV peptide

<400> 909

```
Pro Ile Gly Asp Ile Tyr Lys Leu
 1               5
```

<210> 910
<211> 8
<212> PRT
<213> Artificial Sequence

<220>
<223> HIV peptide

<400> 910

```
Pro Ile Gly Asp Ile Tyr Lys Arg
 1               5
```

<210> 911
<211> 8
<212> PRT '
<213> Artificial Sequence

<220>
<223> HIV peptide

<400> 911

```
Pro Ile Gly Asp Ile Tyr Lys Val
 1               5
```

<210> 912
<211> 8
<212> PRT
<213> Artificial Sequence

<220>
<223> HIV peptide

<400> 912

```
Pro Ile Gly Glu Ile Tyr Lys Ile
 1               5
```

<210> 913
<211> 8
<212> PRT
<213> Artificial Sequence

<220>
<223> HIV peptide

<400> 913

```
                    Pro Ile Gly Glu Ile Tyr Lys Leu
                     1                5
```

<210> 914
<211> 8
<212> PRT
<213> Artificial Sequence

<220>
<223> HIV peptide

<400> 914

```
                    Pro Ile Gly Glu Ile Tyr Lys Val
                     1                5
```

<210> 915
<211> 8
<212> PRT
<213> Artificial Sequence

<220>
<223> HIV peptide

<400> 915

```
                    Pro Leu Gly Asp Ile Tyr Lys Ala
                     1                5
```

<210> 916
<211> 8
<212> PRT
<213> Artificial Sequence

<220>
<223> HIV peptide

<400> 916

```
                    Pro Leu Gly Asp Ile Tyr Lys Ile
                     1                5
```

<210> 917
<211> 8
<212> PRT
<213> Artificial Sequence

<220>
<223> HIV peptide

<400> 917

```
Pro Leu Gly Asp Ile Tyr Lys Leu
 1               5
```

<210> 918
<211> 8
<212> PRT
<213> Artificial Sequence

<220>
<223> HIV peptide

<400> 918

```
Pro Leu Gly Asp Ile Tyr Lys Arg
 1               5
```

<210> 919
<211> 8
<212> PRT
<213> Artificial Sequence

<220>
<223> HIV peptide

<400> 919

```
Pro Leu Gly Glu Ile Tyr Lys Ala
 1               5
```

<210> 920
<211> 8
<212> PRT
<213> Artificial Sequence

<220>
<223> HIV peptide

<400> 920

```
Pro Leu Gly Glu Ile Tyr Lys Ile
 1               5
```

<210> 921
<211> 8
<212> PRT
<213> Artificial Sequence

<220>
<223> HIV peptide

<400> 921

```
                        Pro Leu Gly Glu Ile Tyr Lys Leu
                         1               5
```

<210> 922
<211> 8
<212> PRT
<213> Artificial Sequence

<220>
<223> HIV peptide

<400> 922

```
                        Pro Met Gly Asp Ile Tyr Lys Ala
                         1               5
```

<210> 923
<211> 8
<212> PRT
<213> Artificial Sequence

<220>
<223> HIV peptide

<400> 923

```
                        Pro Met Gly Asp Ile Tyr Lys Ile
                         1               5
```

<210> 924
<211> 8
<212> PRT
<213> Artificial Sequence

<220>
<223> HIV peptide

<400> 924

```
                        Pro Met Gly Asp Ile Tyr Lys Leu
                         1               5
```

<210> 925
<211> 8
<212> PRT
<213> Artificial Sequence

<220>
<223> HIV peptide

<400> 925

```
Pro Met Gly Asp Ile Tyr Lys Arg
1               5
```

<210> 926
<211> 8
<212> PRT
<213> Artificial Sequence

<220>
<223> HIV peptide

<400> 926

```
Pro Met Gly Asp Ile Tyr Lys Val
1               5
```

<210> 927
<211> 8
<212> PRT
<213> Artificial Sequence

<220>
<223> HIV peptide

<400> 927

```
Pro Met Gly Glu Ile Tyr Lys Ile
1               5
```

<210> 928
<211> 8
<212> PRT
<213> Artificial Sequence

<220>
<223> HIV peptide

<400> 928

```
Pro Met Gly Glu Ile Tyr Lys Leu
1               5
```

<210> 929
<211> 8
<212> PRT
<213> Artificial Sequence

<220>
<223> HIV peptide

<400> 929

Pro Met Gly Glu Ile Tyr Lys Val
1                   5

<210> 930
<211> 8
<212> PRT
<213> Artificial Sequence

<220>
<223> HIV peptide

<400> 930

Pro Gln Gly Asp Ile Tyr Lys Ile
1                   5

<210> 931
<211> 8
<212> PRT
<213> Artificial Sequence

<220>
<223> HIV peptide

<400> 931

Pro Gln Gly Asp Ile Tyr Lys Leu
1                   5

<210> 932
<211> 8
<212> PRT
<213> Artificial Sequence

<220>
<223> HIV peptide

<400> 932

Pro Gln Gly Asp Ile Tyr Lys Val
1                   5

<210> 933
<211> 8
<212> PRT
<213> Artificial Sequence

<220>
<223> HIV peptide

<400> 933

```
Pro Gln Gly Glu Ile Tyr Lys Leu
1               5
```

<210> 934
<211> 8
<212> PRT
<213> Artificial Sequence

<220>
<223> HIV peptide

<400> 934

```
Pro Gln Gly Glu Ile Tyr Lys Val·
1               5
```

<210> 935
<211> 8
<212> PRT
<213> Artificial Sequence

<220>
<223> HIV peptide

<400> 935

```
Pro Val Gly Asp Ile Tyr Lys Ile
1               5
```

<210> 936
<211> 8
<212> PRT
<213> Artificial Sequence

<220>
<223> HIV peptide

<400> 936

```
Pro Val Gly Asp Ile Tyr Lys Leu
1               5
```

<210> 937
<211> 8
<212> PRT
<213> Artificial Sequence

<220>
<223> HIV peptide

<400> 937

```
Pro Val Gly Asp Ile Tyr Lys Val
 1               5
```

<210> 938
<211> 8
<212> PRT
<213> Artificial Sequence

<220>
<223> HIV peptide

<400> 938

```
Pro Val Gly Glu Ile Tyr Lys Leu
 1               5
```

<210> 939
<211> 8
<212> PRT
<213> Artificial Sequence

<220>
<223> HIV peptide

<400> 939

```
Pro Val Gly Glu Ile Tyr Lys Val
 1               5
```

<210> 940
<211> 8
<212> PRT
<213> Artificial Sequence

<220>
<223> HIV peptide

<400> 940

```
Gln Ile Gly Ile Pro His Pro Ile
 1               5
```

<210> 941
<211> 8
<212> PRT
<213> Artificial Sequence

<220>
<223> HIV peptide

<400> 941

```
Gln Ile Gly Ile Pro His Pro Leu
 1               5
```

<210> 942
<211> 8
<212> PRT
<213> Artificial Sequence

<220>
<223> HIV peptide

<400> 942

```
Gln Ile Gly Ile Pro His Pro Val
 1               5
```

<210> 943
<211> 8
<212> PRT
<213> Artificial Sequence

<220>
<223> HIV peptide

<400> 943

```
Gln Leu Gly Ile Pro His Pro Ile
 1               5
```

<210> 944
<211> 8
<212> PRT
<213> Artificial Sequence

<220>
<223> HIV peptide

<400> 944

```
Gln Leu Gly Ile Pro His Pro Leu
 1               5
```

<210> 945
<211> 9
<212> PRT
<213> Artificial Sequence

<220>
<223> HIV peptide

<400> 945

```
Gln Leu Thr Glu Ala Val Gln Lys Leu
 1               5
```

<210> 946
<211> 8
<212> PRT
<213> Artificial Sequence

<220>
<223> HIV peptide

<400> 946

```
Gln Met Gly Ile Pro His Pro Ile
 1               5
```

<210> 947
<211> 8
<212> PRT
<213> Artificial Sequence

<220>
<223> HIV peptide

<400> 947

```
Gln Met Gly Ile Pro His Pro Leu
 1               5
```

<210> 948
<211> 8
<212> PRT
<213> Artificial Sequence

<220>
<223> HIV peptide

<400> 948

```
Gln Met Gly Ile Pro His Pro Val
 1               5
```

<210> 949
<211> 9
<212> PRT
<213> Artificial Sequence

<220>
<223> HIV peptide

<400> 949

```
Gln Met Thr Glu Ala Val Gln Lys Val
 1               5
```

<210> 950
<211> 8
<212> PRT
<213> Artificial Sequence

<220>
<223> HIV peptide

<400> 950

```
Gln Gln Gly Ile Pro His Pro Leu
 1               5
```

<210> 951
<211> 8
<212> PRT
<213> Artificial Sequence

<220>
<223> HIV peptide

<400> 951

```
Gln Gln Gly Ile Pro His Pro Val
 1               5
```

<210> 952
<211> 8
<212> PRT
<213> Artificial Sequence

<220>
<223> HIV peptide

<400> 952

```
Arg Ile Ala Glu Pro Val Pro Leu
 1               5
```

<210> 953
<211> 8
<212> PRT
<213> Artificial Sequence

<220>
<223> HIV peptide

<400> 953

```
                    Arg Ile Ala Glu Pro Val Pro Val
                     1               5
```

<210> 954
<211> 9
<212> PRT
<213> Artificial Sequence

<220>
<223> HIV peptide

<400> 954

```
                    Arg Ile Ile Glu Ala Gln Gln His Val
                     1               5
```

<210> 955
<211> 8
<212> PRT
<213> Artificial Sequence

<220>
<223> HIV peptide

<400> 955

```
                    Arg Leu Ala Glu Pro Val Pro Leu
                     1               5
```

<210> 956
<211> 9
<212> PRT
<213> Artificial Sequence

<220>
<223> HIV peptide

<400> 956

```
                    Arg Leu Ala Phe His His Met Ala Ile
                     1               5
```

<210> 957
<211> 9
<212> PRT
<213> Artificial Sequence

<220>
<223> HIV peptide

<400> 957

Arg Leu Ala Phe His His Met Ala Leu
1                   5

<210> 958
<211> 9
<212> PRT
<213> Artificial Sequence

<220>
<223> HIV peptide

<400> 958

Arg Leu Ala Phe His His Met Ala Ala
1                   5

<210> 959
<211> 9
<212> PRT
<213> Artificial Sequence

<220>
<223> HIV peptide

<400> 959

Arg Leu His Pro Val Gln Ala Gly Ala
1                   5

<210> 960
<211> 9
<212> PRT
<213> Artificial Sequence

<220>
<223> HIV peptide

<400> 960

Arg Leu His Pro Val Gln Ala Gly Ile
1                   5

<210> 961
<211> 9
<212> PRT
<213> Artificial Sequence

<220>
<223> HIV peptide

<400> 961

```
Arg Leu His Pro Val Gln Ala Gly Leu
 1               5
```

<210> 962
<211> 9
<212> PRT
<213> Artificial Sequence

<220>
<223> HIV peptide

<400> 962

```
Arg Leu Ile Glu Ala Gln Gln His Ala
 1               5
```

<210> 963
<211> 9
<212> PRT
<213> Artificial Sequence

<220>
<223> HIV peptide

<400> 963

```
Arg Leu Ile Glu Ala Gln Gln His Ile
 1               5
```

<210> 964
<211> 9
<212> PRT
<213> Artificial Sequence

<220>
<223> HIV peptide

<400> 964

```
Arg Leu Ile Glu Ala Gln Gln His Leu
 1               5
```

<210> 965
<211> 9
<212> PRT
<213> Artificial Sequence

<220>
<223> HIV peptide

<400> 965

```
                              Arg Leu Tyr Ser Pro Val Ser Ile Leu
                               1               5
```

<210> 966
<211> 8
<212> PRT
<213> Artificial Sequence

<220>
<223> HIV peptide

<400> 966

```
                              Arg Met Ala Glu Pro Val Pro Leu
                               1               5
```

<210> 967
<211> 8
<212> PRT
<213> Artificial Sequence

<220>
<223> HIV peptide

<400> 967

```
                              Arg Met Ala Glu Pro Val Pro Val
                               1               5
```

<210> 968
<211> 9
<212> PRT
<213> Artificial Sequence

<220>
<223> HIV peptide

<400> 968

```
                              Arg Met Ala Phe His His Met Ala Ile
                               1               5
```

<210> 969
<211> 9
<212> PRT
<213> Artificial Sequence

<220>
<223> HIV peptide

<400> 969

```
                    Arg Met Ala Phe His His Met Ala Leu
                     1                   5
```

<210> 970
<211> 9
<212> PRT
<213> Artificial Sequence

<220>
<223> HIV peptide

<400> 970

```
                    Arg Met Ala Phe His His Met Ala Val
                     1                   5
```

<210> 971
<211> 9
<212> PRT
<213> Artificial Sequence

<220>
<223> HIV peptide

<400> 971

```
                    Arg Met Ala Phe His His Met Ala Ala
                     1                   5
```

<210> 972
<211> 9
<212> PRT
<213> Artificial Sequence

<220>
<223> HIV peptide

<400> 972

```
                    Arg Met His Pro Val Gln Ala Gly Ala
                     1                   5
```

<210> 973
<211> 9
<212> PRT
<213> Artificial Sequence

<220>
<223> HIV peptide

<400> 973

```
                    Arg Met His Pro Val Gln Ala Gly Leu
                     1                 5
```

<210> 974
<211> 9
<212> PRT
<213> Artificial Sequence

<220>
<223> HIV peptide

<400> 974

```
                    Arg Met His Pro Val Gln Ala Gly Val
                     1                 5
```

<210> 975
<211> 9
<212> PRT
<213> Artificial Sequence

<220>
<223> HIV peptide

<400> 975

```
                    Arg Met Ile Glu Ala Gln Gln His Ala
                     1                 5
```

<210> 976
<211> 9
<212> PRT
<213> Artificial Sequence

<220>
<223> HIV peptide

<400> 976

```
                    Arg Met Ile Glu Ala Gln Gln His Ile
                     1                 5
```

<210> 977
<211> 9
<212> PRT
<213> Artificial Sequence

<220>
<223> HIV peptide

<400> 977

```
Arg Met Ile Glu Ala Gln Gln His Leu
 1               5
```

<210> 978
<211> 9
<212> PRT
<213> Artificial Sequence

<220>
<223> HIV peptide

<400> 978

```
Arg Met Ile Glu Ala Gln Gln His Val
 1               5
```

<210> 979
<211> 9
<212> PRT
<213> Artificial Sequence

<220>
<223> HIV peptide

<400> 979

```
Arg Met Tyr Ser Pro Val Ser Ile Val
 1               5
```

<210> 980
<211> 8
<212> PRT
<213> Artificial Sequence

<220>
<223> HIV peptide

<400> 980

```
Arg Gln Ala Glu Pro Val Pro Val
 1               5
```

<210> 981
<211> 8
<212> PRT
<213> Artificial Sequence

<220>
<223> HIV peptide

<400> 981

```
Ser Ile Gly Asp Pro Glu Ile Leu
 1                   5
```

<210> 982
<211> 8
<212> PRT
<213> Artificial Sequence

<220>
<223> HIV peptide

<400> 982

```
Ser Ile Gly Asp Pro Glu Ile Val
 1                   5
```

<210> 983
<211> 9
<212> PRT
<213> Artificial Sequence

<220>
<223> HIV peptide

<400> 983

```
Ser Leu Ala Glu Glu Glu Val Val Leu
 1                   5
```

<210> 984
<211> 9
<212> PRT
<213> Artificial Sequence

<220>
<223> HIV peptide

<400> 984

```
Ser Leu Glu Glu Met Met Thr Ala Leu
 1                   5
```

<210> 985
<211> 8
<212> PRT
<213> Artificial Sequence

<220>
<223> HIV peptide

<400> 985

```
                        Ser Leu Gly Asp Pro Glu Ile Ile
                        1                   5
```

<210> 986
<211> 8
<212> PRT
<213> Artificial Sequence

<220>
<223> HIV peptide

<400> 986

```
                        Ser Leu Gly Asp Pro Glu Ile Leu
                        1                   5
```

<210> 987
<211> 9
<212> PRT
<213> Artificial Sequence

<220>
<223> HIV peptide

<400> 987

```
                        Ser Leu Gly Gln His Ile Tyr Glu Ala
                        1                   5
```

<210> 988
<211> 9
<212> PRT
<213> Artificial Sequence

<220>
<223> HIV peptide

<400> 988

```
                        Ser Leu Gly Gln His Ile Tyr Glu Ile
                        1                   5
```

<210> 989
<211> 9
<212> PRT
<213> Artificial Sequence

<220>
<223> HIV peptide

<400> 989

```
Ser Leu Gly Gln His Ile Tyr Glu Leu
 1               5
```

<210> 990
<211> 9
<212> PRT
<213> Artificial Sequence

<220>
<223> HIV peptide

<400> 990

```
Ser Leu Gly Gln Tyr Ile Tyr Glu Ala
 1               5
```

<210> 991
<211> 9
<212> PRT
<213> Artificial Sequence

<220>
<223> HIV peptide

<400> 991

```
Ser Leu Gly Gln Tyr Ile Tyr Glu Leu
 1               5
```

<210> 992
<211> 9
<212> PRT
<213> Artificial Sequence

<220>
<223> HIV peptide

<400> 992

```
Ser Leu Ile Tyr Ala Gly Ile Lys Ala
 1               5
```

<210> 993
<211> 9
<212> PRT
<213> Artificial Sequence

<220>
<223> HIV peptide

<400> 993

```
                    Ser Leu Ile Tyr Ala Gly Ile Lys Leu
                     1                   5
```

<210> 994
<211> 9
<212> PRT
<213> Artificial Sequence

<220>
<223> HIV peptide

<400> 994

```
                    Ser Leu Ile Tyr Pro Gly Ile Lys Ala
                     1                   5
```

<210> 995
<211> 9
<212> PRT
<213> Artificial Sequence

<220>
<223> HIV peptide

<400> 995

```
                    Ser Leu Ile Tyr Pro Gly Ile Lys Ile
                     1                   5
```

<210> 996
<211> 9
<212> PRT
<213> Artificial Sequence

<220>
<223> HIV peptide

<400> 996

```
                    Ser Leu Ile Tyr Pro Gly Ile Lys Leu
                     1                   5
```

<210> 997
<211> 9
<212> PRT
<213> Artificial Sequence

<220>
<223> HIV peptide

<400> 997

```
                        Ser Leu Val Lys His His Met Tyr Ala
                         1               5
```

<210> 998
<211> 9
<212> PRT
<213> Artificial Sequence

<220>
<223> HIV peptide

<400> 998

```
                        Ser Leu Val Lys His His Met Tyr Leu
                         1               5
```

<210> 999
<211> 9
<212> PRT
<213> Artificial Sequence

<220>
<223> HIV peptide

<400> 999

```
                        Ser Leu Trp Asn Trp Phe Ser Ile Leu
                         1               5
```

<210> 1000
<211> 9
<212> PRT
<213> Artificial Sequence

<220>
<223> HIV peptide

<400> 1000

```
                        Ser Met Ala Glu Glu Glu Val Val Val
                         1               5
```

<210> 1001
<211> 9
<212> PRT
<213> Artificial Sequence

<220>
<223> HIV peptide

<400> 1001

```
                    Ser Met Glu Glu Met Met Thr Ala Leu
                     1                   5
```

<210> 1002
<211> 9
<212> PRT
<213> Artificial Sequence

<220>
<223> HIV peptide

<400> 1002

```
                    Ser Met Glu Glu Met Met Thr Ala Val
                     1                   5
```

<210> 1003
<211> 8
<212> PRT
<213> Artificial Sequence

<220>
<223> HIV peptide

<400> 1003

```
                    Ser Met Gly Asp Pro Glu Ile Leu
                     1                   5
```

<210> 1004
<211> 8
<212> PRT
<213> Artificial Sequence

<220>
<223> HIV peptide

<400> 1004

```
                    Ser Met Gly Asp Pro Glu Ile Val
                     1                   5
```

<210> 1005
<211> 9
<212> PRT
<213> Artificial Sequence

<220>
<223> HIV peptide

<400> 1005

```
Ser Met Gly Gln His Ile Tyr Glu Ala
 1               5
```

<210> 1006
<211> 9
<212> PRT
<213> Artificial Sequence

<220>
<223> HIV peptide

<400> 1006

```
Ser Met Gly Gln His Ile Tyr Glu Leu
 1               5
```

<210> 1007
<211> 9
<212> PRT
<213> Artificial Sequence

<220>
<223> HIV peptide

<400> 1007

```
Ser Met Gly Gln His Ile Tyr Glu Val
 1               5·
```

<210> 1008
<211> 9
<212> PRT
<213> Artificial Sequence

<220>
<223> HIV peptide

<400> 1008

```
Ser Met Gly Gln Tyr Ile Tyr Glu Leu
 1               5
```

<210> 1009
<211> 9
<212> PRT
<213> Artificial Sequence

<220>
<223> HIV peptide

<400> 1009

```
Ser Met Gly Gln Tyr Ile Tyr Glu Val
 1               5
```

<210> 1010
<211> 9
<212> PRT
<213> Artificial Sequence

<220>
<223> HIV peptide

<400> 1010

```
Ser Met Ile Tyr Ala Gly Ile Lys Leu
 1               5
```

<210> 1011
<211> 9
<212> PRT
<213> Artificial Sequence

<220>
<223> HIV peptide

<400> 1011

```
Ser Met Ile Tyr Ala Gly Ile Lys Val
 1               5
```

<210> 1012
<211> 9
<212> PRT
<213> Artificial Sequence

<220>
<223> HIV peptide

<400> 1012

```
Ser Met Ile Tyr Pro Gly Ile Lys Ala
 1               5
```

<210> 1013
<211> 9
<212> PRT
<213> Artificial Sequence

<220>
<223> HIV peptide

<400> 1013

```
Ser Met Ile Tyr Pro Gly Ile Lys Leu
 1               5
```

<210> 1014
<211> 9
<212> PRT
<213> Artificial Sequence

<220>
<223> HIV peptide

<400> 1014

```
Ser Met Ile Tyr Pro Gly Ile Lys Val
 1               5
```

<210> 1015
<211> 9
<212> PRT
<213> Artificial Sequence

<220>
<223> HIV peptide

<400> 1015

```
Ser Met Val Lys His His Met Tyr Leu
 1               5
```

<210> 1016
<211> 9
<212> PRT
<213> Artificial Sequence

<220>
<223> HIV peptide

<400> 1016

```
Ser Met Val Lys His His Met Tyr Val
 1               5
```

<210> 1017
<211> 9
<212> PRT
<213> Artificial Sequence

<220>
<223> HIV peptide

<400> 1017

Ser Met Trp Asn Trp Phe Ser Ile Val
1                   5

<210> 1018
<211> 9
<212> PRT
<213> Artificial Sequence

<220>
<223> HIV peptide

<400> 1018

Ser Met Tyr Asn Thr Val Ala Thr Val
1                   5

<210> 1019
<211> 8
<212> PRT
<213> Artificial Sequence

<220>
<223> HIV peptide

<400> 1019

Ser Gln Gly Asp Pro Glu Ile Leu
1                   5

<210> 1020
<211> 8
<212> PRT
<213> Artificial Sequence

<220>
<223> HIV peptide

<400> 1020

Ser Gln Gly Asp Pro Glu Ile Val
1                   5

<210> 1021
<211> 8
<212> PRT
<213> Artificial Sequence

<220>
<223> HIV peptide

<400> 1021

```
                        Thr Ile Gly Trp Cys Phe Lys Leu
                         1               5
```

<210> 1022
<211> 8
<212> PRT
<213> Artificial Sequence

<220>
<223> HIV peptide

<400> 1022

```
                        Thr Ile Gly Trp Cys Phe Lys Val
                         1               5
```

<210> 1023
<211> 8
<212> PRT
<213> Artificial Sequence

<220>
<223> HIV peptide

<400> 1023

```
                        Thr Ile Asn Asp Ile Gln Lys Leu
                         1               5
```

<210> 1024
<211> 8
<212> PRT
<213> Artificial Sequence

<220>
<223> HIV peptide

<400> 1024

```
                        Thr Ile Asn Asp Ile Gln Lys Val
                         1               5
```

<210> 1025
<211> 8
<212> PRT
<213> Artificial Sequence

<220>
<223> HIV peptide

<400> 1025

```
Thr Ile Asn Pro Pro Ile Pro Leu
 1               5
```

<210> 1026
<211> 8
<212> PRT
<213> Artificial Sequence

<220>
<223> HIV peptide

<400> 1026

```
Thr Ile Asn Pro Pro Ile Pro Leu
 1               5
```

<210> 1027
<211> 8
<212> PRT
<213> Artificial Sequence

<220>
<223> HIV peptide

<400> 1027

```
Thr Ile Asn Pro Pro Ile Pro Val
 1               5
```

<210> 1028
<211> 8
<212> PRT
<213> Artificial Sequence

<220>
<223> HIV peptide

<400> 1028

```
Thr Ile Asn Pro Pro Ile Pro Val
 1               5
```

<210> 1029
<211> 8
<212> PRT
<213> Artificial Sequence

<220>
<223> HIV peptide

<400> 1029

```
                        Thr Leu Gly Trp Cys Phe Lys Leu
                         1               5
```

<210> 1030
<211> 9
<212> PRT
<213> Artificial Sequence

<220>
<223> HIV peptide

<400> 1030

```
                        Thr Leu Gly Ala Ala Ser Ile Thr Leu
                         1               5
```

<210> 1031
<211> 8
<212> PRT
<213> Artificial Sequence

<220>
<223> HIV peptide

<400> 1031

```
                        Thr Leu Asn Asp Ile Gln Lys Ile
                         1               5
```

<210> 1032
<211> 8
<212> PRT
<213> Artificial Sequence

<220>
<223> HIV peptide

<400> 1032

```
                        Thr Leu Asn Asp Ile Gln Lys Leu
                         1               5
```

<210> 1033
<211> 8
<212> PRT
<213> Artificial Sequence

<220>
<223> HIV peptide

<400> 1033

```
                            Thr Leu Asn Pro Pro Ile Pro Leu
                             1               5
```

<210> 1034
<211> 8
<212> PRT
<213> Artificial Sequence

<220>
<223> HIV peptide

<400> 1034

```
                            Thr Leu Asn Pro Pro Ile Pro Leu
                             1               5
```

<210> 1035
<211> 9
<212> PRT
<213> Artificial Sequence

<220>
<223> HIV peptide

<400> 1035

```
                            Thr Leu Gln Glu Gln Ile Ala Trp Leu
                             1               5
```

<210> 1036
<211> 8
<212> PRT
<213> Artificial Sequence

<220>
<223> HIV peptide

<400> 1036

```
                            Thr Met Gly Trp Cys Phe Lys Leu
                             1               5
```

<210> 1037
<211> 8
<212> PRT
<213> Artificial Sequence

<220>
<223> HIV peptide

<400> 1037

```
Thr Met Gly Trp Cys Phe Lys Val
 1               5
```

<210> 1038
<211> 9
<212> PRT
<213> Artificial Sequence

<220>
<223> HIV peptide

<400> 1038

```
Thr Met Gly Ala Ala Ser Ile Thr Val
 1               5
```

<210> 1039
<211> 8
<212> PRT
<213> Artificial Sequence

<220>
<223> HIV peptide

<400> 1039

```
Thr Met Asn Asp Ile Gln Lys Leu
 1               5
```

<210> 1040
<211> 8
<212> PRT
<213> Artificial Sequence

<220>
<223> HIV peptide

<400> 1040

```
Thr Met Asn Asp Ile Gln Lys Val
 1               5
```

<210> 1041
<211> 8
<212> PRT
<213> Artificial Sequence

<220>
<223> HIV peptide

<400> 1041

```
Thr Met Asn Pro Pro Ile Pro Leu
 1               5
```

<210> 1042
<211> 8
<212> PRT
<213> Artificial Sequence

<220>
<223> HIV peptide

<400> 1042

```
Thr Met Asn Pro Pro Ile Pro Leu
 1               5
```

<210> 1043
<211> 8
<212> PRT
<213> Artificial Sequence

<220>
<223> HIV peptide

<400> 1043

```
Thr Met Asn Pro Pro Ile Pro Val
 1               5
```

<210> 1044
<211> 8
<212> PRT
<213> Artificial Sequence

<220>
<223> HIV peptide

<400> 1044

```
Thr Met Asn Pro Pro Ile Pro Val
 1               5
```

<210> 1045
<211> 9
<212> PRT
<213> Artificial Sequence

<220>
<223> HIV peptide

<400> 1045

```
                         Thr Met Gln Glu Gln Ile Ala Trp Leu
                          1               5
```

<210> 1046
<211> 9
<212> PRT
<213> Artificial Sequence

<220>
<223> HIV peptide

<400> 1046

```
                         Thr Met Gln Glu Gln Ile Ala Trp Val
                          1               5
```

<210> 1047
<211> 9
<212> PRT
<213> Artificial Sequence

<220>
<223> HIV peptide

<400> 1047

```
                         Thr Met Gln Glu Gln Ile Gly Trp Val
                          1               5
```

<210> 1048
<211> 8
<212> PRT
<213> Artificial Sequence

<220>
<223> HIV peptide

<400> 1048

```
                         Thr Gln Gly Trp Cys Phe Lys Leu
                          1               5
```

<210> 1049
<211> 8
<212> PRT
<213> Artificial Sequence

<220>
<223> HIV peptide

<400> 1049

```
                    Thr Gln Gly Trp Cys Phe Lys Val
                     1               5
```

<210> 1050
<211> 8
<212> PRT
<213> Artificial Sequence

<220>
<223> HIV peptide

<400> 1050

```
                    Thr Gln Asn Asp Ile Gln Lys Leu
                     1               5
```

<210> 1051
<211> 8
<212> PRT
<213> Artificial Sequence

<220>
<223> HIV peptide

<400> 1051

```
                    Thr Gln Asn Asp Ile Gln Lys Val
                     1               5
```

<210> 1052
<211> 8
<212> PRT
<213> Artificial Sequence

<220>
<223> HIV peptide

<400> 1052

```
                    Thr Gln Asn Pro Pro Ile Pro Leu
                     1               5
```

<210> 1053
<211> 8
<212> PRT
<213> Artificial Sequence

<220>
<223> HIV peptide

<400> 1053

```
                            Thr Gln Asn Pro Pro Ile Pro Leu
                             1               5
```

<210> 1054
<211> 8
<212> PRT
<213> Artificial Sequence

<220>
<223> HIV peptide

<400> 1054

```
                            Thr Gln Asn Pro Pro Ile Pro Val
                             1               5
```

<210> 1055
<211> 8
<212> PRT
<213> Artificial Sequence

<220>
<223> HIV peptide

<400> 1055

```
                            Thr Gln Asn Pro Pro Ile Pro Val
                             1               5
```

<210> 1056
<211> 8
<212> PRT
<213> Artificial Sequence

<220>
<223> HIV peptide

<400> 1056

```
                            Thr Val Asn Asp Ile Gln Lys Val
                             1               5
```

<210> 1057
<211> 9
<212> PRT
<213> Artificial Sequence

<220>
<223> HIV peptide

<400> 1057

Val Leu Ala Glu Ala Met Ser Gln Leu
1                       5

<210> 1058
<211> 9
<212> PRT
<213> Artificial Sequence

<220>
<223> HIV peptide

<400> 1058

Val Met Ala Glu Ala Met Ser Gln Val
1                       5

<210> 1059
<211> 9
<212> PRT
<213> Artificial Sequence

<220>
<223> HIV peptide

<400> 1059

Val Met Val Tyr Tyr Gly Val Pro Val
1                       5

<210> 1060
<211> 8
<212> PRT
<213> Artificial Sequence

<220>
<223> HIV peptide

<400> 1060

Trp Ile Phe Lys Leu Val Pro Val
1                       5

<210> 1061
<211> 8
<212> PRT
<213> Artificial Sequence

<220>
<223> HIV peptide

<400> 1061

```
Trp Leu Phe Lys Leu Val Pro Leu
1               5
```

<210> 1062
<211> 8
<212> PRT
<213> Artificial Sequence

<220>
<223> HIV peptide

<400> 1062

```
Trp Met Phe Lys Leu Val Pro Val
1               5
```

<210> 1063
<211> 9
<212> PRT
<213> Artificial Sequence

<220>
<223> HIV peptide

<400> 1063

```
Tyr Cys Ala Pro Ala Gly Phe Ala Leu
1               5
```

<210> 1064
<211> 9
<212> PRT
<213> Artificial Sequence

<220>
<223> HIV peptide

<400> 1064

```
Tyr Cys Ala Pro Ala Gly Phe Ala Val
1               5
```

<210> 1065
<211> 9
<212> PRT
<213> Artificial Sequence

<220>
<223> HIV peptide

<400> 1065

```
                          Tyr Cys Ala Pro Ala Gly Tyr Ala Val
                           1               5
```

<210> 1066
<211> 9
<212> PRT
<213> Artificial Sequence

<220>
<223> HIV peptide

<400> 1066

```
                          Tyr Ile Ala Pro Ala Gly Phe Ala Ile
                           1               5
```

<210> 1067
<211> 9
<212> PRT
<213> Artificial Sequence

<220>
<223> HIV peptide

<400> 1067

```
                          Tyr Ile Ala Pro Ala Gly Phe Ala Leu
                           1               5
```

<210> 1068
<211> 9
<212> PRT
<213> Artificial Sequence

<220>
<223> HIV peptide

<400> 1068

```
                          Tyr Ile Ala Pro Ala Gly Phe Ala Val
                           1               5
```

<210> 1069
<211> 9
<212> PRT
<213> Artificial Sequence

<220>
<223> HIV peptide

<400> 1069

```
Tyr Ile Ala Pro Ala Gly Phe Ala Ala
 1                   5
```

<210> 1070
<211> 9
<212> PRT
<213> Artificial Sequence

<220>
<223> HIV peptide

<400> 1070

```
Tyr Ile Ala Pro Ala Gly Tyr Ala Leu
 1                   5
```

<210> 1071
<211> 9
<212> PRT
<213> Artificial Sequence

<220>
<223> HIV peptide

<400> 1071

```
Tyr Ile Ala Pro Ala Gly Tyr Ala Val
 1                   5
```

<210> 1072
<211> 8
<212> PRT
<213> Artificial Sequence

<220>
<223> HIV peptide

<400> 1072

```
Tyr Ile Ser Asn Pro Tyr Pro Ile
 1                   5
```

<210> 1073
<211> 8
<212> PRT
<213> Artificial Sequence

<220>
<223> HIV peptide

<400> 1073

```
Tyr Ile Ser Asn Pro Tyr Pro Leu
 1               5
```

<210> 1074
<211> 8
<212> PRT
<213> Artificial Sequence

<220>
<223> HIV peptide

<400> 1074

```
Tyr Ile Ser Asn Pro Tyr Pro Val
 1               5
```

<210> 1075
<211> 9
<212> PRT
<213> Artificial Sequence

<220>
<223> HIV peptide

<400> 1075

```
Tyr Leu Ala Pro Ala Gly Phe Ala Ile
 1               5
```

<210> 1076
<211> 9
<212> PRT
<213> Artificial Sequence

<220>
<223> HIV peptide

<400> 1076

```
Tyr Leu Ala Pro Ala Gly Phe Ala Leu
 1               5
```

<210> 1077
<211> 9
<212> PRT
<213> Artificial Sequence

<220>
<223> HIV peptide

<400> 1077

```
Tyr Leu Ala Pro Ala Gly Phe Ala Ala
 1               5
```

<210> 1078
<211> 9
<212> PRT
<213> Artificial Sequence

<220>
<223> HIV peptide

<400> 1078

```
Tyr Leu Ala Pro Ala Gly Tyr Ala Ile
 1               5
```

<210> 1079
<211> 9
<212> PRT
<213> Artificial Sequence

<220>
<223> HIV peptide

<400> 1079

```
Tyr Leu Ala Pro Ala Gly Tyr Ala Leu
 1               5
```

<210> 1080
<211> 9
<212> PRT
<213> Artificial Sequence

<220>
<223> HIV peptide

<400> 1080

```
Tyr Leu Ala Pro Ala Gly Tyr Ala Ala
 1               5
```

<210> 1081
<211> 9
<212> PRT
<213> Artificial Sequence

<220>
<223> HIV peptide

<400> 1081

```
Tyr Leu Ala Trp Val Pro Ala His Ala
 1                   5
```

<210> 1082
<211> 9
<212> PRT
<213> Artificial Sequence

<220>
<223> HIV peptide

<400> 1082

```
Tyr Leu Ala Trp Val Pro Ala His Ile
 1                   5
```

<210> 1083
<211> 9
<212> PRT
<213> Artificial Sequence

<220>
<223> HIV peptide

<400> 1083

```
Tyr Leu Ala Trp Val Pro Ala His Leu
 1                   5
```

<210> 1084
<211> 9
<212> PRT
<213> Artificial Sequence

<220>
<223> HIV peptide

<400> 1084

```
Tyr Leu Lys Ile Phe Ile Met Ile Leu
 1                   5
```

<210> 1085
<211> 8
<212> PRT
<213> Artificial Sequence

<220>
<223> HIV peptide

<400> 1085

```
Tyr Leu Ser Asn Pro Tyr Pro Ile
 1               5
```

<210> 1086
<211> 8
<212> PRT
<213> Artificial Sequence

<220>
<223> HIV peptide

<400> 1086

```
Tyr Leu Ser Asn Pro Tyr Pro Lys
 1               5                  .
```

<210> 1087
<211> 8
<212> PRT
<213> Artificial Sequence

<220>
<223> HIV peptide

<400> 1087

```
Tyr Leu Ser Asn Pro Tyr Pro Leu
 1               5
```

<210> 1088
<211> 9
<212> PRT
<213> Artificial Sequence

<220>
<223> HIV peptide

<400> 1088

```
Tyr Met Ala Phe Thr Ile Pro Ser Val
 1               5
```

<210> 1089
<211> 9
<212> PRT
<213> Artificial Sequence

<220>
<223> HIV peptide

<400> 1089

```
                    Tyr Met Ala Leu Gln Asp Ser Gly Val
                     1               5
```

<210> 1090
<211> 9
<212> PRT
<213> Artificial Sequence

<220>
<223> HIV peptide

<400> 1090

```
                    Tyr Met Ala Pro Ala Gly Phe Ala Ile
                     1               5
```

<210> 1091
<211> 9
<212> PRT
<213> Artificial Sequence

<220>
<223> HIV peptide

<400> 1091

```
                    Tyr Met Ala Pro Ala Gly Phe Ala Leu
                     1               5
```

<210> 1092
<211> 9
<212> PRT
<213> Artificial Sequence

<220>
<223> HIV peptide

<400> 1092

```
                    Tyr Met Ala Pro Ala Gly Phe Ala Val
                     1               5
```

<210> 1093
<211> 9
<212> PRT
<213> Artificial Sequence

<220>
<223> HIV peptide

<400> 1093

```
                        Tyr Met Ala Pro Ala Gly Phe Ala Ala
                         1               5
```

<210> 1094
<211> 9
<212> PRT
<213> Artificial Sequence

<220>
<223> HIV peptide

<400> 1094

```
                        Tyr Met Ala Pro Ala Gly Tyr Ala Ile
                         1               5
```

<210> 1095
<211> 9
<212> PRT
<213> Artificial Sequence

<220>
<223> HIV peptide

<400> 1095

```
                        Tyr Met Ala Pro Ala Gly Tyr Ala Leu
                         1               5
```

<210> 1096
<211> 9
<212> PRT
<213> Artificial Sequence

<220>
<223> HIV peptide

<400> 1096

```
                        Tyr Met Ala Pro Ala Gly Tyr Ala Val
                         1               5
```

<210> 1097
<211> 9
<212> PRT
<213> Artificial Sequence

<220>
<223> HIV peptide

<400> 1097

```
                        Tyr Met Ala Pro Ala Gly Tyr Ala Ala
                         1                   5
```

<210> 1098
<211> 9
<212> PRT
<213> Artificial Sequence

<220>
<223> HIV peptide

<400> 1098

```
                        Tyr Met Ala Trp Val Pro Ala His Ala
                         1                   5
```

<210> 1099
<211> 9
<212> PRT
<213> Artificial Sequence

<220>
<223> HIV peptide

<400> 1099

```
                        Tyr Met Ala Trp Val Pro Ala His Ile
                         1                   5
```

<210> 1100
<211> 9
<212> PRT
<213> Artificial Sequence

<220>
<223> HIV peptide

<400> 1100

```
                        Tyr Met Ala Trp Val Pro Ala His Leu
                         1                   5
```

<210> 1101
<211> 9
<212> PRT
<213> Artificial Sequence

<220>
<223> HIV peptide

<400> 1101

```
                    Tyr Met Ala Trp Val Pro Ala His Val
                     1               5
```

<210> 1102
<211> 9
<212> PRT
<213> Artificial Sequence

<220>
<223> HIV peptide

<400> 1102

```
                    Tyr Met Lys Ile Phe Ile Met Ile Leu
                     1               5
```

<210> 1103
<211> 9
<212> PRT
<213> Artificial Sequence

<220>
<223> HIV peptide

<400> 1103

```
                    Tyr Met Lys Ile Phe Ile Met Ile Val
                     1               5
```

<210> 1104
<211> 8
<212> PRT
<213> Artificial Sequence

<220>
<223> HIV peptide

<400> 1104

```
                    Tyr Met Ser Asn Pro Tyr Pro Ile
                     1               5
```

<210> 1105
<211> 8
<212> PRT
<213> Artificial Sequence

<220>
<223> HIV peptide

<400> 1105

```
Tyr Met Ser Asn Pro Tyr Pro Leu
 1               5
```

<210> 1106
<211> 8
<212> PRT
<213> Artificial Sequence

<220>
<223> HIV peptide

<400> 1106

```
Tyr Met Ser Asn Pro Tyr Pro Val
 1               5
```

<210> 1107
<211> 9
<212> PRT
<213> Artificial Sequence

<220>
<223> HIV peptide

<400> 1107

```
Tyr Gln Ala Pro Ala Gly Phe Ala Leu
 1               5
```

<210> 1108
<211> 9
<212> PRT
<213> Artificial Sequence

<220>
<223> HIV peptide

<400> 1108

```
Tyr Gln Ala Pro Ala Gly Phe Ala Val
 1               5
```

<210> 1109
<211> 9
<212> PRT
<213> Artificial Sequence

<220>
<223> HIV peptide

<400> 1109

```
Tyr Gln Ala Pro Ala Gly Phe Ala Ala
 1                   5
```

<210> 1110
<211> 9
<212> PRT
<213> Artificial Sequence

<220>
<223> HIV peptide

<400> 1110

```
Tyr Gln Ala Pro Ala Gly Tyr Ala Leu
 1                   5
```

<210> 1111
<211> 9
<212> PRT
<213> Artificial Sequence

<220>
<223> HIV peptide

<400> 1111

```
Tyr Gln Ala Pro Ala Gly Tyr Ala Val
 1                   5
```

<210> 1112
<211> 8
<212> PRT
<213> Artificial Sequence

<220>
<223> HIV peptide

<400> 1112

```
Tyr Gln Ser Asn Pro Tyr Pro Leu
 1                   5
```

<210> 1113
<211> 8
<212> PRT
<213> Artificial Sequence

<220>
<223> HIV peptide

<400> 1113

```
                         Tyr Gln Ser Asn Pro Tyr Pro Val
                          1               5
```

<210> 1114
<211> 9
<212> PRT
<213> Artificial Sequence

<220>
<223> HIV peptide

<400> 1114

```
                         Ala Ile Tyr Ala Pro Pro Ile Ala Val
                          1               5
```

<210> 1115
<211> 9
<212> PRT
<213> Artificial Sequence

<220>
<223> HIV peptide

<400> 1115

```
                         Ala Ile Tyr Ala Pro Pro Ile Lys Leu
                          1               5
```

<210> 1116
<211> 9
<212> PRT
<213> Artificial Sequence

<220>
<223> HIV peptide

<400> 1116

```
                         Ala Ile Tyr Ala Pro Pro Ile Lys Val
                          1               5
```

<210> 1117
<211> 9
<212> PRT
<213> Artificial Sequence

<220>
<223> HIV peptide

<400> 1117

```
Ala Leu Tyr Ala Pro Pro Ile Ala Ala
 1                   5
```

<210> 1118
<211> 9
<212> PRT
<213> Artificial Sequence

<220>
<223> HIV peptide

<400> 1118

```
Ala Leu Tyr Ala Pro Pro Ile Ala Ile
 1                   5
```

<210> 1119
<211> 9
<212> PRT
<213> Artificial Sequence

<220>
<223> HIV peptide

<400> 1119

```
Ala Leu Tyr Ala Pro Pro Ile Ala Leu
 1                   5
```

<210> 1120
<211> 9
<212> PRT
<213> Artificial Sequence

<220>
<223> HIV peptide

<400> 1120

```
Ala Leu Tyr Ala Pro Pro Ile Ala Val
 1                   5
```

<210> 1121
<211> 9
<212> PRT
<213> Artificial Sequence

<220>
<223> HIV peptide

<400> 1121

```
Ala Leu Tyr Ala Pro Pro Ile Lys Ala
 1               5
```

<210> 1122
<211> 9
<212> PRT
<213> Artificial Sequence

<220>
<223> HIV peptide

<400> 1122

```
Ala Leu Tyr Ala Pro Pro Ile Lys Ile
 1               5
```

<210> 1123
<211> 9
<212> PRT
<213> Artificial Sequence

<220>
<223> HIV peptide

<400> 1123

```
Ala Leu Tyr Ala Pro Pro Ile Lys Leu
 1               5
```

<210> 1124
<211> 9
<212> PRT
<213> Artificial Sequence

<220>
<223> HIV peptide

<400> 1124

```
Ala Leu Tyr Ala Pro Pro Ile Lys Val
 1               5
```

<210> 1125
<211> 9
<212> PRT
<213> Artificial Sequence

<220>
<223> HIV peptide

<400> 1125

```
Ala Met Tyr Ala Pro Pro Ile Ala Ala
 1               5
```

<210> 1126
<211> 9
<212> PRT
<213> Artificial Sequence

<220>
<223> HIV peptide

<400> 1126

```
Ala Met Tyr Ala Pro Pro Ile Ala Ile
 1               5
```

<210> 1127
<211> 9
<212> PRT
<213> Artificial Sequence

<220>
<223> HIV peptide

<400> 1127

```
Ala Met Tyr Ala Pro Pro Ile Ala Leu
 1               5
```

<210> 1128
<211> 9
<212> PRT
<213> Artificial Sequence

<220>
<223> HIV peptide

<400> 1128

```
Ala Met Tyr Ala Pro Pro Ile Lys Ala
 1               5
```

<210> 1129
<211> 9
<212> PRT
<213> Artificial Sequence

<220>
<223> HIV peptide

<400> 1129

```
Ala Met Tyr Ala Pro Pro Ile Lys Ile
 1               5
```

<210> 1130
<211> 9
<212> PRT
<213> Artificial Sequence

<220>
<223> HIV peptide

<400> 1130

```
Ala Met Tyr Ala Pro Pro Ile Lys Leu
 1               5
```

<210> 1131
<211> 9
<212> PRT
<213> Artificial Sequence

<220>
<223> HIV peptide

<400> 1131

```
Ala Gln Tyr Ala Pro Pro Ile Lys Val
 1               5
```

<210> 1132
<211> 9
<212> PRT
<213> Artificial Sequence

<220>
<223> HIV peptide

<400> 1132

```
Lys Leu Met Ala Gly Ala Asp Cys Leu
 1               5
```

<210> 1133
<211> 9
<212> PRT
<213> Artificial Sequence

<220>
<223> HIV peptide

<400> 1133

```
                    Lys Met Met Ala Gly Ala Asp Cys Val
                     1                   5
```

<210> 1134
<211> 9
<212> PRT
<213> Artificial Sequence

<220>
<223> HIV peptide

<400> 1134

```
                    Leu Ile Val Arg Thr Tyr Trp Gly Val
                     1                   5
```

<210> 1135
<211> 9
<212> PRT
<213> Artificial Sequence

<220>
<223> HIV peptide

<400> 1135

```
                    Leu Leu Val Ala Arg Ile Val Glu Ala
                     1                   5
```

<210> 1136
<211> 9
<212> PRT
<213> Artificial Sequence

<220>
<223> HIV peptide

<400> 1136

```
                    Leu Leu Val Ala Arg Ile Val Glu Ile
                     1                   5
```

<210> 1137
<211> 9
<212> PRT
<213> Artificial Sequence

<220>
<223> HIV peptide

<400> 1137

```
Leu Leu Val Ala Arg Ile Val Glu Leu
 1               5
```

<210> 1138
<211> 9
<212> PRT
<213> Artificial Sequence

<220>
<223> HIV peptide

<400> 1138

```
Leu Leu Val Arg Thr Tyr Trp Gly Ala
 1               5
```

<210> 1139
<211> 9
<212> PRT
<213> Artificial Sequence

<220>
<223> HIV peptide

<400> 1139

```
Leu Leu Val Arg Thr Tyr Trp Gly Ile
 1               5
```

<210> 1140
<211> 9
<212> PRT
<213> Artificial Sequence

<220>
<223> HIV peptide

<400> 1140

```
Leu Leu Val Arg Thr Tyr Trp Gly Leu
 1               5
```

<210> 1141
<211> 9
<212> PRT
<213> Artificial Sequence

<220>
<223> HIV peptide

<400> 1141

```
Leu Leu Val Thr Thr Tyr Trp Gly Ala
 1               5
```

<210> 1142
<211> 9
<212> PRT
<213> Artificial Sequence

<220>
<223> HIV peptide

<400> 1142

```
Leu Leu Val Thr Thr Tyr Trp Gly Ile
 1               5
```

<210> 1143
<211> 9
<212> PRT
<213> Artificial Sequence

<220>
<223> HIV peptide

<400> 1143

```
Leu Leu Val Thr Thr Tyr Trp Gly Leu
 1               5
```

<210> 1144
<211> 9
<212> PRT
<213> Artificial Sequence

<220>
<223> HIV peptide

<400> 1144

```
Leu Met Val Ala Arg Ile Val Glu Ala
 1               5
```

<210> 1145
<211> 9
<212> PRT
<213> Artificial Sequence

<220>
<223> HIV peptide

<400> 1145

```
                        Leu Met Val Ala Arg Ile Val Glu Leu
                         1               5
```

<210> 1146
<211> 9
<212> PRT
<213> Artificial Sequence

<220>
<223> HIV peptide

<400> 1146

```
                        Leu Met Val Ala Arg Ile Val Glu Val
                         1               5
```

<210> 1147
<211> 9
<212> PRT
<213> Artificial Sequence

<220>
<223> HIV peptide

<400> 1147

```
                        Leu Met Val Arg Thr Tyr Trp Gly Ala
                         1               5
```

<210> 1148
<211> 9
<212> PRT
<213> Artificial Sequence

<220>
<223> HIV peptide

<400> 1148

```
                        Leu Met Val Arg Thr Tyr Trp Gly Ile
                         1               5
```

<210> 1149
<211> 9
<212> PRT
<213> Artificial Sequence

<220>
<223> HIV peptide

<400> 1149

Leu Met Val Arg Thr Tyr Trp Gly Leu
1     5

<210> 1150
<211> 9
<212> PRT
<213> Artificial Sequence

<220>
<223> HIV peptide

<400> 1150

Leu Met Val Arg Thr Tyr Trp Gly Val
1     5

<210> 1151
<211> 9
<212> PRT
<213> Artificial Sequence

<220>
<223> HIV peptide

<400> 1151

Leu Met Val Thr Thr Tyr Trp Gly Ala
1     5

<210> 1152
<211> 9
<212> PRT
<213> Artificial Sequence

<220>
<223> HIV peptide

<400> 1152

Leu Met Val Thr Thr Tyr Trp Gly Leu
1     5

<210> 1153
<211> 9
<212> PRT
<213> Artificial Sequence

<220>
<223> HIV peptide

<400> 1153

```
Leu Met Val Thr Thr Tyr Trp Gly Val
 1               5
```

<210> 1154
<211> 9
<212> PRT
<213> Artificial Sequence

<220>
<223> HIV peptide

<400> 1154

```
Leu Gln Val Arg Thr Tyr Trp Gly Val
 1               5
```

<210> 1155
<211> 9
<212> PRT
<213> Artificial Sequence

<220>
<223> HIV peptide

<400> 1155

```
Leu Val Val Arg Thr Tyr Trp Gly Val
 1               5
```

<210> 1156
<211> 9
<212> PRT
<213> Artificial Sequence

<220>
<223> HIV peptide

<400> 1156

```
Met Leu Ser Asn Pro Pro Val Pro Ala
 1               5
```

<210> 1157
<211> 9
<212> PRT
<213> Artificial Sequence

<220>
<223> HIV peptide

<400> 1157

```
Met Leu Ser Asn Pro Pro Val Pro Leu
 1               5
```

<210> 1158
<211> 9
<212> PRT
<213> Artificial Sequence

<220>
<223> HIV peptide

<400> 1158

```
Met Met Ser Asn Pro Pro Val Pro Leu
 1               5
```

<210> 1159
<211> 9
<212> PRT
<213> Artificial Sequence

<220>
<223> HIV peptide

<400> 1159

```
Met Met Ser Asn Pro Pro Val Pro Val
 1               5
```

<210> 1160
<211> 9
<212> PRT
<213> Artificial Sequence

<220>
<223> HIV peptide

<400> 1160

```
Asn Leu Val Ala Val Leu Tyr Cys Leu
 1               5
```

<210> 1161
<211> 9
<212> PRT
<213> Artificial Sequence

<220>
<223> HIV peptide

<400> 1161

```
                        Asn Met Val Ala Val Leu Tyr Cys Val
                         1               5
```

<210> 1162
<211> 9
<212> PRT
<213> Artificial Sequence

<220>
<223> HIV peptide

<400> 1162

```
                        Gln Met Thr Glu Val Val Gln Lys Val
                         1               5
```

<210> 1163
<211> 9
<212> PRT
<213> Artificial Sequence

<220>
<223> HIV peptide

<400> 1163

```
                        Ser Met Ala Glu Glu Glu Ile Ile Val
                         1               5
```

<210> 1164
<211> 9
<212> PRT
<213> Artificial Sequence

<220>
<223> HIV peptide

<400> 1164

```
                        Val Leu Ala Ala Ile Ile Ala Ile Ala
                         1               5
```

<210> 1165
<211> 9
<212> PRT
<213> Artificial Sequence

<220>
<223> HIV peptide

<400> 1165

```
                              Val Leu Ala Ala Ile Ile Ala Ile Ile
                               1                   5
```

<210> 1166
<211> 9
<212> PRT
<213> Artificial Sequence

<220>
<223> HIV peptide

<400> 1166

```
                              Val Leu Ala Ala Ile Ile Ala Ile Leu
                               1                   5
```

<210> 1167
<211> 9
<212> PRT
<213> Artificial Sequence

<220>
<223> HIV peptide

<400> 1167

```
                              Val Met Ala Ala Ile Ile Ala Ile Ala
                               1                   5
```

<210> 1168
<211> 9
<212> PRT
<213> Artificial Sequence

<220>
<223> HIV peptide

<400> 1168

```
                              Val Met Ala Ala Ile Ile Ala Ile Leu
                               1                   5
```

<210> 1169
<211> 9
<212> PRT
<213> Artificial Sequence

<220>
<223> HIV peptide

<400> 1169

```
                              Val Leu Ala Ala Ile Ile Ala Ile Ile
                               1                   5
```

```
Val Met Ala Ala Ile Ile Ala Ile Val
 1               5
```

<210> 1170
<211> 9
<212> PRT
<213> Artificial Sequence

<220>
<223> HIV peptide

<400> 1170

```
Tyr Leu Arg Ile Phe Ile Met Ile Leu
 1               5
```

<210> 1171
<211> 9
<212> PRT
<213> Artificial Sequence

<220>
<223> HIV peptide

<400> 1171

```
Tyr Met Arg Ile Phe Ile Met Ile Val
 1               5
```

<210> 1172
<211> 9
<212> PRT
<213> Artificial Sequence

<220>
<223> HIV peptide

<400> 1172

```
Ser Leu Gly Gln His Ile Tyr Glu Thr
 1               5
```

<210> 1173
<211> 9
<212> PRT
<213> Artificial Sequence

<400> 1173

```
Leu Gln Leu Pro Pro Ile Glu Arg Leu
 1               5
```

<210> 1174

<211> 9
<212> PRT
<213> Artificial Sequence

<400> 1174

```
Ile Leu Val Glu Ser Pro Ala Val Leu
 1               5
```

<210> 1175
<211> 9
<212> PRT
<213> Artificial Sequence

<400> 1175

```
Tyr Ile Lys Ile Phe Ile Met Ile Val
 1               5
```

<210> 1176
<211> 9
<212> PRT
<213> Artificial Sequence

<400> 1176

```
Val Leu Ala Glu Ala Met Ser Gln Ala
 1               5
```

<210> 1177
<211> 8
<212> PRT
<213> Artificial Sequence

<400> 1177

```
Leu Asn Phe Pro Ile Ser Pro Ile
 1               5
```

<210> 1178
<211> 9
<212> PRT
<213> Artificial Sequence

<400> 1178

```
Lys Ile Lys Pro Pro Leu Pro Ser Val
 1               5
```

<210> 1179
<211> 9
<212> PRT
<213> Artificial Sequence

<400> 1179

```
Lys Thr Lys Pro Pro Leu Pro Ser Val
1               5
```

<210> 1180
<211> 9
<212> PRT
<213> Artificial Sequence

<400> 1180

```
Asn Val Trp Ala Thr His Ala Cys Val
1               5
```

<210> 1181
<211> 9
<212> PRT
<213> Artificial Sequence

<400> 1181

```
Glu Met Met Thr Ala Cys Gln Gly Val
1               5
```

<210> 1182
<211> 9
<212> PRT
<213> Artificial Sequence

<400> 1182

```
Asn Ile Trp Ala Thr His Ala Cys Val
1               5
```

<210> 1183
<211> 9
<212> PRT
<213> Artificial Sequence

<400> 1183

```
Phe Pro Arg Pro Trp Leu His Gly Leu
1               5
```

<210> 1184
<211> 9
<212> PRT
<213> Artificial Sequence

<400> 1184

```
Ala Leu Ala Ala Leu Ile Thr Pro Lys
 1               5
```

<210> 1185
<211> 9
<212> PRT
<213> Artificial Sequence

<400> 1185

```
Ala Thr Leu Glu Glu Met Met Thr Ala
 1               5
```

<210> 1186
<211> 8
<212> PRT
<213> Artificial Sequence

<400> 1186

```
Ala Leu Gly Ile Ile Gln Ala Gln
 1               5
```

<210> 1187
<211> 9
<212> PRT
<213> Artificial Sequence

<400> 1187

```
Ile Thr Leu Trp Gln Arg Pro Leu Val
 1               5
```

<210> 1188
<211> 9
<212> PRT
<213> Artificial Sequence

<400> 1188

```
Ser Leu Val Lys His His Met Tyr Ile
 1               5
```

<210> 1189
<211> 9
<212> PRT
<213> Artificial Sequence

<400> 1189

```
Gly Leu Ala Asp Gln Leu Ile His Met
 1               5
```

<210> 1190
<211> 9
<212> PRT
<213> Artificial Sequence

<400> 1190

```
Ala Leu Val Glu Met Gly His His Ala
 1               5
```

<210> 1191
<211> 9
<212> PRT
<213> Artificial Sequence

<400> 1191

```
Arg Thr Leu Asn Ala Trp Val Lys Val
 1               5
```

<210> 1192
<211> 9
<212> PRT
<213> Artificial Sequence

<400> 1192

```
Lys Leu Gly Lys Ala Gly Tyr Val Thr
 1               5
```

<210> 1193
<211> 8
<212> PRT
<213> Artificial Sequence

<400> 1193

```
Trp Cys Phe Lys Leu Val Pro Val
 1               5
```

<210> 1194
<211> 9
<212> PRT
<213> Artificial Sequence

<400> 1194

```
Tyr Leu Ala Trp Val Pro Ala His Lys
 1               5
```

<210> 1195
<211> 9
<212> PRT

<213> Artificial Sequence

<400> 1195

```
                        Thr Leu Gln Glu Gln Ile Gly Trp Met
                         1               5
```

<210> 1196
<211> 9
<212> PRT
<213> Artificial Sequence

<400> 1196

```
                        Thr Met Gly Ala Ala Ser Ile Thr Leu
                         1               5
```

<210> 1197
<211> 9
<212> PRT
<213> Artificial Sequence

<400> 1197

```
                        Val Val Ala Ala Ile Ile Ala Ile Val
                         1               5
```

<210> 1198
<211> 9
<212> PRT
<213> Artificial Sequence

<400> 1198

```
                        Phe Leu Trp Met Gly Tyr Glu Leu His
                         1               5
```

<210> 1199
<211> 9
<212> PRT
<213> Artificial Sequence

<400> 1199

```
                        Leu Cys Phe Gly Trp Cys Phe Lys Leu
                         1               5
```

<210> 1200
<211> 9
<212> PRT
<213> Artificial Sequence

<400> 1200

```
Arg Leu Ala Phe His His Met Ala Arg
 1               5
```

<210> 1201
<211> 8
<212> PRT
<213> Artificial Sequence

<400> 1201

```
Thr Gly Asn Pro Pro Ile Pro Val
 1               5
```

<210> 1202
<211> 9
<212> PRT
<213> Artificial Sequence

<400> 1202

```
Ala Leu Gly Pro Ala Ala Thr Leu Glu
 1               5
```

<210> 1203
<211> 9
<212> PRT
<213> Artificial Sequence

<400> 1203

```
Ala Ile Glu Ala Gln Gln His Leu Leu
 1               5
```

<210> 1204
<211> 8
<212> PRT
<213> Artificial Sequence

<400> 1204

```
Phe Leu Gly Ala Ala Gly Ser Thr
 1               5
```

<210> 1205
<211> 8
<212> PRT
<213> Artificial Sequence

<400> 1205

```
Ser Gly Gly Asp Pro Glu Ile Val
 1               5
```

<210> 1206
<211> 9
<212> PRT
<213> Artificial Sequence

<400> 1206

```
Leu Lys Thr Ala Val Gln Met Ala Val
 1                   5
```

<210> 1207
<211> 8
<212> PRT
<213> Artificial Sequence

<400> 1207

```
Arg Ser Ala Glu Pro Val Pro Leu
 1                   5
```

<210> 1208
<211> 9
<212> PRT
<213> Artificial Sequence

<400> 1208

```
Gly Val Gly Ser Pro Gln Ile Leu Val
 1                   5
```

<210> 1209
<211> 9
<212> PRT
<213> Artificial Sequence

<400> 1209

```
Ala Thr Asn Ala Asp Cys Ala Trp Leu
 1                   5
```

<210> 1210
<211> 8
<212> PRT
<213> Artificial Sequence

<400> 1210

```
Ile Ile Gly Asp Ile Arg Gln Ala
 1                   5
```

<210> 1211
<211> 8
<212> PRT

<213> Artificial Sequence

<400> 1211

```
Lys Val Gly Ser Leu Gln Tyr Leu
 1                   5
```

<210> 1212
<211> 8
<212> PRT
<213> Artificial Sequence

<400> 1212

```
Leu Leu Phe Ile His Phe Arg Ile
 1                   5
```

<210> 1213
<211> 8
<212> PRT
<213> Artificial Sequence

<400> 1213

```
Pro Val Gly Glu Ile Tyr Lys Arg
 1                   5
```

<210> 1214
<211> 9
<212> PRT
<213> Artificial Sequence

<400> 1214

```
Lys Leu Trp Tyr Gln Leu Glu Lys Glu
 1                   5
```

<210> 1215
<211> 9
<212> PRT
<213> Artificial Sequence

<400> 1215

```
Leu Val Ile Thr Thr Tyr Trp Gly Leu
 1                   5
```

<210> 1216
<211> 9
<212> PRT
<213> Artificial Sequence

<400> 1216

```
                    Ile Ile Ala Ile Val Val Trp Thr Ile
                     1               5
```

<210> 1217
<211> 9
<212> PRT
<213> Artificial Sequence

<400> 1217

```
                    Phe Pro Val Arg Pro Gln Val Pro Leu
                     1               5
```

<210> 1218
<211> 9
<212> PRT
<213> Artificial Sequence

<400> 1218

```
                    Ala Leu Val Glu Ile Cys Thr Glu Met
                     1               5
```

<210> 1219
<211> 9
<212> PRT
<213> Artificial Sequence

<400> 1219

```
                    Ser Leu Gly Gln Tyr Ile Tyr Glu Thr
                     1               5
```

<210> 1220
<211> 9
<212> PRT
<213> Artificial Sequence

<400> 1220

```
                    Leu Val Val Arg Thr Tyr Trp Gly Leu
                     1               5
```

<210> 1221
<211> 9
<212> PRT
<213> Artificial Sequence

<400> 1221

```
                    Leu Val Val Thr Thr Tyr Trp Gly Leu
                     1               5
```

<210> 1222
<211> 9
<212> PRT
<213> Artificial Sequence

<400> 1222

```
Ala Leu Thr Ala Leu Ile Thr Pro Lys
 1               5
```

<210> 1223
<211> 9
<212> PRT
<213> Artificial Sequence

<400> 1223

```
Ala Leu Thr Glu Ile Cys Thr Glu Met
 1               5
```

<210> 1224
<211> 9
<212> PRT
<213> Artificial Sequence

<400> 1224

```
Leu Gly Ile Pro His Pro Ala Gly Leu
 1               5
```

<210> 1225
<211> 9
<212> PRT
<213> Artificial Sequence

<400> 1225

```
Tyr Thr Ala Phe Thr Ile Pro Ser Val
 1               5
```

<210> 1226
<211> 9
<212> PRT
<213> Artificial Sequence

<400> 1226

```
His Leu Leu Arg Trp Gly Phe Thr Thr
 1               5
```

<210> 1227
<211> 9
<212> PRT

<213> Artificial Sequence

<400> 1227

```
Lys Leu Asn Trp Ala Ser Gln Ile Tyr
 1               5
```

<210> 1228
<211> 9
<212> PRT
<213> Artificial Sequence

<400> 1228

```
Ser Gln Ile Tyr Ala Gly Ile Lys Val
 1               5
```

<210> 1229
<211> 9
<212> PRT
<213> Artificial Sequence

<400> 1229

```
Ser Gln Ile Tyr Pro Gly Ile Lys Val
 1               5
```

<210> 1230
<211> 9
<212> PRT
<213> Artificial Sequence

<400> 1230

```
Ala Leu Thr Glu Val Ile Pro Leu Thr
 1               5
```

<210> 1231
<211> 9
<212> PRT
<213> Artificial Sequence

<400> 1231

```
Ala Leu Thr Asp Ile Val Pro Leu Thr
 1               5
```

<210> 1232
<211> 9
<212> PRT
<213> Artificial Sequence

<400> 1232

```
Ala Leu Thr Asp Ile Val Thr Leu Thr
 1               5
```

<210> 1233
<211> 9
<212> PRT
<213> Artificial Sequence

<400> 1233

```
Ala Leu Thr Glu Val Val Pro Leu Thr
 1               5
```

<210> 1234
<211> 9
<212> PRT
<213> Artificial Sequence

<400> 1234

```
Lys Leu Trp Tyr Gln Leu Glu Lys Asp
 1               5
```

<210> 1235
<211> 9
<212> PRT
<213> Artificial Sequence

<400> 1235

```
Ala Val Lys Ala Ala Cys Trp Trp Ala
 1               5
```

<210> 1236
<211> 9
<212> PRT
<213> Artificial Sequence

<400> 1236

```
Lys Gln Met Ala Gly Ala Asp Cys Val
 1               5
```

<210> 1237
<211> 9
<212> PRT
<213> Artificial Sequence

<400> 1237

```
Asn Leu Ala Phe Pro Gln Gly Glu Ala
 1               5
```

<210> 1238
<211> 9
<212> PRT
<213> Artificial Sequence

<400> 1238

```
                              Leu Trp Gln Arg Pro Leu Val Thr Val
                               1                   5
```

<210> 1239
<211> 9
<212> PRT
<213> Artificial Sequence

<400> 1239

```
                              Ile Thr Leu Trp Gln Arg Pro Ile Val
                               1                   5
```

<210> 1240
<211> 9
<212> PRT
<213> Artificial Sequence

<400> 1240

```
                              Leu Met Trp Lys Phe Asp Ser Arg Leu
                               1                   5
```

<210> 1241
<211> 9
<212> PRT
<213> Artificial Sequence

<400> 1241

```
                              Asn Thr Val Ala Thr Leu Tyr Cys Val
                               1                   5
```

<210> 1242
<211> 9
<212> PRT
<213> Artificial Sequence

<400> 1242

```
                              Asn Thr Val Ala Val Leu Tyr Cys Val
                               1                   5
```

<210> 1243
<211> 9
<212> PRT

<213> Artificial Sequence

<400> 1243

Thr Leu Tyr Cys Val His Gln Lys Ile
1               5

<210> 1244
<211> 9
<212> PRT
<213> Artificial Sequence

<400> 1244

Thr Leu Trp Cys Val His Gln Arg Ile
1               5

<210> 1245
<211> 9
<212> PRT
<213> Artificial Sequence

<400> 1245

Leu Gln Gly Gln Met Val His Gln Ala
1               5

<210> 1246
<211> 9
<212> PRT
<213> Artificial Sequence

<400> 1246

Arg Leu His Pro Val Gln Ala Gly Pro
1               5

<210> 1247
<211> 9
<212> PRT
<213> Artificial Sequence

<400> 1247

Thr Leu Gln Glu Gln Ile Ala Trp Met
1               5

<210> 1248
<211> 9
<212> PRT
<213> Artificial Sequence

<400> 1248

```
Lys Ile Val Arg Met Tyr Ser Pro Val
 1               5
```

<210> 1249
<211> 9
<212> PRT
<213> Artificial Sequence

<400> 1249

```
Arg Met Tyr Ser Pro Val Ser Ile Leu
 1               5
```

<210> 1250
<211> 9
<212> PRT
<213> Artificial Sequence

<400> 1250

```
Arg Met Tyr Ser Pro Thr Ser Ile Leu
 1               5
```

<210> 1251
<211> 9
<212> PRT
<213> Artificial Sequence

<400> 1251

```
Ser Leu Glu Glu Met Met Thr Ala Cys ·
 1               5
```

<210> 1252
<211> 9
<212> PRT
<213> Artificial Sequence

<400> 1252

```
Met Met Gln Arg Gly Asn Phe Arg Asn
 1               5
```

<210> 1253
<211> 9
<212> PRT
<213> Artificial Sequence

<400> 1253

```
Met Met Gln Arg Gly Asn Phe Arg Gly
 1               5
```

<210> 1254
<211> 9
<212> PRT
<213> Artificial Sequence

<400> 1254

```
Met Met Gln Arg Gly Asn Phe Lys Gly
 1                   5
```

<210> 1255
<211> 9
<212> PRT
<213> Artificial Sequence

<400> 1255

```
Phe Leu Gln Asn Arg Pro Glu Pro Thr
 1                   5
```

<210> 1256
<211> 9
<212> PRT
<213> Artificial Sequence

<400> 1256

```
His Leu Trp Arg Trp Gly Thr Met Leu
 1                   5
```

<210> 1257
<211> 9
<212> PRT
<213> Artificial Sequence

<400> 1257

```
Met Leu Leu Gly Met Leu Met Ile Cys
 1                   5
```

<210> 1258
<211> 9
<212> PRT
<213> Artificial Sequence

<400> 1258

```
Lys Leu Trp Val Thr Val Tyr Tyr Gly
 1                   5
```

<210> 1259
<211> 9
<212> PRT

<213> Artificial Sequence

<400> 1259

```
Val Thr Val Tyr Tyr Gly Val Pro Val
 1               5
```

<210> 1260
<211> 9
<212> PRT
<213> Artificial Sequence

<400> 1260

```
Tyr Cys Ala Pro Ala Gly Tyr Ala Ile
 1               5
```

<210> 1261
<211> 9
<212> PRT
<213> Artificial Sequence

<400> 1261

```
Ser Leu Ala Glu Glu Glu Val Val Ile
 1               5
```

<210> 1262
<211> 9
<212> PRT
<213> Artificial Sequence

<400> 1262

```
Ser Leu Ala Glu Glu Glu Ile Ile Ile
 1               5
```

<210> 1263
<211> 9
<212> PRT
<213> Artificial Sequence

<400> 1263

```
Ala Met Tyr Ala Pro Pro Ile Arg Gly
 1               5
```

<210> 1264
<211> 9
<212> PRT
<213> Artificial Sequence

<400> 1264

```
Ala Met Tyr Ala Pro Pro Ile Glu Gly
 1               5
```

<210> 1265
<211> 9
<212> PRT
<213> Artificial Sequence

<400> 1265

```
Ala Met Tyr Ala Pro Pro Ile Lys Gly
 1               5
```

<210> 1266
<211> 9
<212> PRT
<213> Artificial Sequence

<400> 1266

```
Ala Met Tyr Ala Pro Pro Ile Ala Gly
 1               5
```

<210> 1267
<211> 9
<212> PRT
<213> Artificial Sequence

<400> 1267

```
Pro Leu Gly Ile Ala Pro Thr Lys Ala
 1               5
```

<210> 1268
<211> 9
<212> PRT
<213> Artificial Sequence

<400> 1268

```
Thr Met Gly Ala Ala Ser Leu Thr Leu
 1               5
```

<210> 1269
<211> 9
<212> PRT
<213> Artificial Sequence

<400> 1269

```
Arg Ala Ile Glu Ala Gln Gln His Leu
 1               5
```

<210> 1270
<211> 9
<212> PRT
<213> Artificial Sequence

<400> 1270

```
                    Ala Ile Glu Ala Gln Gln His Met Leu
                     1                   5
```

<210> 1271
<211> 9
<212> PRT
<213> Artificial Sequence

<400> 1271

```
                    Leu Leu Lys Leu Thr Val Trp Gly Ile
                     1                   5
```

<210> 1272
<211> 9
<212> PRT
<213> Artificial Sequence

<400> 1272

```
                    Cys Thr Thr Ala Val Pro Trp Asn Ala
                     1                   5
```

<210> 1273
<211> 9
<212> PRT
<213> Artificial Sequence

<400> 1273

```
                    Ser Leu Trp Asn Trp Phe Ser Ile Thr
                     1                   5
```

<210> 1274
<211> 9
<212> PRT
<213> Artificial Sequence

<400> 1274

```
                    Tyr Ile Arg Ile Phe Ile Met Ile Val
                     1                   5
```

<210> 1275
<211> 9
<212> PRT

<213> Artificial Sequence

<400> 1275

```
Phe Ile Met Ile Val Gly Gly Leu Val
1               5
```

<210> 1276
<211> 9
<212> PRT
<213> Artificial Sequence

<400> 1276

```
Ile Val Phe Ala Val Leu Ser Ile Val
1               5
```

<210> 1277
<211> 9
<212> PRT
<213> Artificial Sequence

<400> 1277

```
Ile Ile Phe Ala Val Leu Ser Ile Val
1               5
```

<210> 1278
<211> 9
<212> PRT
<213> Artificial Sequence

<400> 1278

```
Leu Ile Ala Ala Arg Thr Val Glu Leu
1               5
```

<210> 1279
<211> 9
<212> PRT
<213> Artificial Sequence

<400> 1279

```
Leu Ile Val Ala Arg Ile Val Glu Leu
1               5
```

<210> 1280
<211> 8
<212> PRT
<213> Artificial Sequence

<400> 1280

```
                    Leu Leu Phe Val His Phe Arg Ile
                     1               5
```

<210> 1281
<211> 8
<212> PRT
<213> Artificial Sequence

<400> 1281

```
                    Ile Leu Gly His Ile Val Ser Pro
                     1               5
```

<210> 1282
<211> 8
<212> PRT
<213> Artificial Sequence

<400> 1282

```
                    Tyr Gln Ser Asn Pro Tyr Pro Lys
                     1               5
```

<210> 1283
<211> 8
<212> PRT
<213> Artificial Sequence

<400> 1283

```
                    Gly Val Gly Ser Pro Gln Ile Leu
                     1               5
```

<210> 1284
<211> 8
<212> PRT
<213> Artificial Sequence

<400> 1284

```
                    Lys Ile Gly Pro Glu Asn Pro Tyr
                     1               5
```

<210> 1285
<211> 8
<212> PRT
<213> Artificial Sequence

<400> 1285

```
                    Gln Leu Gly Ile Pro His Pro Ala
                     1               5
```

<210> 1286
<211> 8
<212> PRT
<213> Artificial Sequence

<400> 1286

```
Leu Ile Ala Glu Ile Gln Lys Gln
 1               5
```

<210> 1287
<211> 8
<212> PRT
<213> Artificial Sequence

<400> 1287

```
Leu Ala Glu Val Val Gln Lys Val
 1               5
```

<210> 1288
<211> 8
<212> PRT
<213> Artificial Sequence

<400> 1288

```
Phe Ser Phe Pro Gln Ile Thr Leu
 1               5
```

<210> 1289
<211> 8
<212> PRT
<213> Artificial Sequence

<400> 1289

```
Phe Asn Phe Pro Gln Ile Thr Leu
 1               5
```

<210> 1290
<211> 8
<212> PRT
<213> Artificial Sequence

<400> 1290

```
Phe Ile Lys Val Lys Gln Tyr Asp
 1               5
```

<210> 1291
<211> 8
<212> PRT
<213> Artificial Sequence

<400> 1291

```
                              Gly Pro Gly Val Arg Tyr Pro Leu
                               1                   5
```

<210> 1292
<211> 8
<212> PRT
<213> Artificial Sequence

<400> 1292

```
                              Gly Pro Gly Thr Arg Phe Pro Leu
                               1                   5
```

<210> 1293
<211> 8
<212> PRT
<213> Artificial Sequence

<400> 1293

```
                              Thr Phe Gly Trp Cys Phe Lys Leu
                               1                   5
```

<210> 1294
<211> 8
<212> PRT
<213> Artificial Sequence

<400> 1294

```
                              Cys Phe Gly Trp Cys Phe Lys Leu
                               1                   5
```

<210> 1295
<211> 8
<212> PRT
<213> Artificial Sequence

<400> 1295

```
                              Thr Asn Asn Pro Pro Ile Pro Val
                               1                   5
```

<210> 1296
<211> 8
<212> PRT
<213> Artificial Sequence

<400> 1296

Thr Ser Asn Pro Pro Ile Pro Val
1                   5

<210> 1297
<211> 8
<212> PRT
<213> Artificial Sequence

<400> 1297

Pro Val Gly Asp Ile Tyr Lys Arg
1                   5

<210> 1298
<211> 8
<212> PRT
<213> Artificial Sequence

<400> 1298

Leu Gln Asn Arg Pro Glu Pro Thr
1                   5

<210> 1299
<211> 8
<212> PRT
<213> Artificial Sequence

<400> 1299

Cys Arg Gly Glu Phe Phe Tyr Cys
1                   5

<210> 1300
<211> 8
<212> PRT
<213> Artificial Sequence

<400> 1300

Ile Ile Asn Met Trp Gln Gly Val
1                   5

<210> 1301
<211> 8
<212> PRT
<213> Artificial Sequence

<400> 1301

Phe Leu Gly Phe Leu Gly Ala Ala
1                   5

<210> 1302
<211> 8
<212> PRT
<213> Artificial Sequence

<400> 1302

```
Leu Gln Ala Arg Ile Leu Ala Val
 1               5
```

<210> 1303
<211> 8
<212> PRT
<213> Artificial Sequence

<400> 1303

```
Ile Val Gly Gly Leu Val Gly Leu
 1               5
```

<210> 1304
<211> 8
<212> PRT
<213> Artificial Sequence

<400> 1304

```
Ile Ile Phe Ala Val Leu Ser Ile
 1               5
```

<210> 1305
<211> 8
<212> PRT
<213> Artificial Sequence

<400> 1305

```
Leu Val Asn Gly Phe Leu Ala Leu
 1               5
```

<210> 1306
<211> 8
<212> PRT
<213> Artificial Sequence

<400> 1306

```
Ile Ile Arg Ile Leu Gln Gln Leu
 1               5
```

<210> 1307
<211> 9
<212> PRT

<213> Artificial Sequence

<400> 1307

```
                        Asp Leu Ala Asp Gln Leu Ile His Leu
                         1                   5
```

<210> 1308
<211> 8
<212> PRT
<213> Artificial Sequence

<400> 1308

```
                        Ser Leu Phe Gly Asn Asp Pro Leu
                         1                   5
```

<210> 1309
<211> 8
<212> PRT
<213> Artificial Sequence

<400> 1309

```
                        Ser Leu Phe Gly Ser Asp Pro Leu
                         1                   5
```

<210> 1310
<211> 8
<212> PRT
<213> Artificial Sequence

<400> 1310

```
                        Ala Leu Gly Thr Gly Ala Thr Leu
                         1                   5
```

<210> 1311
<211> 9
<212> PRT
<213> Artificial Sequence

<400> 1311

```
                        Ala Leu Gln Asp Ser Gly Ser Glu Val
                         1                   5
```

<210> 1312
<211> 9
<212> PRT
<213> Artificial Sequence

<400> 1312

```
Glu Leu Gln Ala Ile Gln Leu Ala Leu
 1               5
```

<210> 1313
<211> 9
<212> PRT
<213> Artificial Sequence

<400> 1313

```
Glu Leu Gln Ala Ile Gln Leu Ala Leu
 1               5
```

<210> 1314
<211> 9
<212> PRT
<213> Artificial Sequence

<400> 1314

```
Asn Leu Ala Phe Gln Gln Gly Glu Ala
 1               5
```

<210> 1315
<211> 8
<212> PRT
<213> Artificial Sequence

<400> 1315

```
Lys Leu Val Asp Phe Arg Glu Leu
 1               5
```

<210> 1316
<211> 8
<212> PRT
<213> Artificial Sequence

<400> 1316

```
Ala Leu Thr Asp Ile Val Thr Leu
 1               5
```

<210> 1317
<211> 8
<212> PRT
<213> Artificial Sequence

<400> 1317

```
Ala Leu Thr Glu Val Val Pro Leu
 1               5
```

<210> 1318
<211> 9
<212> PRT
<213> Artificial Sequence

<400> 1318

```
Glu Leu His Pro Asp Lys Trp Thr Val
 1               5
```

<210> 1319
<211> 8
<212> PRT
<213> Artificial Sequence

<400> 1319

```
Val Ile Leu Val Ala Val His Val
 1               5
```

<210> 1320
<211> 8
<212> PRT
<213> Artificial Sequence

<400> 1320

```
Ile Ile Leu Val Ala Val His Val
 1               5
```

<210> 1321
<211> 9
<212> PRT
<213> Artificial Sequence

<400> 1321

```
Pro Leu Trp Lys Gly Pro Ala Lys Leu
 1               5
```

<210> 1322
<211> 8
<212> PRT
<213> Artificial Sequence

<400> 1322

```
Arg Gln Gly Phe Glu Arg Ala Leu
 1               5
```

<210> 1323
<211> 8
<212> PRT

<213> Artificial Sequence

<400> 1323

```
Arg Gln Gly Phe Glu Arg Ala Leu
 1               5
```

<210> 1324
<211> 9
<212> PRT
<213> Artificial Sequence

<400> 1324

```
Ser Leu Leu Asn Ala Thr Ala Ile Ala
 1               5
```

<210> 1325
<211> 9
<212> PRT
<213> Artificial Sequence

<400> 1325

```
Ser Leu Leu Asn Ala Thr Ala Ile Ala
 1.              5
```

<210> 1326
<211> 9
<212> PRT
<213> Artificial Sequence

<400> 1326

```
Ala Leu Lys Tyr Trp Trp Asn Leu Leu
 1               5
```

<210> 1327
<211> 9
<212> PRT
<213> Artificial Sequence

<400> 1327

```
Leu Ile Val Ala Arg Ile Val Glu Leu
 1               5
```

<210> 1328
<211> 9
<212> PRT
<213> Artificial Sequence

<400> 1328

```
                Arg Leu Arg Asp Phe Ile Leu Ile Ala
                1               5
```

<210> 1329
<211> 9
<212> PRT
<213> Artificial Sequence

<400> 1329

```
                Arg Leu Arg Asp Phe Ile Leu Ile Ala
                1               5
```

<210> 1330
<211> 9
<212> PRT
<213> Artificial Sequence

<400> 1330

```
                Phe Leu Ala Leu Ala Trp Asp Asp Leu
                1               5
```

<210> 1331
<211> 9
<212> PRT
<213> Artificial Sequence

<400> 1331

```
                Phe Leu Ala Ile Ile Trp Val Asp Leu
                1               5
```

<210> 1332
<211> 9
<212> PRT
<213> Artificial Sequence

<400> 1332

```
                Arg Leu Val Ser Gly Phe Leu Ala Leu
                1               5
```

<210> 1333
<211> 8
<212> PRT
<213> Artificial Sequence

<400> 1333

```
Leu Gln Ala Arg Val Leu Ala Val
1               5
```

<210> 1334
<211> 9
<212> PRT
<213> Artificial Sequence

<400> 1334

```
Gln Leu Gln Ala Arg Val Leu Ala Val
1               5
```

<210> 1335
<211> 9
<212> PRT
<213> Artificial Sequence

<400> 1335

```
Arg Leu Ile Ser Cys Asn Thr Ser Val
1               5
```

<210> 1336
<211> 9
<212> PRT
<213> Artificial Sequence

<400> 1336

```
Arg Leu Ile Asn Cys Asn Thr Ser Val
1               5
```

<210> 1337
<211> 9
<212> PRT
<213> Artificial Sequence

<400> 1337

```
Ala Leu Phe Tyr Lys Leu Asp Val Val
1               5
```

<210> 1338
<211> 8
<212> PRT
<213> Artificial Sequence

<400> 1338

```
Ile Ile Arg Ile Leu Gln Gln Leu
1               5
```

<210> 1339
<211> 9
<212> PRT
<213> Artificial Sequence

<400> 1339

```
Asp Leu Ala Asp Gln Leu Ile His Leu
1               5
```

<210> 1340
<211> 8
<212> PRT
<213> Artificial Sequence

<400> 1340

```
Ser Leu Phe Gly Asn Asp Pro Leu
1               5
```

<210> 1341
<211> 8
<212> PRT
<213> Artificial Sequence

<400> 1341

```
Ser Leu Phe Gly Ser Asp Pro Leu
1               5
```

<210> 1342
<211> 8
<212> PRT
<213> Artificial Sequence

<400> 1342

```
Ala Leu Gly Thr Gly Ala Thr Leu
1               5
```

<210> 1343
<211> 9
<212> PRT
<213> Artificial Sequence

<400> 1343

```
Ala Leu Gln Asp Ser Gly Ser Glu Val
1               5
```

<210> 1344
<211> 9
<212> PRT

<213> Artificial Sequence

<400> 1344

```
Glu Leu Gln Ala Ile Gln Leu Ala Leu
 1                   5
```

<210> 1345
<211> 9
<212> PRT
<213> Artificial Sequence

<400> 1345

```
Glu Leu Gln Ala Ile Gln Leu Ala Leu
 1                   5
```

<210> 1346
<211> 9
<212> PRT
<213> Artificial Sequence

<400> 1346

```
Asn Leu Ala Phe Gln Gln Gly Glu Ala
 1                   5
```

<210> 1347
<211> 8
<212> PRT
<213> Artificial Sequence

<400> 1347

```
Lys Leu Val Asp Phe Arg Glu Leu
 1                   5
```

<210> 1348
<211> 8
<212> PRT
<213> Artificial Sequence

<400> 1348

```
Ala Leu Thr Asp Ile Val Thr Leu
 1                   5
```

<210> 1349
<211> 8
<212> PRT
<213> Artificial Sequence

<400> 1349

```
                    Ala Leu Thr Glu Val Val Pro Leu
                     1               5
```

<210> 1350
<211> 9
<212> PRT
<213> Artificial Sequence

<400> 1350

```
                    Glu Leu His Pro Asp Lys Trp Thr Val
                     1               5
```

<210> 1351
<211> 8
<212> PRT
<213> Artificial Sequence

<400> 1351

```
                    Val Ile Leu Val Ala Val His Val
                     1               5
```

<210> 1352
<211> 8
<212> PRT
<213> Artificial Sequence

<400> 1352

```
                    Ile Ile Leu Val Ala Val His Val
                     1               5
```

<210> 1353
<211> 9
<212> PRT
<213> Artificial Sequence

<400> 1353

```
                    Pro Leu Trp Lys Gly Pro Ala Lys Leu
                     1               5
```

<210> 1354
<211> 8
<212> PRT
<213> Artificial Sequence

<400> 1354

```
                    Arg Gln Gly Phe Glu Arg Ala Leu
                     1               5
```

<210> 1355
<211> 8
<212> PRT
<213> Artificial Sequence

<400> 1355

```
Arg Gln Gly Phe Glu Arg Ala Leu
 1               5
```

<210> 1356
<211> 9
<212> PRT
<213> Artificial Sequence

<400> 1356

```
Ser Leu Leu Asn Ala Thr Ala Ile Ala
 1               5
```

<210> 1357
<211> 9
<212> PRT
<213> Artificial Sequence

<400> 1357

```
Ser Leu Leu Asn Ala Thr Ala Ile Ala
 1               5
```

<210> 1358
<211> 9
<212> PRT
<213> Artificial Sequence

<400> 1358

```
Ala Leu Lys Tyr Trp Trp Asn Leu Leu
 1               5
```

<210> 1359
<211> 9
<212> PRT
<213> Artificial Sequence

<400> 1359

```
Leu Ile Val Ala Arg Ile Val Glu Leu
 1               5
```

<210> 1360
<211> 9
<212> PRT

<213> Artificial Sequence

<400> 1360

Arg Leu Arg Asp Phe Ile Leu Ile Ala
1               5

<210> 1361
<211> 9
<212> PRT
<213> Artificial Sequence

<400> 1361

Arg Leu Arg Asp Phe Ile Leu Ile Ala
1               5

<210> 1362
<211> 9
<212> PRT
<213> Artificial Sequence

<400> 1362

Phe Leu Ala Leu Ala Trp Asp Asp Leu
1               5

<210> 1363
<211> 9
<212> PRT
<213> Artificial Sequence

<400> 1363

Phe Leu Ala Ile Ile Trp Val Asp Leu
1               5

<210> 1364
<211> 9
<212> PRT
<213> Artificial Sequence

<400> 1364

Arg Leu Val Ser Gly Phe Leu Ala Leu
1               5

<210> 1365
<211> 8
<212> PRT
<213> Artificial Sequence

<400> 1365

```
              Leu Gln Ala Arg Val Leu Ala Val
               1             5
```

<210> 1366
<211> 9
<212> PRT
<213> Artificial Sequence

<400> 1366

```
              Gln Leu Gln Ala Arg Val Leu Ala Val
               1             5
```

<210> 1367
<211> 9
<212> PRT
<213> Artificial Sequence

<400> 1367

```
              Arg Leu Ile Ser Cys Asn Thr Ser Val
               1             5
```

<210> 1368
<211> 9
<212> PRT
<213> Artificial Sequence

<400> 1368

```
              Arg Leu Ile Asn Cys Asn Thr Ser Val
               1             5
```

<210> 1369
<211> 9
<212> PRT
<213> Artificial Sequence

<400> 1369

```
              Ala Leu Phe Tyr Lys Leu Asp Val Val
               1        5
```